# EUROPEAN PATENT SPECIFICATION

(11) **EP 3 947 723 B1**
(45) Date of publication and mention of the grant of the patent: **18.12.2024**
(21) Application number: 20722098.9
(22) Date of filing: 02.04.2020
(51) Int. Cl.: C12Q 1/6806, C12Q 1/6869

(54) **METHODS AND COMPOSITIONS FOR ANALYZING NUCLEIC ACID**
VERFAHREN UND ZUSAMMENSETZUNGEN ZUR NUKLEINSÄURE-ANALYSE
METHODES ET COMPOSITIONS POUR L'ANALYSE D'ACIDES NUCLEIQUES

(30) Priority: 05.04.2019 US 201962830211 P; 14.06.2019 US 201962861594 P; 23.10.2019 US 201962925132 P
(43) Date of publication of application: 09.02.2022
(73) Proprietor: Claret Bioscience, LLC, Scotts Valley, CA 95066 (US)
(72) Inventor: HARKINS KINCAID, Kelly M., Scotts Valley CA 95066 (US); NAUGHTON, Colin, Patrick, Scotts Valley, CA 95066 (US); RAO, Varsha, Scotts Valley, CA 95066 (US); TROLL, Christopher, J., Scotts Valley, CA 95066 (US); KAPP, Joshua, D., Scotts Valley, CA 95066 (US)
(74) Representative: Vossius & Partner Patentanwälte Rechtsanwälte mbB
(86) International application number: PCT/US2020/026421
(87) International publication number: WO 2020/206143

(56) References cited:
- WO-A1-2014/164466
- WO-A1-2018/013837
- WO-A1-2018/175258
- WO-A1-2018/175997
- KELLY M HARKINS ET AL: "Abstract", NUCLEIC ACIDS RESEARCH, 20 February 2020 (2020-02-20), XP055679066, ISSN: 0305-1048, DOI: 10.1093/nar/gkaa128

## Description

### Field

The present invention is defined by the claims. The technology as described herein generally relates in part to methods and compositions for analyzing nucleic acid. In some aspects, the technology relates to methods and compositions for preparing a nucleic acid library from single-stranded nucleic acid fragments.

### Background

Genetic information of living organisms (e.g., animals, plants and microorganisms) and other forms of replicating genetic information (e.g., viruses) is encoded in nucleic acid (i.e., deoxyribonucleic acid (DNA) or ribonucleic acid (RNA)). Genetic information is a succession of nucleotides or modified nucleotides representing the primary structure of chemical or hypothetical nucleic acids.

A variety of high-throughput sequencing platforms are used for analyzing nucleic acid. The ILLUMINA platform, for example, involves clonal amplification of adaptor-ligated DNA fragments. Another platform is nanopore-based sequencing, which relies on the transition of nucleic acid molecules or individual nucleotides through a small channel. Library preparation for certain sequencing platforms often includes fragmentation of DNA, modification of fragment ends, and ligation of adapters, and may include amplification of nucleic acid fragments (e.g., PCR amplification).

The selection of an appropriate sequencing platform for particular types of nucleic acid analysis requires a detailed understanding of the technologies available, including sources of error, error rate, as well as the speed and cost of sequencing. While sequencing costs have decreased, the throughput and costs of library preparation can be a limiting factor. One aspect of library preparation includes modification of the ends of nucleic acid fragments such that they are suitable for a particular sequencing platform. Nucleic acid ends may contain useful information. Accordingly, methods that modify nucleic acid ends (e.g., for library preparation) while preserving the information contained in the nucleic acid ends would be useful for processing and analyzing nucleic acid.

Another aspect of library preparation includes capturing single stranded nucleic acid fragments. In certain instances, single-stranded library preparation methods can generate better and more complex libraries compared to traditional double-stranded DNA (dsDNA) preparation methods.

Drawbacks to producing single-stranded DNA (ssDNA) libraries include labor intensive, expensive, and time-consuming protocols, and exotic or custom reagent requirements. Accordingly, methods that capture single-stranded nucleic acids (e.g., for library preparation), without requiring labor intensive, expensive, and time-consuming protocols, and/or exotic or custom reagents would be useful for processing and analyzing nucleic acid (e.g., single-stranded nucleic acid, denatured double-stranded nucleic acid, or mixtures containing single-stranded nucleic acid).

Another aspect of library preparation includes capturing single stranded RNA fragments. Generally, existing RNA library preparation methods necessitate not only first strand DNA synthesis from RNA using a reverse transcriptase, but also second strand synthesis in order to make a cDNA molecule that is compatible with downstream double-stranded sequencing adapter ligation. Often, it is desirable to generate stranded RNA sequencing libraries so that an accurate assessment can be made as to which genomic DNA strand the transcript is being transcribed from. To create stranded RNA sequencing libraries, methods may include degrading the second DNA strand after sequencing adapter ligation. However, performing second strand DNA synthesis before RNA pre-degradation is problematic in that it can create third and sometimes fourth strand synthesis byproducts that convolute the resulting RNA sequencing data and partially obfuscate the strandedness of the library. Accordingly, methods that capture single-stranded RNA (e.g., for library preparation), without requiring second strand synthesis would be useful for processing and analyzing nucleic acid containing RNA.

Moreover, methods for the identification of variant recognition sites for rare cutting engineered double strand break inducing agents and compositions thereof are provided in WO 2014/164466.

### Summary

The present invention is defined by the claims.

Accordingly, the present invention relates to a method of producing a nucleic acid library, comprising: combining (i) a nucleic acid composition comprising single-stranded nucleic acid (ssNA), (ii) a first oligonucleotide, and (iii) a plurality of first scaffold polynucleotide species, wherein: (a) each polynucleotide in the plurality of first scaffold polynucleotide species comprises an ssNA hybridization region and a first oligonucleotide hybridization region; (b) the nucleic acid composition, the first oligonucleotide, and the plurality of first scaffold polynucleotide species are combined under conditions in which a molecule of the first scaffold polynucleotide species is hybridized to (i) a first ssNA terminal region and (ii) a molecule of the first oligonucleotide, thereby forming hybridization products in which an end of the molecule of the first oligonucleotide is adjacent to an end of the first ssNA terminal region; (c) the ssNA is not combined with a single-stranded nucleic acid binding protein (SSB) prior to the combining or during the combining; and (d) the ssNA is not modified prior to the combining.

In accordance with a preferred embodiment the method further comprises combining the nucleic acid composition with (iv) a second oligonucleotide, and (v) a plurality of second scaffold polynucleotide species, wherein: (e) each polynucleotide in the plurality of second scaffold polynucleotide species comprises an ssNA hybridization region and a second oligonucleotide hybridization region; and (f) the nucleic acid composition, the second oligonucleotide, and the plurality of second scaffold polynucleotide species are combined under conditions in which a molecule of the second scaffold polynucleotide species is hybridized to (i) a second ssNA terminal region and (ii) a molecule of the second oligonucleotide, thereby forming hybridization products in which an end of the molecule of the second oligonucleotide is adjacent to an end of the second ssNA terminal region.

In accordance with a more preferred embodiment prior to the combining, each of the first scaffold polynucleotide species is hybridized to a first oligonucleotide to form a plurality of first scaffold duplex species, and each of the second scaffold polynucleotide species is hybridized to a second oligonucleotide to form a plurality of second scaffold duplex species.

In accordance with a preferred embodiment the method further comprises covalently linking the adjacent ends of the first oligonucleotide and the first ssNA terminal region, and covalently linking the adjacent ends of the second oligonucleotide and the second ssNA terminal region, thereby generating covalently linked hybridization products.

In accordance with a more preferred embodiment the covalently linking comprises contacting the hybridization products with an agent comprising a ligase activity under conditions in which an end of the first ssNA terminal region is covalently linked to an end of the first oligonucleotide and an end of the second ssNA terminal region is covalently linked to an end of the second oligonucleotide.

In accordance with another more preferred embodiment some or all of the duplexes in the plurality of first scaffold duplex species comprise an adenylation modification at the 5' end of the first oligonucleotide, and the plurality of first scaffold duplex species are combined with and covalently linked to the ssNA in the absence of ATP, thereby forming intermediate covalently linked hybridization products, wherein the intermediate covalently linked hybridization products are combined with and covalently linked to the plurality of second scaffold duplex species and ATP, thereby forming covalently linked hybridization products.

In accordance with a preferred embodiment the ssNA hybridization region of each of the first polynucleotide species is different than the ssNA hybridization region in other first polynucleotide species in the plurality of first polynucleotide species, and the ssNA hybridization region of each of the second polynucleotide species is different than the ssNA hybridization region in other second polynucleotide species in the plurality of second polynucleotide species.

In accordance with another preferred embodiment (1) the ssNA hybridization region comprises a random sequence, and/or (2) the ssNA hybridization region comprises one or more universal bases.

Also in accordance with a preferred embodiment (1) the first oligonucleotide comprises one or more modified nucleotides, some or all of the first scaffold polynucleotide species comprise one or more modified nucleotides, or the first oligonucleotide comprises one or more modified nucleotides and some or all of the first scaffold polynucleotide species comprise one or more modified nucleotides; and/or (2) the second oligonucleotide comprises one or more modified nucleotides, some or all of the second scaffold polynucleotide species comprise one or more modified nucleotides, or the second oligonucleotide comprises one or more modified nucleotides and some or all of the second scaffold polynucleotide species comprise one or more modified nucleotides, wherein the one or more modified nucleotides are capable of blocking covalent linkage of the oligonucleotide to another oligonucleotide, polynucleotide, or nucleic acid molecule, and wherein the oligonucleotide comprises the one or more modified nucleotides at an end not adjacent to the ssNA, and/or wherein the one or more modified nucleotides are capable of blocking covalent linkage of the scaffold polynucleotide to another oligonucleotide, polynucleotide, or nucleic acid molecule, and wherein the scaffold polynucleotide comprises the one or more modified nucleotides at one or both ends of the polynucleotide.

In accordance with a more preferred embodiment the one or more modified nucleotides comprise a ligation-blocking modification.

In accordance with a more preferred embodiment the method further comprises denaturing the covalently linked hybridization products, thereby generating single-stranded ligation products.

In accordance with an even more preferred embodiment the method further comprises: (1) combining the single-stranded ligation products with a third oligonucleotide under conditions in which the third oligonucleotide is hybridized to a dimer of the first oligonucleotide and the second oligonucleotide, thereby forming an oligonucleotide dimer hybridization product, wherein the oligonucleotide dimer hybridization product comprises a cleavage site, and (2) contacting the oligonucleotide dimer hybridization product with a cleavage agent.

In accordance with a preferred embodiment the nucleic acid composition comprises single-stranded DNA (ssDNA), single-stranded RNA (ssRNA), or ssDNA and ssRNA.

In accordance with another preferred embodiment one or both native ends of the ssNA are present when the ssNA is combined with the first oligonucleotide and the plurality of first scaffold polynucleotide species.

Finally, in accordance with a preferred embodiment the ssNA is from cell-free nucleic acid.

Certain embodiments are described further in the following description, examples, claims and drawings.

### Brief Description of the Drawings

The drawings illustrate certain embodiments of the technology and are not limiting. For clarity and ease of illustration, the drawings are not made to scale and, in some instances, various aspects may be shown exaggerated or enlarged to facilitate an understanding of particular embodiments.

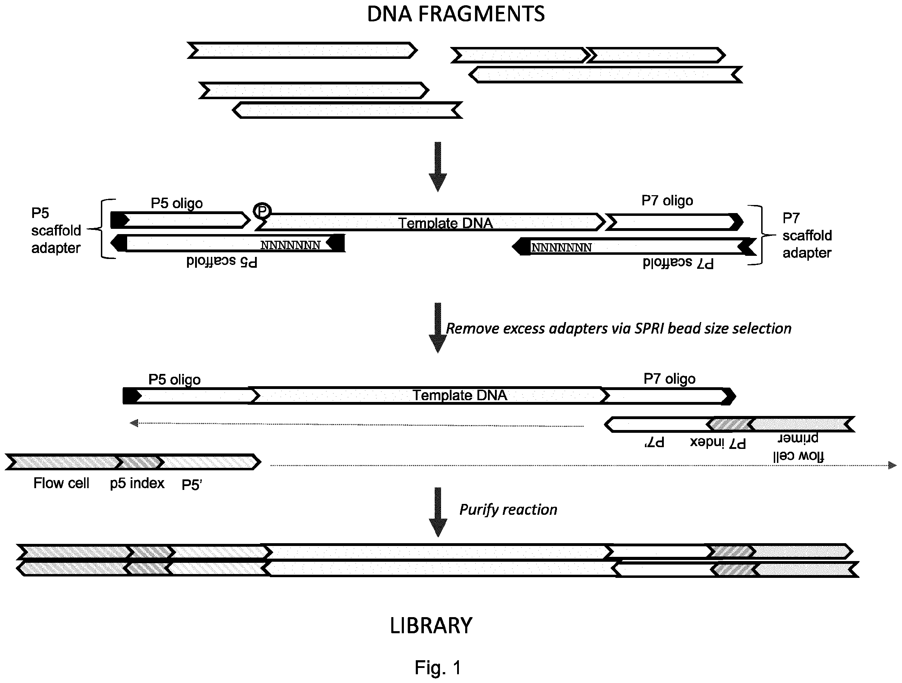

Fig. 1 shows a general workflow for certain library preparation methods described herein. The solid black chevrons at the end of the P5 adapter, at the end of the P7 adapter, and at the ends of the P5/P7 scaffold polynucleotides represent blocking modifications.

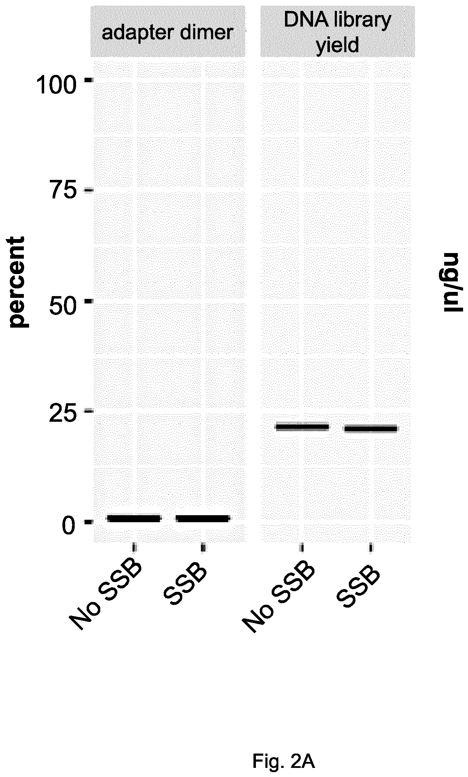

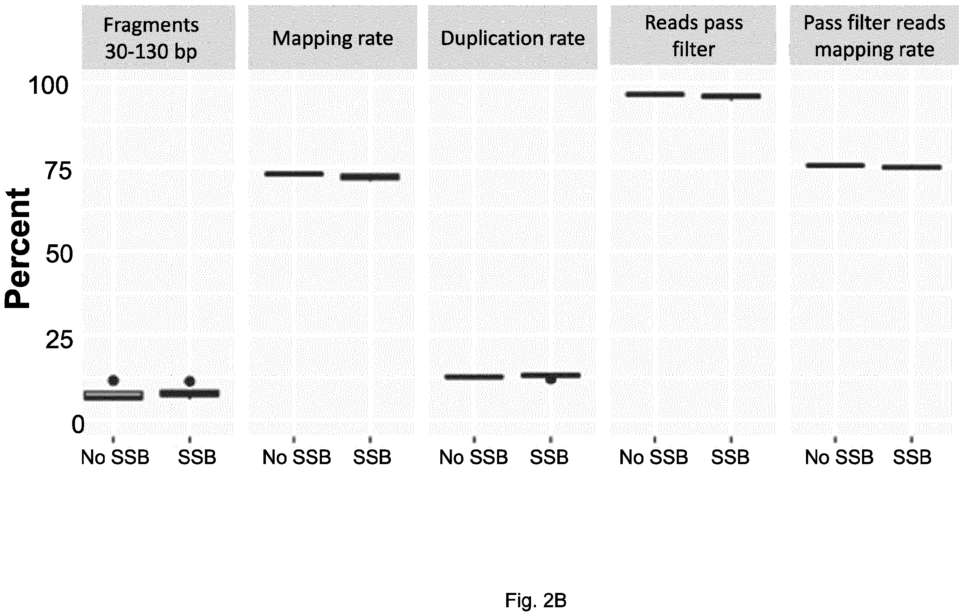

Fig. 2A shows molecular QC metrics for ssDNA libraries made with and without the use of extreme thermostable single-stranded DNA binding protein (ET SSB). Fig. 2B shows ILLUMINA HISEQ sequencing metrics for ssDNA libraries generated with and without the use of ET SSB. Two sample equal variance T-tests showed no significant difference in sequencing metrics.

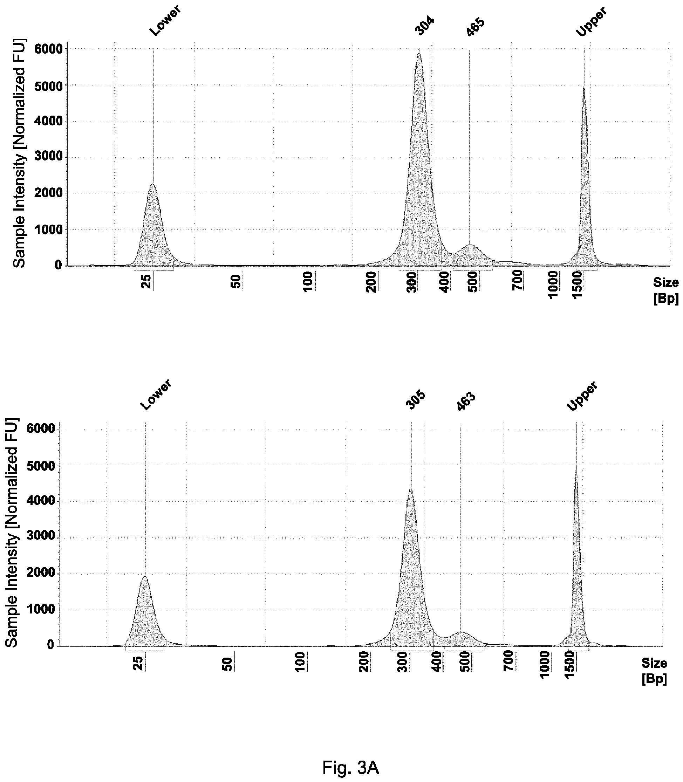

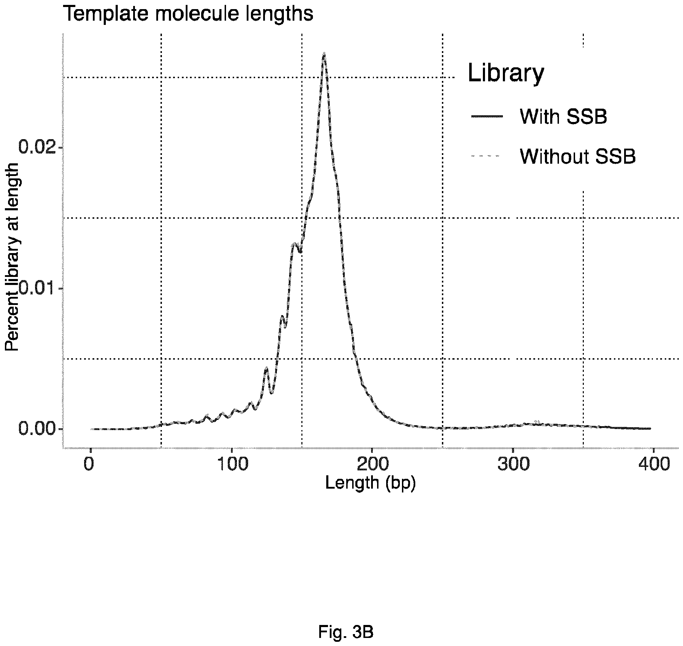

Fig. 3A shows ssDNA final library product size distribution from 1 ng cell-free DNA generated with (top) and without (bottom) the presence of SSB. Fig. 3B shows an overlap of insert length distribution inferred from sequencing data of the two cfDNA libraries (generated with or without SSB).

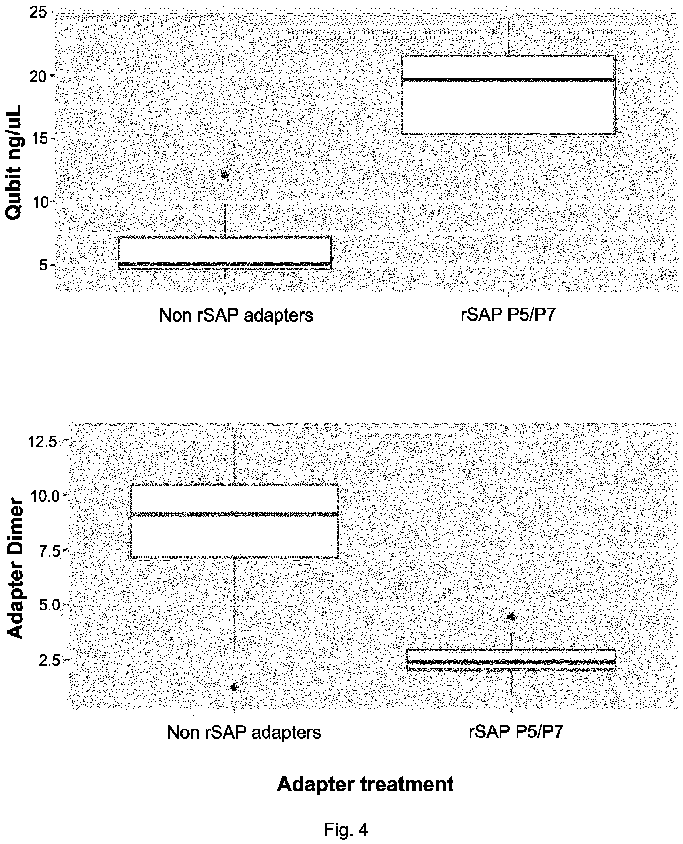

Fig. 4 shows library yield as measured by Qubit fluorometer (top) and adapter dimer % (bottom) following dephosphorylation pre-treatment of P5/P7 adapters using rapid shrimp alkaline phosphatase (rSAP). After rSAP treatment, library yield increases, and the amount of artifacts caused by adapter dimers decreases.

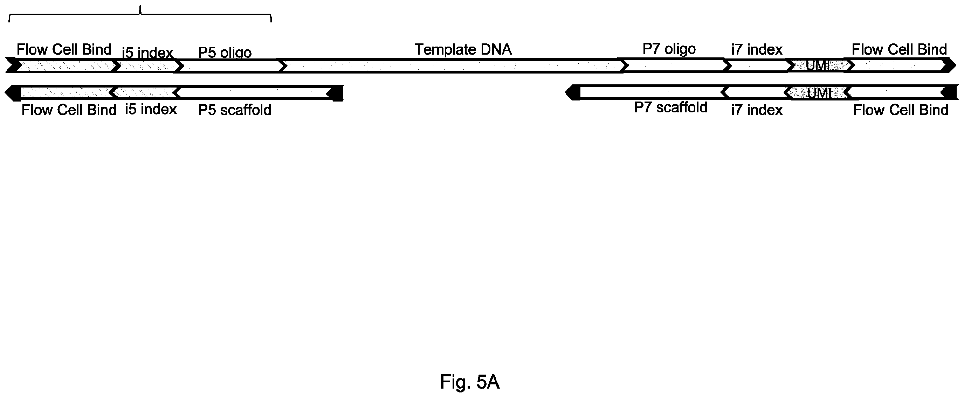

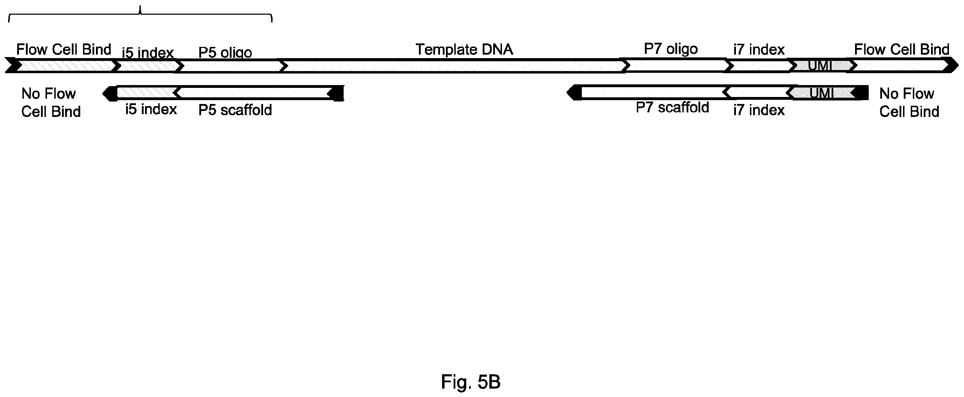

Fig. 5A and Fig. 5B show PCR free approaches which include ligating complete indexing sequencing adapters plus scaffolds during an ssDNA library ligation step. Such adapters may or may not contain a unique molecular identifier (UMI).

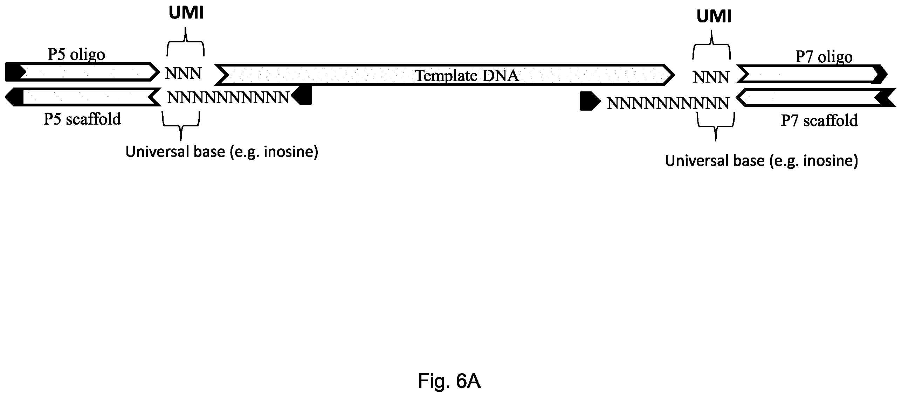

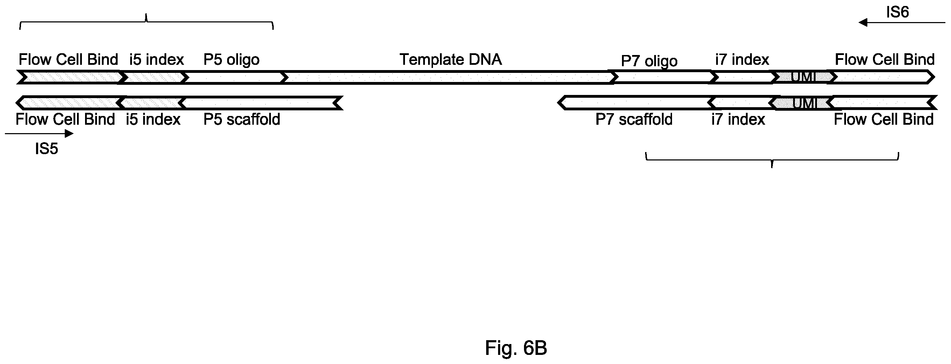

Fig. 6A and Fig. 6B show scaffold adapter configurations having unique molecular identifiers (UMIs). Fig. 6A shows a configuration with the UMI adjacent to the template. Fig. 6B shows a configuration with the UMI adjacent to an index polynucleotide.

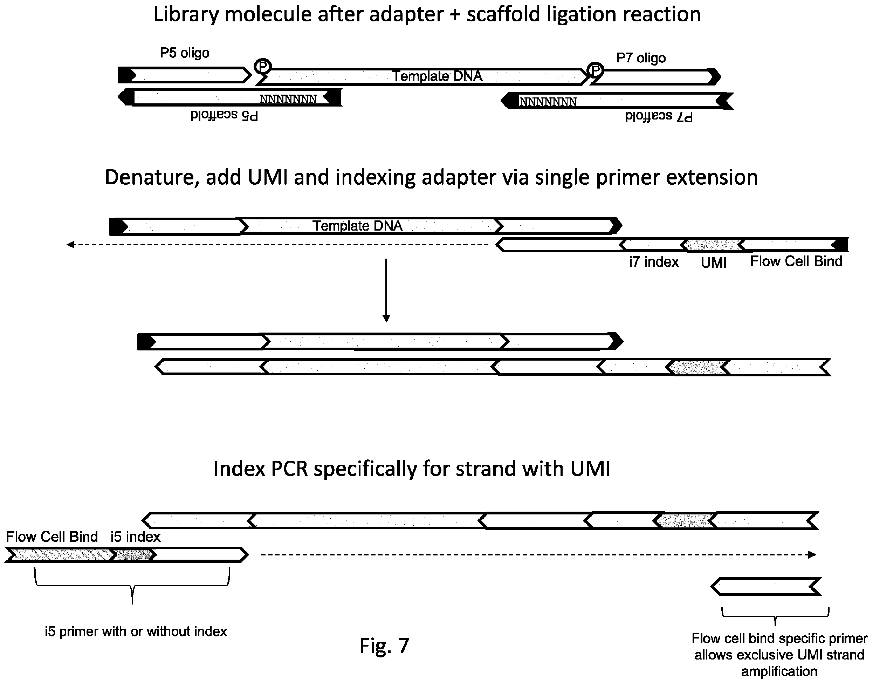

Fig. 7 shows an example of incorporation of unique molecular identifiers (UMIs) and P7 indexing adapter after scaffold adapter ligation prior to index PCR using a single primer extension.

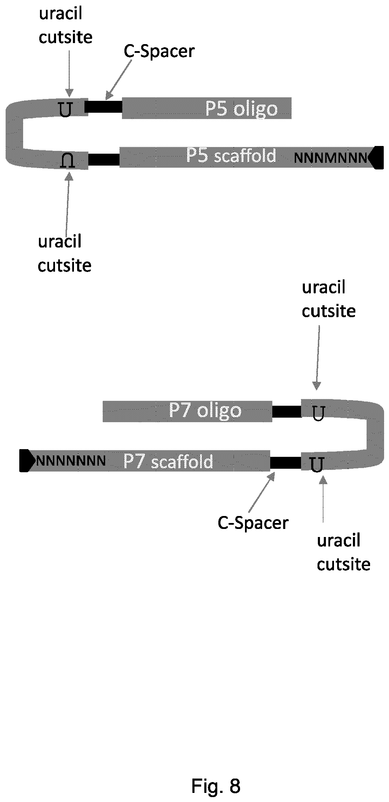

Fig. 8 shows an example of hairpin scaffold adapter design.

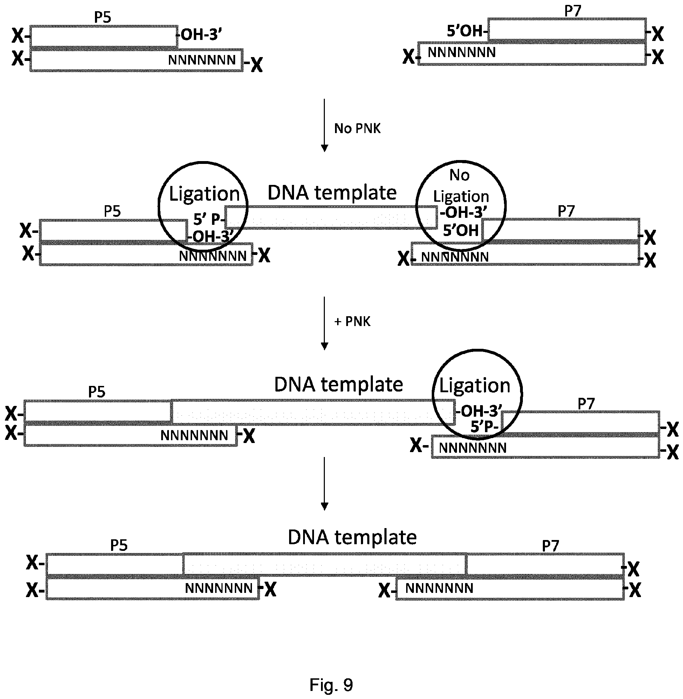

Fig. 9 shows an example workflow with staged ligation and enzyme delay. X, blocking modification. 5' P, 5' phosphate. 5' OH, 5' hydroxyl. 3' OH, 3' hydroxyl. N, any nucleotide.

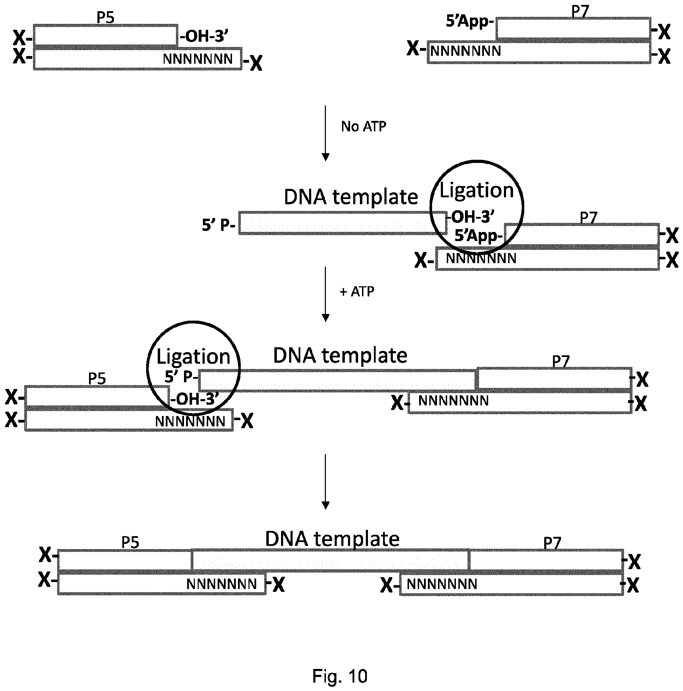

Fig. 10 shows an example workflow with staged ligation using a P7 scaffold adapter having a 5' App modification and ATP delay. X, blocking modification. 5' App, 5'-adenylated DNA. 5' P, 5' phosphate. 3' OH, 3' hydroxyl. N, any nucleotide.

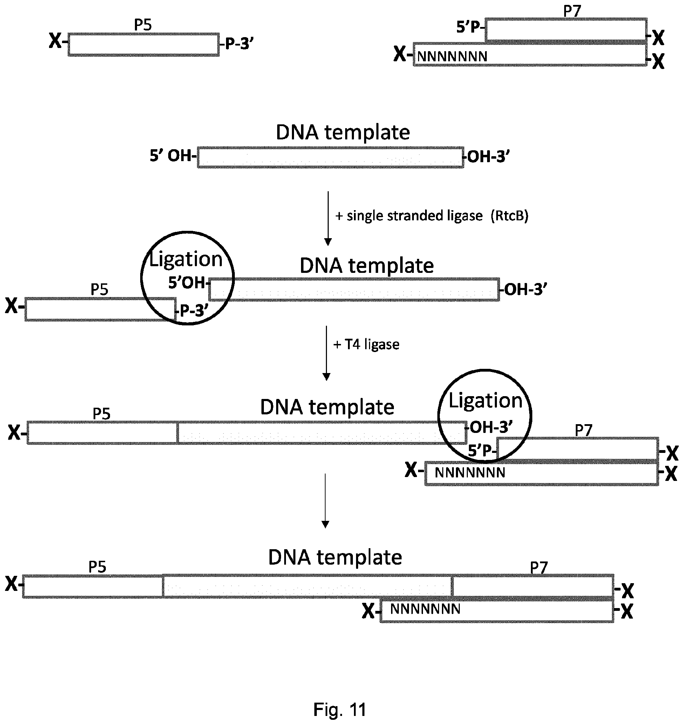

Fig. 11 shows an example workflow with staged ligation using a single-stranded P5 adapter having a 3' phosphate. X, blocking modification. 3' P, 3' phosphate. 5' P, 5' phosphate. 3' OH, 3' hydroxyl. 5' OH, 5' hydroxyl. N, any nucleotide.

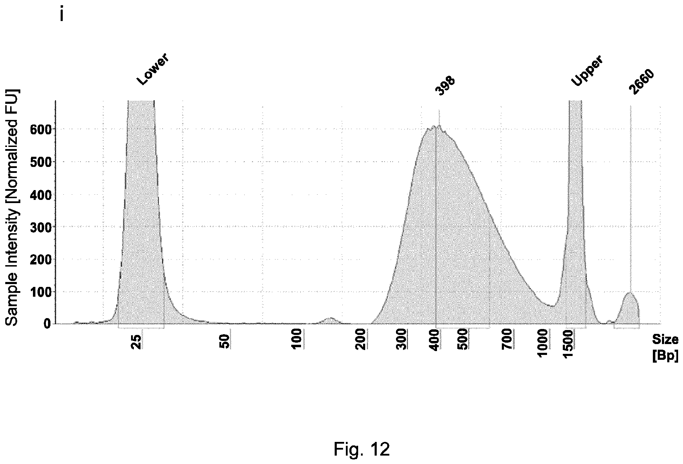

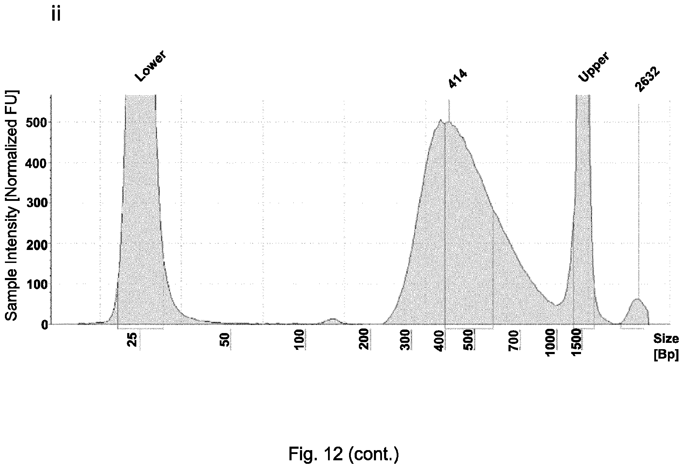

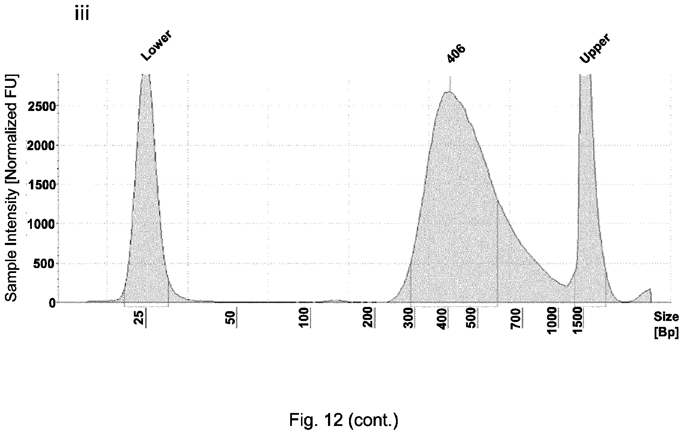

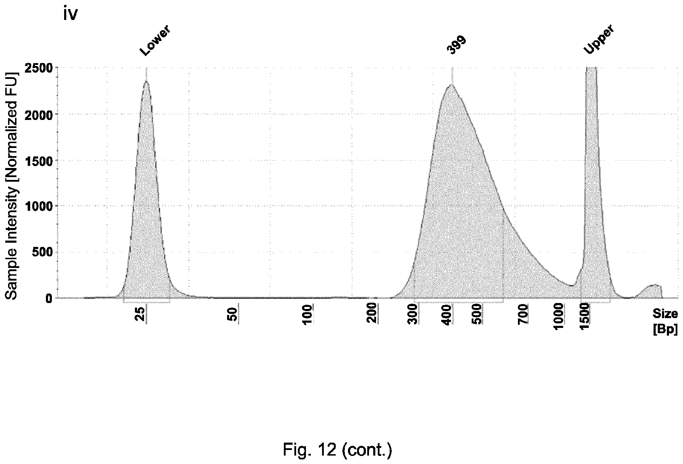

Fig. 12 shows tapestation traces of four ssDNA libraries generated from first strand cDNA synthesized using random hexamer, octamer and anchored poly-T primers and Mu-MLV reverse transcriptase (in-house protocol), or New England Biolabs Ultra II RNA First Strand synthesis module (commercial protocol). i and ii are technical replicates of the in-house protocol, iii and iv are technical replicates of the commercial protocol.

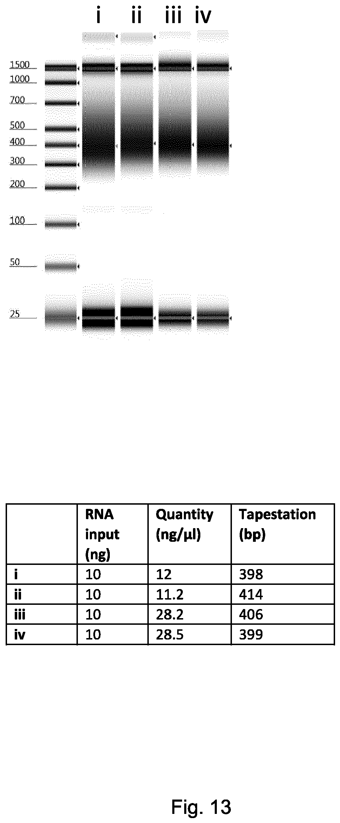

Fig. 13 shows molecular metrics of four ssDNA libraries generated from first strand cDNA synthesized using random hexamer, octamer and anchored poly-T primers and Mu-MLV reverse transcriptase (in-house protocol), or New England Biolabs Ultra II RNA First Strand synthesis module (commercial protocol). Top panel: gel image of the final RNA-Seq libraries. Bottom panel: table indicating the library yield (ng/pl) and size (bp). i and ii are technical replicates of the in-house protocol, iii and iv are technical replicates of the commercial protocol.

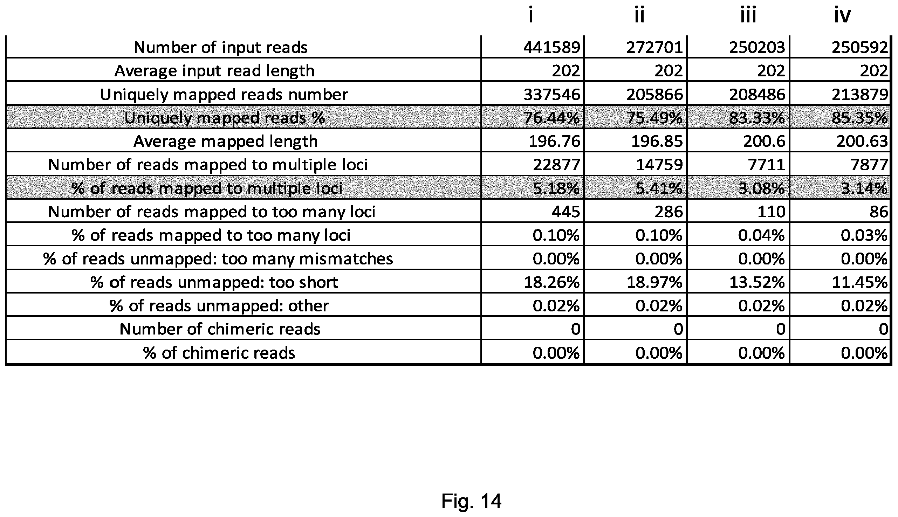

Fig. 14 provides a table showing sequencing metrics generated using STAR-aligner for ssDNA libraries generated from first strand cDNA synthesized using an in-house protocol (random hexamer, octamer and anchored poly-T primers and Mu-MLV reverse transcriptase), or a commercial protocol (New England Biolabs Ultra II RNA First Strand synthesis module). Typical RNA-Seq metrics for a good quality library may include: a) uniquely mapped reads for 70-90% of the library, and b) % reads mapped to multiple loci ~5 (such metrics are marked in grey). The data provided in the table shows successful RNA-seq library generation.

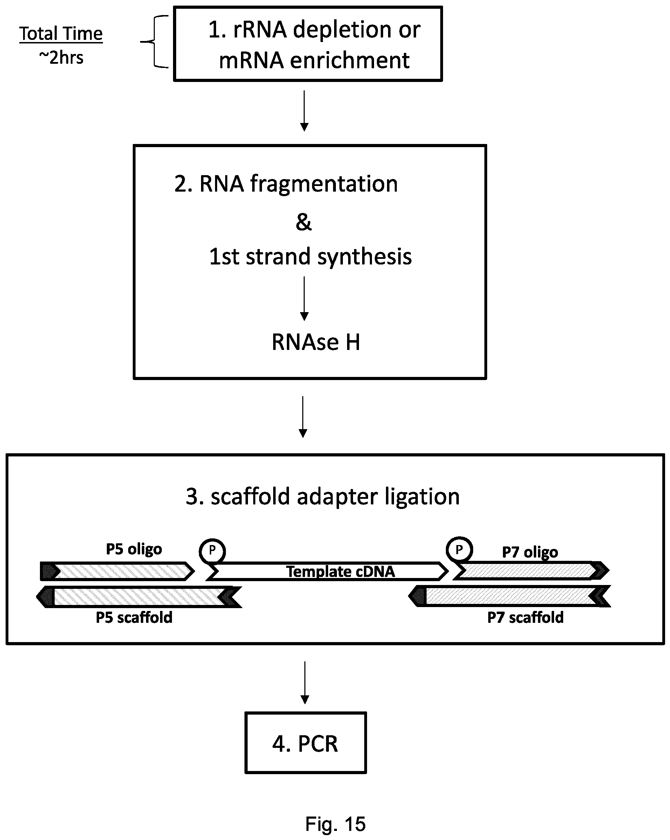

Fig. 15 shows an overview of a method for single-stranded preparation for RNA-Seq as well as a schematic detailing single-stranded scaffold adapter ligation technology post 1st strand cDNA synthesis.

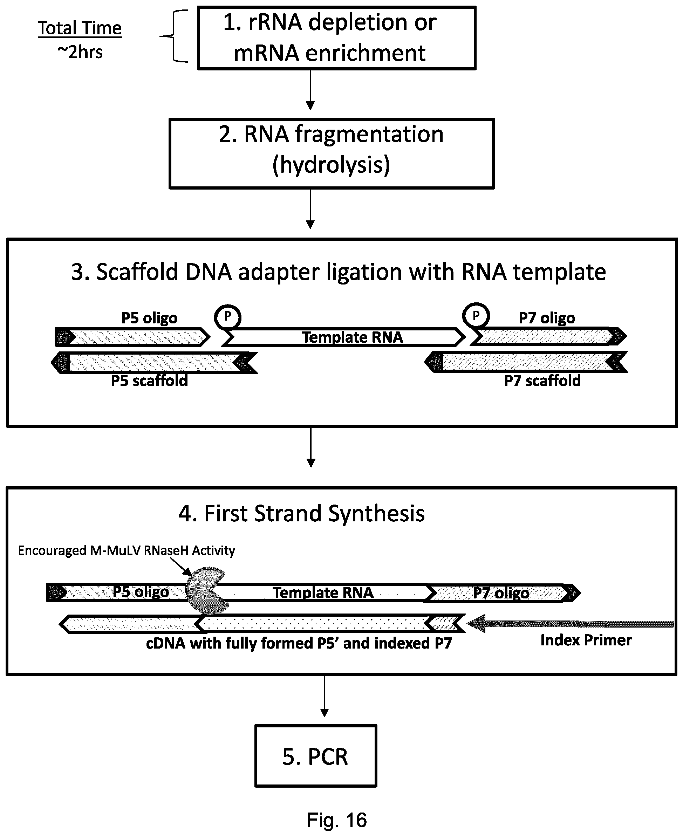

Fig. 16 shows an overview of a method for single-stranded preparation for RNA-Seq as well as a schematic detailing single-stranded scaffold adapter ligation technology created DNA/RNA hybrids followed by cDNA first strand synthesis.

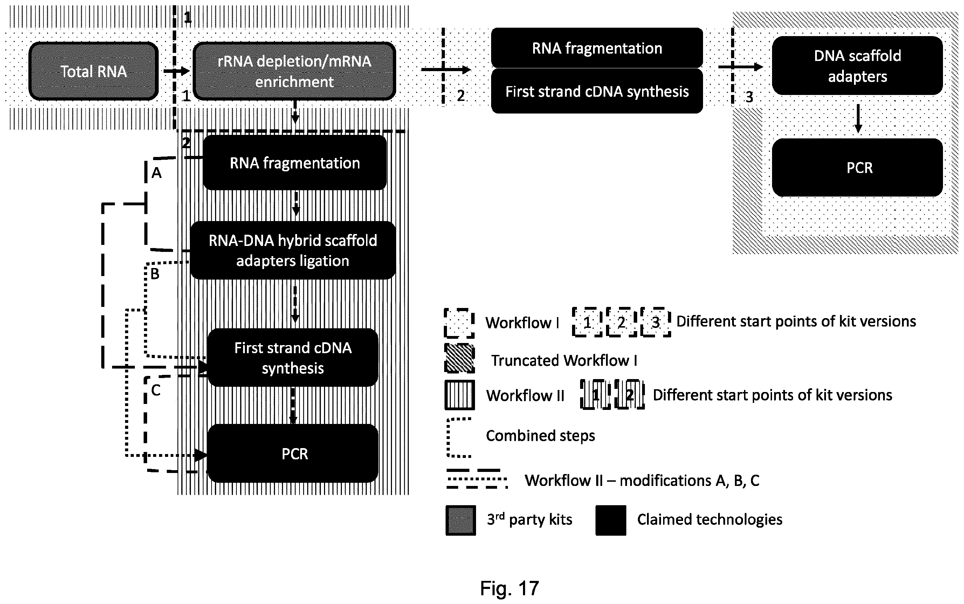

Fig. 17 shows various workflows that can precede or follow an ssDNA/ssRNA ligation reaction described herein.

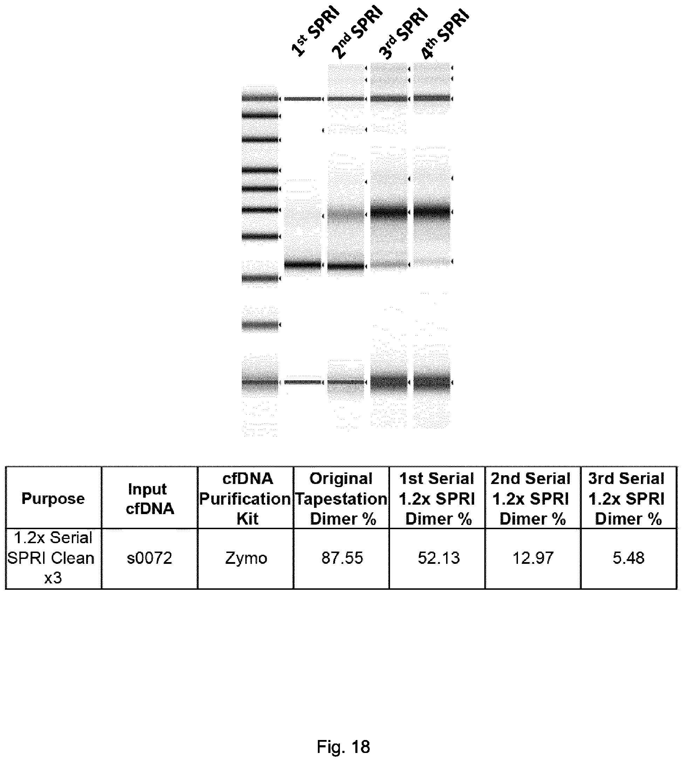

Fig. 18 shows results of serial 1.2x solid phase reversible immobilization (SPRI) cleans for a sample having a high percentage of adapter dimers.

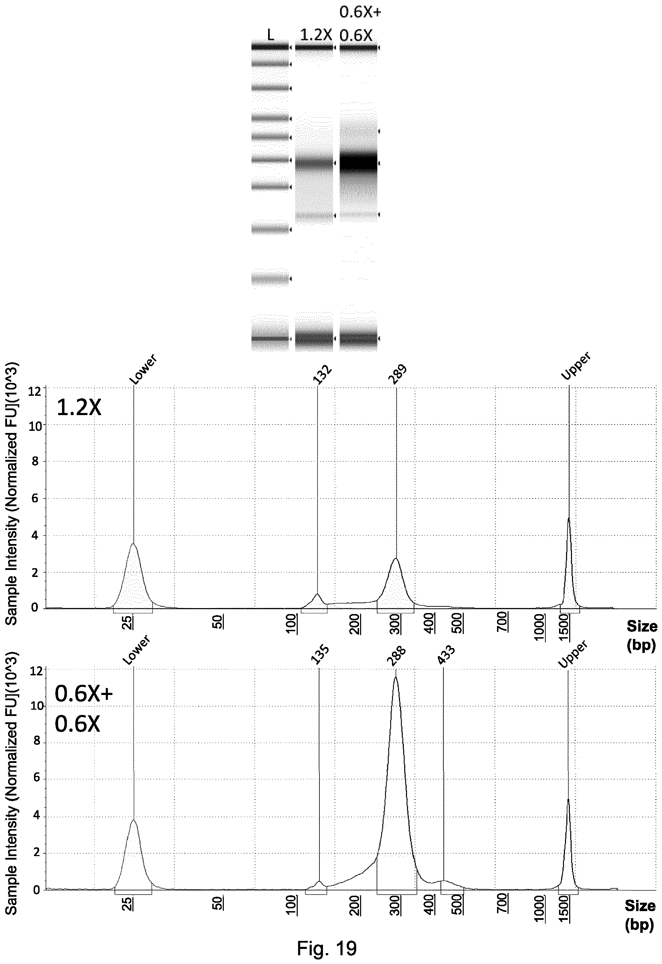

Fig. 19 shows results of sequential 0.6x SPRI + 0.6x SPRI cleans.

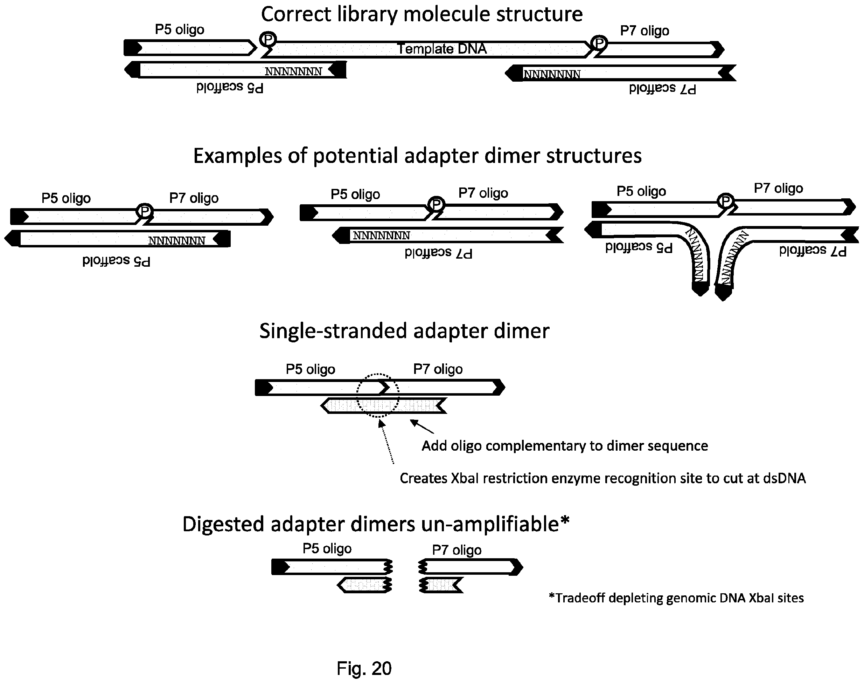

Fig. 20 shows examples of presumed adapter dimer formations, the single-stranded form of an adapter dimer, and the addition of an oligo that anneals only to such single-stranded adapter dimer. In one example, an Xbal recognition site forms when the double-stranded hybridization product is formed.

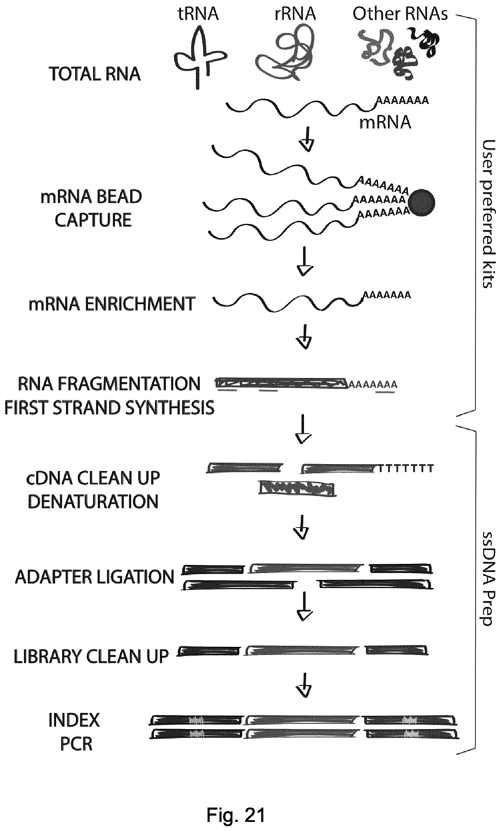

Fig. 21 shows an example workflow for a directional RNA-Seq library preparation NGS assay.

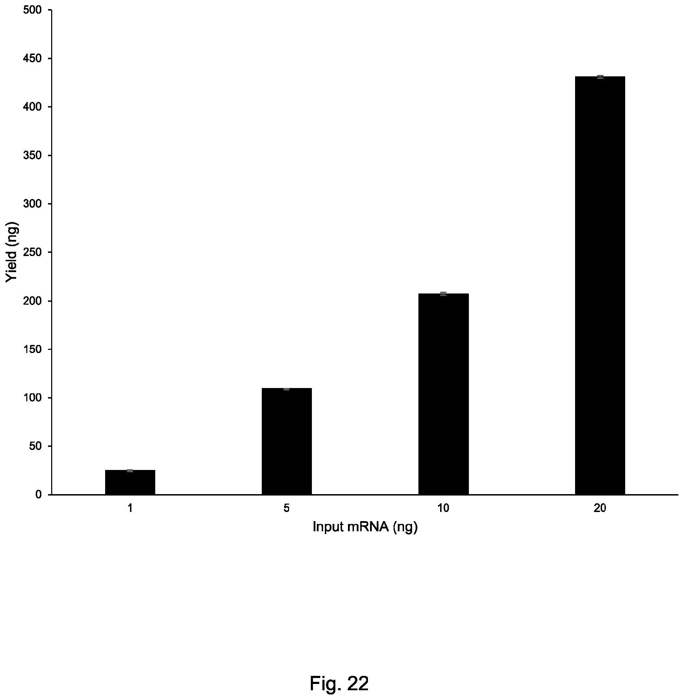

Fig. 22 shows library yields from 1 to 20 ng of mRNA input for a single-stranded library prep (ssPrep) for RNA described herein. Average of replicates per input concentration are shown. Except for the 1 ng input (which was amplified for 11 PCR cycles) all libraries were amplified for 9 cycles. Libraries were quantified using the QUANT-IT dsDNA High Sensitivity kit.

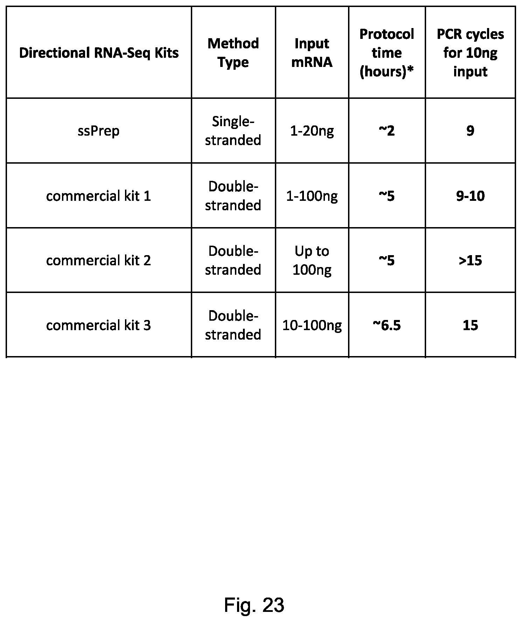

Fig. 23 provides a table showing a comparison of a single-stranded library prep (ssPrep) for RNA described herein with three commercially available double-stranded library prep (dsPrep) kits (i.e., NEBNEXT ULTRA II Directional RNA-Seq kit; NUGEN Universal mRNA library kit; and TRUSEQ mRNA Stranded library kit). Shown are comparisons of input range, workflow time, and PCR cycles.

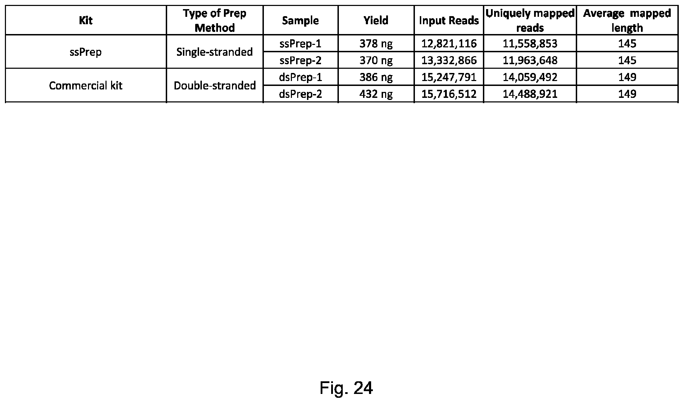

Fig. 24 provides a table showing a comparison of yields and mapping metrics for human reads generated from a single-stranded library prep (ssPrep) for RNA described herein or a commercially available double-stranded library prep (dsPrep) kit (i.e., NEBNEXT ULTRA II Directional RNA-Seq kit). Sequencing data from replicate libraries for ssPrep and dsPrep were mapped to the human reference genome (hg19). The ssPrep library had shorter mapped lengths presumably due to lack of end polishing.

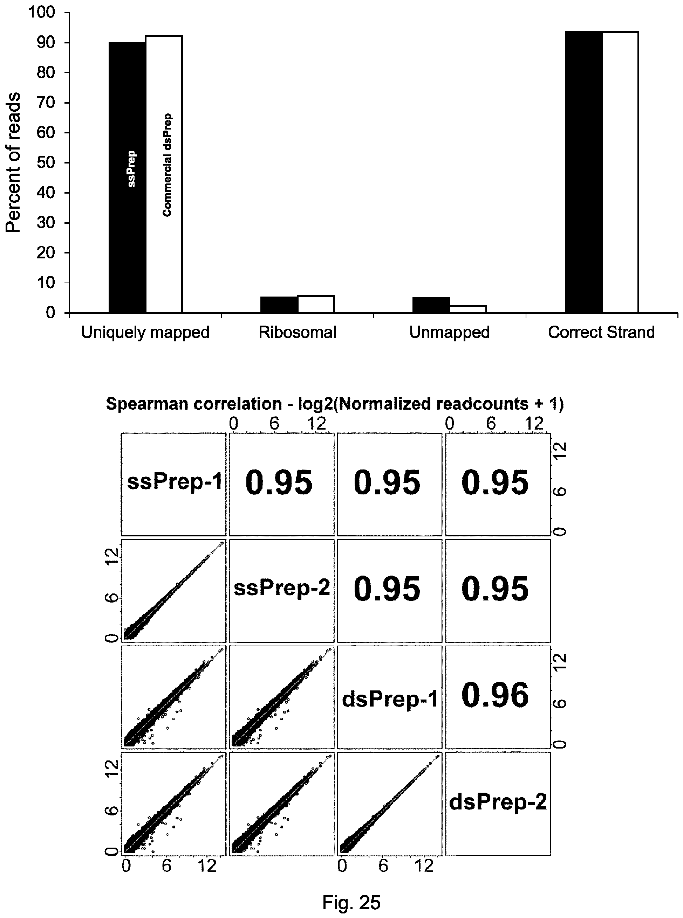

Fig. 25 shows performance metrics for a single-stranded library prep (ssPrep) for RNA described herein vs. a commercially available double-stranded library prep (dsPrep) kit (i.e., NEBNEXT ULTRA II Directional RNA-Seq kit). Top panel: Average mapping metrics for replicate libraries generated by both methods. Both libraries had ~90% uniquely mapped reads and ~5% ribosomal reads. ~93% of the reads mapped to the correct strand. Bottom panel: Spearman correlation coefficient of p~0.95 was observed between normalized read counts (human) of the replicates for both methods and with each other. Pairwise correlation coefficients are provided in the squares.

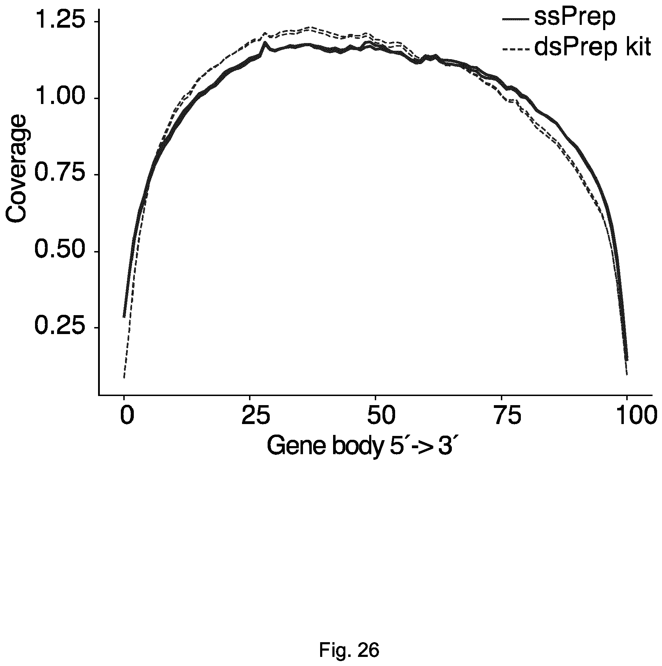

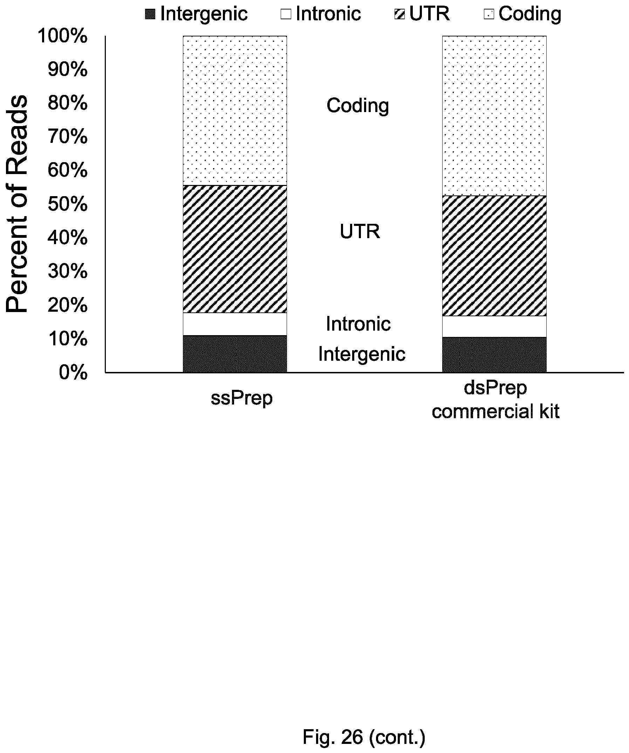

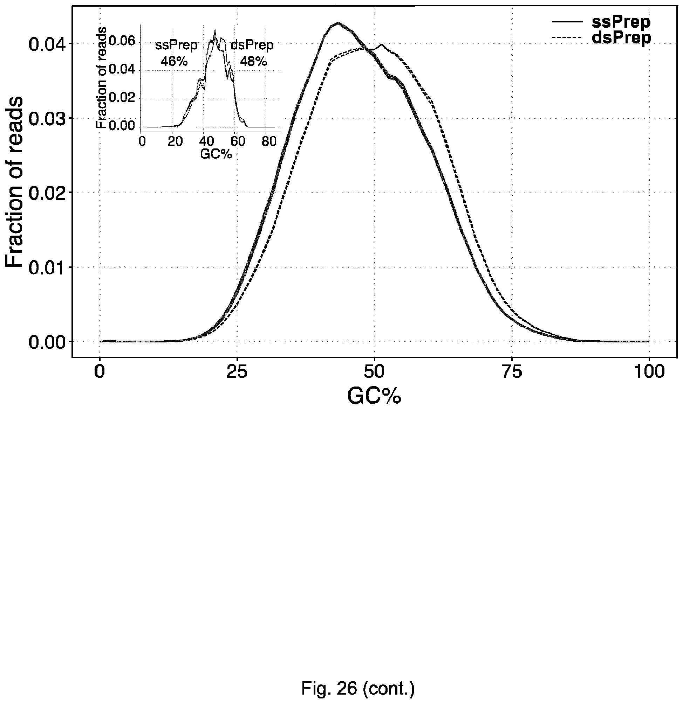

Fig. 26 shows data comparing a single-stranded library prep (ssPrep) for RNA described herein vs. a commercially available double-stranded library prep (dsPrep) kit (i.e., NEBNEXT ULTRA II Directional RNA-Seq kit). Top panel: Gene body coverage was calculated using Picard Tools CollectRNASeqMetrics; normalized coverage across the entire length of the transcript is shown for replicate libraries for ssPrep and dsPrep. Middle panel: Genomic distribution of reads was calculated using the Picard Tools CollectRNASeqMetrics and the average composition of coding, untranslated (UTR), intronic and intergenic regions were plotted. Both methods captured minimal intergenic regions. ssPrep captured more of the untranslated region owing to the more uniform gene body coverage. Bottom panel: Differential GC composition captured by the two methods. GC distribution of the External RNA Control Consortium (ERCC) spike-in control is shown in the inset. Percentages indicate observed GC composition for each library.

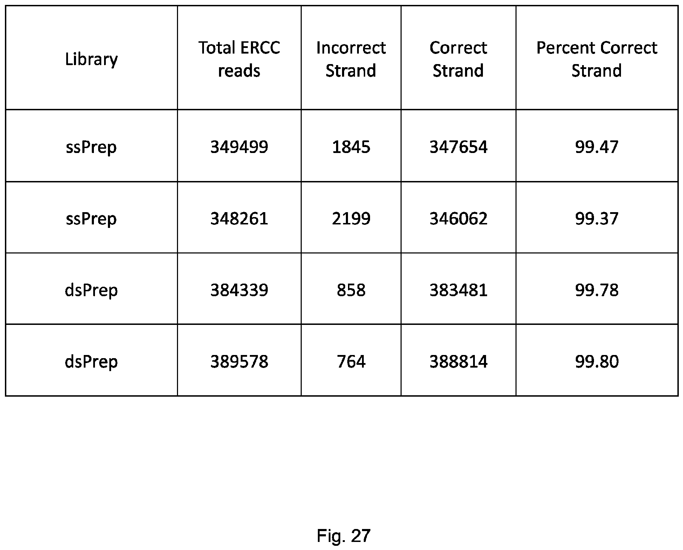

Fig. 27 provides a table showing number of reads mapping to the ERCC reference genome and percent mapping to the correct strand for libraries generated using a single-stranded library prep (ssPrep) for RNA described herein or a commercially available double-stranded library prep (dsPrep) kit (i.e., NEBNEXT ULTRA II Directional RNA-Seq kit). Both types of libraries had >300,000 control reads, >99% of the reads mapped to the correct strand.

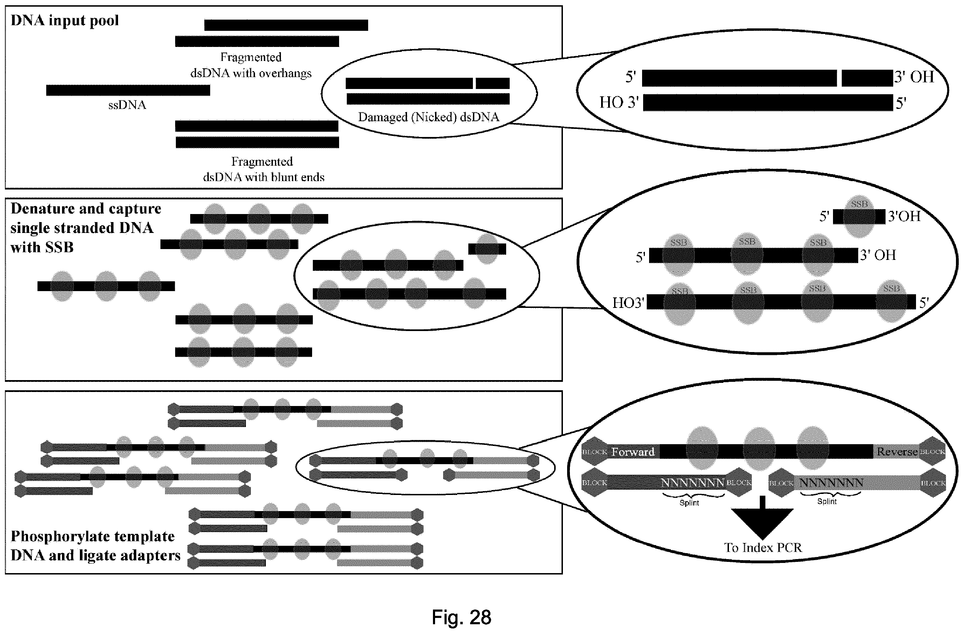

Fig. 28 shows a schematic overview of a single-stranded library prep (ssPrep) method. A DNA input pool of diverse template molecules is denatured with heat and maintained as single-stranded molecules through a cold-snap and use of a thermostable single-stranded DNA binding protein (SSB). Template DNA is phosphorylated and ssPrep scaffold adapters, which contain a randomized 7-bp single-stranded scaffold overhang and ligation blocking modification on all termini except for the ones that facilitate correctly oriented library molecules, are ligated in a combined phosphorylation/ligation reaction. After clean up, molecules are ready for index PCR.

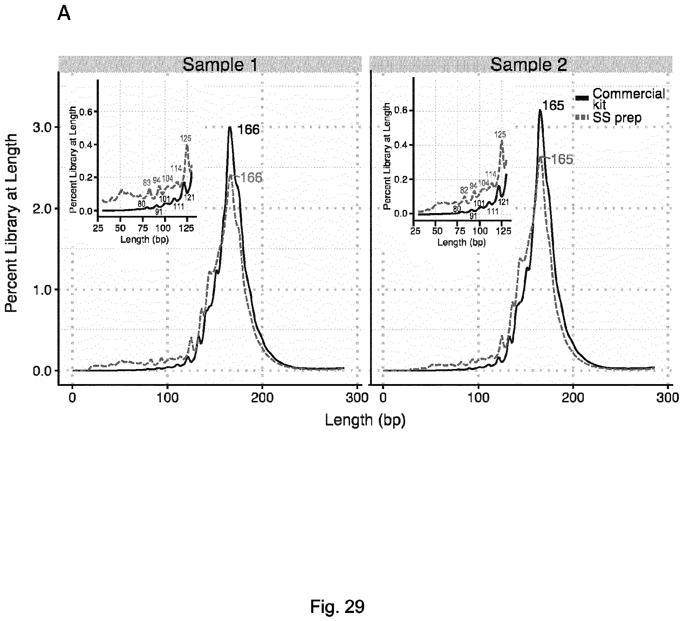

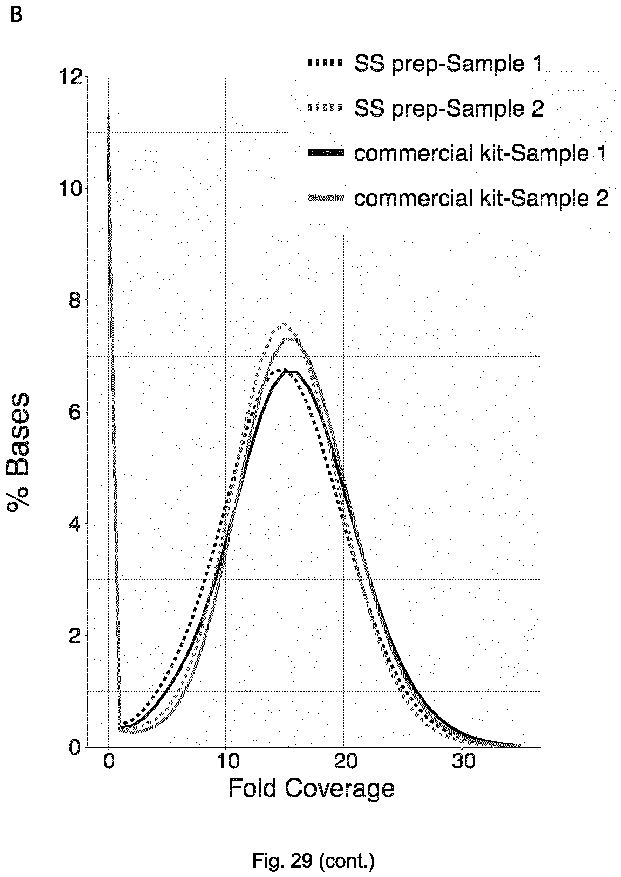

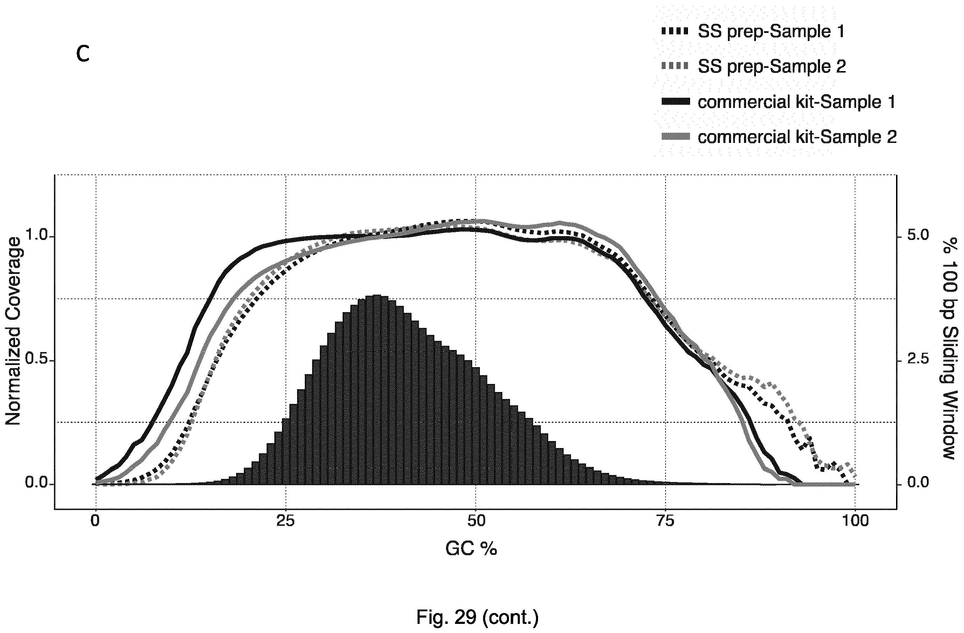

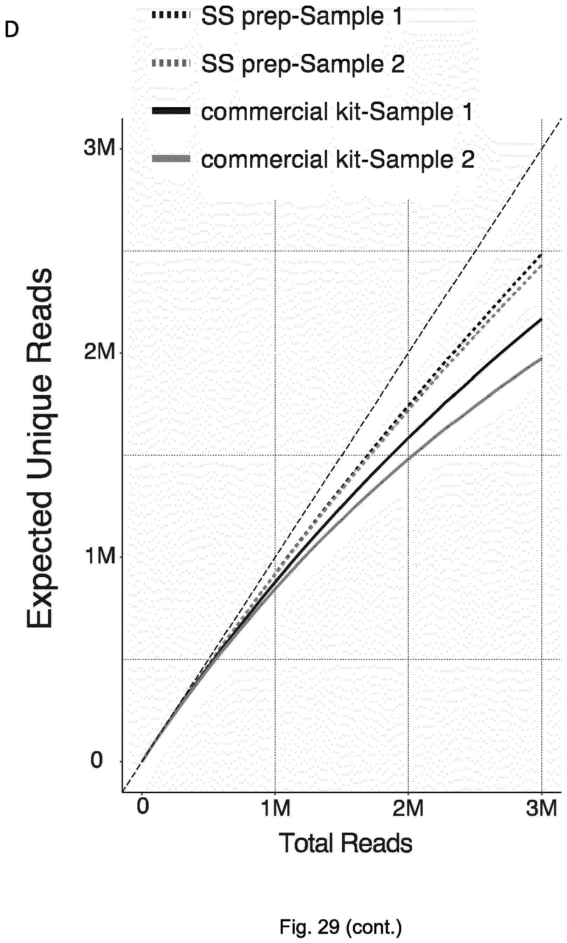

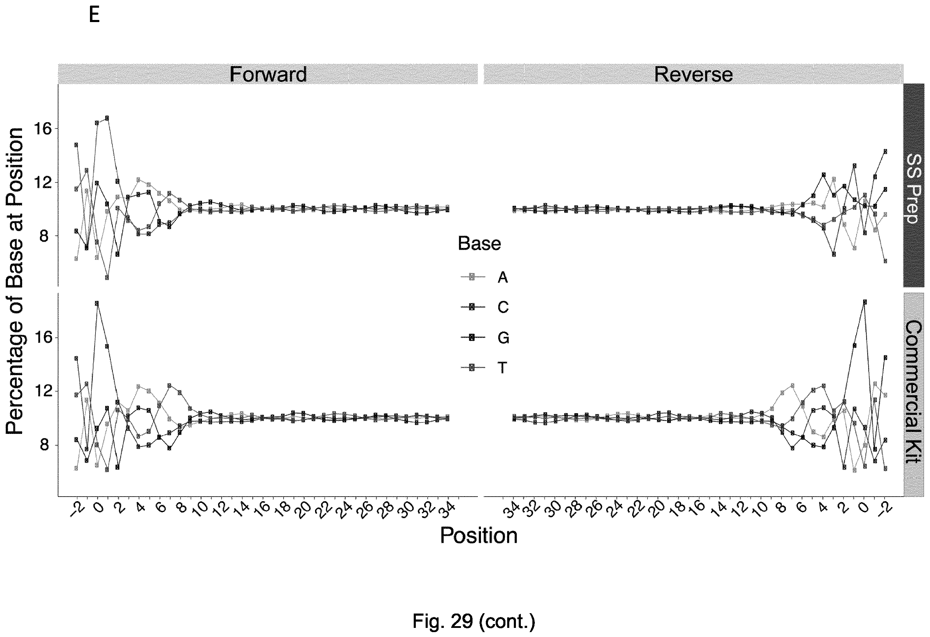

Fig. 29 shows standard NGS metrics for merged reads from single-stranded library prep (ssPrep) and commercial kit (dsPrep; i.e., NEBNEXT ULTRA II) libraries from healthy human cfDNA extracts (A, sample A; B, sample B). Unless otherwise stated, all libraries for each method were combined by cfDNA extract prior to analysis and filtered for PCR duplicates and a quality score equal to or greater than q20. Panel A: Insert distribution plots for cfDNA extracts A and B, respectively. Panel B: Fold coverage by base percent across the human genome (hg19) for ssPrep and commercial kit by cfDNA extract. Combined libraries were subsampled to similar read depth prior to fold coverage calculations. Subsampled depth was set at 295M reads, the limit of sequenced reads for ssPrep-B. Panel C: Normalized coverage as a function of GC content over 100 bp sliding scale across the human genome for ssPrep and commercial kit by cfDNA extract. Shaded histogram represents the human genome GC across the 100 bp sliding window. Panel D: Preseq complexity estimate for ssPrep and commercial kit by cfDNA extract. To get the most accurate preseq estimate possible, three libraries of equivalent sequencing depth were combined per method, since more libraries were made via ssPrep than commercial kit. Files containing the PCR duplicate reads were used to facilitate complexity estimates. Panel E: Normalized, log-transformed base composition at each position of read termini starting 2 bp upstream and extending to 34 bp downstream of read start site. All reads regardless of insert length were considered.

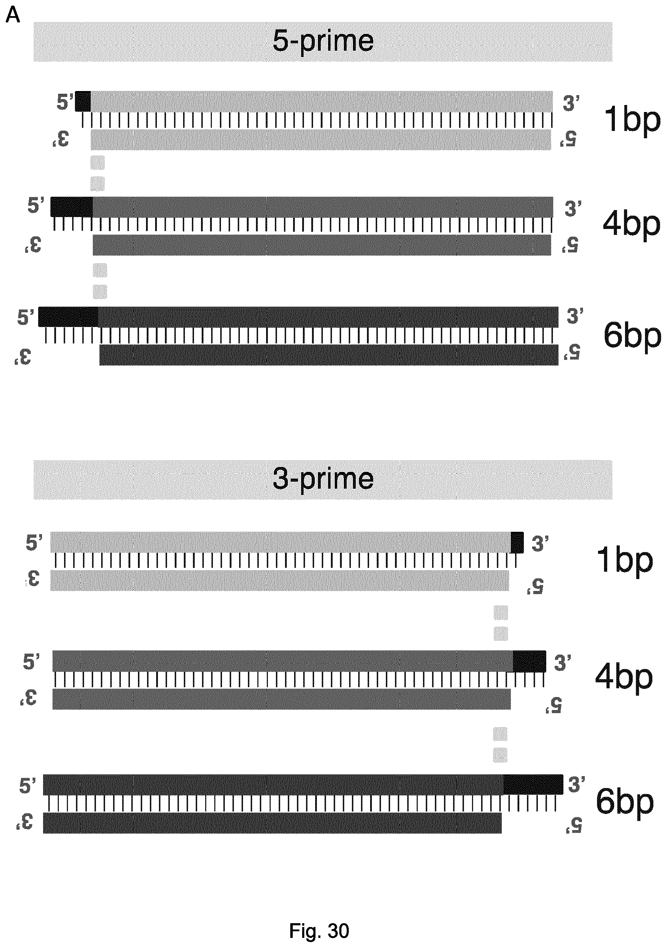

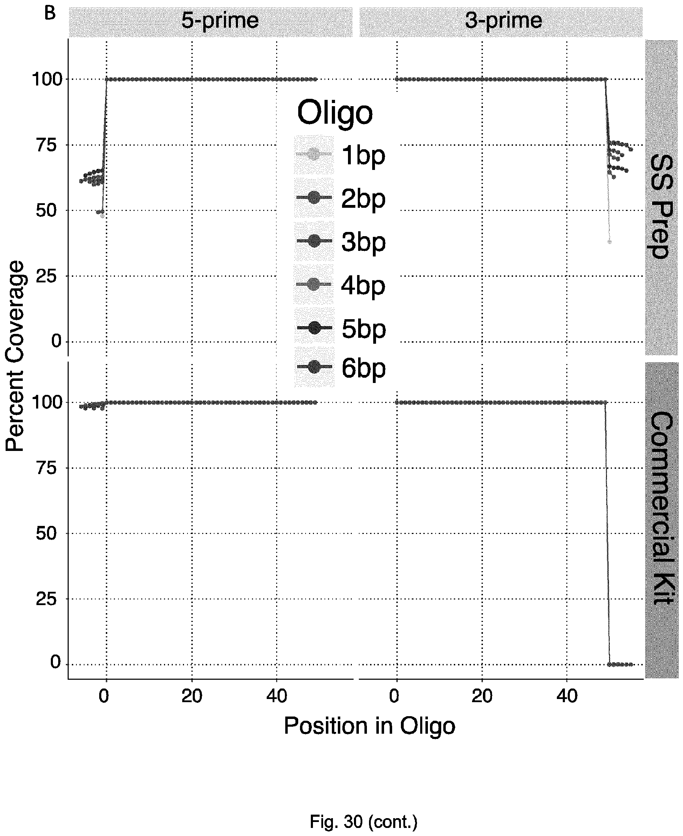

Fig. 30 shows coverage of duplexed oligos containing single-stranded overhangs for single-stranded library prep (ssPrep) and commercial kit (dsPrep; i.e., NEBNEXT ULTRA II). Panel A: Cartoon schematic of duplexed synthetic oligos - one blunt end, an identifiable 50 nt complementary region, and an overhang of specific length and type. Panel B: Average coverage per base across the length of all duplexed oligos for three technical replicates in 0 base coordinates for both ssPrep and commercial kit methods. Technical replicates were not statistically different from each other (Students t-test: ssPrep p=0.714, commercial kit p=0.985). Each oligo sequenced > 5,000 reads.

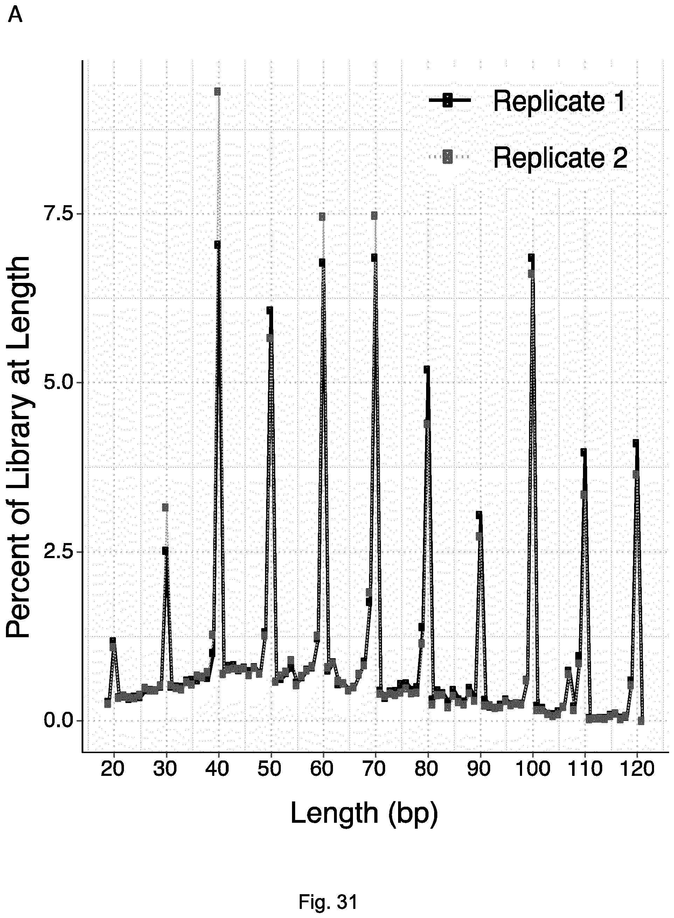

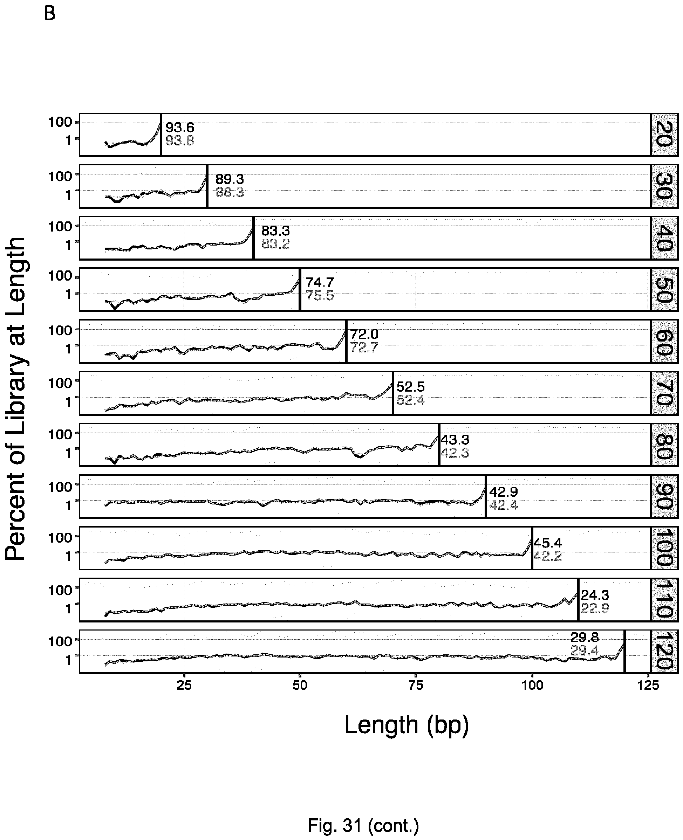

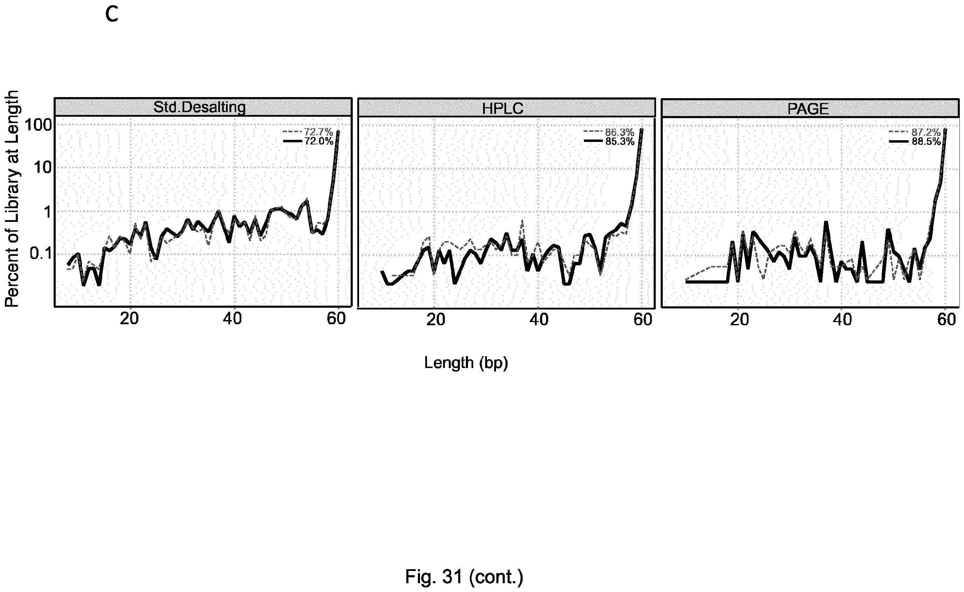

Fig. 31 shows single-stranded oligo analyses by ssPrep. Gray and black lines and dots represent technical replicates. Panel A: Insert distribution of equimolar pooled single-stranded oligo libraries. Oligos from 20 -120 nt synthesized at 10 nt intervals were purified by standard desalting. Raw unfiltered sequencing data. Panel B: Mapped sequencing data for technical replicates separated by oligo. Represented as a function of oligo length. Black vertical bar and associated black and gray numbers indicate percent of full-length product per oligo length present in the library pool. Each library was sequenced to a depth of ~100,000 read pairs (10,000 read pairs per oligo, excluding 20 and 30 nt lengths). Panel C: Effects for various purification methods on oligo purity as a function of oligo length for a 60 nt synthesized oligo. Associated black and gray numbers indicate percent of full-length product per oligo. Data for the standard desalted 60 nt synthetic oligo pulled from Panel B.

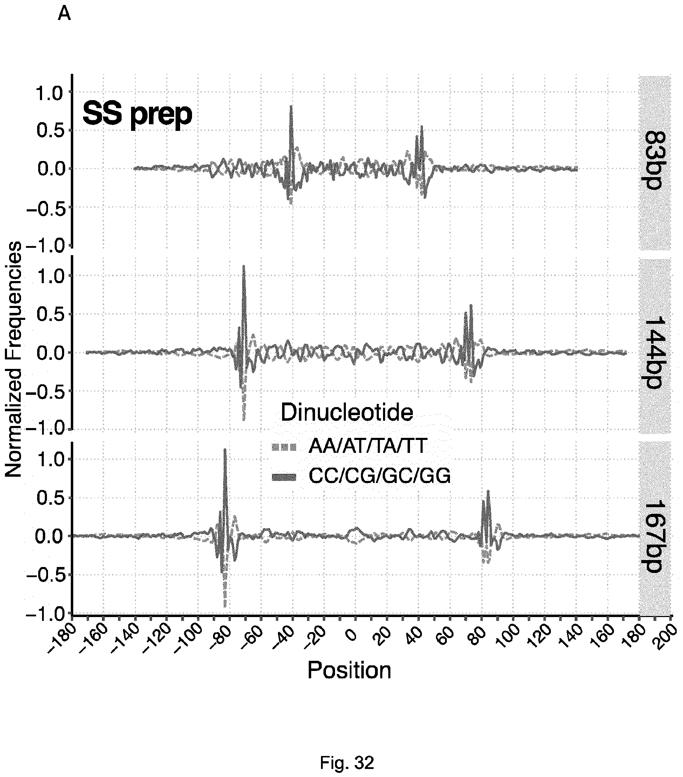

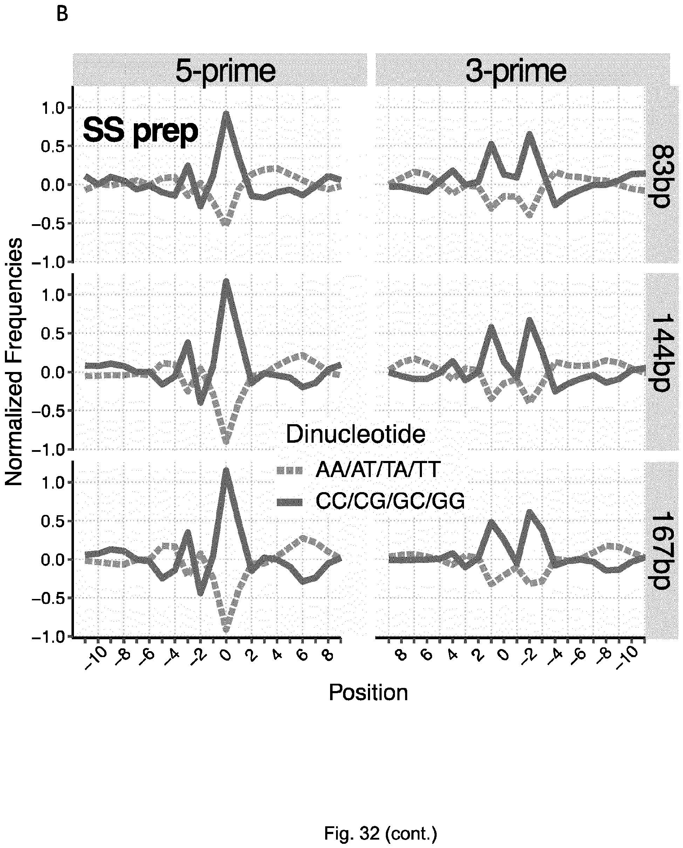

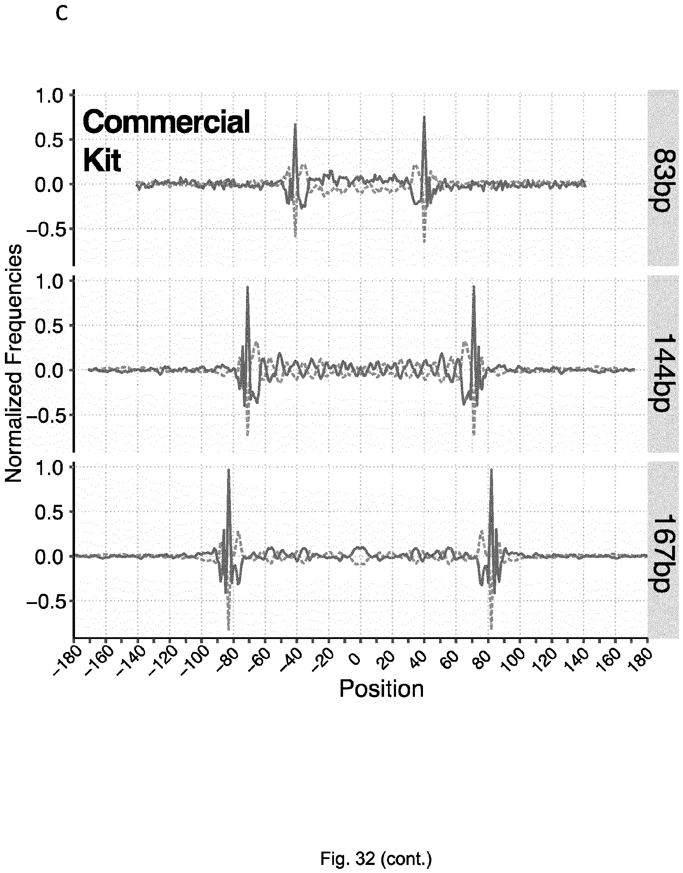

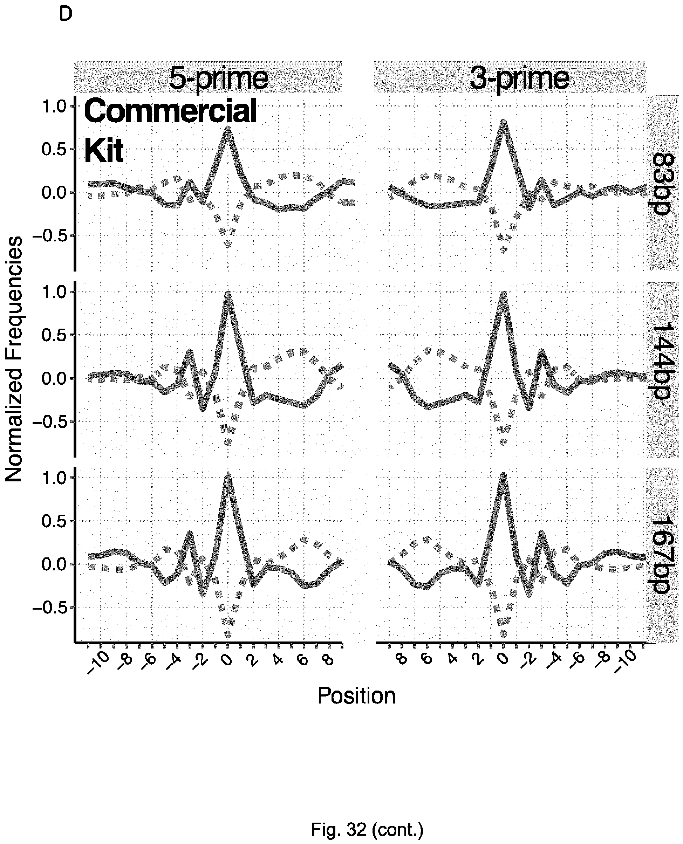

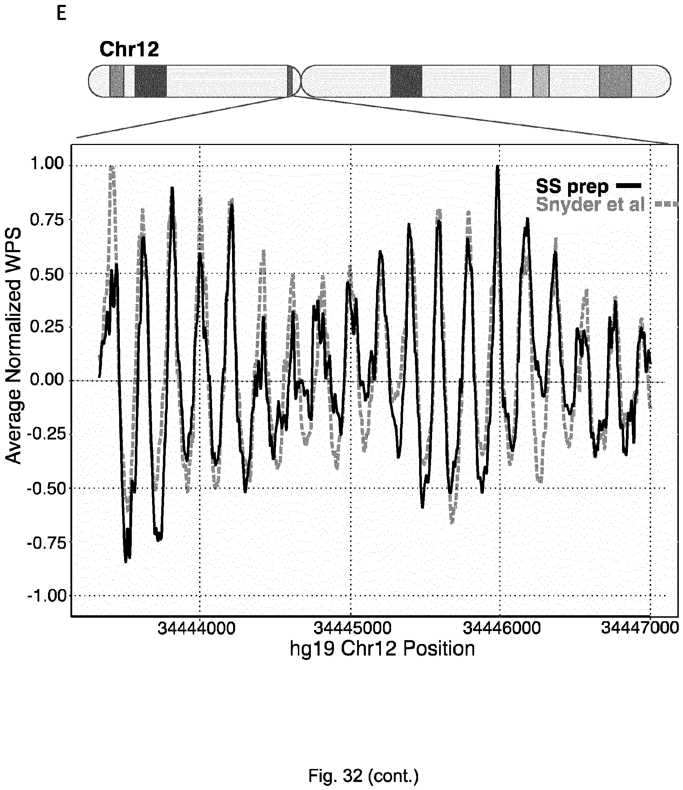

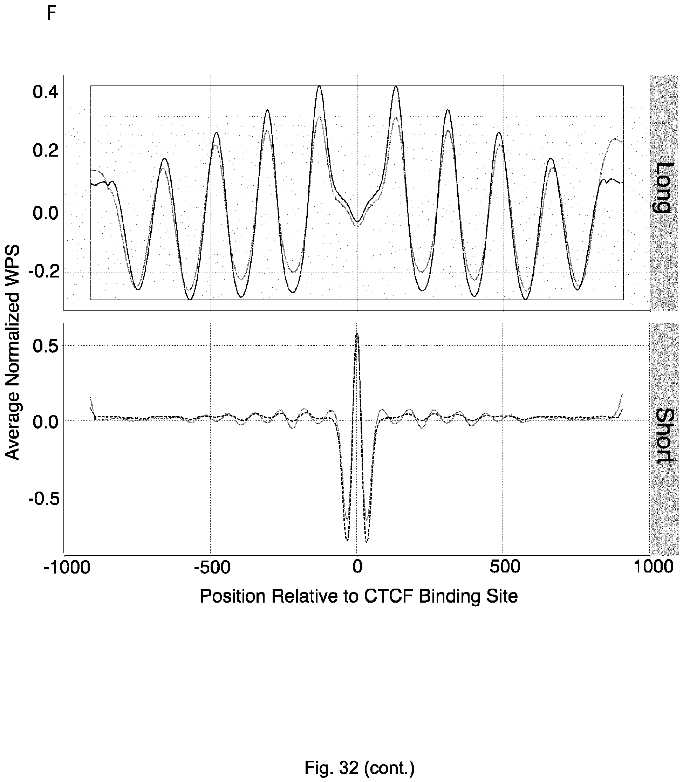

Fig. 32 shows a cfDNA analysis. Panel A: Normalized genomic dinculeotide frequencies as a function of read length for ssPrep data for three discrete fragment lengths including 100 bp ± the read mapped coordinates. Read midpoint is centered at 0. Negative numbers denote genomic regions upstream (5-prime) of the midpoint and positive numbers denote genomic regions downstream (3-prime) of the midpoint. Input data is from the combined sample A and sample B ssPrep datasets. Panel B: Normalized genomic dinucleotide frequency as a function of read length for ssPrep data for the termini of three discrete fragment lengths including a 9 bp region into the read (positive numbers) and 10 bp outside the read (negative numbers). Read start and end coordinates are centered on 0. Input data is from the combined sample A and sample B ssPrep datasets. Panel C: Same as Panel A except for commercial kit data. Panel D: Same as Panel B except for commercial kit data. Panel E: Normalized WPS values (120 bp window; 120-180 bp fragments) for ssPrep data compared to sample CH01 at the same pericentromeric locus on chromosome 12 used to initially showcase WPS. Panel F: Average normalized WPS score within ± 1 kb of annotated CTCF binding sites for long fragment length binned data (120 bp window; 120-180 bp fragments) and short fragment length binned data (16 bp window; 35-80 bp fragments) for ssPrep data compared to sample CH01.

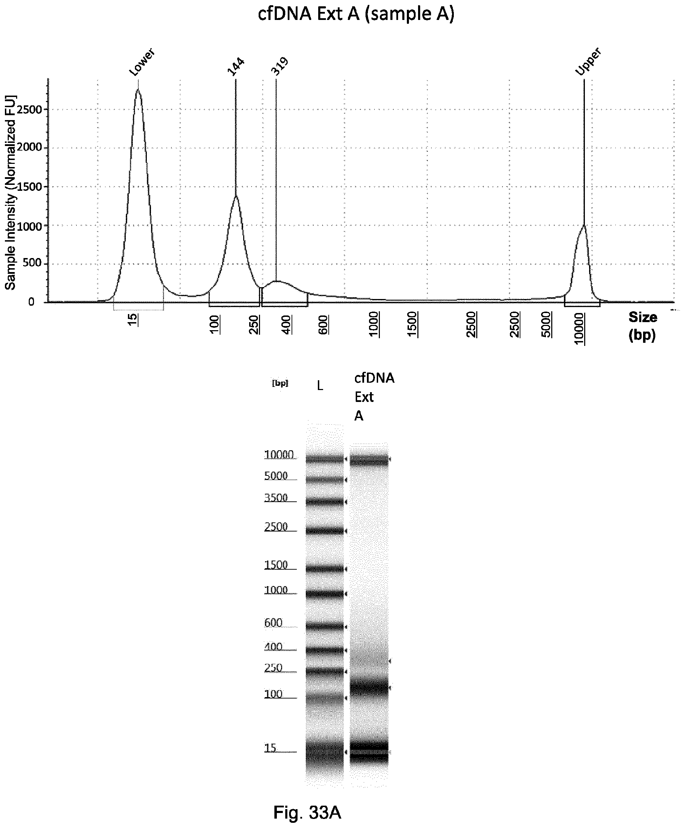

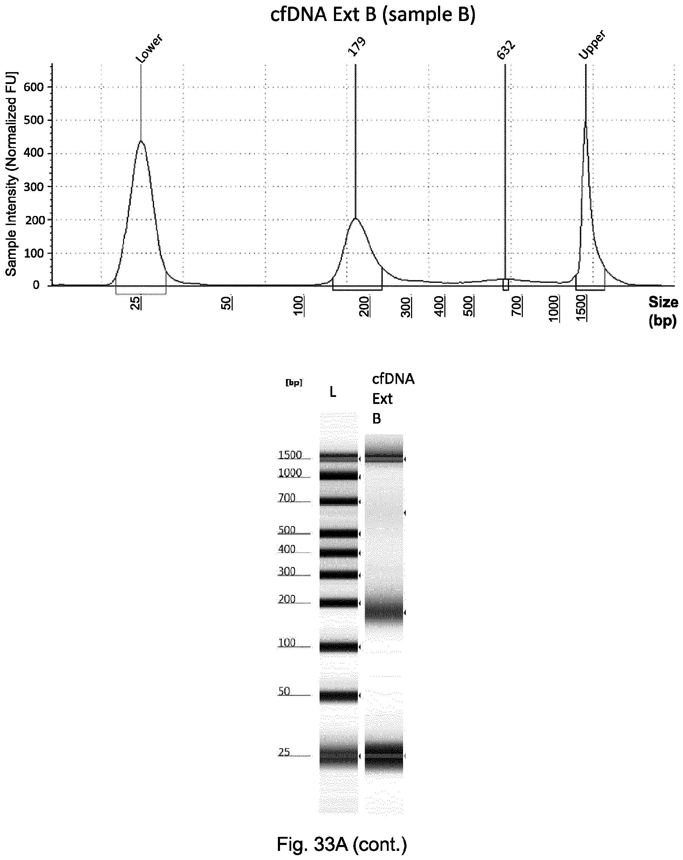

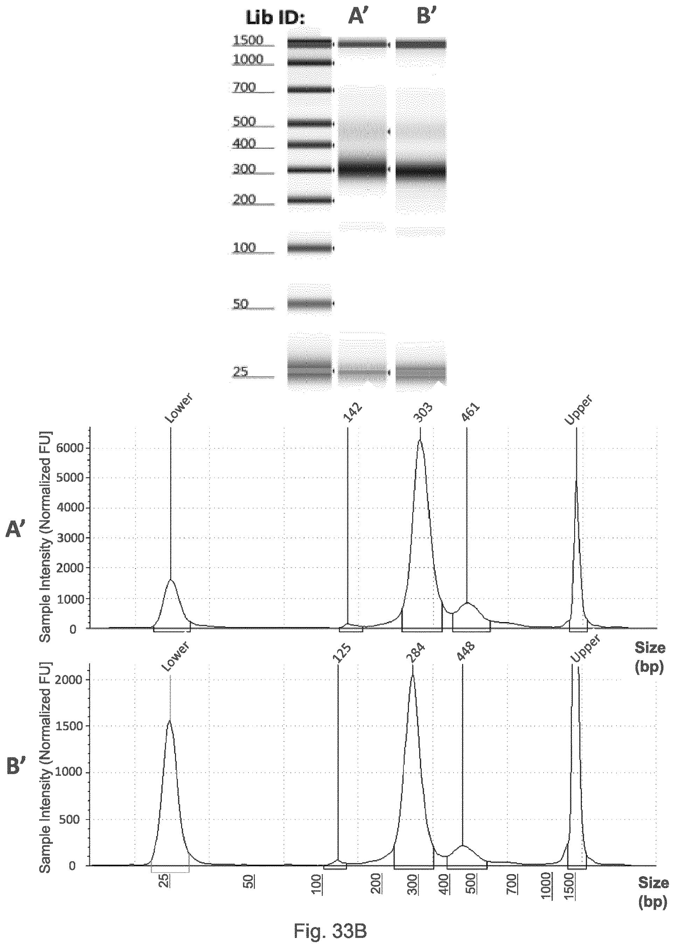

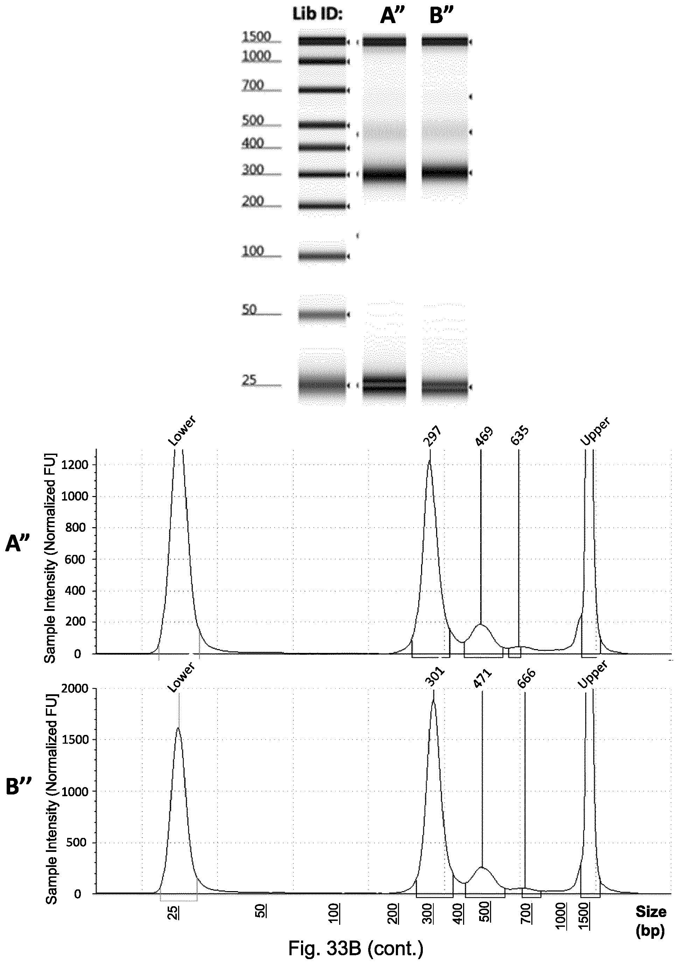

Figs. 33A and 33B show input cfDNA and representative ssPrep and dsPrep libraries. Fig 33A, top panel: Cell-free DNA extract (sample A) after plasma extraction/purification and before NGS library preparation. Analyzed on a D5000 HS tape and an associated Tapestation 4200 (Agilent) according to manufacturer's instructions. Gel image and electropherogram shown. Fig. 33A, bottom panel: Cell-free DNA extract (sample B) after plasma extraction/purification and before NGS library preparation. Analyzed on a D1000 HS tape and an associated Tapestation 4200 (Agilent) according to manufacturer's instructions. Gel image and electropherogram shown. Fig. 33B, top panel: One representative ssPrep library from cfDNA extract (sample A, library ID: A') and one representative ssPrep library from cfDNA extract (sample B, library ID: B') analyzed post index PCR on a D1000 HS tape and an associated Tapestation 4200 (Agilent) according to manufacturer's instructions. Gel image and electropherogram shown. Fig. 33B, bottom panel: One representative dsPrep (i.e., NEBNEXT ULTRA II) library from cfDNA extract (sample A, library ID: A") and one representative dsPrep (i.e., NEBNEXT ULTRA II) library from cfDNA extract (sample B, library ID B") analyzed post index PCR on a D1000 HS tape and an associated Tapestation 4200 (Agilent) according to manufacturer's instructions. Gel image and electropherogram shown.

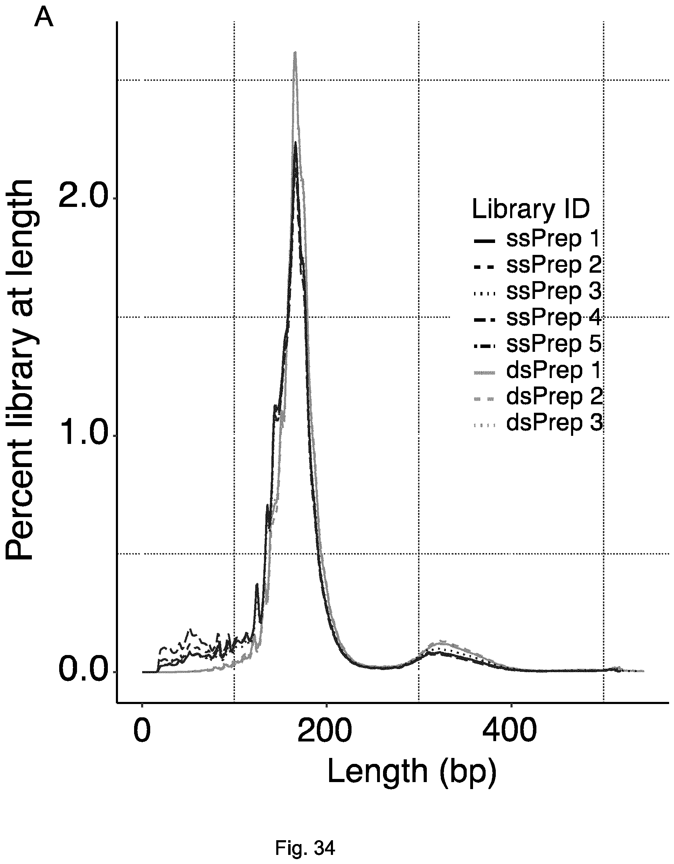

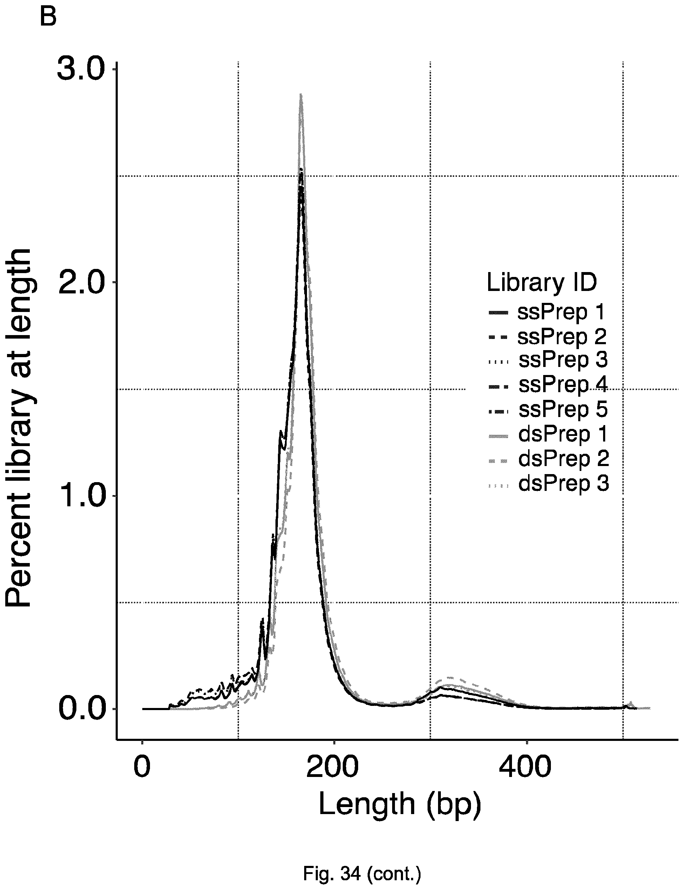

Fig. 34 shows insert distributions for replicate libraries for sample A and sample B. Panel A: Insert distribution for all libraries made for sample A cfDNA extract. Panel B: Insert distribution for all libraries made for sample B cfDNA extract. The order of the library IDs listed in each legend corresponds to the order of the traces shown in the legend.

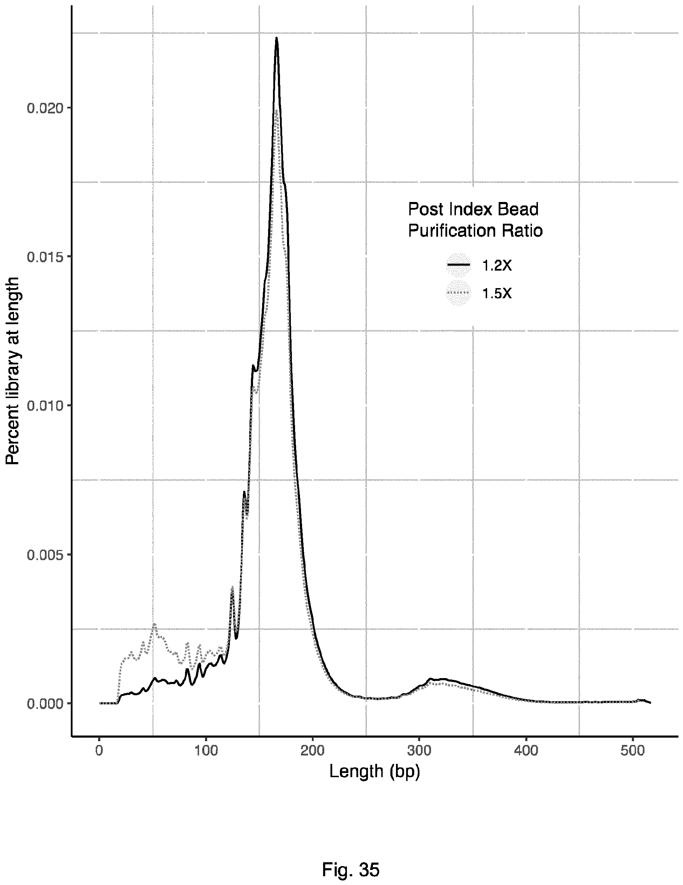

Fig. 35 shows effect of post index PCR DNA purification on fragment length retention. ssPrep libraries for cfDNA (sample A) were purified using either a 1.2x or 1.5x DNA purification bead volume:lndex PCR reaction volume ratio. Recovery of <100 bp fragments changed from 9.3% to 14.7% for the higher ratio from the lower ratio.

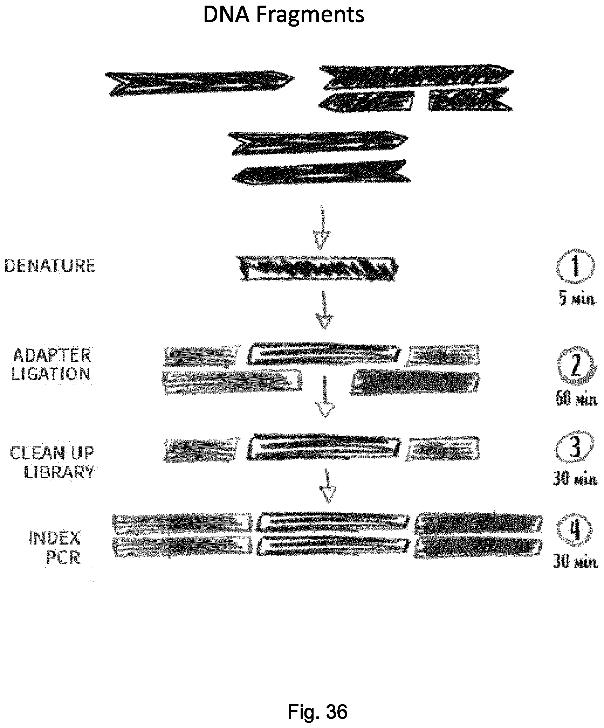

Fig. 36 shows an example workflow for single-stranded DNA library preparation (ssPrep). ssPrep works in a one-step combined phosphorylation/ligation step that simultaneously prepares template DNA molecules for ligation without end-polishing (i.e., end repair) and ligates ILLUMINA adapters by utilizing scaffold adapters described herein.

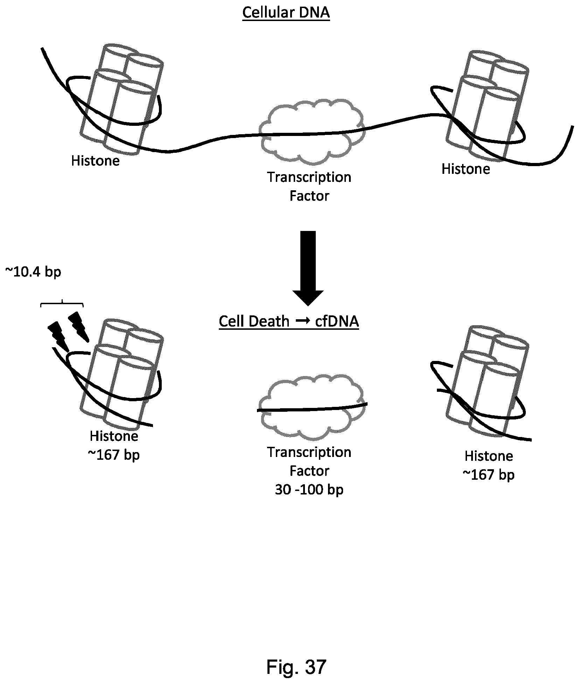

Fig. 37 shows an illustration of the cfDNA Protection Model.

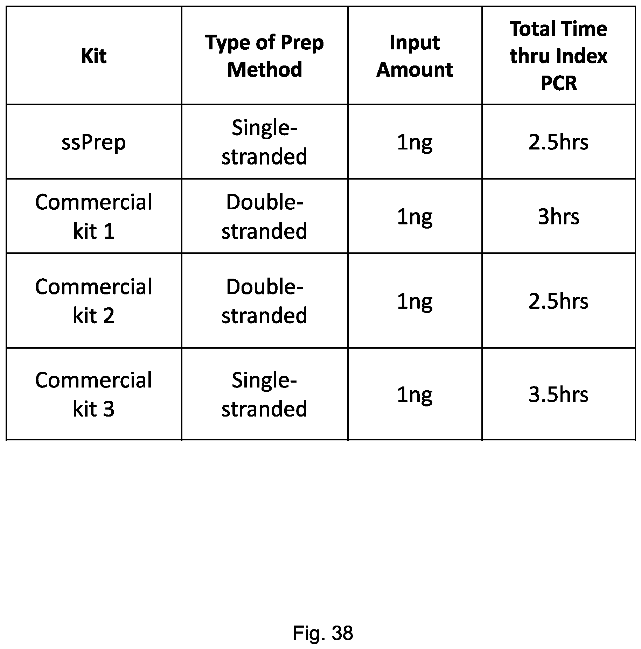

Fig. 38 provides a table showing a library prep kit and input cfDNA summary. Sequencing libraries were generated from two healthy individual cfDNA extracts (Sample 1 and Sample 2) utilizing ssPrep, two commercially available end-polished dsDNA library prep kits, and a commercially available ssDNA kit. Libraries were sequenced 2 x 151 bp on ILLUMINA HISEQ X. Commercial kit 1, NEBNext^{®} Ultra II^{™}; Commercial kit 2, TaKaRa ThruPLEX@ Plasma-Seq; Commercial kit 3, Swift Accel-NGS^{®} 1S Plus.

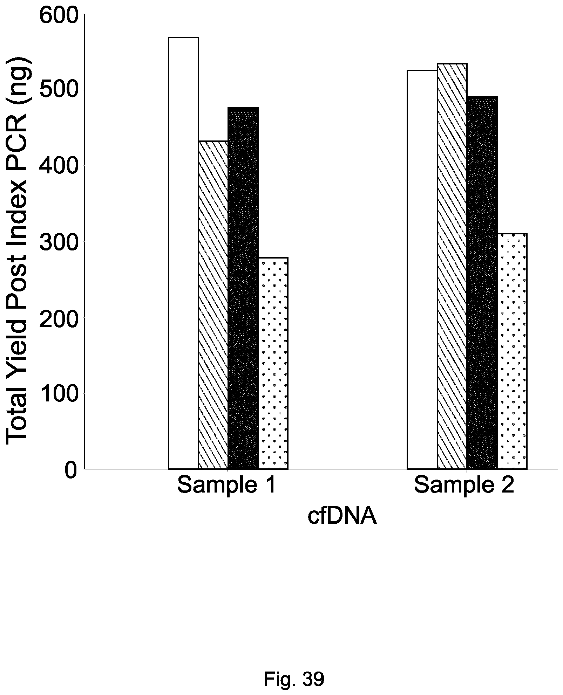

Fig. 39 shows yields post index PCR. Libraries were quantified using a Qubit 3.0 with 2 µl of final purified library post index PCR. All libraries indexed for 10 cycles of PCR using kit-supplied polymerase master mix and primers. Prep kits are represented in each histogram from left to right: ssPrep, Swift, TaKaRa, NEB.

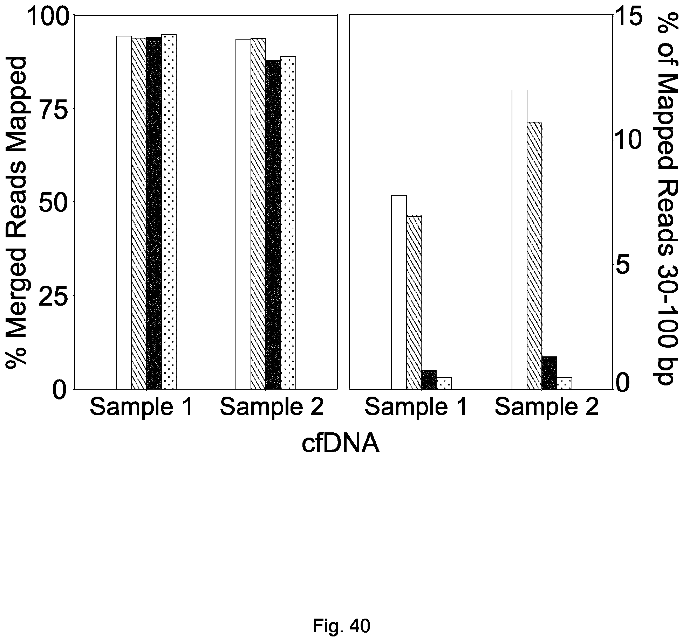

Fig. 40 shows mapping and short insert bin data. Libraries were trimmed/merged using SeqPrep2.0, discarding merged reads < 30 bp. Remaining reads mapped to human reference genome hg19 with BWA aln. Size binning was performed with a custom script. Prep kits are represented in each histogram from left to right: ssPrep, Swift (Swift Accel-NGS^{®} 1S Plus), TaKaRa (Swift Accel-NGS^{®} 1S Plus), NEB (NEBNext^{®} Ultra II^{™}).

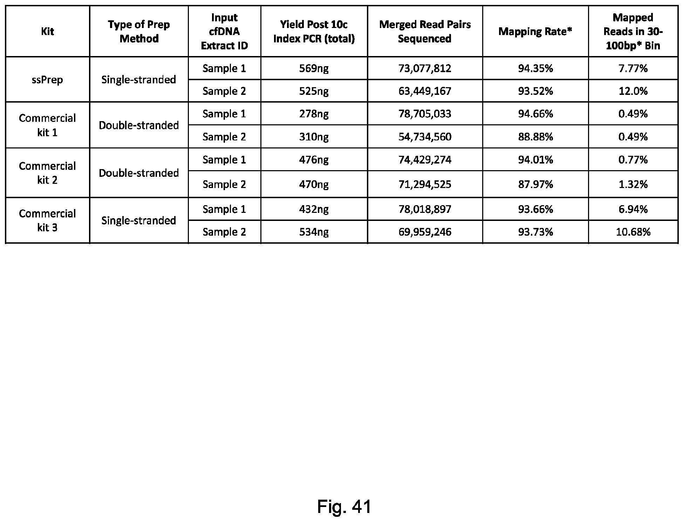

Fig. 41 provides a table showing post index PCR yields and mapping stats. *Due to tailing artifacts created during library prep, Commercial kit 3 recommends trimming 10 bp from forward and reverse reads prior to mapping. Accordingly, the upper limit for the bin size of 30-100 bp was reduced to 90 bp instead of 100 bp. Commercial kit 1, NEBNext^{®} Ultra II^{™}; Commercial kit 2, TaKaRa ThruPLEX^{®} Plasma-Seq; Commercial kit 3, Swift Accel-NGS^{®} 1S Plus.

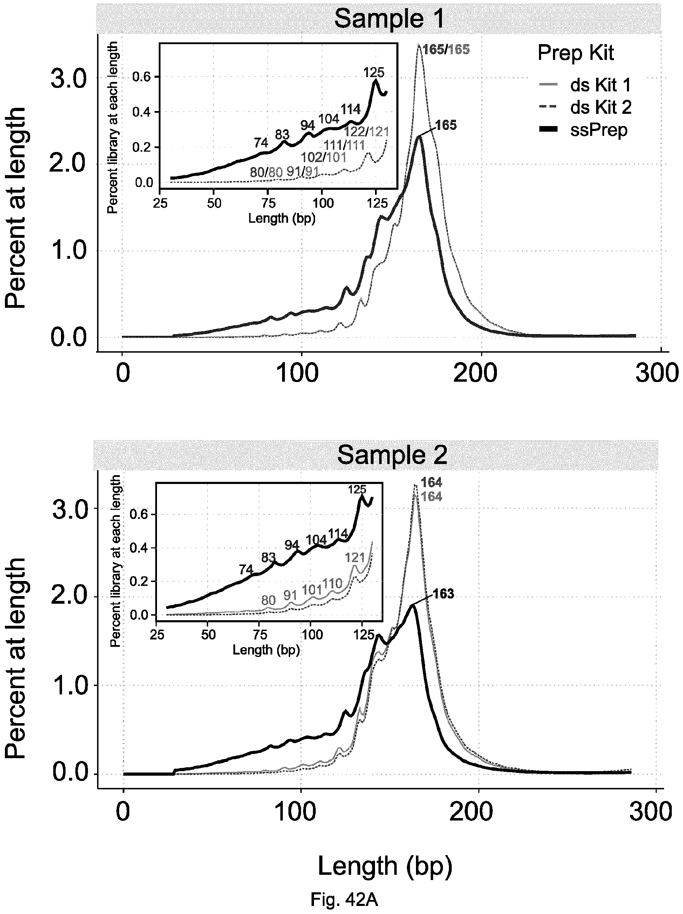

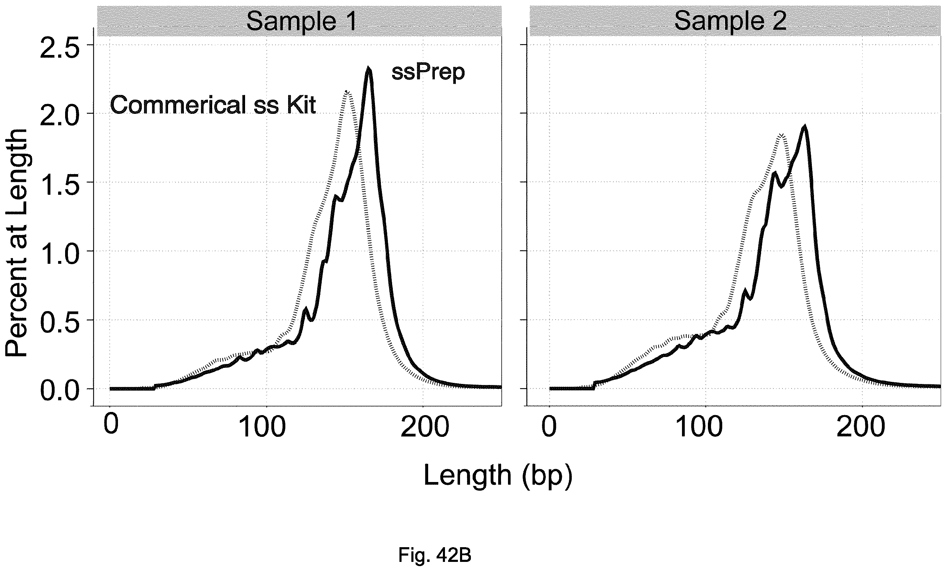

Figs. 42A and 42B show mapped insert length distributions. Fig. 42A shows ssPrep versus dsDNA preps. Kit 2, TaKaRa ThruPLEX^{®} Plasma-Seq; Kit 1, NEBNext^{®} Ultra II^{™}. Fig. 42B shows ssPrep vs. a commercial ssDNA prep kit (Swift Accel-NGS^{®} 1S Plus). Read lengths for all molecules contained in each library were extracted from properly mapped and sorted bam files and then plotted.

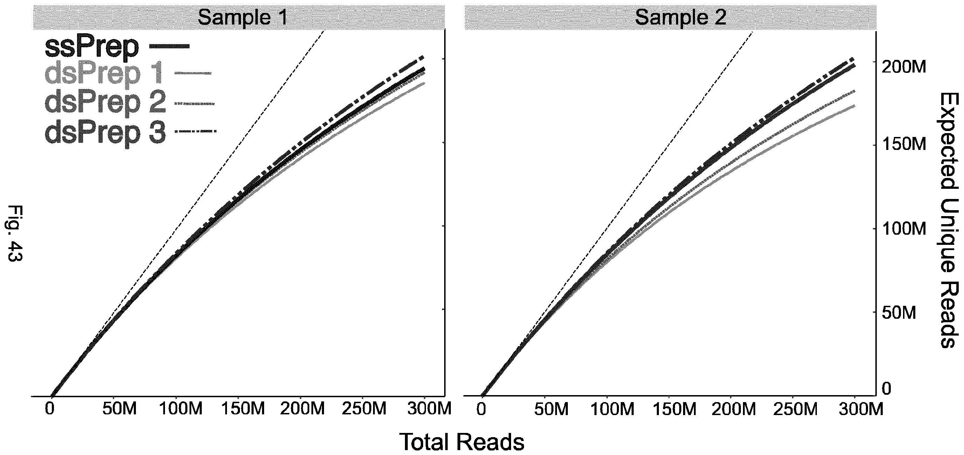

Fig. 43 shows complexity estimates. Complexity estimate output for library prep kits was from Preseq algorithm. Reads from the table in Fig. 41 were used as input and number of unique molecules was extrapolated out to 300M read pairs.

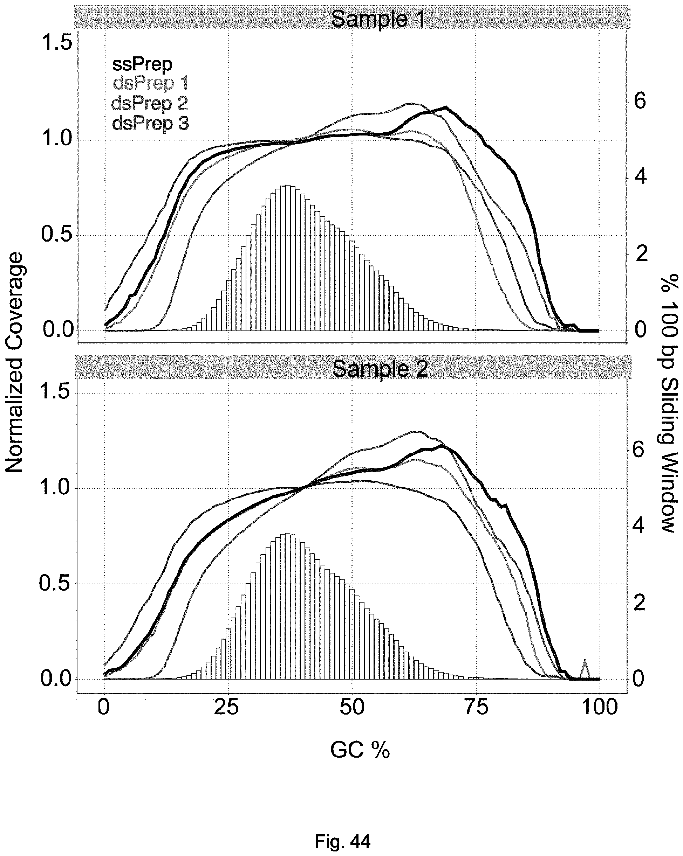

Fig. 44 shows GC coverage as normalized coverage of libraries for each cfDNA extract as a function of the human reference genome's (hg19) GC content over a 100 bp sliding window. Left Y-axis refers to cfDNA (lines). Right axis refers to the reference genome (histogram in gray). Data pulled from Picard Tools CollectGcBiasMetrics.

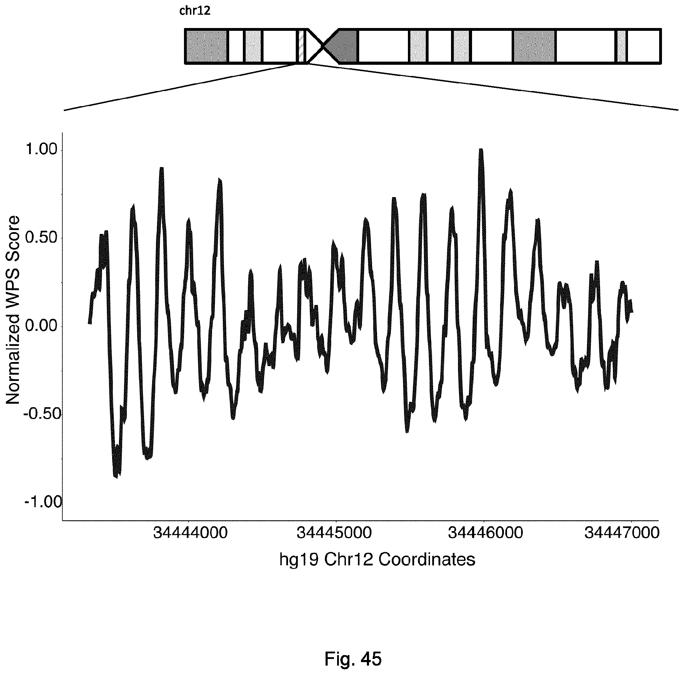

Fig. 45 shows nucleosome occupancy. WPS calculations were generated and normalized to absolute highest WPS value for an alpha-satellite array sub region on chr12. cfDNA data was obtained by combining ssPrep bam files for healthy cfDNA samples 1 and 2 and filtering for reads with nucleosome-associated inserts prior to WPS calculations.

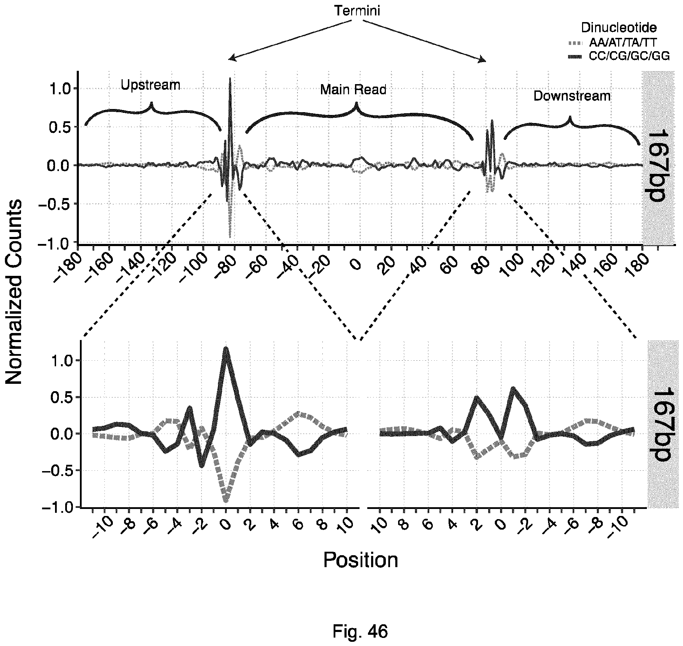

Fig. 46 shows dinucleotide frequencies. Dinucleotide frequency for AT and CG containing dinculeotides was plotted for the 5' and 3' termini of all reads equal to the main histone monomer peak for Sample 1 at 1657bp.

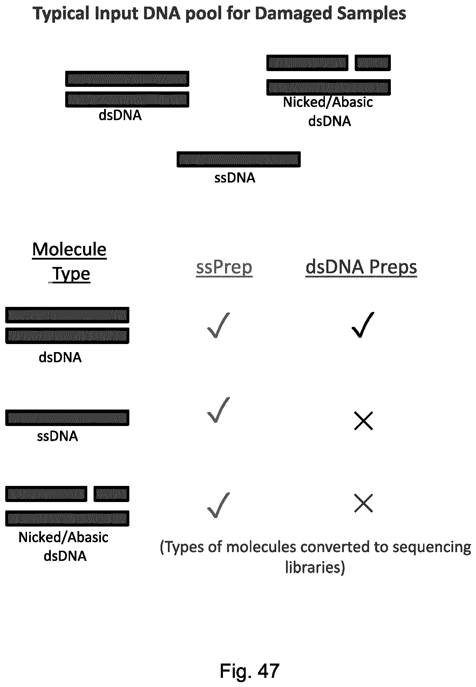

Fig. 47 shows a comparison of library prep methods for damaged DNA.

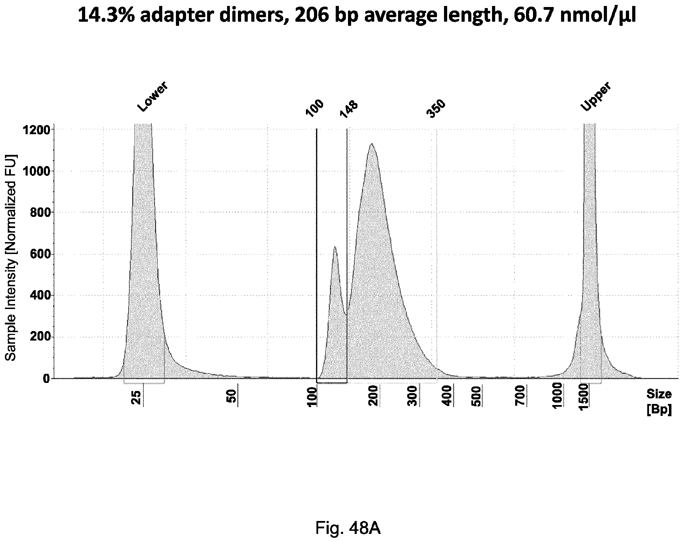

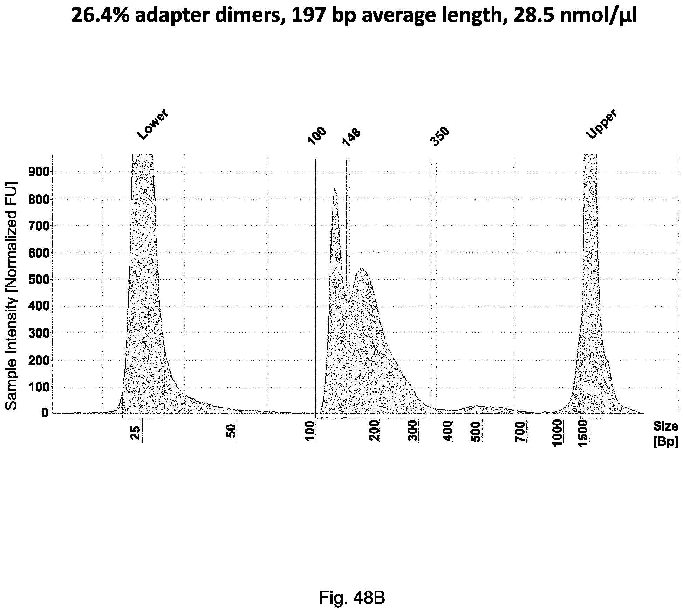

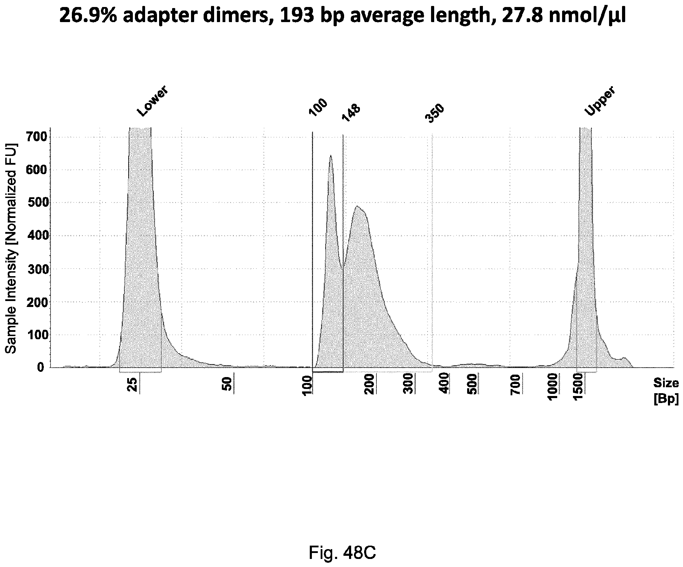

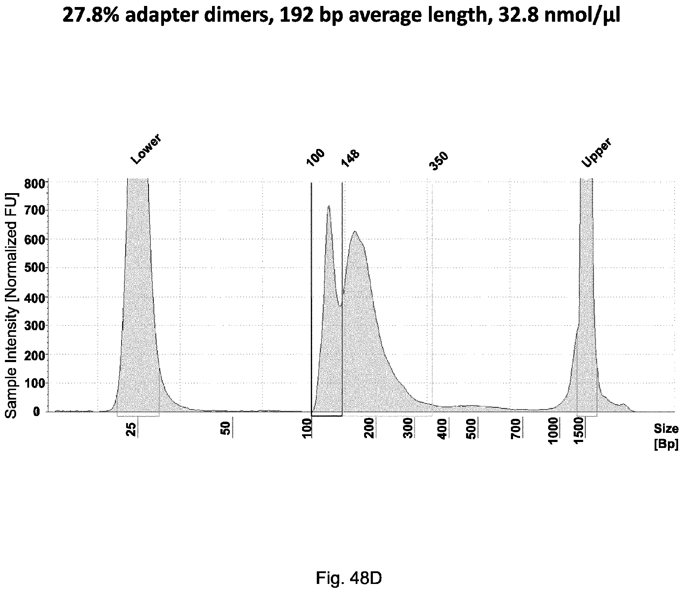

Fig. 48A to Fig. 48D show a comparison of fragment lengths and percent adapter dimers recovered after SPRI purification of fragment-scaffold adapter ligation products under various purification conditions. Fig. 48A shows adapter dimer and fragment length peaks for fragment-scaffold adapter ligation products purified using an 18% PEG SPRI purification with the addition of 50µl Tris buffer (i.e., added to 25µl of ligation products). Fig. 48B shows adapter dimer and fragment length peaks for fragment-scaffold adapter ligation products purified using an 18% PEG SPRI purification with the addition of 25 µl isopropanol and 25 µl Tris buffer. Fig. 48C shows adapter dimer and fragment length peaks for fragment-scaffold adapter ligation products purified using an 18% PEG SPRI purification with the addition of 50 µl isopropanol. Fig. 48D shows adapter dimer and fragment length peaks for fragment-scaffold adapter ligation products purified using an SPRI bead solution buffer containing 38% PEG.

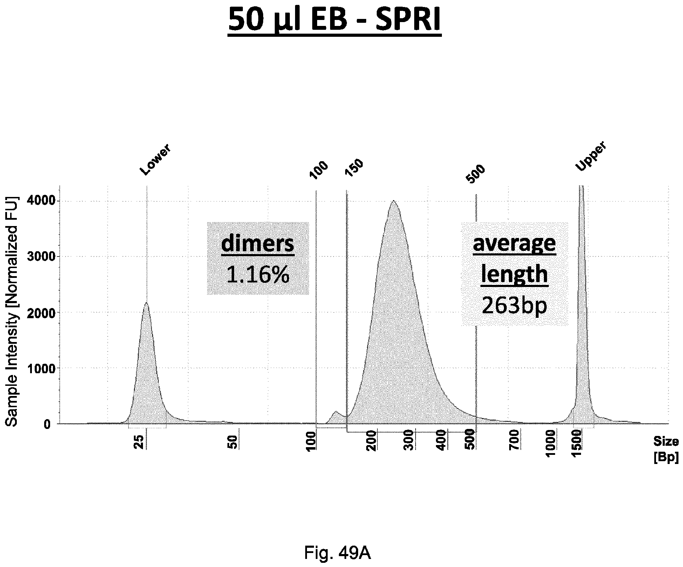

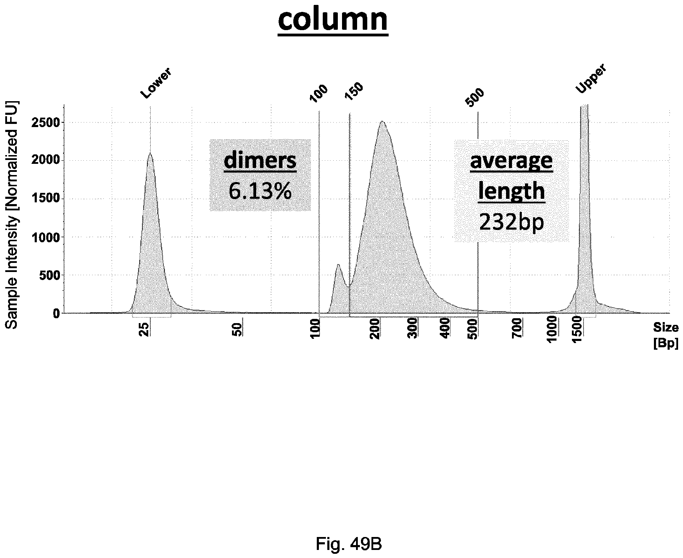

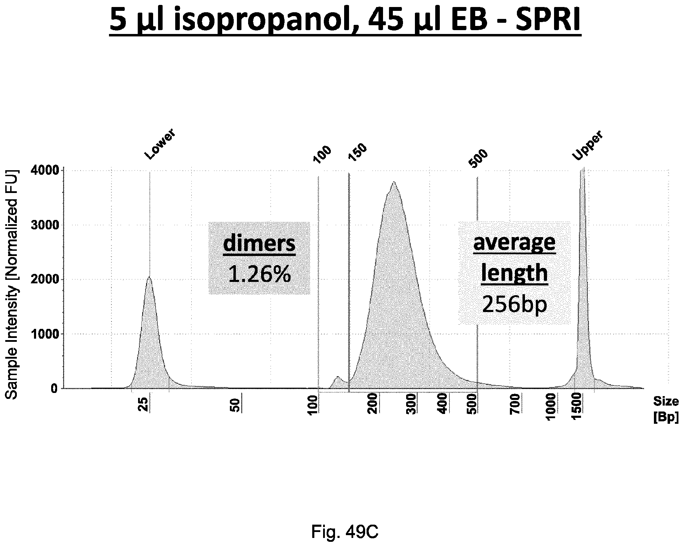

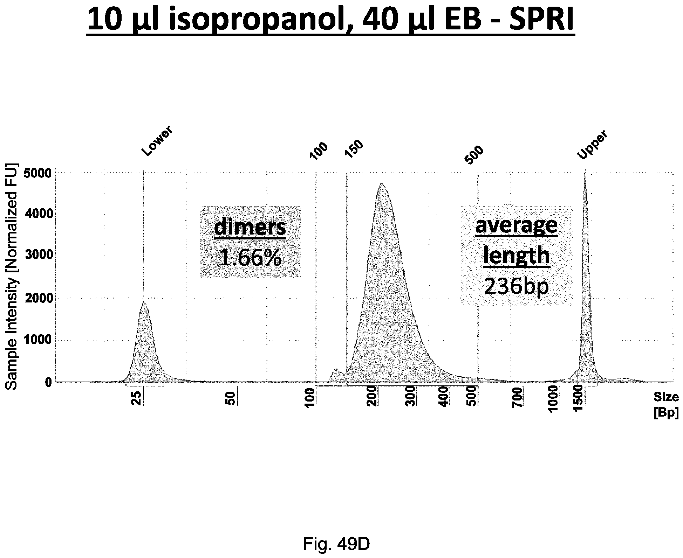

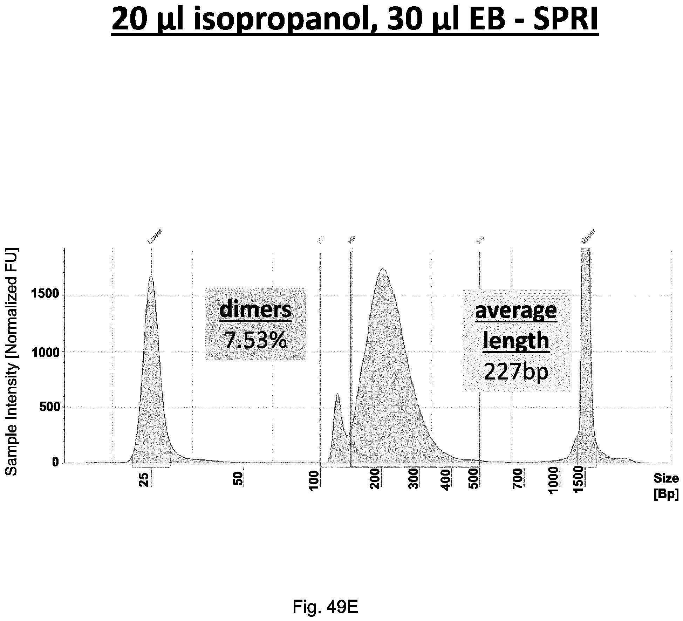

Fig. 49A to Fig. 49E show a comparison of fragment lengths and percent adapter dimers recovered after purification of fragment-scaffold adapter ligation products under various purification conditions. Fig. 49A shows adapter dimer and fragment length peaks for fragment-scaffold adapter ligation products purified using an 18% PEG SPRI purification with the addition of 50 µl Tris buffer. Fig. 49B shows adapter dimer and fragment length peaks for fragment-scaffold adapter ligation products purified using column purification. Fig. 49C shows adapter dimer and fragment length peaks for fragment-scaffold adapter ligation products purified using an 18% PEG SPRI purification with the addition of 5 µl isopropanol and 45 µl Tris buffer. Fig. 49D shows adapter dimer and fragment length peaks for fragment-scaffold adapter ligation products purified using an 18% PEG SPRI purification with the addition of 10 µl isopropanol and 40 µl Tris buffer. Fig. 49E shows adapter dimer and fragment length peaks for fragment-scaffold adapter ligation products purified using an 18% PEG SPRI purification with the addition of 20 µl isopropanol and 30 µl Tris buffer.

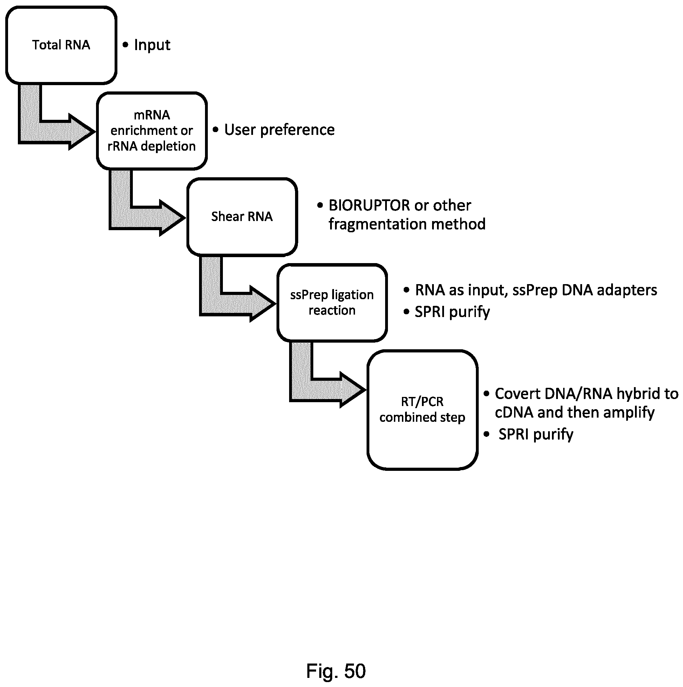

Fig. 50 shows an example workflow for generating a sequencing library from single-stranded RNA.

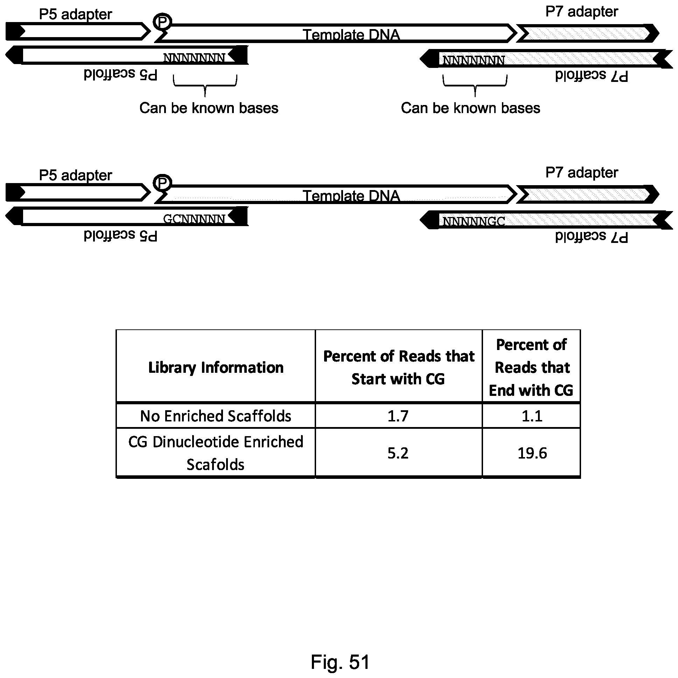

Fig. 51 shows example scaffold adapters designs where some or all of the bases in the ssNA hybridization region are defined or known bases.

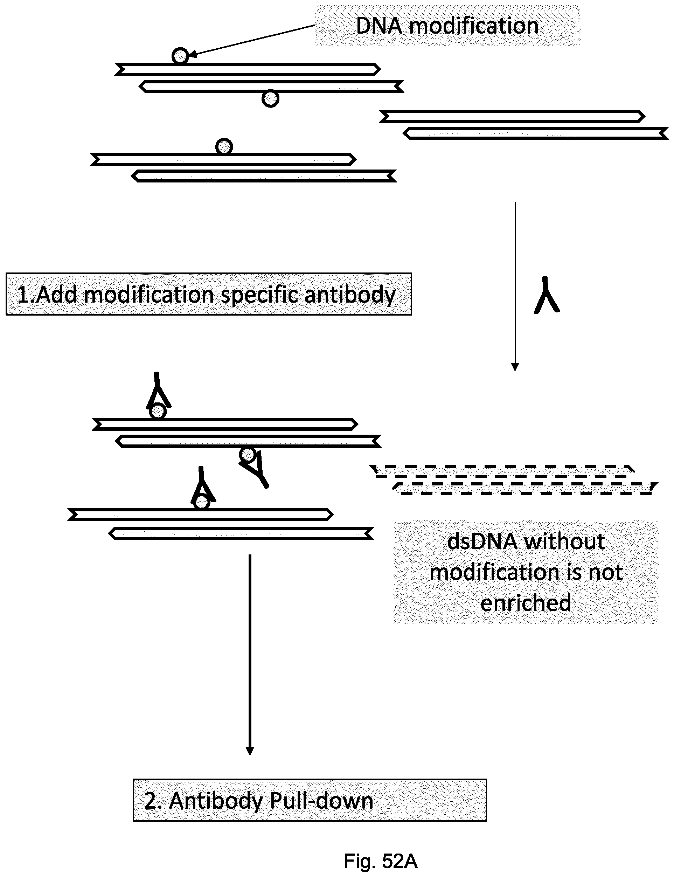

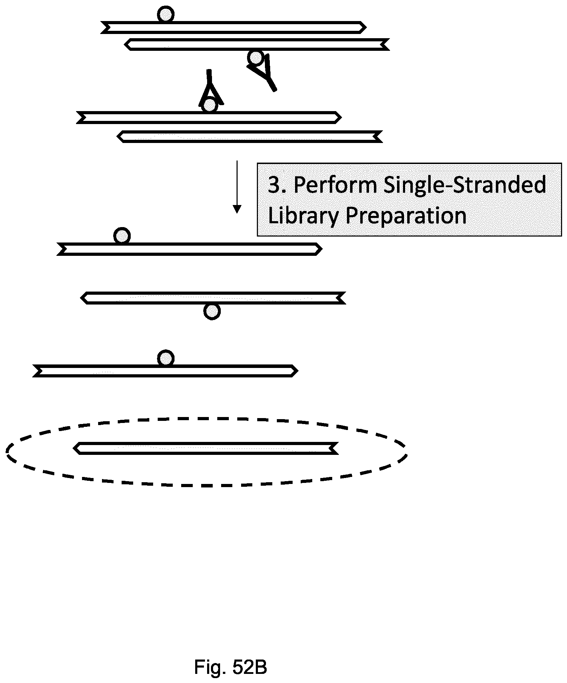

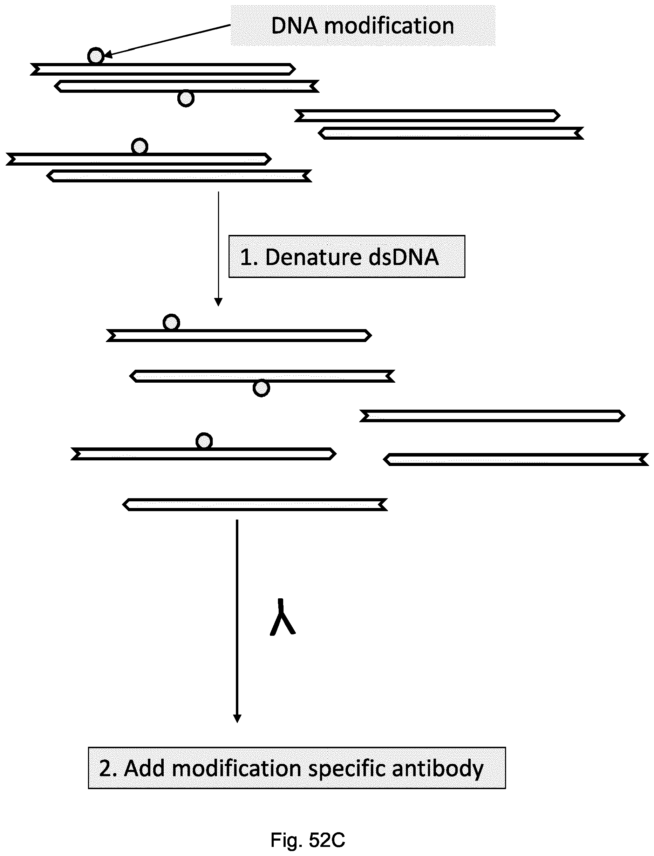

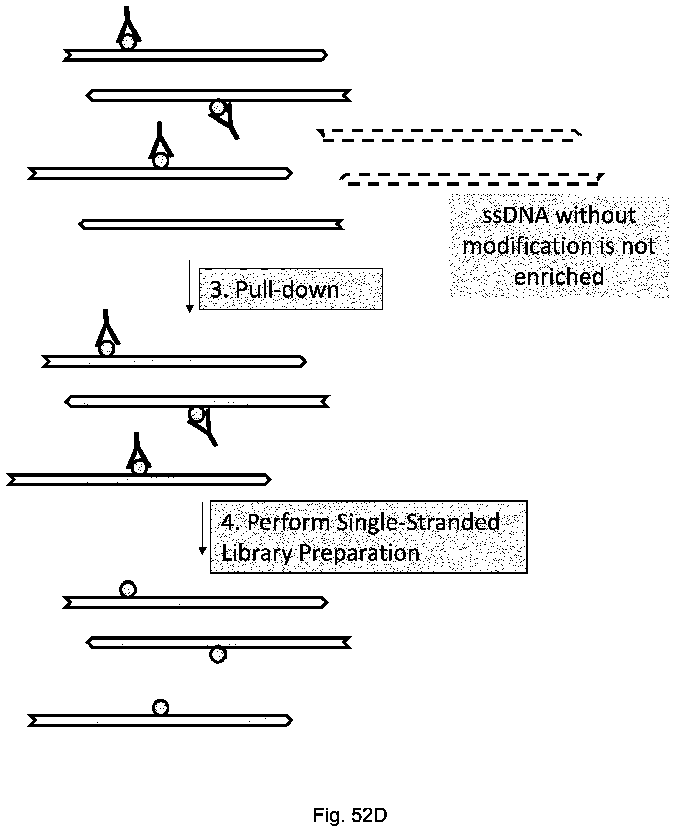

Fig. 52A to Fig. 52D show example workflows for enrichment of modified nucleic acids. Modified nucleic acid enrichment can be conducted before (Figs. 52A, 52B) or after (Figs. 52C, 52D) denaturation of dsDNA.

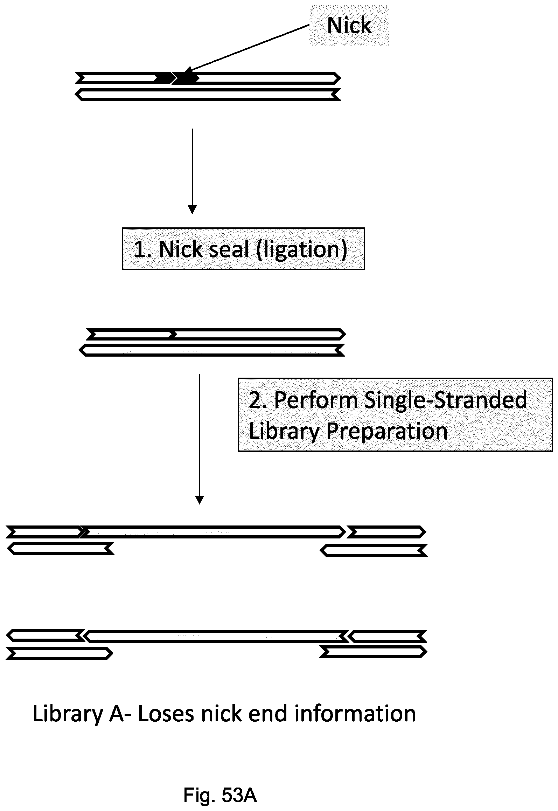

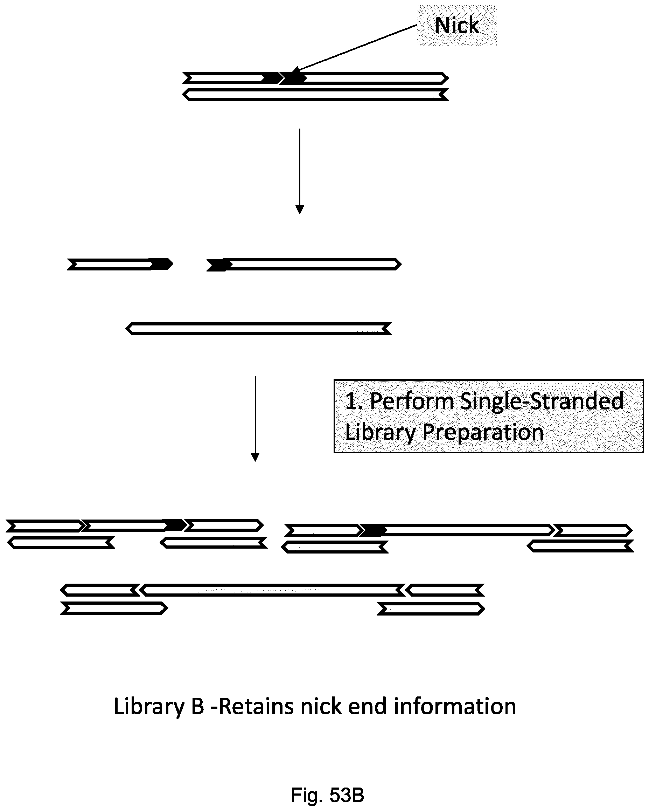

Fig. 53A and Fig. 53B show example workflows for generating libraries from nicked DNA.

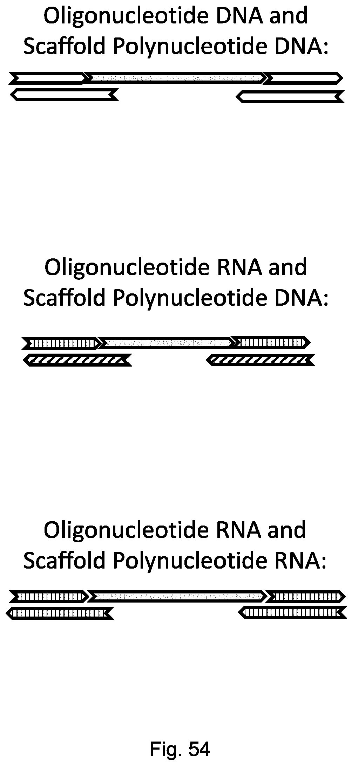

Fig. 54 shows example scaffold adapter configurations comprising DNA, RNA, or a combination thereof.

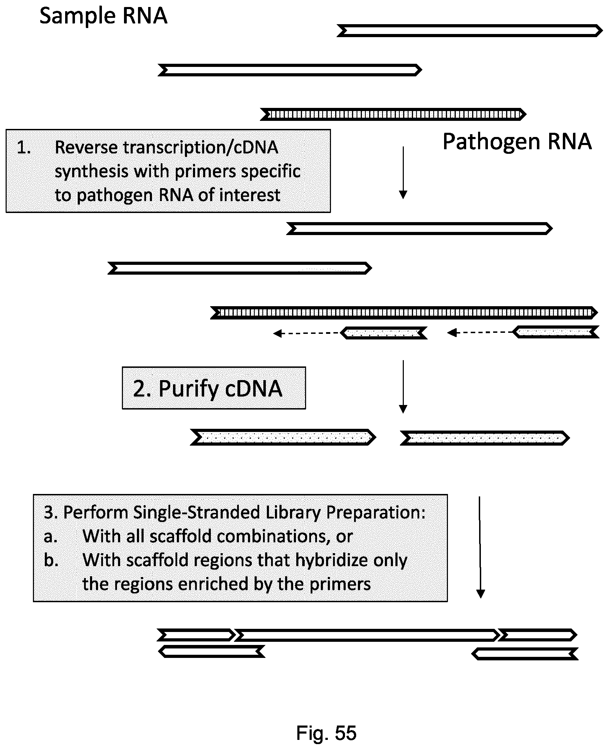

Fig. 55 shows an example workflow for generating a sequencing library from samples containing pathogen RNA.

### Detailed Description

While the present invention is defined by the claims, also provided herein are methods and compositions useful for analyzing nucleic acid. Also provided herein are methods and compositions useful for producing nucleic acid libraries. Also provided herein are methods and compositions useful for analyzing single-stranded nucleic acid fragments. In certain aspects, the methods include combining sample nucleic acid comprising single-stranded nucleic acid fragments and specialized adapters. In some implementations, the specialized adapters include a scaffold polynucleotide capable of hybridizing to an end of a single-stranded nucleic acid. Products of such hybridization may be useful for producing a nucleic acid library and/or further analysis or processing, for example.

### Scaffold adapters

Certain methods herein comprise combining ssNA with scaffold adapters, or components thereof. Scaffold adapters generally include a scaffold polynucleotide and an oligonucleotide. Accordingly, a "component" of a scaffold adapter may refer to a scaffold polynucleotide and/or an oligonucleotide, or a subcomponent or region thereof. The oligonucleotide and/or the scaffold polynucleotide can be composed of pyrimidine (C, T, U) and/or purine (A, G) nucleotides. Additional components or subcomponents may include one or more of an index polynucleotide, a unique molecular identifier (UMI), primer binding site (e.g., sequencing primer binding site, P5 primer binding site, P7 primer binding site), flow cell binding region, and the like, and complements thereto. Scaffold adapters comprising a P5 primer binding site may be referred to as P5 adapters or P5 scaffold adapters. Scaffold adapters comprising a P7 primer binding site may be referred to as P7 adapters or P7 scaffold adapters.

A scaffold polynucleotide is a single-stranded component of a scaffold adapter. A polynucleotide herein generally refers to a single-stranded multimer of nucleotide from 5 to 500 nucleotides, e.g., 5 to 100 nucleotides. Polynucleotides may be synthetic or may be made enzymatically, and, in some implementations, are about 5 to 50 nucleotides in length. Polynucleotides may contain ribonucleotide monomers (i.e., may be polyribonucleotides or "RNA polynucleotides"), deoxyribonucleotide monomers (i.e., may be polydeoxyribonucleotides or "DNA polynucleotides"), or a combination thereof. Polynucleotides may be 10 to 20, 20 to 30, 30 to 40, 40 to 50, 50 to 60, 60 to 70, 70 to 80, 80 to 100, 100 to 150 or 150 to 200, or up to 500 nucleotides in length, for example. The terms polynucleotide and oligonucleotide may be used interchangeably.

A scaffold polynucleotide may include an ssNA hybridization region (also referred to as scaffold, scaffold region, single-stranded scaffold, single-stranded scaffold region) and an oligonucleotide hybridization region. An ssNA hybridization region and an oligonucleotide hybridization region may be referred to as subcomponents of a scaffold polynucleotide. An ssNA hybridization region typically comprises a polynucleotide that hybridizes, or is capable of hybridizing, to an ssNA terminal region. An oligonucleotide hybridization region typically comprises a polynucleotide that hybridizes, or is capable of hybridizing, to all or a portion of the oligonucleotide component of the scaffold adapter.

An ssNA hybridization region of a scaffold polynucleotide may comprise a polynucleotide that is complementary, or substantially complementary, to an ssNA terminal region. In some implementations, an ssNA hybridization region comprises a random sequence. In some implementations, an ssNA hybridization region comprises a sequence complementary to an ssNA terminal region sequence of interest (e.g., targeted sequence). In certain implementations, an ssNA hybridization region comprises one or more nucleotides that are all capable of non-specific base pairing to bases in the ssNA. Nucleotides capable of non-specific base pairing may be referred to as universal bases. A universal base is a base capable of indiscriminately base pairing with each of the four standard nucleotide bases: A, C, G and T. Universal bases that may be incorporated into the ssNA hybridization region include, but are not limited to, inosine, deoxyinosine, 2'-deoxyinosine (dl, dlnosine), nitroindole, 5-nitroindole, and 3-nitropyrrole. In certain implementations, an ssNA hybridization region comprises one or more degenerate/wobble bases which can replace two or three (but not all) of the four typical bases (e.g., non-natural base P and K).

An ssNA hybridization region of a scaffold polynucleotide may have any suitable length and sequence. In some implementations, the length of the ssNA hybridization region is 10 nucleotides or less. In certain aspects, the ssNA hybridization region is from 4 to 100 nucleotides in length, e.g., about 5, 10, 15, 20, 25, 30, 35, 40, 45, 50, 55, 60, 65, 70, 75, 80, 85, 90, 95, or 100 nucleotides in length. In certain aspects, the ssNA hybridization region is from 4 to 20 nucleotides in length, e.g., from 5 to 15, 5 to 10, 5 to 9, 5 to 8, or 5 to 7 (e.g., 6 or 7) nucleotides in length. In some implementations, the ssNA hybridization region is 7 nucleotides in length. In some implementations, the ssNA hybridization region comprises or consists of a random nucleotide sequence, such that when a plurality of heterogeneous scaffold polynucleotides having various random ssNA hybridization regions are employed, the collection is capable of acting as scaffold polynucleotides for a heterogeneous population of ssNAs irrespective of the sequences of the terminal regions of the ssNAs. Each scaffold polynucleotide having a unique ssNA hybridization region sequence may be referred to as a scaffold polynucleotide species and a collection of multiple scaffold polynucleotide species may be referred to as a plurality of scaffold polynucleotide species (e.g., for a scaffold polynucleotide designed to have 7 random bases in the ssNA hybridization region, a plurality of scaffold polynucleotide species would include 4⁷ unique ssNA hybridization region sequences). Accordingly, each scaffold adapter having a unique scaffold polynucleotide (i.e., comprising a unique ssNA hybridization region sequence) may be referred to as a scaffold adapter species and a collection of multiple scaffold adapter species may be referred to as a plurality of scaffold adapter species. A species of scaffold polynucleotide generally contains a feature that is unique with respect to other scaffold polynucleotide species. For example, a scaffold polynucleotide species may contain a unique sequence feature. A unique sequence feature may include a unique sequence length, a unique nucleotide sequence (e.g., a unique random sequence, a unique targeted sequence), or a combination of a unique sequence length and nucleotide sequence.

A scaffold polynucleotide may comprise one or more additional subcomponents including an index polynucleotide, a unique molecular identifier (UMI), primer binding site (e.g., P5 primer binding site, P7 primer binding site), flow cell binding region, and the like, or complementary polynucleotides thereof. A scaffold polynucleotide may comprise a primer binding site (or a polynucleotide complementary to a primer binding site). Scaffold polynucleotides comprising a P5 primer binding site (or complement thereof) may be referred to as P5 scaffolds or P5 scaffold polynucleotides. Scaffold polynucleotides comprising a P7 primer binding site (or complement thereof) may be referred to as P7 scaffolds or P7 scaffold polynucleotides.

An oligonucleotide can be a further single-stranded component of a scaffold adapter. An oligonucleotide herein generally refers to a single-stranded multimer of nucleotides from 5 to 500 nucleotides, e.g., 5 to 100 nucleotides. Oligonucleotides may be synthetic or may be made enzymatically, and, in some implementations, are 5 to 50 nucleotides in length. Oligonucleotides may contain ribonucleotide monomers (i.e., may be oligoribonucleotides or "RNA oligonucleotides"), deoxyribonucleotide monomers (i.e., may be oligodeoxyribonucleotides or "DNA oligonucleotides"), or a combination thereof. Oligonucleotides may be 10 to 20, 20 to 30, 30 to 40, 40 to 50, 50 to 60, 60 to 70, 70 to 80, 80 to 100, 100 to 150 or 150 to 200, or up to 500 nucleotides in length, for example. The terms oligonucleotide and polynucleotide may be used interchangeably.

An oligonucleotide component of a scaffold adapter generally comprises a nucleic acid sequence that is complementary or substantially complementary to an oligonucleotide hybridization region of a scaffold polynucleotide. An oligonucleotide component of a scaffold adapter may include one or more subcomponents useful for one or more downstream applications such as, for example, PCR amplification of the ssNA fragment or derivative thereof, sequencing of the ssNA or derivative thereof, and the like. In some implementations, a subcomponent of an oligonucleotide is a sequencing adapter. Sequencing adapter generally refers to one or more nucleic acid domains that include at least a portion of a nucleotide sequence (or complement thereof) utilized by a sequencing platform of interest, such as a sequencing platform provided by Illumina^{®} (e.g., the HiSeq^{™}, MiSeq^{™} and/or Genome Analyzer^{™} sequencing systems); Oxford Nanopore^{™} Technologies (e.g., the MinION^{™} sequencing system), Ion Torrent^{™} (e.g., the Ion PGM^{™} and/or Ion Proton^{™} sequencing systems); Pacific Biosciences (e.g., a Sequel or PACBIO RS II sequencing system); Life Technologies^{™} (e.g., a SOLiD^{™} sequencing system); Roche (e.g., the 454 GS FLX+ and/or GS Junior sequencing systems); Genapsys; BGI; or any sequencing platform of interest.

In some implementations, an oligonucleotide component of a scaffold adapter is, or comprises, a nucleic acid domain selected from: a domain (e.g., a "capture site" or "capture sequence") that specifically binds to a surface-attached sequencing platform oligonucleotide (e.g., a P5 or P7 oligonucleotide attached to the surface of a flow cell in an Illumina^{®} sequencing system); a sequencing primer binding domain (e.g., a domain to which the Read 1 or Read 2 primers of the Illumina^{®} platform may bind); a unique identifier or index (e.g., a barcode or other domain that uniquely identifies the sample source of the ssNA being sequenced to enable sample multiplexing by marking every molecule from a given sample with a specific barcode or "tag"); a barcode sequencing primer binding domain (a domain to which a primer used for sequencing a barcode binds); a molecular identification domain or unique molecular identifier (UMI) (e.g., a molecular index tag, such as a randomized tag of 4, 6, or other number of nucleotides) for uniquely marking molecules of interest, e.g., to determine expression levels based on the number of instances a unique tag is sequenced; a complement of any such domains; or any combination thereof. In some implementations, a barcode domain (e.g., sample index tag) and a molecular identification domain (e.g., a molecular index tag; UMI) may be included in the same nucleic acid. Sequencing platform oligonucleotides, sequencing primers, and their corresponding binding domains can be designed to be compatible with a variety of available sequencing platforms and technologies, including but not limited to those discussed herein.

When an oligonucleotide component of a scaffold adapter includes one or a portion of a sequencing adapter, one or more additional sequencing adapters and/or a remaining portion of the sequencing adapter may be added using a variety of approaches. For example, additional and/or remaining portions of sequencing adapters may be added by any one of ligation, reverse transcription, PCR amplification, and the like. In the case of PCR, an amplification primer pair may be employed that includes a first amplification primer that includes a 3' hybridization region (e.g., for hybridizing to an adapter region of the oligonucleotide) and a 5' region including an additional and/or remaining portion of a sequencing adapter, and a second amplification primer that includes a 3' hybridization region (e.g., for hybridizing to an adapter region of a second oligonucleotide added to the opposite end of an ssNA molecule) and optionally a 5' region including an additional and/or remaining portion of a sequencing adapter.

An oligonucleotide component of a scaffold adapter may comprise one or more additional subcomponents including an index polynucleotide, a unique molecular identifier (UMI), primer binding site (e.g., P5 primer binding site, P7 primer binding site), flow cell binding region or sequencing adapter, and the like, or complementary polynucleotides thereof. An oligonucleotide may comprise a primer binding site (or a polynucleotide complementary to a primer binding site). Oligonucleotides comprising a P5 primer binding site (or complement thereof) may be referred to as P5 oligos or P5 oligonucleotides. Oligonucleotides comprising a P7 primer binding site (or complement thereof) may be referred to as P7 oligos or P7 oligonucleotides.

The scaffold polynucleotide may be hybridized to the oligonucleotide, forming a duplex in the scaffold adapter. Accordingly, a scaffold adapter may be referred to as a scaffold duplex, a duplex adapter, a duplex oligonucleotide, or a duplex polynucleotide. Each scaffold duplex having a unique scaffold polynucleotide (i.e., comprising a unique ssNA hybridization region sequence) may be referred to as a scaffold duplex species and a collection of multiple scaffold duplex species may be referred to as a plurality of scaffold duplex species. In some implementations, the scaffold polynucleotide and the oligonucleotide are on separate DNA strands. In some implementations, the scaffold polynucleotide and the oligonucleotide are on a single DNA strand (e.g., a single DNA strand capable of forming a hairpin structure).

Scaffold adapters can comprise DNA, RNA, or a combination thereof. Scaffold adapters can comprise a DNA scaffold polynucleotide and a DNA oligonucleotide, a DNA scaffold polynucleotide and an RNA oligonucleotide, an RNA scaffold polynucleotide and a DNA oligonucleotide, or an RNA scaffold polynucleotide and an RNA oligonucleotide. Example scaffold adapter compositions and designs are shown in FIG. 54 (vertical line shading denotes RNA, slanted line shading denotes DNA). FIG. 54, top, shows a scaffold adapter comprising a DNA scaffold polynucleotide and a DNA oligonucleotide, with an RNA sample nucleic acid; example ligases for use with such an adapter include T4 RNA ligase 2 and T4 DNA ligase. FIG. 54, middle, shows a scaffold adapter comprising a DNA scaffold polynucleotide and an RNA oligonucleotide, with an RNA sample nucleic acid; example ligases for use with such an adapter include T4 RNA ligase 1. FIG. 54, bottom, shows a scaffold adapter comprising an RNA scaffold polynucleotide and an RNA oligonucleotide, with an RNA sample nucleic acid; example ligases for use with such an adapter include T4 RNA ligase 1. In some instances, the adapter nucleotide composition is selected to provide homogeneity between sample nucleic acids and scaffold adapter nucleic acids (e.g., such that at least the oligonucleotide is homogenous to the sample nucleic acids). In some instances, the adapter nucleotide composition is selected to provide homogeneity between the oligonucleotide and the sample nucleic acids and heterogeneity between the scaffold polynucleotide and the sample nucleic acids.

### Combining scaffold adapters, or components thereof, and ssNA

A method herein may comprise combining one or more scaffold adapters, or components thereof, with a composition comprising single-stranded nucleic acid (ssNA) to form one or more complexes. The scaffold polynucleotide is designed for simultaneous hybridization to an ssNA fragment and an oligonucleotide component such that, upon complex formation, an end of the oligonucleotide component is adjacent to an end of the terminal region of the ssNA fragment. Typically, upon complex formation, a 5' end of the oligonucleotide component is adjacent to a 3' end of the terminal region of the ssNA, or a 5' end of the oligonucleotide component is adjacent to a 3' end of the terminal region of the ssNA. Upon complex formation in instances where a scaffold adapter is attached to both ends of an ssNA fragment, a 5' end of one oligonucleotide component is adjacent to a 3' end of one terminal region of the ssNA, and a 5' end of a second oligonucleotide component is adjacent to a 3' end of a second terminal region of the ssNA.

In some implementations, a method includes forming complexes by combining an ssNA composition, an oligonucleotide, and a plurality of heterogeneous scaffold polynucleotides having various random ssNA hybridization regions capable of acting as scaffolds for a heterogeneous population of ssNA having terminal regions of undetermined sequence.

In some implementations, an ssNA hybridization region includes a known sequence designed to hybridize to an ssNA terminal region of known sequence. In some implementations, two or more heterogeneous scaffold polynucleotides having different ssNA hybridization regions of known sequence are designed to hybridize to respective ssNA terminal regions of known sequence. Implementations in which the ssNA hybridization regions have a known sequence may be useful, for example, for producing a nucleic acid library from a subset of ssNAs having terminal regions of known sequence. Accordingly, in certain implementations, a method herein comprises forming complexes by combining an ssNA composition, an oligonucleotide, and one or more heterogeneous scaffold polynucleotides having one or more different ssNA hybridization regions of known sequence capable of acting as scaffolds for one or more ssNAs having one or more terminal regions of known sequence.

An ssNA fragment, an oligonucleotide, and scaffold polynucleotide may be combined in various ways. In some configurations, the combining includes combining 1) a complex comprising the scaffold polynucleotide hybridized to the oligonucleotide component via the oligonucleotide hybridization region, and 2) the ssNA fragment. In another configuration, the combining includes combining 1) a complex comprising the scaffold polynucleotide hybridized to the ssNA fragment via the ssNA hybridization region, and 2) the oligonucleotide component. In another configuration, the combining includes combining 1) the ssNA fragment, 2) the oligonucleotide, and 3) the scaffold polynucleotide, where none of the three components are pre-complexed with, or hybridized to, another component prior to the combining.

The combining may be carried out under hybridization conditions such that complexes form including a scaffold polynucleotide hybridized to a terminal region of an ssNA fragment via the ssNA hybridization region, and the scaffold polynucleotide hybridized to an oligonucleotide component via the oligonucleotide hybridization region. Whether specific hybridization occurs may be determined by factors such as the degree of complementarity between the hybridizing regions of the scaffold polynucleotide, the terminal region of the ssNA fragment, and the oligonucleotide component, as well as the length thereof, salt concentration, and the temperature at which the hybridization occurs, which may be informed by the melting temperatures (Tm) of the relevant regions.

Complexes may be formed such that an end of an oligonucleotide component is adjacent to an end of a terminal region of an ssNA fragment. Adjacent to refers the terminal nucleotide at the end of the oligonucleotide and the terminal nucleotide end of the terminal region of the ssNA fragment are sufficiently proximal to each other that the terminal nucleotides may be covalently linked, for example, by chemical ligation, enzymatic ligation, or the like. In some implementations, the ends are adjacent to each other by virtue of the terminal nucleotide at the end of the oligonucleotide and the terminal nucleotide end of the terminal region of the ssNA being hybridized to adjacent nucleotides of the scaffold polynucleotide. The scaffold polynucleotide may be designed to ensure that an end of the oligonucleotide is adjacent to an end of the terminal region of the ssNA fragment.

A scaffold polynucleotide may be designed with one or more uracil bases in place of thymine. In some implementations, one of the strands in a scaffold adapter duplex may be degraded by generating multiple cut sites at uracil bases, for example by using a uracil-DNA glycosylase and an endonuclease.

Scaffold adapters, oligonucleotide components, and scaffold polynucleotides may be referred to herein as first scaffold adapters (or first scaffold duplexes), first oligonucleotide components (or first oligonucleotides), and first scaffold polynucleotides; or second scaffold adapters (or second scaffold duplexes), second oligonucleotide components (or second oligonucleotides), and second scaffold polynucleotides. The terms first and second generally refer to scaffold adapters, or components thereof, that hybridize to and/or are covalently linked to a first end and second end of an ssNA fragment terminus (i.e., a 5' end and a 3' end). The terms first end and second end do not always refer to a particular directionality of the ssNA fragment. Accordingly, a first end of an ssNA terminus may be a 5' end or a 3' end, and a second end of an ssNA terminus may be a 5' end or a 3' end. A first scaffold adapter, or component thereof, may refer to a P5 adapter, or component thereof, or a P7 adapter, or component thereof. A second scaffold adapter, or component thereof, may refer to a P5 adapter, or component thereof, or a P7 adapter, or component thereof.

In some instances, prior to combining scaffold adapters or components thereof with a nucleic acid sample comprising ssNA, the nucleic acid sample can be treated with a nuclease to remove unwanted nucleic acids. For example, a double-stranded specific nuclease (e.g., T7 nuclease) can be used to digest some or all double-stranded DNA, and scaffolding adapters can then be used to prepare a sequencing library of the remaining nucleic acids as disclosed herein. In an example, a double-stranded specific nuclease is used to digest double-stranded nucleic acids in a sample, leaving intact single-stranded nucleic acids such as those from single-stranded DNA viruses, single-stranded RNA viruses, and single-stranded DNA (e.g., damaged DNA) while digesting double-stranded DNA from a host organism and/or bacteria.

### Combining scaffold adapters, or components thereof, and ssRNA or sscDNA

A method herein may comprise combining one or more scaffold adapters, or components thereof, with a composition comprising single-stranded ribonucleic acid (ssRNA) or single-stranded complementary deoxyribonucleic acid (sscDNA) to form one or more complexes. The scaffold polynucleotide is designed for simultaneous hybridization to an ssRNA or sscDNA fragment and an oligonucleotide component such that, upon complex formation, an end of the oligonucleotide component is adjacent to an end of the terminal region of the ssRNA or sscDNA fragment, as described above for ssNA.

In some implementations, a nucleic acid composition comprises sscDNA. In some implementations, a method comprises prior to the combining, generating sscDNA from single-stranded ribonucleic acid (ssRNA). Typically, when a nucleic acid composition comprises sscDNA, a method herein uses a first-strand cDNA and does not require generating a second-strand cDNA. Thus, in some implementations, a nucleic acid composition comprises first-strand sscDNA. In some implementations, a nucleic acid composition consists essentially of first-strand sscDNA. A nucleic acid composition "consisting essentially of" first-strand sscDNA generally includes first-strand sscDNA and no additional protein or nucleic acid components. A nucleic acid composition consisting essentially of first-strand sscDNA generally does not comprise second-strand sscDNA. Additionally, for example, a nucleic acid composition "consisting essentially of" first-strand sscDNA may exclude double-stranded cDNA (dscDNA) or may include a low percentage of dscDNA (e.g., less than 10% dscDNA, less than 5% dscDNA, less than 1% dscDNA). A nucleic acid composition "consisting essentially of" first-strand sscDNA may exclude proteins. For example, a nucleic acid composition "consisting essentially of" first-strand sscDNA may exclude single-stranded binding proteins (SSBs) or other proteins useful for stabilizing first-strand sscDNA. A nucleic acid composition "consisting essentially of" first-strand sscDNA may include chemical components typically present in nucleic acid compositions such as buffers, salts, alcohols, crowding agents (e.g., PEG), and the like; and may include residual components (e.g., nucleic acids (e.g., residual RNA), proteins, cell membrane components) from the nucleic acid source (e.g., sample), from nucleic acid extraction, or from cDNA synthesis. A nucleic acid composition "consisting essentially of" first-strand sscDNA may include first-strand sscDNA fragments having one or more phosphates (e.g., a terminal phosphate, a 5' terminal phosphate). A nucleic acid composition "consisting essentially of" first-strand sscDNA may include first-strand sscDNA fragments comprising one or more modified nucleotides.

In some implementations, generating the sscDNA comprises contacting the ssRNA with a primer and an agent comprising a reverse transcriptase activity, thereby generating a DNA-RNA duplex. In some implementations, generating the sscDNA may further comprise contacting the DNA-RNA duplex with an agent comprising an RNAse activity, thereby digesting the RNA and generating an sscDNA product. In some implementations, the agent comprising a reverse transcriptase activity also comprises an RNAse activity. Accordingly, in some implementations, reverse transcription and RNAse digestion are combined into one step. In some implementations, the agent comprising a reverse transcriptase activity and an RNAse activity is an M-MuLV reverse transcriptase (also referred to as M-MLV reverse transcriptase). The primer or primers may be any primer or primers suitable for use in conjunction with a reverse transcriptase. The primer or primers may be chosen from one or more of a random primer (e.g., random hexamer primer, random octamer primer), and a poly(T) primer. An sscDNA product may be purified by a suitable purification or wash method, e.g., a purification or wash method described herein.

In some implementations, a nucleic acid composition comprises ssRNA. In such implementations, scaffold adapters are directly hybridized to the ssRNA fragments, and the oligonucleotide component(s) is/are covalently linked to one or more ends of the ssRNA termini, thereby forming hybridization products containing one or more scaffold adapters and an ssRNA fragment. In some implementations, a method further comprises generating single-stranded ligation products from the hybridization products (e.g., by denaturing the hybridization products). In such implementations, single-stranded ligation products comprise an ssRNA fragment covalently linked to one or more oligonucleotide components. In some implementations, a method further comprises contacting the single-stranded ligation products with a primer and an agent comprising a reverse transcriptase activity, thereby generating a DNA-RNA duplex. In some implementations, a method further comprises contacting the DNA-RNA duplex with an agent comprising an RNAse activity, thereby digesting the RNA and generating a single-stranded cDNA (sscDNA) product. In some implementations, the agent comprising a reverse transcriptase activity also comprises an RNAse activity. Accordingly, in some implementations, reverse transcription and RNAse digestion are combined into one step. In some implementations, the agent comprising a reverse transcriptase activity and an RNAse activity is an M-MuLV reverse transcriptase (also referred to as M-MLV reverse transcriptase). The primer may be any primer suitable for use in conjunction with a reverse transcriptase. In some implementations, the primer comprises a nucleotide sequence complementary to a sequence in an oligonucleotide component (i.e., an oligonucleotide component covalently linked to an ssRNA fragment). An sscDNA product may be purified by a suitable purification or wash method, e.g., a purification or wash method described herein.

In some implementations, an sscDNA product is amplified. An sscDNA product may be amplified by a suitable amplification method, e.g., an amplification method described herein. In some implementations, amplifying an sscDNA product may be combined (e.g., combined in a single step, reaction, vessel, and/or volume) with generating a DNA-RNA duplex and/or generating an sscDNA product. Accordingly, reagents for generating a DNA-RNA duplex (e.g., one or more agents comprising a reverse transcriptase activity), reagents for generating an sscDNA product (e.g., one or more agents comprising an RNAse activity), and reagents for amplifying an sscDNA product (e.g., primers, an agent comprising a polymerase activity), may be combined for use in a single step, reaction, vessel, and/or volume. In some implementations, reagents for amplifying an sscDNA product comprise amplification primers that hybridize to a component (e.g., first oligonucleotide) of the scaffold adapters described herein. The amplification primers may be any primer suitable for use in conjunction with a polymerase. In some implementations, each primer comprises a nucleotide sequence complementary to a sequence in an sscDNA product corresponding to an oligonucleotide component (i.e., an oligonucleotide component covalently linked to an ssRNA fragment). An amplified sscDNA product may be purified by a suitable purification or wash method, e.g., a purification or wash method described herein.

In some implementations, a method herein comprises prior to combining the ssRNA with scaffold adapters, or components thereof, or prior to generating the sscDNA, fragmenting the ssRNA, thereby generating ssRNA fragments. Any suitable fragmentation method may be used, such as, for example, a fragmentation method described herein. In some implementations, a method herein comprises prior to combining the ssRNA with scaffold adapters, or components thereof, or prior to generating the sscDNA, depleting ribosomal RNA (rRNA) and/or enriching messenger RNA (mRNA). Any suitable rRNA depletion method and/or mRNA enrichment method may be used, such as, for example, an rRNA depletion method and/or mRNA enrichment method described herein.

### Hybridization and ligation

Nucleic acid fragments (e.g., ssNA fragments) may be combined with scaffold adapters, or components thereof, thereby generating combined products. Combining ssNA fragments with scaffold adapters, or components thereof, may comprise hybridization and/or ligation (e.g., ligation of hybridization products). A combined product may include an ssNA fragment connected to (e.g., hybridized to and/or ligated to) a scaffold adapter, or component thereof, at one or both ends of the ssNA fragment. A combined product may include an ssNA fragment hybridized to a scaffold adapter, or component thereof, at one or both ends of the ssNA fragment, which may be referred to as a hybridization product. A combined product may include an ssNA fragment ligated to a scaffold adapter, or component thereof, at one or both ends of the ssNA fragment, which may be referred to as a ligation product. In some implementations, products from a cleavage step (i.e., cleaved products) may be combined with scaffold adapters, or components thereof, thereby generating combined products. Certain methods herein comprise generating sets of combined products (e.g., a first set of combined products and a second set of combined products). In some implementations, a first set of combined products includes ssNAs connected to (e.g., hybridized to and/or ligated to) scaffold adapters, or components thereof, from a first set of scaffold adapters, or components thereof. In some implementations, a second set of combined products includes the first set of combined products connected to (e.g., hybridized to and/or ligated to) scaffold adapters, or components thereof, from a second set of scaffold adapters, or components thereof.

ssNAs may be combined with scaffold adapters, or components thereof, under hybridization conditions, thereby generating hybridization products. In some implementations, the scaffold adapters are provided as pre-hybridized products and the hybridization step includes hybridizing the scaffold adapters to the ssNA. In some implementations, the scaffold adapter components (i.e., oligonucleotides and scaffold polynucleotides) are provided as individual components and the hybridization step includes hybridizing the scaffold adapter components 1) to each other and 2) to the ssNA. In some implementations, the scaffold adapter components (i.e., oligonucleotides and scaffold polynucleotides) are provided sequentially as individual components and the hybridization steps includes 1) hybridizing the scaffold polynucleotides to the ssNA, and then 2) hybridizing the oligonucleotides to the oligonucleotide hybridization region of the scaffold polynucleotides. The conditions during the combining step are those conditions in which scaffold adapters, or components thereof (e.g., single-stranded scaffold regions), specifically hybridize to ssNAs having a terminal region or terminal regions that are complementary in sequence with respect to the single-stranded scaffold regions. The conditions during the combining step also may include those conditions in which components of the scaffold adapters (e.g., oligonucleotides and oligonucleotide hybridization regions within the scaffold polynucleotides), specifically hybridize, or remain hybridized, to each other.

Specific hybridization may be affected or influenced by factors such as the degree of complementarity between the single-stranded scaffold regions and the ssNA terminal region(s), or between the oligonucleotides and oligonucleotide hybridization regions, the length thereof, and the temperature at which the hybridization occurs, which may be informed by melting temperatures (Tm) of the single-stranded scaffold regions. Melting temperature generally refers to the temperature at which half of the single-stranded scaffold regions /ssNA terminal regions remain hybridized and half of the single-stranded scaffold regions /ssNA terminal regions dissociate into single strands. The Tm of a duplex may be experimentally determined or predicted using the following formula Tm = 81.5 + 16.6(log₁₀[Na+]) + 0.41 (fraction G+C) - (60/N), where N is the chain length and [Na+] is less than 1 M. Additional models that depend on various parameters also may be used to predict Tm of relevant regions depending on various hybridization conditions. Approaches for achieving specific nucleic acid hybridization are described, e.g., Tijssen, Laboratory Techniques in Biochemistry and Molecular Biology-Hybridization with Nucleic Acid Probes, part I, chapter 2, "Overview of principles of hybridization and the strategy of nucleic acid probe assays," Elsevier (1993).

In some implementations, a method herein comprises exposing hybridization products to conditions under which an end of an ssNA is joined to an end of a scaffold adapter to which it is hybridized. In particular, a method herein may comprise exposing hybridization products to conditions under which an end of an ssNA is joined to an end of an oligonucleotide component of a scaffold adapter to which it is hybridized. Joining may be achieved by any suitable approach that permits covalent attachment of ssNA to the scaffold adapter and/or oligonucleotide component of a scaffold adapter to which it is hybridized. When one end of an ssNA is joined to an end of a scaffold adapter and/or oligonucleotide component of a scaffold adapter to which it is hybridized, typically one of two attachment events is conducted: 1) the 3' end of the ssNA to the 5' end of the oligonucleotide component of the scaffold adapter, or 2) the 5' end of the ssNA to the 3' end of the oligonucleotide component of the scaffold adapter. When both ends of an ssNA are each joined to an end of a scaffold adapter and/or oligonucleotide component of a scaffold adapter to which it is hybridized, typically two attachment events are conducted: 1) the 3' end of the ssNA to the 5' end of the oligonucleotide component of a first scaffold adapter, and 2) the 5' end of the ssNA to the 3' end of the oligonucleotide component of a second scaffold adapter.

In some implementations, a method herein comprises contacting hybridization products with an agent comprising a ligase activity under conditions in which an end of an ssNA is covalently linked to an end of a scaffold adapter and/or oligonucleotide component of a scaffold adapter to which the target nucleic acid (ssNA) is hybridized. Ligase activity may include, for example, blunt-end ligase activity, nick-sealing ligase activity, sticky end ligase activity, circularization ligase activity, cohesive end ligase activity, DNA ligase activity, RNA ligase activity, single-stranded ligase activity, and double-stranded ligase activity. Ligase activity may include ligating a 5' phosphorylated end of one polynucleotide to a 3' OH end of another polynucleotide (5'P to 3'OH). Ligase activity may include ligating a 3' phosphorylated end of one polynucleotide to a 5' OH end of another polynucleotide (3'P to 5'OH). Ligase activity may include ligating a 5' end of an ssNA to a 3' end of a scaffold adapter and/or oligonucleotide component of a scaffold adapter hybridized thereto in a ligation reaction. Ligase activity may include ligating a 3' end of an ssNA to a 5' end of a scaffold adapter and/or oligonucleotide component of a scaffold adapter hybridized thereto in a ligation reaction. Suitable reagents (e.g., ligases) and kits for performing ligation reactions are known and available. For example, Instant Sticky-end Ligase Master Mix available from New England Biolabs (Ipswich, MA) may be used. Ligases that may be used include but are not limited to, for example, T3 ligase, T4 DNA ligase (e.g., at low or high concentration), T7 DNA Ligase, E. coli DNA Ligase, Electro Ligase^{®}, RNA ligases, T4 RNA ligase 2, SplintR^{®} Ligase, RtcB ligase, Taq ligase, and the like and combinations thereof. When needed, a phosphate group may be added at the 5' end of the oligonucleotide component or ssNA fragment using a suitable kinase, for example, such as T4 polynucleotide kinase (PNK). Such kinases and guidance for using such kinases to phosphorylate 5' ends are available, for example, from New England BioLabs, Inc. (Ipswich, MA).

In some implementations, a method comprises covalently linking the adjacent ends of an oligonucleotide component and an ssNA terminal region, thereby generating covalently linked hybridization products. In some implementations, the covalently linking comprises contacting the hybridization products (e.g., ssNA fragments hybridized to at least one scaffold adapter herein) with an agent comprising a ligase activity under conditions in which the end of an ssNA terminal region is covalently linked to an end of the oligonucleotide component. In some implementations, a method comprises covalently linking the adjacent ends of a first oligonucleotide component and a first ssNA terminal region, and covalently linking the adjacent ends of a second oligonucleotide component and a second ssNA terminal region, thereby generating covalently linked hybridization products. In some implementations, the covalently linking comprises contacting hybridization products (e.g., ssNA fragments each hybridized two scaffold adapters herein) with an agent comprising a ligase activity under conditions in which an end of a first ssNA terminal region is covalently linked to an end of a first oligonucleotide component and an end of a second ssNA terminal region is covalently linked to an end of a second oligonucleotide component. In some implementations, the agent comprising a ligase activity is a T4 DNA ligase. In some implementations, the T4 DNA ligase is used at an amount between about 1 unit/µl to about 50 units/µl. In some implementations, the T4 DNA ligase is used at an amount between about 5 unit/µl to about 30 units/µl. In some implementations, the T4 DNA ligase is used at an amount between about 5 unit/µl to about 15 units/µl. In some implementations, the T4 DNA ligase is used at about 10 units/µl. In some implementations, the T4 DNA ligase is used at an amount less than 25 units/µl. In some implementations, the T4 DNA ligase is used at an amount less than 20 units/µl. In some implementations, the T4 DNA ligase is used at an amount less than 15 units/µl. In some implementations, the T4 DNA ligase is used at an amount less than 10 units/µl.

In some implementations, hybridization products are contacted with a first agent comprising a first ligase activity and a second agent comprising a second ligase activity different than the first ligase activity. For example, the first ligase activity and the second ligase activity independently may be chosen from blunt-end ligase activity, nick-sealing ligase activity, sticky end ligase activity, circularization ligase activity, and cohesive end ligase activity, double-stranded ligase activity, single-stranded ligase activity, 5'P to 3'OH ligase activity, and 3'P to 5'OH ligase activity.

In some implementations, a method herein comprises joining ssNAs to scaffold adapters and/or oligonucleotide components of scaffold adapters via biocompatible attachments. Methods may include, for example, click chemistry or tagging, which include biocompatible reactions useful for joining biomolecules. In some implementations, an end of each of the oligonucleotide components comprises a first chemically reactive moiety and an end of each of the ssNAs includes a second chemically reactive moiety. In such implementations, the first chemically reactive moiety typically is capable of reacting with the second chemically reactive moiety and forming a covalent bond between an oligonucleotide component of a scaffold adapter and an ssNA to which the scaffold adapter is hybridized. In some implementations, a method herein includes contacting ssNA with one or more chemical agents under conditions in which the second chemically reactive moiety is incorporated at an end of each of the ssNA fragments. In some implementations, a method herein includes exposing hybridization products to conditions in which the first chemically reactive moiety reacts with the second chemically reactive moiety forming a covalent bond between an oligonucleotide component and an ssNA to which the scaffold adapter is hybridized. In some implementations, the first chemically reactive moiety is capable of reacting with the second chemically reactive moiety to form a 1 ,2,3-triazole between the oligonucleotide component and the ssNA to which the scaffold adapter is hybridized. In some implementations, the first chemically reactive moiety is capable of reacting with the second chemically reactive moiety under conditions comprising copper. The first and second chemically reactive moieties may include any suitable pairings. For example, the first chemically reactive moiety may be chosen from an azide-containing moiety and 5-octadiynyl deoxyuracil, and the second chemically reactive moiety may be independently chosen from an azide-containing moiety, hexynyl and 5-octadiynyl deoxyuracil. In some implementations, the azide-containing moiety is N-hydroxysuccinimide (NHS) ester-azide.

Covalently linking the adjacent ends of an oligonucleotide and an ssNA fragment produces a covalently linked product, which may be referred to a ligation product. A covalently linked product that includes an ssNA fragment covalently linked to an oligonucleotide component, which remain hybridized to a scaffold polynucleotide, may be referred to as a covalently linked hybridization product. A covalently linked hybridization product may be denatured (e.g., heat-denatured) to separate the ssNA fragment covalently linked to an oligonucleotide component from the scaffold polynucleotide. A covalently linked product that includes an ssNA fragment covalently linked to an oligonucleotide component, which is no longer hybridized to a scaffold polynucleotide (e.g., after denaturing), may be referred to as a single-stranded ligation product. In some instances, portions of a scaffold polynucleotide can be cleaved and/or degraded, for example by using uracil-DNA glycosylase and an endonuclease at one or more uracil bases in the scaffold polynucleotide.

A covalently linked hybridization product and/or single-stranded ligation product may be purified prior to use as input in a downstream application of interest (e.g., amplification; sequencing). For example, covalently linked hybridization products and/or single-stranded ligation products may be purified from certain components present during the combining, hybridization, and/or covalently linking (ligation) steps (e.g., by solid phase reversible immobilization (SPRI), column purification, and/or the like).

In some implementations, when a method herein include combining an ssNA composition with scaffold adapters herein, or components thereof, and covalently linking the adjacent ends of an oligonucleotide component and an ssNA fragment, the total duration of the combining and covalently linking may be 4 hours or less, 3 hours or less, 2 hours or less, or 1 hour or less. In some implementations, the total duration of the combining and covalently linking is less than 1 hour.

In some implementations, a method herein is performed in a single vessel, a single chamber, and/or a single volume (i.e., contiguous volume), including but not limited to on a microfluidic device. In some implementations, combining an ssNA composition with scaffold adapters herein, or components thereof, and covalently linking the adjacent ends of an oligonucleotide component and an ssNA fragment are performed in a single vessel, a single chamber, and/or a single volume (i.e., contiguous volume), including but not limited to on a microfluidic device. In some implementations, a method herein is performed in a collection of wells, droplets, emulsion, partitions, or other reaction volumes, including but not limited to on a microfluidic device. In some implementations, combining an ssNA composition with scaffold adapters herein, or components thereof, and covalently linking the adjacent ends of an oligonucleotide component and an ssNA fragment are performed in a collection of wells, droplets, emulsion, partitions, or other reaction volumes, including but not limited to on a microfluidic device. In some instances, the collection of reaction volumes are prepared such that a majority or all of the reaction volumes comprise at most one ssNA. In some instances, the collection of reaction volumes are prepared such that a majority or all of the reaction volumes comprise at most 2, 3, 4, 5, 6, 7, 8, 9, 10, 20, 30, 40, 50, 60, 70, 80, 90, 100, 1000, 2000, 3000, 4000, 5000, 6000, 7000, 8000, 9000, 10000, 20000, 30000, 40000, 50000, 60000, 70000, 80000, 90000, 100000, or more ssNA. Partitioning one or a limited number of ssNA into reaction volumes can provide favorable reaction kinetics, such as increasing the library conversion of rare species of sample nucleic acids.

### Adapter dimers

In some implementations, a method herein comprises one or more modifications and/or additional steps for preventing, reducing, or eliminating adapter dimers. Adapter dimers may unintentionally form during a method described herein. Adapter dimers generally refer to two or more scaffold adapters, components thereof, or parts thereof hybridizing, or hybridizing and ligating, to each other. Examples of certain adapter dimer configurations are provided in Fig. 20.

In certain implementations, a scaffold adapter, or a component thereof, is modified to prevent adapter dimer formation. Examples of modifications to a scaffold adapter include modified nucleotides capable of blocking covalent linkage of the scaffold adapter, oligonucleotide component, or scaffold polynucleotide, to another oligonucleotide, polynucleotide, or nucleic acid molecule (e.g., another scaffold adapter, oligonucleotide component, and/or scaffold polynucleotide). Examples of modified nucleotides are described below. Other/additional modifications to a scaffold adapter include configurations such as a Y-configuration or a hairpin configuration, which are described in further detail below. In some implementations, scaffold adapter, oligonucleotide component, and/or scaffold polynucleotide may comprise a phosphorothioate backbone modification (e.g., a phosphorothioate bond between the last two nucleotides on a strand).

In some implementations, a method includes a dephosphorylation step to prevent or reduce adapter dimer formation. In some implementations, a method includes prior to combining scaffold adapters, or components thereof, with ssNA, contacting scaffold adapters, oligonucleotide components, and/or scaffold polynucleotides with an agent comprising a phosphatase activity under conditions in which the scaffold adapters, oligonucleotide components, and/or scaffold polynucleotides is/are dephosphorylated, thereby generating dephosphorylated scaffold adapters, dephosphorylated oligonucleotide components, and/or dephosphorylated scaffold polynucleotides.

In some implementations, a method includes one or more staged ligation approaches to prevent or reduce adapter dimer formation. In some implementations, a method includes staged ligation which comprises delaying addition of an agent comprising a phosphoryl transfer activity (e.g., until after hybridization products are formed) and/or delaying addition of a second scaffold adapter, or components thereof (see Fig. 9). For example, a method may comprise after forming hybridization products and prior to covalently linking the oligonucleotide component(s) to the ssNA terminal region(s), contacting the oligonucleotide component(s) with an agent comprising a phosphoryl transfer activity under conditions in which a 5' phosphate is added to a 5' end of an oligonucleotide component. In another example, a method may comprise combining a first set of scaffold adapters with ssNA. A first set of scaffold adapters may include an oligonucleotide component having a 3' OH. The first set of scaffold adapters are hybridized to the ssNA, and the 3' OH of the oligonucleotide component is covalently linked to the 5' end (e.g., 5' phosphorylated end) of an ssNA terminal region. The products of such first round of hybridizing and covalently linking may be referred to as intermediate covalently linked hybridization products. The intermediate covalently linked hybridization products are then combined with a second set of scaffold adapters. A second set of scaffold adapters may include an oligonucleotide component having a 5' end that may be phosphorylated as described herein. The second set of scaffold adapters are hybridized to the intermediate covalently linked hybridization products, and the 5' phosphorylated end of the oligonucleotide component is covalently linked to the 3' end of the ssNA terminal region.

In some implementations, a method includes staged ligation which comprises use of a scaffold adapter, or component thereof, having an adenylation modification (see Fig. 10). For example, a first set of scaffold adapters may comprise an adenylation modification at the 5' end of the oligonucleotide component (5' App). The first set of scaffold adapters are hybridized to the ssNA, and the 5' App of the oligonucleotide component is covalently linked to the 3' end of an ssNA terminal region. The covalent linking may occur in the absence of ATP. The products of such first round of hybridizing and covalently linking may be referred to as intermediate covalently linked hybridization products. The intermediate covalently linked hybridization products are then combined with a second set of scaffold adapters. A second set of scaffold adapters may include an oligonucleotide component having a 3' OH end. The second set of scaffold adapters are hybridized to the intermediate covalently linked hybridization products, and the 3' OH end of the oligonucleotide component is covalently linked to the 5' end (e.g., 5' phosphorylated end) of the ssNA terminal region (with the addition of ATP). In one variation, the first set of scaffold adapters and the second set of scaffold adapters are combined with ssNA at the same time in the absence of ATP. Ligation of the first set of scaffold adapters may proceed in the absence of ATP, and ligation of the second set of scaffold adapters may proceed only until ATP is added.

In some implementations, a method includes staged ligation which comprises use of an oligonucleotide (i.e., a single stranded oligonucleotide) having a 3' phosphorylated end (see Fig. 11). An oligonucleotide having a 3' phosphorylated end may comprise any of the subcomponents described herein for oligonucleotide components of scaffold adapters (e.g., a primer binding site, an index, a UMI, a flow cell adapter, and the like). An oligonucleotide having a 3' phosphorylated end generally is single-stranded and is not hybridized to a scaffold polynucleotide. In one example, a method may comprise prior to combining scaffold adapters, or components thereof, with ssNA, combining the ssNA with an oligonucleotide comprising a phosphate at the 3' end and covalently linking the 3' phosphorylated end of the oligonucleotide to the 5' end (e.g., 5' non-phosphorylated end) of an ssNA terminal region. In some implementations, prior to the covalently linking of the oligonucleotide to the ssNA, the ssNA is contacted with an agent comprising a phosphatase activity under conditions in which the ssNA is dephosphorylated, thereby generating dephosphorylated ssNA. In some implementations, covalently linking the oligonucleotide to the ssNA comprises contacting the ssNA and the oligonucleotide with an agent comprising a single-stranded ligase activity under conditions in which the 5' end of the ssNA is covalently linked to the 3' end of the oligonucleotide. In some implementations, the agent comprising a ligase activity is an RtcB ligase. The products of such covalently linking may be referred to as intermediate covalently linked products. The intermediate covalently linked products are then combined with a set of scaffold adapters. A set of scaffold adapters may include an oligonucleotide component having a 5' phosphorylated end. The set of scaffold adapters are hybridized to the intermediate covalently linked products, and the 5' phosphorylated end of the oligonucleotide component is covalently linked to the 3' end of the ssNA terminal region.

In some implementations, a method includes use of an oligonucleotide capable of hybridizing to an oligonucleotide dimer product (see Fig. 20) to reduce or eliminate adapter dimers. An oligonucleotide dimer product may be a component of a scaffold adapter dimer, and may contain an oligonucleotide component from a first scaffold adapter covalently linked to an oligonucleotide component from a second scaffold adapter. A method herein may include a denaturing step which can release the oligonucleotide dimer product from the scaffold adapter dimer. The oligonucleotide dimer product may hybridize to an oligonucleotide having a sequence complementary to the oligonucleotide dimer product, or part thereof, thereby forming an oligonucleotide dimer hybridization product. In some implementations, the oligonucleotide dimer hybridization product comprises a cleavage site. In some implementations, the cleavage site is a restriction enzyme recognition site. In some implementations, a method herein further comprises contacting the oligonucleotide dimer hybridization product with a cleavage agent (e.g., a restriction enzyme, a rare-cutter restriction enzyme).

In some implementations, a method includes purifying or washing nucleic acid products at various stages of library preparation to reduce or eliminate adapter dimers. In some instances, purifying or washing nucleic acid products may reduce or eliminate adapter dimers. For example, covalently linked hybridization products (i.e., ssNA hybridized to scaffold adapters and covalently linked to oligonucleotide components), single-stranded ligation products (i.e., denatured covalently linked hybridization products; ssNA covalently linked to oligonucleotide components and no longer hybridized to scaffold polynucleotides), or amplification products thereof, may be purified or washed by any suitable purification or washing method. In some implementations, purifying or washing comprises use of solid phase reversible immobilization (SPRI). SPRI beads can be resuspended in a DNA binding buffer containing, for example, about 2.5 M to about 5 M NaCl, about 0.1 mM to about 1 M EDTA, about 10 mM Tris, about 0.01% to about 0.05% TWEEN-20, and between about 8% and about 38% PEG-8000. For example, 1 ml of SPRI bead suspension can be combined with 2.5 M NaCl, 10 mM Tris, 1 mM EDTA, 0.05% Tween-20 and 20% PEG-8000. In some implementations, SPRI includes serial SPRI (washes performed back to back) and or sequential SPRI (wash comprising sequential addition of SPRI beads and incubations). Serial SPRI may include a plurality of serial (back to back) washes, which may include 2, 3, 4, 5, 6, 7, 8, 9, 10 or more serial washes. Sequential SPRI may include a plurality of sequential addition of SPRI beads (with intervening incubations), which may include 2, 3, 4, 5, 6, 7, 8, 9, 10 or more sequential addition of SPRI beads. In some implementations, the amount of SPRI beads used in an SPRI purification may include an amount between 0.1x to 3x SPRI beads (x is ratio of beads to nucleic acid (e.g., bead volume to reaction volume)). For example, the amount of SPRI beads used in an SPRI purification may include about 0.1x, 0.2x, 0.3x, 0.4x, 0.5x, 0.6x, 0.7x, 0.8x, 0.9x, 1.0x, 1.1x, 1.2x, 1.3x, 1.4x, 1.5x, 1.6x, 1.7x, 1.8x, 1.9x, 2.0x, 2.1x, 2.2x, 2.3x, 2.4x, 2.5x, 2.6x, 2.7x, 2.8x, 2.9x, or 3.0x SPRI beads. In some implementations, the amount of SPRI beads used in an SPRI purification is 1.2x. In some implementations, the amount of SPRI beads used in an SPRI purification is 1.5x. In some implementations, purifying or washing comprises a column purification (e.g., column chromatography). In some implementations, purifying or washing does not comprise a column purification (e.g., column chromatography). In some implementations, covalently linked hybridization products, single-stranded ligation products, and/or amplification products thereof are not purified or washed.

An SPRI purification is typically performed in the presence of a buffer. Any suitable buffer may be used, e.g., Tris buffer, water that is of similar pH, and the like. SPRI purification beads may be added directly to a sample solution (e.g., a sample solution containing covalently linked hybridization products (ligation products), or amplified products thereof). In certain instances, buffer may be added to raise the volume of the reaction so additional beads may be added. In some implementations, an SPRI bead solution is made up of carboxylated magnetic beads added to PEG 8000 dissolved in water, NaCl, Tris, and EDTA. The amount of PEG typically determines the PEG percentage of the SPRI bead solution. For example, adding 9 g of PEG 8000 in a 50 ml SPRI bead solution may be referred to as "18% SPRI." In another example, adding 19 g of PEG 8000 in a 50 ml SPRI solution may be referred to as "38% SPRI." Generally, the higher proportion of PEG, the lower the size of DNA fragments retained.

In some implementations, a purification process comprises contacting covalently linked hybridization products (ligation products) with solid phase reversible immobilization (SPRI) beads and a buffer. In some implementations, some or all SPRI buffer is replaced with isopropanol. In some implementations, SPRI buffer comprises isopropanol. In some implementations, SPRI buffer is completely replaced with isopropanol. In some implementations, SPRI buffer comprises about 5% volume/volume (v/v) isopropanol to about 50% v/v isopropanol. In some implementations, SPRI buffer comprises about 10% v/v isopropanol to about 40% v/v isopropanol. For example, SPRI buffer may comprise about 10% v/v isopropanol, 15% v/v isopropanol, 20% v/v isopropanol, 25% v/v isopropanol, 30% v/v isopropanol, 35% v/v isopropanol, or 40% v/v isopropanol. In some implementations, SPRI buffer comprises about 20% v/v isopropanol.

In some implementations, a purifying or washing step may enrich for nucleic acid fragments, or amplification products thereof, having a particular length or range of lengths. In some implementations, an SPRI purification may enrich for nucleic acid fragments, or amplification products thereof, having a particular length or range of lengths. In some implementations, the amount of PEG 8000 in an SPRI bead solution used in an SPRI purification may affect the length or range of lengths of fragments that are enriched. For example, an SPRI purification at 1.5x v/v ratio may recover more fragments in the < 100 base range than an SPRI purification at 1.2x because the final concentration of PEG 8000 is higher in 1.5x than in 1.2x. In some implementations, a method herein comprises adjusting an SPRI ratio to enrich for a desired fragment length or range of lengths. In some implementations, a method herein comprises adjusting an amount of isopropanol in an SPRI purification to enrich for a desired fragment length or range of lengths. In some implementations, a method herein comprises adjusting an amount of isopropanol in an SPRI purification to enrich for a desired fragment length or range of lengths, while minimizing the amount of unwanted artifacts (e.g., adapter dimers). For example, a method herein may comprise adjusting an amount of isopropanol in an SPRI purification to enrich for a desired fragment length or range of lengths, where the amount of adapter dimers recovered is less than about 10% of the total nucleic acid recovered. In another example, a method herein may comprise adjusting an amount of isopropanol in an SPRI purification to enrich for a desired fragment length or range of lengths, where the amount of adapter dimers recovered is less than about 5% of the total nucleic acid recovered.

In some implementations, a method herein (e.g., combining ssNA with scaffold adapters or components thereof, hybridization, and covalently linking) may be performed in a suitable reaction volume and/or with a suitable amount of ssNA and/or suitable ratio of ssNA to scaffold adapters (or components thereof). A suitable reaction volume and/or a suitable amount of ssNA and/or a suitable ratio of ssNA to scaffold adapters (or components thereof) may include reaction volumes, amounts of ssNA, and/or ratios of ssNA and scaffold adapters that reduce or prevent adapter dimer formation. In some implementations, a suitable amount of ssNA may range from about 250 pg to about 5 ng of ssNA. For example, a suitable amount of ssNA may be about 250 pg, 500 pg, 750 pg, 1 ng, 1.5 ng, 2 ng, 2.5 ng, 3 ng, 3.5 ng, 4 ng, 4.5 ng, or 5 ng. In some implementations, a suitable amount of ssNA may be about 1 ng of ssNA. In some implementations, for a 25 µl final reaction volume, 1 ng ssNA may be combined with between about 1.0 to 2.0 picomoles of each scaffold adapter (i.e., about 1.0 to 2.0 picomoles of scaffold adapters (pool of scaffold adapters that contains a plurality of scaffold adapter species) that hybridize to the 5' end of ssNA terminal regions, and about 1.0 to 2.0 picomoles of scaffold adapters (pool of scaffold adapters that contains a plurality of scaffold adapter species) that hybridize to the 3' end of ssNA terminal regions). For example, for a 25 µl final reaction volume, 1 ng ssNA may be combined with about 1.0, 1.1, 1.2, 1.3, 1.4, 1.5, 1.6, 1.7, 1.8, 1.9, or 2.0 picomoles of each scaffold adapter. In some implementations, for a 25 µl final reaction volume, 1 ng ssNA is combined with about 1.6 picomoles of each scaffold adapter (i.e., about 1.6 picomoles of scaffold adapters that hybridize to the 5' end of ssNA terminal regions and about 1.6 picomoles of scaffold adapters that hybridize to the 3' end of ssNA terminal regions). For larger reaction volumes, amounts of ssNA and scaffold adapters may be scaled up so long as the relative amounts are preserved. For smaller reaction volumes, amounts of ssNA and scaffold adapters may be scaled down so long as the relative amounts are preserved. In some implementations, the scaffold adapters herein are combined with ssNA at a molar ratio between about 5:1 (scaffold adapters to ssNA) to about 50:1 (scaffold adapters to ssNA). For example, scaffold adapters may combined with ssNA at a molar ratio of about 5:1 (scaffold adapters to ssNA), about 10:1 (scaffold adapters to ssNA), about 15:1 (scaffold adapters to ssNA), about 20:1 (scaffold adapters to ssNA), about 25:1 (scaffold adapters to ssNA), about 30:1 (scaffold adapters to ssNA), about 35:1 (scaffold adapters to ssNA), about 40:1 (scaffold adapters to ssNA), about 45:1 (scaffold adapters to ssNA), or about 50:1 (scaffold adapters to ssNA). In some implementations, scaffold adapters are combined with ssNA at a molar ratio of about 15:1 (scaffold adapters to ssNA). In some implementations, scaffold adapters are combined with ssNA at a molar ratio of about 30:1 (scaffold adapters to ssNA).

In some implementations, a method herein comprises use of a crowding agent. A suitable amount of crowding agent may be used to reduce or prevent adapter dimer formation. Crowding agents may include, for example, ficoll 70, dextran 70, polyethylene glycol (PEG) 2000, and polyethylene glycol (PEG) 8000. In some implementations, a method herein comprises use of polyethylene glycol (PEG) 8000. PEG, for example, may be used in an amount between about 15% to about 20%, which percentages refer to final concentrations of PEG in a ligation reaction. For example, PEG may be used at about 15%, 15.5%, 16%, 16.5%, 17%, 17.5%, 18%, 18.5%, 19%, 19.5%, or 20%. In some implementations, 18.5% PEG is used. In some implementations, 18% PEG is used.

During purification, an SPRI bead solution may be added to a sample solution, often with instructions for a v/v ratio. For example, 1.2x 18% SPRI means that, if given a 50 µl sample, add 60 µl (50 x 1.2) of 18% SPRI beads. This v/v ratio leads to a final concentration of PEG at 9.8%, assuming there is in no PEG in the sample solution. However, often after ligation, there is an existing amount of PEG present in the sample solution (i.e., ligation products). Accordingly, a user may adjust the volume of added SPRI beads to reach the desired final concentration of PEG. A desired final concentration of PEG may range from about 5% final PEG to about 15% final PEG. For example, a desired final concentration of PEG may be about 5%, 6%, 7%, 8%, 9%, 10%, 11%, 12%, 13%, 14%, or 15%. In some implementations, a desired final concentration of PEG is about 10% (e.g., for hair samples and cfDNA samples). In some implementations, a desired final concentration of PEG is about 12% (e.g., for formalin-fixed paraffin-embedded (FFPE) samples and samples with large template fragments).

### Y-adapters

In some implementations, scaffold adapters described herein comprise two strands, with single-stranded scaffold region at a first end and two non-complementary strands at a second end. Such scaffold adapters may be referred to as Y-scaffold adapters, Y-adapters, Y-shaped scaffold adapters, Y-shaped adapters, Y-duplexes, Y-shaped duplexes, Y-scaffold duplexes, Y-shaped scaffold duplexes, and the like. A scaffold adapter having a Y-shaped structure generally comprises a double-stranded duplex region, two single stranded "arms" at one end, and single-stranded scaffold region at the other end.

Y-scaffold adapters may comprise a plurality of nucleic acid components and subcomponents. In some implementations, Y-scaffold adapters comprise a first nucleic acid strand and a second nucleic acid strand. In some implementations, a first nucleic acid strand is complementary to a second nucleic acid strand. In some implementations, a portion of a first nucleic acid strand is complementary to a portion of a second nucleic acid strand. In some implementations, a first nucleic acid strand comprises a first region that is complementary to a first region in a second nucleic acid strand, and the first polynucleotide comprises a second region that is not complementary to a second region in the second polynucleotide. The complementary region often forms the duplex region of the Y-scaffold adapter and the non-complementary region often forms the arms, or parts thereof, of the Y-scaffold adapter. The first and second nucleic acid strands may comprise subcomponents (e.g., subcomponents of scaffold polynucleotides, subcomponents of oligonucleotides and subcomponents of sequencing adapters described herein, such as, for example, amplification priming sites and/or specific sequencing adapters (e.g., P5, P7 adapters)). In some implementations, the first and second nucleic acid strands do not comprise certain subcomponents of sequencing adapters described herein, such as, for example, amplification priming sites and/or specific sequencing adapters (e.g., P5, P7 adapters).

In some implementations, a Y-scaffold adapter comprises a single-stranded scaffold region (ssNA hybridization region). The single-stranded scaffold region of a Y-scaffold adapter typically is located adjacent to the double-stranded duplex portion and at the opposite end of the non-complementary strands (or "arms") portion. The single-stranded scaffold region of a Y-scaffold adapter typically is complementary to a terminal region of a target nucleic acid (e.g., a terminal region of a single-stranded nucleic acid).

### Hairpins

In some implementations, a scaffold adapter comprises one strand capable of forming a hairpin structure having a single-stranded loop. In some implementations, a scaffold adapter consists of one strand capable of forming a hairpin structure having a single-stranded loop. A scaffold adapter having a hairpin structure generally comprises a double-stranded "stem" region and a single stranded "loop" region. In some implementations, a scaffold adapter comprises one strand (i.e., one continuous strand) capable of adopting a hairpin structure. In some implementations, a scaffold adapter consists essentially of one strand (i.e., one continuous strand) capable of adopting a hairpin structure. Consisting essentially of one strand means that the scaffold adapter does not include any additional strands of nucleic acid (e.g., hybridized to the scaffold adapter) that are not part of the continuous strand. Thus, "consisting essentially of" here refers to the number of strands in the scaffold adapter, and the scaffold adapter can include other features not essential to the number of strands (e.g., can include a detectable label, can include other regions). A scaffold adapter comprising or consisting essentially of one strand capable of forming a hairpin structure may be referred to herein as a hairpin, hairpin scaffold adapter, or hairpin adapter.

Hairpin scaffold adapters may comprise a plurality of nucleic acid components and subcomponents within the one strand. In some implementations, a hairpin scaffold adapter comprises an oligonucleotide and a scaffold polynucleotide. In some implementations, the oligonucleotide is complementary to an oligonucleotide hybridization region in the scaffold polynucleotide. In some implementations, a portion of the oligonucleotide is complementary to a portion of the oligonucleotide hybridization region in the scaffold polynucleotide. In some implementations, a hairpin scaffold adapter comprises complementary region and a non-complementary region. The complementary region often forms the stem of the hairpin adapter and the non-complementary region often forms the loop, or part thereof, of the hairpin scaffold adapter. The oligonucleotide and the scaffold polynucleotide may comprise subcomponents (e.g., subcomponents of scaffold polynucleotides, subcomponents of oligonucleotides, and subcomponents of sequencing adapters described herein, such as, for example, amplification priming sites and/or specific sequencing adapters (e.g., P5, P7 adapters)). In some implementations, the oligonucleotide and the scaffold polynucleotide do not comprise certain subcomponents of sequencing adapters described herein, such as, for example, amplification priming sites and specific sequencing adapters (e.g., P5, P7 adapters).

Hairpin scaffold adapters may comprise one or more cleavage sites capable of being cleaved under cleavage conditions. In some implementations, a cleavage site is located between an oligonucleotide and a scaffold polynucleotide. Cleavage at a cleavage site often generates two separate strands from the hairpin scaffold adapter. In some implementations, cleavage at a cleavage site generates a partially double stranded scaffold adapter with two unpaired strands forming a "Y" structure. Cleavage sites may include any suitable cleavage site, such as cleavage sites described herein, for example. In some implementations, cleavage sites comprise RNA nucleotides and may be cleaved, for example, using an RNAse. In some implementations, cleavage sites comprise uracil and/or deoxyuridine and may be cleaved, for example, using DNA glycosylase, endonuclease, RNAse, and the like and combinations thereof. In some implementations, cleavage sites do not comprise uracil and/or deoxyuridine. In some implementations, a method herein comprises after combining hairpin scaffold adapters with single-stranded nucleic acids, exposing one or more cleavage sites to cleavage conditions, thereby cleaving the scaffold adapters.

In some implementations, a hairpin scaffold adapter comprises a single-stranded scaffold region (ssNA hybridization region). The single-stranded scaffold region of a hairpin scaffold adapter typically is located adjacent to the double-stranded stem portion and at the opposite end of the loop portion. The single-stranded scaffold region of a hairpin scaffold adapter typically is complementary to a terminal region of a target nucleic acid (e.g., a terminal region of a single-stranded nucleic acid).

In some implementations, a hairpin scaffold adapter comprises in a 5' to 3' orientation: an oligonucleotide, one or more cleavage sites, and a scaffold polynucleotide comprising an oligonucleotide hybridization region and a scaffold region (ssNA hybridization region). In some implementations, a hairpin oligonucleotide comprises in a 5' to 3' orientation: a scaffold polynucleotide comprising a scaffold region (ssNA hybridization region) and an oligonucleotide hybridization region, one or more cleavage sites, and an oligonucleotide. In some implementations, a plurality or pool of hairpin scaffold adapter species comprises a mixture of: 1) hairpin scaffold adapters comprising in a 5' to 3' orientation: an oligonucleotide, one or more cleavage sites, and a scaffold polynucleotide comprising an oligonucleotide hybridization region and a scaffold region (ssNA hybridization region); and 2) hairpin scaffold adapters comprising in a 5' to 3' orientation: a scaffold polynucleotide comprising a scaffold region (ssNA hybridization region) and an oligonucleotide hybridization region, one or more cleavage sites, and an oligonucleotide.

### Modified nucleotides

In some implementations, a scaffold adapter, or component thereof, comprises one or more modified nucleotides. Modified nucleotides may be referred to as modified bases and may include, for example, nucleotides conjugated to a member of a binding pair, blocked nucleotides, non-natural nucleotides, nucleotide analogues, peptide nucleic acid (PNA) nucleotides, Morpholino nucleotides, locked nucleic acid (LNA) nucleotides, bridged nucleic acid (BNA) nucleotides, glycol nucleic acid (GNA) nucleotides, threose nucleic acid (TNA) nucleotides, and the like and combinations thereof. In some implementations, scaffold adapter, or component thereof, comprises one or more modified nucleotides within a duplex region, within a scaffold region, at one end, or at both ends of the scaffold adapter, or component thereof. In some implementations, a scaffold adapter, or component thereof, comprises one or more unpaired modified nucleotides. In some implementations, a scaffold adapter, or component thereof, comprises one or more unpaired modified nucleotides at one end of the adapter. In some implementations, a scaffold adapter, or component thereof, comprises one or more unpaired modified nucleotides at the end of the adapter opposite to the end that hybridizes to a target nucleic acid (e.g., an end comprising a single-stranded scaffold region). A modified nucleotide may be present at the end of the strand having a 3' terminus or at the end of the strand having a 5' terminus.

In some implementations, an oligonucleotide component comprises one or more modified nucleotides. In some implementations, the one or more modified nucleotides are capable of blocking covalent linkage of the oligonucleotide component to another oligonucleotide, polynucleotide, or nucleic acid molecule. In some implementations, an oligonucleotide component comprises one or more modified nucleotides at an end not adjacent to the ssNA. In some implementations, a scaffold polynucleotide comprises one or more modified nucleotides. In some implementations, the one or more modified nucleotides are capable of blocking covalent linkage of the scaffold polynucleotide to another oligonucleotide, polynucleotide, or nucleic acid molecule. A scaffold polynucleotide may comprise the one or more modified nucleotides at one or both ends of the polynucleotide. In some implementations, the one or more modified nucleotides comprise a ligation-blocking modification.

In some implementations, a scaffold adapter, or component thereof, comprises one or more blocked nucleotides. In one example, a scaffold adapter, or component thereof, may comprise one or more modified nucleotides that are capable of blocking hybridization to a nucleotide in another scaffold adapter, or component thereof. In some instances, the one or more modified nucleotides are capable of blocking ligation to a nucleotide in another scaffold adapter, or component thereof. In another example, a scaffold adapter, or component thereof, may comprise one or more modified nucleotides that are capable of blocking hybridization to a nucleotide in a target nucleic acid (e.g., ssNA). In some instances, the one or more modified nucleotides are capable of blocking ligation to a nucleotide in a target nucleic acid. In some implementations, one or both ends of a scaffold polynucleotide include a blocking modification and/or the end of an oligonucleotide component not adjacent to an ssNA fragment may include a blocking modification. A blocking modification refers to a modified end that cannot be linked to the end of another nucleic acid component using an approach employed to covalently link the adjacent ends of an oligonucleotide component and an ssNA fragment. In certain implementations, the blocking modification is a ligation-blocking modification. Examples of blocking modifications which may be included at one or both ends of a scaffold polynucleotide and/or the end of an oligonucleotide component not adjacent to the ssNA, include the absence of a 3' OH, and an inaccessible 3' OH. Non-limiting examples of blocking modifications in which an end has an inaccessible 3' OH include: an amino modifier, an amino linker, a spacer, an isodeoxy-base, a dideoxy base, an inverted dideoxy base, a 3' phosphate, and the like. In some implementations, a scaffold adapter, or component thereof, comprises one or more modified nucleotides that are incapable of binding to a natural nucleotide.

In some implementations, one or more modified nucleotides comprise an isodeoxy-base. In some implementations, one or more modified nucleotides comprise isodeoxy-guanine (iso-dG). In some implementations, one or more modified nucleotides comprise isodeoxy-cytosine (iso-dC). Iso-dC and iso-dG are chemical variants of cytosine and guanine, respectively. Iso-dC can hydrogen bond with iso-dG but not with unmodified guanine (natural guanine). Iso-dG can base pair with Iso-dC but not with unmodified cytosine (natural cytosine). A scaffold adapter, or component thereof, containing iso-dC can be designed so that it hybridizes to a complementary oligo containing iso-dG but cannot hybridize to any naturally occurring nucleic acid sequence.

In some implementations, one or more modified nucleotides comprise epigenetic-associated modifications, including but not limited to methylation, hydroxymethylation, and carboxylation. Example epigenetic-associated modifications include carboxycytosine, 5-methylcytosine (5mC) and its oxidative derivatives (e.g., 5-hydroxymethylcytosine (5hmC), 5-formylcytosine (5fC), and 5-arboxylcytosine (5caC)), N(6)-methyladenine (6mA), N4-methylcytosine (4mC), N(6)-methyladenosine (m(6)A), pseudouridine (Ψ), 5-methylcytidine (m(5)C), hydroxymethyl uracil, 2'-O-methylation at the 3' end, tRNA modifications, miRNA modifications, and snRNA modifications.

In some implementations, one or more modified nucleotides comprise a dideoxy-base. In some implementations, one or more modified nucleotides comprise dideoxy-cytosine. In some implementations, one or more modified nucleotides comprise an inverted dideoxy-base. In some implementations, one or more modified nucleotides comprise inverted dideoxy-thymine. For example, an inverted dideoxy-thymine located at the 5' end of a sequence can prevent unwanted 5' ligations.

In some implementations, one or more modified nucleotides comprise a spacer. In some implementations, one or more modified nucleotides comprise a C3 spacer. A C3 spacer phosphoramidite can be incorporated internally or at the 5'-end of an oligonucleotide. Multiple C3 spacers can be added at either end of a scaffold adapter, or component thereof, to introduce a long hydrophilic spacer arm (e.g., for the attachment of fluorophores or other pendent groups). Other spacers include, for example, photo-cleavable (PC) spacers, hexanediol, spacer 9, spacer 18, 1',2'-dideoxyribose (dSpacer), and the like.

In some implementations, a modified nucleotide comprises an amino linker or amino blocker. In some implementations, a modified nucleotide comprises an amino linker C6 (e.g., a 5' amino linker C6 or a 3' amino linker C6). In one example, an amino linker C6 can be used to incorporate an active primary amino group onto the 5'-end of an oligonucleotide. This can then be conjugated to a ligand. The amino group then becomes internal to the 5' end ligand. The amino group is separated from the 5'-end nucleotide base by a 6-carbon spacer arm to reduce steric interaction between the amino group and the oligo. In some implementations, a modified nucleotide comprises an amino linker C12 (e.g., a 5' amino linker C12 or a 3' amino linker C12). In one example, an amino linker C12 can be used to incorporate an active primary amino group onto the 5'-end of an oligonucleotide. The amino group is separated from the 5'-end nucleotide base by a 12-carbon spacer arm to minimize steric interaction between the amino group and the oligo.

In some implementations, a modified nucleotide comprises a member of a binding pair. Binding pairs may include, for example, antibody/antigen, antibody/antibody, antibody/antibody fragment, antibody/antibody receptor, antibody/protein A or protein G, hapten/anti-hapten, biotin/avidin, biotin/streptavidin, folic acid/folate binding protein, vitamin B12/intrinsic factor, chemical reactive group/complementary chemical reactive group, digoxigenin moiety/anti-digoxigenin antibody, fluorescein moiety/anti-fluorescein antibody, steroid/steroid-binding protein, operator/ repressor, nuclease/nucleotide, lectin/polysaccharide, active compound/active compound receptor, hormone/hormone receptor, enzyme/substrate, oligonucleotide or polynucleotide/its corresponding complement, the like or combinations thereof. In some implementations, a modified nucleotide comprises biotin.

In some implementations, a modified nucleotide comprises a first member of a binding pair (e.g., biotin); and a second member of a binding pair (e.g., streptavidin) is conjugated to a solid support or substrate. A solid support or substrate can be any physically separable solid to which a member of a binding pair can be directly or indirectly attached including, but not limited to, surfaces provided by microarrays and wells, and particles such as beads (e.g., paramagnetic beads, magnetic beads, microbeads, nanobeads), microparticles, and nanoparticles. Solid supports also can include, for example, chips, columns, optical fibers, wipes, filters (e.g., flat surface filters), one or more capillaries, glass and modified or functionalized glass (e.g., controlled-pore glass (CPG)), quartz, mica, diazotized membranes (paper or nylon), polyformaldehyde, cellulose, cellulose acetate, paper, ceramics, metals, metalloids, semiconductive materials, quantum dots, coated beads or particles, other chromatographic materials, magnetic particles; plastics (including acrylics, polystyrene, copolymers of styrene or other materials, polybutylene, polyurethanes, TEFLON^{™}, polyethylene, polypropylene, polyamide, polyester, polyvinylidenedifluoride (PVDF), and the like), polysaccharides, nylon or nitrocellulose, resins, silica or silica-based materials including silicon, silica gel, and modified silicon, Sephadex^{®}, Sepharose^{®}, carbon, metals (e.g., steel, gold, silver, aluminum, silicon and copper), inorganic glasses, conducting polymers (including polymers such as polypyrole and polyindole); micro or nanostructured surfaces such as nucleic acid tiling arrays, nanotube, nanowire, or nanoparticulate decorated surfaces; or porous surfaces or gels such as methacrylates, acrylamides, sugar polymers, cellulose, silicates, or other fibrous or stranded polymers. In some implementations, a solid support or substrate may be coated using passive or chemically-derivatized coatings with any number of materials, including polymers, such as dextrans, acrylamides, gelatins or agarose. Beads and/or particles may be free or in connection with one another (e.g., sintered). In some implementations, a solid support can be a collection of particles. In some implementations, the particles can comprise silica, and the silica may comprise silica dioxide. In some implementations, the silica can be porous, and in certain implementations the silica can be non-porous. In some implementations, the particles further comprise an agent that confers a paramagnetic property to the particles. In certain implementations, the agent comprises a metal, and in certain implementations the agent is a metal oxide, (e.g., iron or iron oxides, where the iron oxide contains a mixture of Fe2+ and Fe3+). A member of a binding pair may be linked to a solid support by covalent bonds or by non-covalent interactions and may be linked to a solid support directly or indirectly (e.g., via an intermediary agent such as a spacer molecule or biotin).

In some implementations, a scaffold polynucleotide, an oligonucleotide component, or both, include one or more non-natural nucleotides, also referred to as nucleotide analogs. Non-limiting examples of non-natural nucleotides that may be included in a scaffold polynucleotide, an oligonucleotide component, or both include LNA (locked nucleic acid), PNA (peptide nucleic acid), FANA (2'-deoxy-2'-fluoroarabinonucleotide), GNA (glycol nucleic acid), TNA (threose nucleic acid), 2'-O-Me RNA, 2'-fluoro RNA, Morpholino nucleotides, and any combination thereof.

### End Treatments

In some implementations, a method herein comprises contacting a nucleic acid composition comprising single-stranded nucleic acid (ssNA) with an agent comprising an end treatment activity under conditions in which single-stranded nucleic acid (ssNA) molecules are end treated, thereby generating an end treated ssNA composition. End treatments can include but are not limited to phosphorylation, dephosphorylation, methylation, demethylation, oxidation, de-oxidation, base modification, extension, polymerization, and combinations thereof. End treatments can be conducted with enzymes, including but not limited to ligases, polynucleotide kinases (PNK), terminal transferases, methyltransferases, methylases (e.g., 3' methylases, 5' methylases), polymerases (e.g., poly A polymerases), oxidases, and combinations thereof.

In some implementations, a method herein comprises contacting a nucleic acid composition comprising single-stranded nucleic acid (ssNA) with an agent comprising a phosphatase activity under conditions in which single-stranded nucleic acid (ssNA) molecules are dephosphorylated, thereby generating a dephosphorylated ssNA composition. In some implementations, a method herein comprises contacting a scaffold adapter, or component thereof, with an agent comprising a phosphatase activity under conditions in which the scaffold adapter, or component thereof, is dephosphorylated, thereby generating a dephosphorylated scaffold adapter, or component thereof (e.g., a dephosphorylated oligonucleotide; a dephosphorylated scaffold polynucleotide). Generally, an ssNA composition and/or scaffold adapters, or components thereof, are dephosphorylated prior to a combining step (i.e., prior to hybridization). ssNAs may be dephosphorylated and then subsequently phosphorylated prior to a combining step (i.e., prior to hybridization). Scaffold adapters, or components thereof, may be dephosphorylated and then subsequently phosphorylated prior to a combining step (i.e., prior to hybridization). Scaffold adapters, or components thereof, may be dephosphorylated and then not phosphorylated prior to a combining step (i.e., prior to hybridization). Scaffold adapters, or components thereof, may be dephosphorylated, not phosphorylated prior to a combining step (i.e., prior to hybridization), and then phosphorylated after a combining step (i.e., after hybridization) and prior to or during a ligation step. Reagents and kits for carrying out dephosphorylation of nucleic acids are known and available. For example, target nucleic acids (e.g., ssNAs) and/or scaffold adapters, or components thereof, can be treated with a phosphatase (i.e., an enzyme that uses water to cleave a phosphoric acid monoester into a phosphate ion and an alcohol).

In some implementations, a method herein comprises contacting a nucleic acid composition comprising single-stranded nucleic acid (ssNA) with an agent comprising a phosphoryl transfer activity under conditions in which a 5' phosphate is added to a 5' end of ssNAs. In some implementations, a method herein comprises contacting a dephosphorylated ssNA composition with an agent comprising a phosphoryl transfer activity under conditions in which a 5' phosphate is added to a 5' end of an ssNA. In some implementations, a method herein comprises contacting a scaffold adapter, or component thereof, with an agent comprising a phosphoryl transfer activity under conditions in which a 5' phosphate is added to a 5' end of a scaffold adapter, or component thereof. In some implementations, a method herein comprises contacting a dephosphorylated scaffold adapter, or component thereof, with an agent comprising a phosphoryl transfer activity under conditions in which a 5' phosphate is added to a 5' end of a scaffold adapter, or component thereof. In certain instances, an ssNA composition and/or scaffold adapters, or components thereof, are phosphorylated prior to a combining step (i.e., prior to hybridization). 5' phosphorylation of nucleic acids can be conducted by a variety of techniques. For example an ssNA composition and/or scaffold adapters, or components thereof, can be treated with a polynucleotide kinase (PNK) (e.g., T4 PNK), which catalyzes the transfer and exchange of Pi from the γ position of ATP to the 5'-hydroxyl terminus of polynucleotides (double-and single-stranded DNA and RNA) and nucleoside 3'-monophosphates. Suitable reaction conditions include, e.g., incubation of the nucleic acids with PNK in 1X PNK reaction buffer (e.g., 70 mM Tris-HCl, 10 mM MgCl₂, 5 mM DTT, pH 7.6 @ 25°C) for 30 minutes at 37°C; and incubation of the nucleic acids with PNK in T4 DNA ligase buffer (e.g., 50 mM Tris-HCl, 10 mM MgCl₂, 1 mM ATP, 10 mM DTT, pH 7.5 @ 25°C) for 30 minutes at 37°C. Optionally, following the phosphorylation reaction, the PNK may be heat inactivated, e.g., at 65°C for 20 minutes.

In some implementations, a method herein does not include use of an agent comprising a phosphoryl transfer activity. In some implementations, methods do not include producing the 5' phosphorylated ssNAs by phosphorylating the 5' ends of ssNAs from a nucleic acid sample. In certain instances, a nucleic acid sample comprises ssNAs with natively phosphorylated 5' ends. In some implementations, methods do not include producing the 5' phosphorylated scaffold adapters, or components thereof, by phosphorylating the 5' ends of scaffold adapters, or components thereof.

### Cleavage

In some implementations, ssNAs, scaffold adapters, and/or hybridization products (e.g., scaffold adapters hybridized to ssNAs) are cleaved or sheared prior to, during, or after a method described herein. In some implementations, ssNAs, scaffold adapters, and/or hybridization products are cleaved or sheared at a cleavage site. In some implementations, scaffold adapters and/or hybridization products are cleaved or sheared at a cleavage site within a hairpin loop. In some implementations, scaffold adapters and/or hybridization products are cleaved or sheared at a cleavage site at an internal location in a scaffold adapter (e.g., within a duplex region of a scaffold adapter). In some implementations, scaffold adapters are cleaved at a cleavage site (e.g., a uracil) at an internal location present only on the scaffold polynucleotide but not the complementary oligonucleotide component. Thus, in some implementations, a scaffold polynucleotide comprises one or more uracil bases, and an oligonucleotide component comprises no uracil bases. In some implementations, circular hybridization products are cleaved or sheared prior to, during, or after a method described herein. In some implementations, nucleic acids, such as, for example, cellular nucleic acids and/or large fragments (e.g., greater than 500 base pairs in length) are cleaved or sheared prior to, during, or after a method described herein. Large fragments may be referred to as high molecular weight (HMW) nucleic acid, HMW DNA or HMW RNA. HMW nucleic acid fragments may include fragments greater than about 500 bp, about 600 bp, about 700 bp, about 800 bp, about 900 bp, about 1000 bp, about 2000 bp, about 3000 bp, about 4000 bp, about 5000 bp, about 10,000 bp, or more. The term "shearing" or "cleavage" generally refers to a procedure or conditions in which a nucleic acid molecule may be severed into two (or more) smaller nucleic acid molecules. Such shearing or cleavage can be sequence specific, base specific, or nonspecific, and can be accomplished by any of a variety of methods, reagents or conditions, including, for example, chemical, enzymatic, and physical (e.g., physical fragmentation). Sheared or cleaved nucleic acids may have a nominal, average or mean length of about 5 to about 10,000 base pairs, about 100 to about 1,000 base pairs, about 100 to about 500 base pairs, or about 10, 15, 20, 25, 30, 35, 40, 45, 50, 55, 60, 65, 70, 75, 80, 85, 90, 95, 100, 200, 300, 400, 500, 600, 700, 800, 900, 1000, 2000, 3000, 4000, 5000, 6000, 7000, 8000 or 9000 base pairs.

Sheared or cleaved nucleic acids can be generated by a suitable method, non-limiting examples of which include physical methods (e.g., shearing, e.g., sonication, ultrasonication, French press, heat, UV irradiation, the like), enzymatic processes (e.g., enzymatic cleavage agents (e.g., a suitable nuclease, a suitable restriction enzyme), chemical methods (e.g., alkylation, DMS, piperidine, acid hydrolysis, base hydrolysis, heat, the like, or combinations thereof), ultraviolet (UV) light (e.g., at a photo-cleavable site (e.g., comprising a photo-cleavable spacer), the like or combinations thereof. The average, mean or nominal length of the resulting nucleic acid fragments can be controlled by selecting an appropriate fragment-generating method.

The term "cleavage agent" generally refers to an agent, sometimes a chemical or an enzyme that can cleave a nucleic acid at one or more specific or non-specific sites. Specific cleavage agents often cleave specifically according to a particular nucleotide sequence at a particular site, which may be referred to as a cleavage site. Cleavage agents may include enzymatic cleavage agents, chemical cleaving agents, and light (e.g., ultraviolet (UV) light).

Examples of enzymatic cleavage agents include without limitation endonucleases; deoxyribonucleases (DNase; e.g., DNase I, II); ribonucleases (RNase; e.g., RNAse A, RNAse E, RNAse F, RNAse H, RNAse III, RNAse L, RNAse P, RNAse PhyM, RNAse T1, RNAse T2, RNAse U2, and RNAse V); endonuclease VIII; CLEAVASE enzyme; TAQ DNA polymerase; E. coli DNA polymerase I; eukaryotic structure-specific endonucleases; murine FEN-1 endonucleases; nicking enzymes; type I, II or III restriction endonucleases (i.e., restriction enzymes) such as Acc I, Acil, Afl III, Alu I, Alw44 I, Apa I, Asn I, Ava I, Ava II, BamH I, Ban II, Bcl I, Bgl I, Bgl II, Bln I, Bsm I, BssH II, BstE II, BstUI, Cfo I, Cla I, Dde I, Dpn I, Dra I, EclX I, EcoR I, EcoR I, EcoR II, EcoR V, Hae II, Hae II, Hhal, Hind II, Hind III, Hpa I, Hpa II, Kpn I, Ksp I, Maell, McrBC, Mlu I, MIuN I, Msp I, Nci I, Nco I, Nde I, Nde II, Nhe I, Not I, Nru I, Nsi I, Pst I, Pvu I, Pvu II, Rsa I, Sac I, Sal I, Sau3A I, Sca I, ScrF I, Sfi I, Sma I, Spe I, Sph I, Ssp I, Stu I, Sty I, Swa I, Taq I, Xba I, Xho I; glycosylases (e.g., uracil-DNA glycolsylase (UDG), 3-methyladenine DNA glycosylase, 3-methyladenine DNA glycosylase II, pyrimidine hydrate-DNA glycosylase, FaPy-DNA glycosylase, thymine mismatch-DNA glycosylase (e.g., hypoxanthine-DNA glycosylase, uracil DNA glycosylase (UDG), 5-Hydroxymethyluracil DNA glycosylase (HmUDG), 5-Hydroxymethylcytosine DNA glycosylase, or 1,N6-etheno-adenine DNA glycosylase); exonucleases (e.g., exonuclease I, exonuclease II, exonuclease III, exonuclease IV, exonuclease V, exonuclease VI, exonuclease VII, exonuclease VIII); 5' to 3' exonucleases (e.g. exonuclease II); 3' to 5' exonucleases (e.g. exonuclease I); poly(A)-specific 3' to 5' exonucleases; ribozymes; DNAzymes; and the like and combinations thereof.

In some implementations, a cleavage site comprises a restriction enzyme recognition site. In some implementations, a cleavage agent comprises a restriction enzyme. In some implementations, a cleavage site comprises a rare-cutter restriction enzyme recognition site (e.g., a Notl recognition sequence). In some implementations, a cleavage agent comprises a rare-cutter enzyme (e.g., a rare-cutter restriction enzyme). A rare-cutter enzyme generally refers to a restriction enzyme with a recognition sequence which occurs only rarely in a genome (e.g., a human genome). An example is Notl, which cuts after the first GC of a 5'-GCGGCCGC-3' sequence. Restriction enzymes with seven and eight base pair recognition sequences often are considered as rare-cutter enzymes.

Cleavage methods and procedures for selecting restriction enzymes for cutting DNA at specific sites are well known to the skilled artisan. For example, many suppliers of restriction enzymes provide information on conditions and types of DNA sequences cut by specific restriction enzymes, including New England BioLabs, Pro-Mega Biochems, Boehringer-Mannheim, and the like. Enzymes often are used under conditions that will enable cleavage of the DNA with about 95%-100% efficiency, preferably with about 98%-100% efficiency.

In some implementations, a cleavage site comprises one or more ribonucleic acid (RNA) nucleotides. In some implementations, a cleavage site comprises a single stranded portion comprising one or more RNA nucleotides. In some implementations, the singe stranded portion is flanked by duplex portions. In some implementations, the singe stranded portion is a hairpin loop. In some implementations, a cleavage site comprises one RNA nucleotide. In some implementations, a cleavage site comprises two RNA nucleotides. In some implementations, a cleavage site comprises three RNA nucleotides. In some implementations, a cleavage site comprises four RNA nucleotides. In some implementations, a cleavage site comprises five RNA nucleotides. In some implementations, a cleavage site comprises more than five RNA nucleotides. In some implementations, a cleavage site comprises one or more RNA nucleotides chosen from adenine (A), cytosine (C), guanine (G), and uracil (U). In some implementations, a cleavage site comprises one or more RNA nucleotides chosen from adenine (A), cytosine (C), and guanine (G). In some implementations, a cleavage site comprises no uracil (U). In some implementations, a cleavage site comprises one or more RNA nucleotides comprising guanine (G). In some implementations, a cleavage site comprises one or more RNA nucleotides consisting of guanine (G). In some implementations, a cleavage site comprises one or more RNA nucleotides comprising cytosine (C). In some implementations, a cleavage site comprises one or more RNA nucleotides consisting of cytosine (C). In some implementations, a cleavage site comprises one or more RNA nucleotides comprising adenine (A). In some implementations, a cleavage site comprises one or more RNA nucleotides consisting of adenine (A). In some implementations, a cleavage site comprises one or more RNA nucleotides consisting of adenine (A), cytosine (C), and guanine (G). In some implementations, a cleavage site comprises one or more RNA nucleotides consisting of adenine (A) and cytosine (C). In some implementations, a cleavage site comprises one or more RNA nucleotides consisting of adenine (A) and guanine (G). In some implementations, a cleavage site comprises one or more RNA nucleotides consisting of cytosine (C) and guanine (G). In some implementations, a cleavage agent comprises a ribonuclease (RNAse). In some implementations, an RNAse is an endoribonuclease. An RNAse may be chosen from one or more of RNAse A, RNAse E, RNAse F, RNAse H, RNAse III, RNAse L, RNAse P, RNAse PhyM, RNAse T1, RNAse T2, RNAse U2, and RNAse V.

In some implementations, a cleavage site comprises a photo-cleavable spacer or photo-cleavable modification. Photo-cleavable modifications may contain, for example, a photolabile functional group that is cleavable by ultraviolet (UV) light of specific wavelength (e.g., 300-350 nm). An example photo-cleavable spacer (available from Integrated DNA Technologies; product no. 1707) is a 10-atom linker arm that can only be cleaved when exposed to UV light within the appropriate spectral range. An oligonucleotide comprising a photo-cleavable spacer can have a 5' phosphate group that is available for subsequent ligase reactions. Photo-cleavable spacers can be placed between DNA bases or between an oligo and a terminal modification (e.g., a fluorophore). In such implementations, ultraviolet (UV) light may be considered as a cleavage agent.

In some implementations, a cleavage site comprises a diol. For example, a cleavage site may comprise vicinal diol incorporated in a 5' to 5' linkage. Cleavage sites comprising a diol may be chemically cleaved, for example, using a periodate. In some implementations, a cleavage site comprises a blunt end restriction enzyme recognition site. Cleavage sites comprising a blunt end restriction enzyme recognition site may be cleaved by a blunt end restriction enzyme.

### Nick seal and fill-in

In some implementations, a method herein comprises performing a nick seal reaction (e.g., using a DNA ligase or other suitable enzyme, and, in certain instances, a kinase adapted to 5' phosphorylate nucleic acids (e.g., a polynucleotide kinase (PNK)). In some implementations, a method herein comprises performing a fill-in reaction. For example, when scaffold adapters are present as duplexes, some or all of the duplexes may include an overhang at the end of the duplex opposite the end that hybridizes to the ssNAs. When such duplex overhangs exist, subsequent to the combining, a method herein may further include filling in the overhangs formed by the duplexes. In some implementations, a fill-in reaction is performed to generate a blunt-ended hybridization product. Any suitable reagent for carrying out a fill-in reaction may be used. Polymerases suitable for performing fill-in reactions include, e.g., DNA polymerase I, large (Klenow) fragment, Bacillus stearothermophilus (Bst) DNA polymerase, and the like. In some implementations, a strand displacing polymerase is used (e.g., Bst DNA polymerase).

### Exonuclease treatment

In some implementations, nucleic acid (e.g., RNA-DNA duplexes, hybridization products; circularized hybridization products) is treated with an exonuclease. In some implementations, RNA in an RNA-DNA duplex (e.g., an RNA-DNA duplex generated by first strand cDNA synthesis) is treated with an exonuclease. Exonucleases are enzymes that work by cleaving nucleotides one at a time from the end of a polynucleotide chain through a hydrolyzing reaction that breaks phosphodiester bonds at either the 3' or the 5' end. Exonucleases include, for example, DNAses, RNAses (e.g., RNAseH), 5' to 3' exonucleases (e.g. exonuclease II), 3' to 5' exonucleases (e.g. exonuclease I), and poly(A)-specific 3' to 5' exonucleases. In some implementations, exonuclease activity is provided by a reverse transcriptase (e.g., RNAse activity provided by M-MLV reverse transcriptase having a fully functional RNAseH domain). In some implementations, hybridization products are treated with an exonuclease to remove contaminating nucleic acids such as, for example, single stranded oligonucleotides, nucleic acid fragments, or RNA from an RNA-DNA duplex. In some implementations, circularized hybridization products are treated with an exonuclease to remove any non-circularized hybridization products, non-hybridized oligonucleotides, non-hybridized target nucleic acids, oligonucleotide dimers, and the like and combinations thereof.

### Samples

Provided herein are methods and compositions for processing and/or analyzing nucleic acid. Nucleic acid or a nucleic acid mixture utilized in methods and compositions described herein may be isolated from a sample obtained from a subject (e.g., a test subject). A subject can be any living or non-living organism, including but not limited to a human, a non-human animal, a plant, a bacterium, a fungus, a protist or a pathogen. Any human or non-human animal can be selected, and may include, for example, mammal, reptile, avian, amphibian, fish, ungulate, ruminant, bovine (e.g., cattle), equine (e.g., horse), caprine and ovine (e.g., sheep, goat), swine (e.g., pig), camelid (e.g., camel, llama, alpaca), monkey, ape (e.g., gorilla, chimpanzee), ursid (e.g., bear), poultry, dog, cat, mouse, rat, fish, dolphin, whale and shark. A subject may be a male or female (e.g., woman, a pregnant woman). A subject may be any age (e.g., an embryo, a fetus, an infant, a child, an adult). A subject may be a cancer patient, a patient suspected of having cancer, a patient in remission, a patient with a family history of cancer, and/or a subject obtaining a cancer screen. A subject may be a patient having an infection or infectious disease or infected with a pathogen (e.g., bacteria, virus, fungus, protozoa, and the like), a patient suspected of having an infection or infectious disease or being infected with a pathogen, a patient recovering from an infection, infectious disease, or pathogenic infection, a patient with a history of infections, infectious disease, pathogenic infections, and/or a subject obtaining an infectious disease or pathogen screen. A subject may be a transplant recipient. A subject may be a patient undergoing a microbiome analysis. In some implementations, a test subject is a female. In some implementations, a test subject is a human female. In some implementations, a test subject is a male. In some implementations, a test subject is a human male.

A nucleic acid sample may be isolated or obtained from any type of suitable biological specimen or sample (e.g., a test sample). A nucleic acid sample may be isolated or obtained from a single cell, a plurality of cells (e.g., cultured cells), cell culture media, conditioned media, a tissue, an organ, or an organism (e.g., bacteria, yeast, or the like). In some implementations, a nucleic acid sample is isolated or obtained from a cell(s), tissue, organ, and/or the like of an animal (e.g., an animal subject). In some implementations, a nucleic acid sample is isolated or obtained from a source such as bacteria, yeast, insects (e.g., drosophila), mammals, amphibians (e.g., frogs (e.g., Xenopus)), viruses, plants, or any other mammalian or non-mammalian nucleic acid sample source.

A nucleic acid sample may be isolated or obtained from an extant organism or animal. In some instances, a nucleic acid sample may be isolated or obtained from an extinct (or "ancient") organism or animal (e.g., an extinct mammal; an extinct mammal from the genus Homo). In some instances, a nucleic acid sample may be obtained as part of a diagnostic analysis.

In some instances, a nucleic acid sample may be obtained as part of a forensics analysis. In some implementations, a single-stranded nucleic acid library preparation (ssPrep) method described herein is applied to a forensic sample or specimen. A forensic sample or specimen may include any biological substance that contains nucleic acid. For example, a forensic sample or specimen may include blood, semen, hair, skin, sweat, saliva, decomposed tissue, bone, fingernail scrapings, licked stamps/envelopes, sluff, touch DNA, razor residue, and the like.

A sample or test sample may be any specimen that is isolated or obtained from a subject or part thereof (e.g., a human subject, a pregnant female, a cancer patient, a patient having an infection or infectious disease, a transplant recipient, a fetus, a tumor, an infected organ or tissue, a transplanted organ or tissue, a microbiome). A sample sometimes is from a pregnant female subject bearing a fetus at any stage of gestation (e.g., first, second or third trimester for a human subject), and sometimes is from a postnatal subject. A sample sometimes is from a pregnant subject bearing a fetus that is euploid for all chromosomes, and sometimes is from a pregnant subject bearing a fetus having a chromosome aneuploidy (e.g., one, three (i.e., trisomy (e.g., T21, T18, T13)), or four copies of a chromosome) or other genetic variation. Non-limiting examples of specimens include fluid or tissue from a subject, including, without limitation, blood or a blood product (e.g., serum, plasma, or the like), umbilical cord blood, chorionic villi, amniotic fluid, cerebrospinal fluid, spinal fluid, lavage fluid (e.g., bronchoalveolar, gastric, peritoneal, ductal, ear, arthroscopic), biopsy sample (e.g., from pre-implantation embryo; cancer biopsy), celocentesis sample, cells (blood cells, placental cells, embryo or fetal cells, fetal nucleated cells or fetal cellular remnants, normal cells, abnormal cells (e.g., cancer cells)) or parts thereof (e.g., mitochondrial, nucleus, extracts, or the like), washings of female reproductive tract, urine, feces, sputum, saliva, nasal mucous, prostate fluid, lavage, semen, lymphatic fluid, bile, tears, sweat, breast milk, breast fluid, the like or combinations thereof. In some implementations, a biological sample is a cervical swab from a subject. A fluid or tissue sample from which nucleic acid is extracted may be acellular (e.g., cell-free). In some implementations, a fluid or tissue sample may contain cellular elements or cellular remnants. In some implementations, fetal cells or cancer cells may be included in the sample.

A sample can be a liquid sample. A liquid sample can comprise extracellular nucleic acid (e.g., circulating cell-free DNA). Examples of liquid samples include, but are not limited to, blood or a blood product (e.g., serum, plasma, or the like), urine, cerebral spinal fluid, saliva, sputum, biopsy sample (e.g., liquid biopsy for the detection of cancer), a liquid sample described above, the like or combinations thereof. In certain implementations, a sample is a liquid biopsy, which generally refers to an assessment of a liquid sample from a subject for the presence, absence, progression or remission of a disease (e.g., cancer). A liquid biopsy can be used in conjunction with, or as an alternative to, a sold biopsy (e.g., tumor biopsy). In certain instances, extracellular nucleic acid is analyzed in a liquid biopsy.

In some implementations, a biological sample may be blood, plasma or serum. The term "blood" encompasses whole blood, blood product or any fraction of blood, such as serum, plasma, buffy coat, or the like as conventionally defined. Blood or fractions thereof often comprise nucleosomes. Nucleosomes comprise nucleic acids and are sometimes cell-free or intracellular. Blood also comprises buffy coats. Buffy coats are sometimes isolated by utilizing a ficoll gradient. Buffy coats can comprise white blood cells (e.g., leukocytes, T-cells, B-cells, platelets, and the like). Blood plasma refers to the fraction of whole blood resulting from centrifugation of blood treated with anticoagulants. Blood serum refers to the watery portion of fluid remaining after a blood sample has coagulated. Fluid or tissue samples often are collected in accordance with standard protocols hospitals or clinics generally follow. For blood, an appropriate amount of peripheral blood (e.g., between 3 to 40 milliliters, between 5 to 50 milliliters) often is collected and can be stored according to standard procedures prior to or after preparation.

An analysis of nucleic acid found in a subject's blood may be performed using, e.g., whole blood, serum, or plasma. An analysis of fetal DNA found in maternal blood, for example, may be performed using, e.g., whole blood, serum, or plasma. An analysis of tumor or cancer DNA found in a patient's blood, for example, may be performed using, e.g., whole blood, serum, or plasma. An analysis of pathogen DNA found in a patient's blood, for example, may be performed using, e.g., whole blood, serum, or plasma. An analysis of transplant DNA found in a transplant recipient's blood, for example, may be performed using, e.g., whole blood, serum, or plasma. Methods for preparing serum or plasma from blood obtained from a subject (e.g., a maternal subject; patient; cancer patient) are known. For example, a subject's blood (e.g., a pregnant woman's blood; patient's blood; cancer patient's blood) can be placed in a tube containing EDTA or a specialized commercial product such as Cell-Free DNA BCT (Streck, Omaha, NE) or Vacutainer SST (Becton Dickinson, Franklin Lakes, N.J.) to prevent blood clotting, and plasma can then be obtained from whole blood through centrifugation. Serum may be obtained with or without centrifugation-following blood clotting. If centrifugation is used then it is typically, though not exclusively, conducted at an appropriate speed, e.g., 1,500-3,000 times g. Plasma or serum may be subjected to additional centrifugation steps before being transferred to a fresh tube for nucleic acid extraction. In addition to the acellular portion of the whole blood, nucleic acid may also be recovered from the cellular fraction, enriched in the buffy coat portion, which can be obtained following centrifugation of a whole blood sample from the subject and removal of the plasma.

A sample may be a tumor nucleic acid sample (i.e., a nucleic acid sample isolated from a tumor). The term "tumor" generally refers to neoplastic cell growth and proliferation, whether malignant or benign, and may include pre-cancerous and cancerous cells and tissues. The terms "cancer" and "cancerous" generally refer to the physiological condition in mammals that is typically characterized by unregulated cell growth/proliferation. Examples of cancer include, but are not limited to, carcinoma, lymphoma, blastoma, sarcoma, leukemia, squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, various types of head and neck cancer, and the like.

A sample may be heterogeneous. For example, a sample may include more than one cell type and/or one or more nucleic acid species. In some instances, a sample may include (i) fetal cells and maternal cells, (ii) cancer cells and non-cancer cells, and/or (iii) pathogenic cells and host cells. In some instances, a sample may include (i) cancer and non-cancer nucleic acid, (ii) pathogen and host nucleic acid, (iii) fetal derived and maternal derived nucleic acid, and/or more generally, (iv) mutated and wild-type nucleic acid. In some instances, a sample may include a minority nucleic acid species and a majority nucleic acid species, as described in further detail below. In some instances, a sample may include cells and/or nucleic acid from a single subject or may include cells and/or nucleic acid from multiple subjects.

### Nucleic acid

Provided herein are methods and compositions for processing and/or analyzing nucleic acid. The terms nucleic acid(s), nucleic acid molecule(s), nucleic acid fragment(s), target nucleic acid(s), nucleic acid template(s), template nucleic acid(s), nucleic acid target(s), target nucleic acid(s), polynucleotide(s), polynucleotide fragment(s), target polynucleotide(s), polynucleotide target(s), and the like may be used interchangeably throughout the disclosure. The terms refer to nucleic acids of any composition from, such as DNA (e.g., complementary DNA (cDNA; synthesized from any RNA or DNA of interest), genomic DNA (gDNA), genomic DNA fragments, mitochondrial DNA (mtDNA), recombinant DNA (e.g., plasmid DNA), and the like), RNA (e.g., message RNA (mRNA), short inhibitory RNA (siRNA), ribosomal RNA (rRNA), transfer RNA (tRNA), microRNA, transacting small interfering RNA (ta-siRNA), natural small interfering RNA (nat-siRNA), small nucleolar RNA (snoRNA), small nuclear RNA (snRNA), long non-coding RNA (IncRNA), non-coding RNA (ncRNA), transfer-messenger RNA (tmRNA), precursor messenger RNA (pre-mRNA), small Cajal body-specific RNA (scaRNA), piwi-interacting RNA (piRNA), endoribonuclease-prepared siRNA (esiRNA), small temporal RNA (stRNA), signal recognition RNA, telomere RNA, RNA highly expressed by a fetus or placenta, and the like), and/or DNA or RNA analogs (e.g., containing base analogs, sugar analogs and/or a non-native backbone and the like), RNA/DNA hybrids and polyamide nucleic acids (PNAs), all of which can be in single- or double-stranded form, and unless otherwise limited, can encompass known analogs of natural nucleotides that can function in a similar manner as naturally occurring nucleotides. A nucleic acid may be, or may be from, a plasmid, phage, virus, bacterium, autonomously replicating sequence (ARS), mitochondria, centromere, artificial chromosome, chromosome, or other nucleic acid able to replicate or be replicated in vitro or in a host cell, a cell, a cell nucleus or cytoplasm of a cell in certain implementations. A template nucleic acid in some implementations can be from a single chromosome (e.g., a nucleic acid sample may be from one chromosome of a sample obtained from a diploid organism). Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid is used interchangeably with locus, gene, cDNA, and mRNA encoded by a gene. The term also may include, as equivalents, derivatives, variants and analogs of RNA or DNA synthesized from nucleotide analogs, single-stranded ("sense" or "antisense," "plus" strand or "minus" strand, "forward" reading frame or "reverse" reading frame) and double-stranded polynucleotides. The term "gene" refers to a section of DNA involved in producing a polypeptide chain; and generally includes regions preceding and following the coding region (leader and trailer) involved in the transcription/translation of the gene product and the regulation of the transcription/translation, as well as intervening sequences (introns) between individual coding regions (exons). A nucleotide or base generally refers to the purine and pyrimidine molecular units of nucleic acid (e.g., adenine (A), thymine (T), guanine (G), and cytosine (C)). For RNA, the base thymine is replaced with uracil. Nucleic acid length or size may be expressed as a number of bases.

Target nucleic acids may be any nucleic acids of interest. Nucleic acids may be polymers of any length composed of deoxyribonucleotides (i.e., DNA bases), ribonucleotides (i.e., RNA bases), or combinations thereof, e.g., 10 bases or longer, 20 bases or longer, 50 bases or longer, 100 bases or longer, 200 bases or longer, 300 bases or longer, 400 bases or longer, 500 bases or longer, 1000 bases or longer, 2000 bases or longer, 3000 bases or longer, 4000 bases or longer, 5000 bases or longer. In certain aspects, nucleic acids are polymers composed of deoxyribonucleotides (i.e., DNA bases), ribonucleotides (i.e., RNA bases), or combinations thereof, e.g., 10 bases or less, 20 bases or less, 50 bases or less, 100 bases or less, 200 bases or less, 300 bases or less, 400 bases or less, 500 bases or less, 1000 bases or less, 2000 bases or less, 3000 bases or less, 4000 bases or less, or 5000 bases or less.

Nucleic acid may be single or double stranded. Single stranded DNA (ssDNA), for example, can be generated by denaturing double stranded DNA by heating or by treatment with alkali, for example. Accordingly, in some implementations, ssDNA is derived from double-stranded DNA (dsDNA). In some implementations, a method herein comprises prior to combining a nucleic acid composition comprising dsDNA with the scaffold adapters herein, or components thereof, denaturing the dsDNA, thereby generating ssDNA.

In certain implementations, nucleic acid is in a D-loop structure, formed by strand invasion of a duplex DNA molecule by an oligonucleotide or a DNA-like molecule such as peptide nucleic acid (PNA). D loop formation can be facilitated by addition of E. Coli RecA protein and/or by alteration of salt concentration, for example, using methods known in the art.

Nucleic acid (e.g., nucleic acid targets, single-stranded nucleic acid (ssNA), oligonucleotides, overhangs, scaffold polynucleotides and hybridization regions thereof (e.g., ssNA hybridization region, oligonucleotide hybridization region)) may be described herein as being complementary to another nucleic acid, having a complementarity region, being capable of hybridizing to another nucleic acid, or having a hybridization region. The terms "complementary" or "complementarity" or "hybridization" generally refer to a nucleotide sequence that base-pairs by non-covalent bonds to a region of a nucleic acid (e.g., the nucleotide sequence of an ssNA hybridization region that hybridizes to the terminal region of an ssNA fragment, and the nucleotide sequence of an oligonucleotide hybridization region that hybridizes to an oligonucleotide component of a scaffold adapter). In the canonical Watson-Crick base pairing, adenine (A) forms a base pair with thymine (T), and guanine (G) pairs with cytosine (C) in DNA. In RNA, thymine (T) is replaced by uracil (U). As such, A is complementary to T and G is complementary to C. In RNA, A is complementary to U and vice versa. In a DNA-RNA duplex, A (in a DNA strand) is complementary to U (in an RNA strand). In some implementations, one or more thymine (T) bases are replaced by uracil (U) in a scaffold adapter, or a component thereof, and is/are complementary to adenine (A). Typically, "complementary" or "complementarity" or "capable of hybridizing" refer to a nucleotide sequence that is at least partially complementary. These terms may also encompass duplexes that are fully complementary such that every nucleotide in one strand is complementary or hybridizes to every nucleotide in the other strand in corresponding positions.

In certain instances, a nucleotide sequence may be partially complementary to a target, in which not all nucleotides are complementary to every nucleotide in the target nucleic acid in all the corresponding positions. For example, an ssNA hybridization region may be perfectly (i.e., 100%) complementary to a target ssNA terminal region, or an ssNA hybridization region may share some degree of complementarity which is less than perfect (e.g., 70%, 75%, 85%, 90%, 95%, 99%). In another example, an oligonucleotide hybridization region may be perfectly (i.e., 100%) complementary to an oligonucleotide, or an oligonucleotide hybridization region may share some degree of complementarity which is less than perfect (e.g., 70%, 75%, 85%, 90%, 95%, 99%).

The percent identity of two nucleotide sequences can be determined by aligning the sequences for optimal comparison purposes (e.g., gaps can be introduced in the sequence of a first sequence for optimal alignment). The nucleotides at corresponding positions are then compared, and the percent identity between the two sequences is a function of the number of identical positions shared by the sequences (i.e., % identity= # of identical positions/total # of positions× 100). When a position in one sequence is occupied by the same nucleotide as the corresponding position in the other sequence, then the molecules are identical at that position.

In some implementations, nucleic acids in a mixture of nucleic acids are analyzed. A mixture of nucleic acids can comprise two or more nucleic acid species having the same or different nucleotide sequences, different lengths, different origins (e.g., genomic origins, fetal vs. maternal origins, cell or tissue origins, cancer vs. non-cancer origin, tumor vs. non-tumor origin, host vs. pathogen, host vs. transplant, host vs. microbiome, sample origins, subject origins, and the like), different overhang lengths, different overhang types (e.g., 5' overhangs, 3' overhangs, no overhangs), or combinations thereof. In some implementations, a mixture of nucleic acids comprises single-stranded nucleic acid and double-stranded nucleic acid. In some implementation, a mixture of nucleic acids comprises DNA and RNA. In some implementation, a mixture of nucleic acids comprises ribosomal RNA (rRNA) and messenger RNA (mRNA). Nucleic acid provided for processes described herein may contain nucleic acid from one sample or from two or more samples (e.g., from 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more samples).

In some implementations, target nucleic acids (e.g., ssNAs) comprise degraded DNA. Degraded DNA may be referred to as low-quality DNA or highly degraded DNA. Degraded DNA may be highly fragmented, and may include damage such as base analogs and abasic sites subject to miscoding lesions and/or intermolecular crosslinking. For example, sequencing errors resulting from deamination of cytosine residues may be present in certain sequences obtained from degraded DNA (e.g., miscoding of C to T and G to A). In some implementations, target nucleic acids (e.g., ssNAs) are derived from nicked double-stranded nucleic acid fragments. Nicked double-stranded nucleic acid fragments may be denatured (e.g., heat denatured) to generate ssNA fragments. Nucleic acid may be derived from one or more sources (e.g., biological sample, blood, cells, serum, plasma, buffy coat, urine, lymphatic fluid, skin, hair, soil, and the like) by methods known in the art. Any suitable method can be used for isolating, extracting and/or purifying DNA from a biological sample (e.g., from blood or a blood product), non-limiting examples of which include methods of DNA preparation (e.g., described by Sambrook and Russell, Molecular Cloning: A Laboratory Manual 3d ed., 2001), various commercially available reagents or kits, such as DNeasy^{®}, RNeasy^{®}, QIAprep^{®}, QIAquick^{®}, and QIAamp^{®} (e.g., QIAamp^{®} Circulating Nucleic Acid Kit, QiaAmp^{®} DNA Mini Kit or QiaAmp^{®} DNA Blood Mini Kit) nucleic acid isolation/purification kits by Qiagen, Inc. (Germantown, Md); GenomicPrep^{™} Blood DNA Isolation Kit (Promega, Madison, Wis.); GFX^{™} Genomic Blood DNA Purification Kit (Amersham, Piscataway, N.J.); DNAzol^{®}, ChargeSwitch^{®}, Purelink^{®}, GeneCatcher^{®} nucleic acid isolation/purification kits by Life Technologies, Inc. (Carlsbad, CA); NucleoMag^{®}, NucleoSpin^{®}, and NucleoBond^{®} nucleic acid isolation/purification kits by Clontech Laboratories, Inc. (Mountain View, CA); the like or combinations thereof. In certain aspects, the nucleic acid is isolated from a fixed biological sample, e.g., formalin-fixed, paraffin-embedded (FFPE) tissue. Genomic DNA from FFPE tissue may be isolated using commercially available kits - such as the AHPrep^{®} DNA/RNA FFPE kit by Qiagen, Inc. (Germantown, Md), the RecoverAll^{®} Total Nucleic Acid Isolation kit for FFPE by Life Technologies, Inc. (Carlsbad, CA), and the NucleoSpin^{®} FFPE kits by Clontech Laboratories, Inc. (Mountain View, CA).

In some implementations, nucleic acid is extracted from cells using a cell lysis procedure. Cell lysis procedures and reagents are known in the art and may generally be performed by chemical (e.g., detergent, hypotonic solutions, enzymatic procedures, and the like, or combination thereof), physical (e.g., French press, sonication, and the like), or electrolytic lysis methods. Any suitable lysis procedure can be utilized. For example, chemical methods generally employ lysing agents to disrupt cells and extract the nucleic acids from the cells, followed by treatment with chaotropic salts. Physical methods such as freeze/thaw followed by grinding, the use of cell presses and the like also are useful. In some instances, a high salt and/or an alkaline lysis procedure may be utilized. In some instances, a lysis procedure may include a lysis step with EDTA/Proteinase K, a binding buffer step with high amount of salts (e.g., guanidinium chloride (GuHCl), sodium acetate) and isopropanol, and binding DNA in this solution to silica-based column. In some instances, a lysis protocol includes certain procedures described in Dabney et al., Proceedings of the National Academy of Sciences 110, no. 39 (2013): 15758-15763.

Nucleic acids can include extracellular nucleic acid in certain implementations. The term "extracellular nucleic acid" as used herein can refer to nucleic acid isolated from a source having substantially no cells and also is referred to as "cell-free" nucleic acid (cell-free DNA, cell-free RNA, or both), "circulating cell-free nucleic acid" (e.g., CCF fragments, ccf DNA) and/or "cell-free circulating nucleic acid." Extracellular nucleic acid can be present in and obtained from blood (e.g., from the blood of a human subject). Extracellular nucleic acid often includes no detectable cells and may contain cellular elements or cellular remnants. Non-limiting examples of acellular sources for extracellular nucleic acid are blood, blood plasma, blood serum and urine. In certain aspects, cell-free nucleic acid is obtained from a body fluid sample chosen from whole blood, blood plasma, blood serum, amniotic fluid, saliva, urine, pleural effusion, bronchial lavage, bronchial aspirates, breast milk, colostrum, tears, seminal fluid, peritoneal fluid, pleural effusion, and stool. As used herein, the term "obtain cell-free circulating sample nucleic acid" includes obtaining a sample directly (e.g., collecting a sample, e.g., a test sample) or obtaining a sample from another who has collected a sample. Extracellular nucleic acid may be a product of cellular secretion and/or nucleic acid release (e.g., DNA release). Extracellular nucleic acid may be a product of any form of cell death, for example. In some instances, extracellular nucleic acid is a product of any form of type I or type II cell death, including mitotic, oncotic, toxic, ischemic, and the like and combinations thereof. Without being limited by theory, extracellular nucleic acid may be a product of cell apoptosis and cell breakdown, which provides basis for extracellular nucleic acid often having a series of lengths across a spectrum (e.g., a "ladder"). In some instances, extracellular nucleic acid is a product of cell necrosis, necropoptosis, oncosis, entosis, pyrotosis, and the like and combinations thereof. In some implementations, sample nucleic acid from a test subject is circulating cell-free nucleic acid. In some implementations, circulating cell free nucleic acid is from blood plasma or blood serum from a test subject. In some aspects, cell-free nucleic acid is degraded. In some implementations, cell-free nucleic acid comprises cell-free fetal nucleic acid (e.g., cell-free fetal DNA). In certain aspects, cell-free nucleic acid comprises circulating cancer nucleic acid (e.g., cancer DNA). In certain aspects, cell-free nucleic acid comprises circulating tumor nucleic acid (e.g., tumor DNA). In some implementations, cell-free nucleic acid comprises infectious agent nucleic acid (e.g., pathogen DNA). In some implementations, cell-free nucleic acid comprises nucleic acid (e.g., DNA) from a transplant. In some implementations, cell-free nucleic acid comprises nucleic acid (e.g., DNA) from a microbiome (e.g., microbiome of gut, microbiome of blood, microbiome of mouth, microbiome of spinal fluid, microbiome of feces).

Cell-free DNA (cfDNA) may originate from degraded sources and often provides limiting amounts of DNA when extracted. Methods described herein for generating single-stranded DNA (ssDNA) libraries are able to capture a larger amount of short DNA fragments from cfDNA. cfDNA from cancer samples, for example, tends to have a higher population of short fragments. In certain instances, short fragments in cfDNA may be enriched for fragments originating from transcription factors rather than nucleosomes.

Extracellular nucleic acid can include different nucleic acid species, and therefore is referred to herein as "heterogeneous" in certain implementations. For example, blood serum or plasma from a person having a tumor or cancer can include nucleic acid from tumor cells or cancer cells (e.g., neoplasia) and nucleic acid from non-tumor cells or non-cancer cells. In another example, blood serum or plasma from a pregnant female can include maternal nucleic acid and fetal nucleic acid. In another example, blood serum or plasma from a patient having an infection or infectious disease can include host nucleic acid and infectious agent or pathogen nucleic acid. In another example, a sample from a subject having received a transplant can include host nucleic acid and nucleic acid from the donor organ or tissue. In some instances, cancer nucleic acid, tumor nucleic acid, fetal nucleic acid, pathogen nucleic acid, or transplant nucleic acid sometimes is about 5% to about 50% of the overall nucleic acid (e.g., about 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49% of the total nucleic acid is cancer, tumor, fetal, pathogen, transplant, or microbiome nucleic acid). In another example, heterogeneous nucleic acid may include nucleic acid from two or more subjects (e.g., a sample from a crime scene).

At least two different nucleic acid species can exist in different amounts in extracellular nucleic acid and sometimes are referred to as minority species and majority species. In certain instances, a minority species of nucleic acid is from an affected cell type (e.g., cancer cell, wasting cell, cell attacked by immune system). In certain implementations, a genetic variation or genetic alteration (e.g., copy number alteration, copy number variation, single nucleotide alteration, single nucleotide variation, chromosome alteration, and/or translocation) is determined for a minority nucleic acid species. In certain implementations, a genetic variation or genetic alteration is determined for a majority nucleic acid species. Generally, it is not intended that the terms "minority" or "majority" be rigidly defined in any respect. In one aspect, a nucleic acid that is considered "minority," for example, can have an abundance of at least about 0.1% of the total nucleic acid in a sample to less than 50% of the total nucleic acid in a sample. In some implementations, a minority nucleic acid can have an abundance of at least about 1% of the total nucleic acid in a sample to about 40% of the total nucleic acid in a sample. In some implementations, a minority nucleic acid can have an abundance of at least about 2% of the total nucleic acid in a sample to about 30% of the total nucleic acid in a sample. In some implementations, a minority nucleic acid can have an abundance of at least about 3% of the total nucleic acid in a sample to about 25% of the total nucleic acid in a sample. For example, a minority nucleic acid can have an abundance of about 1 %, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% of the total nucleic acid in a sample. In some instances, a minority species of extracellular nucleic acid sometimes is about 1 % to about 40% of the overall nucleic acid (e.g., about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40% of the nucleic acid is minority species nucleic acid). In some implementations, the minority nucleic acid is extracellular DNA. In some implementations, the minority nucleic acid is extracellular DNA from apoptotic tissue. In some implementations, the minority nucleic acid is extracellular DNA from tissue where some cells therein underwent apoptosis. In some implementations, the minority nucleic acid is extracellular DNA from necrotic tissue. In some implementations, the minority nucleic acid is extracellular DNA from tissue where some cells therein underwent necrosis. Necrosis may refer to a post-mortem process following cell death, in certain instances. In some implementations, the minority nucleic acid is extracellular DNA from tissue affected by a cell proliferative disorder (e.g., cancer). In some implementations, the minority nucleic acid is extracellular DNA from a tumor cell. In some implementations, the minority nucleic acid is extracellular fetal DNA. In some implementations, the minority nucleic acid is extracellular DNA from a pathogen. In some implementations, the minority nucleic acid is extracellular DNA from a transplant. In some implementations, the minority nucleic acid is extracellular DNA from a microbiome.

In another aspect, a nucleic acid that is considered "majority," for example, can have an abundance greater than 50% of the total nucleic acid in a sample to about 99.9% of the total nucleic acid in a sample. In some implementations, a majority nucleic acid can have an abundance of at least about 60% of the total nucleic acid in a sample to about 99% of the total nucleic acid in a sample. In some implementations, a majority nucleic acid can have an abundance of at least about 70% of the total nucleic acid in a sample to about 98% of the total nucleic acid in a sample. In some implementations, a majority nucleic acid can have an abundance of at least about 75% of the total nucleic acid in a sample to about 97% of the total nucleic acid in a sample. For example, a majority nucleic acid can have an abundance of at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% of the total nucleic acid in a sample. In some implementations, the majority nucleic acid is extracellular DNA. In some implementations, the majority nucleic acid is extracellular maternal DNA. In some implementations, the majority nucleic acid is DNA from healthy tissue. In some implementations, the majority nucleic acid is DNA from non-tumor cells. In some implementations, the majority nucleic acid is DNA from host cells.

In some implementations, a minority species of extracellular nucleic acid is of a length of about 500 base pairs or less (e.g., about 80, 85, 90, 91, 92, 93, 94, 95, 96, 97, 98, 99 or 100% of minority species nucleic acid is of a length of about 500 base pairs or less). In some implementations, a minority species of extracellular nucleic acid is of a length of about 300 base pairs or less (e.g., about 80, 85, 90, 91, 92, 93, 94, 95, 96, 97, 98, 99 or 100% of minority species nucleic acid is of a length of about 300 base pairs or less). In some implementations, a minority species of extracellular nucleic acid is of a length of about 250 base pairs or less (e.g., about 80, 85, 90, 91, 92, 93, 94, 95, 96, 97, 98, 99 or 100% of minority species nucleic acid is of a length of about 250 base pairs or less). In some implementations, a minority species of extracellular nucleic acid is of a length of about 200 base pairs or less (e.g., about 80, 85, 90, 91, 92, 93, 94, 95, 96, 97, 98, 99 or 100% of minority species nucleic acid is of a length of about 200 base pairs or less). In some implementations, a minority species of extracellular nucleic acid is of a length of about 150 base pairs or less (e.g., about 80, 85, 90, 91, 92, 93, 94, 95, 96, 97, 98, 99 or 100% of minority species nucleic acid is of a length of about 150 base pairs or less). In some implementations, a minority species of extracellular nucleic acid is of a length of about 100 base pairs or less (e.g., about 80, 85, 90, 91, 92, 93, 94, 95, 96, 97, 98, 99 or 100% of minority species nucleic acid is of a length of about 100 base pairs or less). In some implementations, a minority species of extracellular nucleic acid is of a length of about 50 base pairs or less (e.g., about 80, 85, 90, 91, 92, 93, 94, 95, 96, 97, 98, 99 or 100% of minority species nucleic acid is of a length of about 50 base pairs or less).

Nucleic acid may be provided for conducting methods described herein with or without processing of the sample(s) containing the nucleic acid. In some implementations, nucleic acid is provided for conducting methods described herein after processing of the sample(s) containing the nucleic acid. For example, a nucleic acid can be extracted, isolated, purified, partially purified or amplified from the sample(s). The term "isolated" as used herein refers to nucleic acid removed from its original environment (e.g., the natural environment if it is naturally occurring, or a host cell if expressed exogenously), and thus is altered by human intervention (e.g., "by the hand of man") from its original environment. The term "isolated nucleic acid" as used herein can refer to a nucleic acid removed from a subject (e.g., a human subject). An isolated nucleic acid can be provided with fewer non-nucleic acid components (e.g., protein, lipid) than the amount of components present in a source sample. A composition comprising isolated nucleic acid can be about 50% to greater than 99% free of non-nucleic acid components. A composition comprising isolated nucleic acid can be about 90%, 91%, 92%, 93%, 94%, 95%, 96%, 97%, 98%, 99% or greater than 99% free of non-nucleic acid components. The term "purified" as used herein can refer to a nucleic acid provided that contains fewer non-nucleic acid components (e.g., protein, lipid, carbohydrate) than the amount of non-nucleic acid components present prior to subjecting the nucleic acid to a purification procedure. A composition comprising purified nucleic acid may be about 80%, 81%, 82%, 83%, 84%, 85%, 86%, 87%, 88%, 89%, 90%, 91%, 92%, 93%, 94%, 95%, 96%, 97%, 98%, 99% or greater than 99% free of other non-nucleic acid components. The term "purified" as used herein can refer to a nucleic acid provided that contains fewer nucleic acid species than in the sample source from which the nucleic acid is derived. A composition comprising purified nucleic acid may be about 90%, 91%, 92%, 93%, 94%, 95%, 96%, 97%, 98%, 99% or greater than 99% free of other nucleic acid species. For example, fetal nucleic acid can be purified from a mixture comprising maternal and fetal nucleic acid. In certain examples, small fragments of nucleic acid (e.g., 30 to 500 bp fragments) can be purified, or partially purified, from a mixture comprising nucleic acid fragments of different lengths. In certain examples, nucleosomes comprising smaller fragments of nucleic acid can be purified from a mixture of larger nucleosome complexes comprising larger fragments of nucleic acid. In certain examples, larger nucleosome complexes comprising larger fragments of nucleic acid can be purified from nucleosomes comprising smaller fragments of nucleic acid. In certain examples, small fragments of fetal nucleic acid (e.g., 30 to 500 bp fragments) can be purified, or partially purified, from a mixture comprising both fetal and maternal nucleic acid fragments. In certain examples, nucleosomes comprising smaller fragments of fetal nucleic acid can be purified from a mixture of larger nucleosome complexes comprising larger fragments of maternal nucleic acid. In certain examples, cancer cell nucleic acid can be purified from a mixture comprising cancer cell and non-cancer cell nucleic acid. In certain examples, nucleosomes comprising small fragments of cancer cell nucleic acid can be purified from a mixture of larger nucleosome complexes comprising larger fragments of non-cancer nucleic acid. In some implementations, nucleic acid is provided for conducting methods described herein without prior processing of the sample(s) containing the nucleic acid. For example, nucleic acid may be analyzed directly from a sample without prior extraction, purification, partial purification, and/or amplification.

Nucleic acids may be amplified under amplification conditions. The term "amplified" or "amplification" or "amplification conditions" as used herein refers to subjecting a target nucleic acid (e.g., ssNA) in a sample or a nucleic acid product generated by a method herein to a process that linearly or exponentially generates amplicon nucleic acids having the same or substantially the same nucleotide sequence as the target nucleic acid (e.g., ssNA), or part thereof. In certain implementations, the term "amplified" or "amplification" or "amplification conditions" refers to a method that comprises a polymerase chain reaction (PCR). In certain instances, an amplified product can contain one or more nucleotides more than the amplified nucleotide region of a nucleic acid template sequence (e.g., a primer can contain "extra" nucleotides such as a transcriptional initiation sequence, in addition to nucleotides complementary to a nucleic acid template gene molecule, resulting in an amplified product containing "extra" nucleotides or nucleotides not corresponding to the amplified nucleotide region of the nucleic acid template gene molecule).

Nucleic acid also may be exposed to a process that modifies certain nucleotides in the nucleic acid before providing nucleic acid for a method described herein. A process that selectively modifies nucleic acid based upon the methylation state of nucleotides therein can be applied to nucleic acid, for example. In addition, conditions such as high temperature, ultraviolet radiation, x-radiation, can induce changes in the sequence of a nucleic acid molecule. Nucleic acid may be provided in any suitable form useful for conducting a sequence analysis.

In some implementations, target nucleic acids (e.g., ssNAs) are not modified in prior to combining with the scaffold adapters herein, or components thereof. In some implementations, target nucleic acids (e.g., ssNAs) are not modified in length prior to combining with the scaffold adapters herein, or components thereof. In this context, "not modified" means that target nucleic acids are isolated from a sample and then combined with scaffold adapters, or components thereof, without modifying the length or the composition of the target nucleic acids. For example, target nucleic acids (e.g., ssNAs) may not be shortened (e.g., they are not contacted with a restriction enzyme or nuclease or physical condition that reduces length (e.g., shearing condition, cleavage condition)) and may not be increased in length by one or more nucleotides (e.g., ends are not filled in at overhangs; no nucleotides are added to the ends). Adding a phosphate or chemically reactive group to one or both ends of a target nucleic acid (e.g., ssNA) generally is not considered modifying the nucleic acid or modifying the length of the nucleic acid. Denaturing a double-stranded nucleic acid (dsNA) fragment to generate an ssNA fragment generally is not considered modifying the nucleic acid or modifying the length of the nucleic acid.

In some implementations, one or both native ends of target nucleic acids (e.g., ssNAs) are present when the ssNA is combined with the scaffold adapters herein, or components thereof. Native ends generally refer to unmodified ends of a nucleic acid fragment. In some implementations, native ends of target nucleic acids (e.g., ssNAs) are not modified in length prior to combining with the scaffold adapters herein, or components thereof. In this context, "not modified" means that target nucleic acids are isolated from a sample and then combined with scaffold adapters, or components thereof, without modifying the length of the native ends of target nucleic acids. For example, target nucleic acids (e.g., ssNAs) are not shortened (e.g., they are not contacted with a restriction enzyme or nuclease or physical condition that reduces length (e.g., shearing condition, cleavage condition) to generate non-native ends) and are not increased in length by one or more nucleotides (e.g., native ends are not filled in at overhangs; no nucleotides are added to the native ends). Adding a phosphate or chemically reactive group to one or both native ends of a target nucleic acid generally is not considered modifying the length of the nucleic acid.

In some implementations, target nucleic acids (e.g., ssNAs) are not contacting with a cleavage agent (e.g., endonuclease, exonuclease, restriction enzyme) and/or a polymerase prior to combining with the scaffold adapters herein, or components thereof. In some implementations, target nucleic acids are not subjected to mechanical shearing (e.g., ultrasonication (e.g., Adaptive Focused Acoustics^{™} (AFA) process by Covaris)) prior to combining with the scaffold adapters herein, or components thereof. In some implementations, target nucleic acids are not contacting with an exonuclease (e.g., DNAse) prior to combining with the scaffold adapters herein, or components thereof. In some implementations, target nucleic acids are not amplified prior to combining with the scaffold adapters herein, or components thereof. In some implementations, target nucleic acids are not attached to a solid support prior to combining with the scaffold adapters herein, or components thereof. In some implementations, target nucleic acids are not conjugated to another molecule prior to combining with the scaffold adapters herein, or components thereof. In some implementations, target nucleic acids are not cloned into a vector prior to combining with the scaffold adapters herein, or components thereof. In some implementations, target nucleic acids may be subjected to dephosphorylation prior to combining with the scaffold adapters herein, or components thereof. In some implementations, target nucleic acids may be subjected to phosphorylation prior to combining with the scaffold adapters herein, or components thereof.

In some implementations, combining target nucleic acids (e.g., ssNAs) with the scaffold adapters herein, or components thereof, comprises isolating the target nucleic acids, and combining the isolated target nucleic acids with the scaffold adapters herein, or components thereof. In some implementations, combining target nucleic acids with the scaffold adapters herein, or components thereof, comprises isolating the target nucleic acids, phosphorylating the isolated target nucleic acids, and combining the phosphorylated target nucleic acids with the scaffold adapters herein, or components thereof. In some implementations, combining target nucleic acids with the scaffold adapters herein, or components thereof, comprises isolating the target nucleic acids, dephosphorylating the scaffold adapters herein, or components thereof, and combining the isolated target nucleic acids with the dephosphorylated scaffold adapters herein, or dephosphorylated components thereof. In some implementations, combining target nucleic acids with the scaffold adapters herein, or components thereof, comprises isolating the target nucleic acids, dephosphorylating the isolated target nucleic acids, phosphorylating the dephosphorylated target nucleic acids, and combining the phosphorylated target nucleic acids with the scaffold adapters herein, or components thereof. In some implementations, combining target nucleic acids with the scaffold adapters herein, or components thereof, comprises isolating the target nucleic acids, dephosphorylating the isolated target nucleic acids, phosphorylating the dephosphorylated target nucleic acids, dephosphorylating the scaffold adapters, or components thereof, and combining the phosphorylated target nucleic acids with the dephosphorylated scaffold adapters herein, or dephosphorylated components thereof.

In some implementations, combining target nucleic acids (e.g., ssNAs) with the scaffold adapters herein, or components thereof, consists of isolating the target nucleic acids, and combining the isolated target nucleic acids with the scaffold adapters herein, or components thereof. In some implementations, combining target nucleic acids with the scaffold adapters herein, or components thereof, consists of isolating the target nucleic acids, phosphorylating the isolated target nucleic acids, and combining the phosphorylated target nucleic acids with the scaffold adapters herein, or components thereof. In some implementations, combining target nucleic acids with the scaffold adapters herein, or components thereof, consists of isolating the target nucleic acids, dephosphorylating the scaffold adapters, or components thereof, and combining the isolated target nucleic acids with the dephosphorylated scaffold adapters herein, or dephosphorylated components thereof. In some implementations, combining target nucleic acids with the scaffold adapters herein, or components thereof, consists of isolating the target nucleic acids, dephosphorylating the isolated target nucleic acids, phosphorylating the dephosphorylated target nucleic acids, and combining the phosphorylated target nucleic acids with the scaffold adapters herein, or components thereof. In some implementations, combining target nucleic acids with the scaffold adapters herein, or components thereof, consists of isolating the target nucleic acids, dephosphorylating the isolated target nucleic acids, phosphorylating the dephosphorylated target nucleic acids, dephosphorylating the scaffold adapters, or components thereof, and combining the phosphorylated target nucleic acids with the dephosphorylated scaffold adapters herein, or dephosphorylated components thereof.

### Single-stranded nucleic acid

Provided herein are methods and compositions for capturing single-stranded nucleic acid (ssNA) using specialized adapters (e.g., for generating a sequencing library). Single-stranded nucleic acid or ssNA generally refers to a collection of polynucleotides which are single-stranded (i.e., not hybridized intermolecularly or intramolecularly) over 70% or more of their length. In some implementations, ssNA is single-stranded over 75% or more, 80% or more, 85% or more, 90% or more, 95% or more, or 99% or more, of the length of the polynucleotides. In certain aspects, the ssNA is single-stranded over the entire length of the polynucleotides. Single-stranded nucleic acid may be referred to herein as target nucleic acid.

ssNA may include single-stranded deoxyribonucleic acid (ssDNA). In some implementations, ssDNA includes, but is not limited to, ssDNA derived from double-stranded DNA (dsDNA). For example, ssDNA may be derived from double-stranded DNA which is denatured (e.g., heat denatured and/or chemically denatured) to produce ssDNA. In some implementations, a method herein comprises, prior to combining ssDNA with scaffold adapters described herein, or components thereof, generating the ssDNA by denaturing dsDNA.

In some implementations, ssNA includes single-stranded ribonucleic acid (ssRNA). RNA may include, for example, messenger RNA (mRNA), microRNA (miRNA), small interfering RNA (siRNA), transacting small interfering RNA (ta-siRNA), natural small interfering RNA (nat-siRNA), ribosomal RNA (rRNA), transfer RNA (tRNA), small nucleolar RNA (snoRNA), small nuclear RNA (snRNA), long non-coding RNA (IncRNA), non-coding RNA (ncRNA), transfer-messenger RNA (tmRNA), precursor messenger RNA (pre-mRNA), small Cajal body-specific RNA (scaRNA), piwi-interacting RNA (piRNA), endoribonucleaseprepared siRNA (esiRNA), small temporal RNA (stRNA), signal recognition RNA, telomere RNA, ribozyme, or a combination thereof. In some implementations, when the ssNA is ssRNA, the ssRNA is mRNA. In some implementations, ssNA includes single stranded complementary DNA (cDNA).

In some implementations, a method herein comprises contacting ssNA with single-stranded nucleic acid binding protein (SSB) to produce SSB-bound ssNA. SSB generally binds in a cooperative manner to ssNA and typically does not bind well to double-stranded nucleic acid (dsNA). Upon binding ssDNA, SSB destabilizes helical duplexes. SSBs may be prokaryotic SSB (e.g., bacterial or archaeal SSB) or eukaryotic SSB. Examples of SSBs may include E. coli SSB, E. coli RecA, Extreme Thermostable Single-Stranded DNA Binding Protein (ET SSB), Thermus thermophilus (Tth) RecA, T4 Gene 32 Protein, replication protein A (RPA - a eukaryotic SSB), and the like. ET SSB, Tth RecA, E. coli RecA, T4 Gene 32 Protein, as well buffers and detailed protocols for preparing SSB-bound ssNA using such SSBs are commercially available (e.g., New England Biolabs, Inc. (Ipswich, MA)).

In some implementations, a method herein does not comprise contacting ssNA with single-stranded nucleic acid binding protein (SSB) to produce SSB-bound ssNA. Accordingly, a method herein may omit the step of producing SSB-bound ssNA. For example, a method herein may comprise combining ssNA with scaffold adapters described herein, or components thereof, without contacting the ssNA with SSB. In such instances, a method herein may be referred to an "SSB-free" method for producing a nucleic acid library. Certain SSB-free methods described herein may produce libraries having parameters similar to parameters for libraries prepared using SSB, as shown in the Drawings and discussed in the Examples. In some implementations, a method herein comprises contacting ssNA with a single-stranded nucleic acid binding agent other than SSB. Such single-stranded nucleic acid binding agents can stably bind single stranded nucleic acids, can prevent or reduce formation of nucleic acid duplexes, can still allow the bound nucleic acids to be ligated or otherwise terminally modified, and can be thermostable. Example single-stranded nucleic acid binding agents include but are not limited to topoisomerases, helicases, domains thereof, and fusion proteins comprising domains thereof.

In some implementations, a method herein comprises combining a nucleic acid composition comprising single-stranded nucleic acid (ssNA) with scaffold adapters described herein, or components thereof. In some implementations, a method herein comprises combining a nucleic acid composition consisting of single-stranded nucleic acid (ssNA) with scaffold adapters described herein, or components thereof. In some implementations, a method herein comprises combining a nucleic acid composition consisting essentially of single-stranded nucleic acid (ssNA) with scaffold adapters described herein, or components thereof. A nucleic acid composition "consisting essentially of" single-stranded nucleic acid (ssNA) generally includes ssNA and no additional protein or nucleic acid components. For example, a nucleic acid composition "consisting essentially of" single-stranded nucleic acid (ssNA) may exclude double-stranded nucleic acid (dsNA) or may include a low percentage of dsNA (e.g., less than 10% dsNA, less than 5% dsNA, less than 1% dsNA). A nucleic acid composition "consisting essentially of" single-stranded nucleic acid (ssNA) may exclude proteins. For example, a nucleic acid composition "consisting essentially of" single-stranded nucleic acid (ssNA) may exclude single-stranded binding proteins (SSBs) or other proteins useful for stabilizing ssNA. A nucleic acid composition "consisting essentially of" single-stranded nucleic acid (ssNA) may include chemical components typically present in nucleic acid compositions such as buffers, salts, alcohols, crowding agents (e.g., PEG), and the like; and may include residual components (e.g., nucleic acids, proteins, cell membrane components) from the nucleic acid source (e.g., sample) or nucleic acid extraction. A nucleic acid composition "consisting essentially of" single-stranded nucleic acid (ssNA) may include ssNA fragments having one or more phosphates (e.g., a terminal phosphate, a 5' terminal phosphate). A nucleic acid composition "consisting essentially of" single-stranded nucleic acid (ssNA) may include ssNA fragments comprising one or more modified nucleotides.

### Enriching nucleic acids

In some implementations, nucleic acid (e.g., extracellular nucleic acid) is enriched or relatively enriched for a subpopulation or species of nucleic acid. Nucleic acid subpopulations can include, for example, fetal nucleic acid, maternal nucleic acid, cancer nucleic acid, tumor nucleic acid, patient nucleic acid, host nucleic acid, pathogen nucleic acid, transplant nucleic acid, microbiome nucleic acid, nucleic acid comprising fragments of a particular length or range of lengths, or nucleic acid from a particular genome region (e.g., single chromosome, set of chromosomes, and/or certain chromosome regions). Such enriched samples can be used in conjunction with a method provided herein. Thus, in certain implementations, methods of the technology comprise an additional step of enriching for a subpopulation of nucleic acid in a sample. In certain implementations, nucleic acid from normal tissue (e.g., non-cancer cells, host cells) is selectively removed (partially, substantially, almost completely or completely) from the sample. In certain implementations, maternal nucleic acid is selectively removed (partially, substantially, almost completely or completely) from the sample. In certain implementations, enriching for a particular low copy number species nucleic acid (e.g., cancer, tumor, fetal, pathogen, transplant, microbiome nucleic acid) may improve quantitative sensitivity. Methods for enriching a sample for a particular species of nucleic acid are described, for example, in U.S. Patent No. 6,927,028, International Patent Application Publication No. WO2007/140417, International Patent Application Publication No. WO2007/147063, International Patent Application Publication No. WO2009/032779, International Patent Application Publication No. WO2009/032781, International Patent Application Publication No. WO2010/033639, International Patent Application Publication No. WO2011/034631, International Patent Application Publication No. WO2006/056480, and International Patent Application Publication No. WO2011/143659.

In some implementations, nucleic acid is enriched for certain target fragment species and/or reference fragment species. In certain implementations, nucleic acid is enriched for a specific nucleic acid fragment length or range of fragment lengths using one or more length-based separation methods described below. In certain implementations, nucleic acid is enriched for fragments from a select genomic region (e.g., chromosome) using one or more sequence-based separation methods described herein and/or known in the art.

Non-limiting examples of methods for enriching for a nucleic acid subpopulation in a sample include methods that exploit epigenetic differences between nucleic acid species (e.g., methylation-based fetal nucleic acid enrichment methods described in U.S. Patent Application Publication No. 2010/0105049); restriction endonuclease enhanced polymorphic sequence approaches (e.g., such as a method described in U.S. Patent Application Publication No. 2009/0317818); selective enzymatic degradation approaches; massively parallel signature sequencing (MPSS) approaches; amplification (e.g., PCR)-based approaches (e.g., loci-specific amplification methods, multiplex SNP allele PCR approaches; universal amplification methods); pull-down approaches (e.g., biotinylated ultramer pull-down methods); extension and ligation-based methods (e.g., molecular inversion probe (MIP) extension and ligation); and combinations thereof.

In some implementations, modified nucleic acids can be enriched for. Nucleic acid modifications include but are not limited to carboxycytosine, 5-methylcytosine (5mC) and its oxidative derivatives (e.g., 5-hydroxymethylcytosine (5hmC), 5-formylcytosine (5fC), and 5-arboxylcytosine (5caC)), N(6)-methyladenine (6mA), N4-methylcytosine (4mC), N(6)-methyladenosine (m(6)A), pseudouridine (ψ), 5-methylcytidine (m(5)C), hydroxymethyl uracil, 2'-O-methylation at the 3' end, tRNA modifications, miRNA modifications, and snRNA modifications. Nucleic acids comprising one or more modifications can be enriched for by a variety of methods, including but not limited to antibody-based pulldown. Modified nucleic acid enrichment can be conducted before (e.g., FIG. 52A-B) or after (e.g., FIG. 52C-D) denaturation of dsDNA. Enrichment prior to denaturation can result in also enriching for the complementary strand which may lack the modification, while enrichment after denaturation does not enrich for complementary strands lacking modification.

In some implementations, nucleic acid is enriched for fragments from a select genomic region (e.g., chromosome) using one or more sequence-based separation methods described herein. Sequence-based separation generally is based on nucleotide sequences present in the fragments of interest (e.g., target and/or reference fragments) and substantially not present in other fragments of the sample or present in an insubstantial amount of the other fragments (e.g., 5% or less). In some implementations, sequence-based separation can generate separated target fragments and/or separated reference fragments. Separated target fragments and/or separated reference fragments often are isolated away from the remaining fragments in the nucleic acid sample. In certain implementations, the separated target fragments and the separated reference fragments also are isolated away from each other (e.g., isolated in separate assay compartments). In certain implementations, the separated target fragments and the separated reference fragments are isolated together (e.g., isolated in the same assay compartment). In some implementations, unbound fragments can be differentially removed or degraded or digested.

In some implementations, scaffold adapters are used to enrich for target nucleic acids. As shown for example in FIG. 51, scaffold adapters can be designed such that some or all of the bases in the ssNA hybridization region are defined or known bases. These scaffold adapters can hybridize preferentially to target nucleic acids with sequences complementary to the defined or known bases of the scaffold adapter ssNA hybridization region, thereby enriching for the target nucleic acids in the resulting library. For example, as shown in FIG. 51, including a GC dinucleotide in the ssNA hybridization region can be used to enrich for target nucleic acids that have terminal CG (also called CpG) dinucleotides. Any other defined sequence can be targeted in a similar manner, using some or all of the length of the scaffold adapter ssNA hybridization region, including but not limited to nuclease cleavage sites, gene promoter regions, pathogen sequences, tumor-related sequences, and other motifs. In an example, libraries were prepared using non-enriching scaffold adapters and CG dinucleotide enriching scaffold adapters. For libraries prepared without enrichment, 1.7% of reads started with CG and 1.1% of reads ended with CG. For libraries prepared with enrichment, 5.2% of reads started with CG and 19.6% of reads ended with CG. In another example, shown in FIG. 55, a sample comprising RNA (e.g., host and pathogen RNA) is reverse transcribed with primers specific to pathogen RNA of interest to generate cDNA; the cDNA is then purified and prepared with single-stranded library preparation methods as discussed herein, either with standard scaffold adapters or with scaffold adapters with ssNA hybridization regions targeted to the regions enriched by the reverse transcription primers. Pathogenic DNA can be similarly enriched.

In some instances, the target nucleic acid sequence at the 5' or 3' nucleic acid termini is defined or known. In other instances, scaffold adapters can be used to identify novel targets of interest at 5' or 3' nucleic acid termini. Nucleic acid sequences or patterns of interest may be characterized from the scaffold adapter library output with or without enrichment. In some instances, a specific sequence or sequence pattern at 5', 3', or both nucleic acid termini may be associated with a particular state. Such states include but are not limited to disease state, methylation state, and gene expression state. The scaffold adapters can be used to quantify the presence or relative abundance of a known or novel target sequence(s) at nucleic acid termini between samples and controls, for example, cell-free DNA from cancer patients and healthy controls. These data can be used to learn the relationship between the sequence information at DNA termini and a given state. By training on a well-characterized dataset of patient and healthy samples, in one example, an analytical method or algorithm can be used to predict the state or transitions through the state. For example, we observe the increase of AT dinucleotides and reduction of CpG dinucleotides at 5' and 3' DNA termini in cfDNA from patients with Acute Myeloid leukemia (AML) when compared to non-AML patient samples. In this example, an analytical tool may be used cfDNA termini sequence information to predict a person's risk for developing AML.

In some implementations, a selective nucleic acid capture process is used to separate target and/or reference fragments away from a nucleic acid sample. Commercially available nucleic acid capture systems include, for example, Nimblegen sequence capture system (Roche NimbleGen, Madison, Wl); ILLUMINA BEADARRAY platform (Illumina, San Diego, CA); Affymetrix GENECHIP platform (Affymetrix, Santa Clara, CA); Agilent SureSelect Target Enrichment System (Agilent Technologies, Santa Clara, CA); and related platforms. Such methods typically involve hybridization of a capture oligonucleotide to a part or all of the nucleotide sequence of a target or reference fragment and can include use of a solid phase (e.g., solid phase array) and/or a solution based platform. Capture oligonucleotides (sometimes referred to as "bait") can be selected or designed such that they preferentially hybridize to nucleic acid fragments from selected genomic regions or loci, or a particular sequence in a nucleic acid target. In certain implementations, a hybridization-based method (e.g., using oligonucleotide arrays) can be used to enrich for fragments containing certain nucleic acid sequences. Thus, in some implementations, a nucleic acid sample is optionally enriched by capturing a subset of fragments using capture oligonucleotides complementary to, for example, selected sequences in sample nucleic acid. In certain instances, captured fragments are amplified. For example, captured fragments containing adapters may be amplified using primers complementary to the adapter sequences to form collections of amplified fragments, indexed according to adapter sequence. In some implementations, nucleic acid is enriched for fragments from a select genomic region (e.g., chromosome, a gene) by amplification of one or more regions of interest using oligonucleotides (e.g., PCR primers) complementary to sequences in fragments containing the region(s) of interest, or part(s) thereof.

In some implementations, nucleic acid is enriched for a particular nucleic acid fragment length, range of lengths, or lengths under or over a particular threshold or cutoff using one or more length-based separation methods. Nucleic acid fragment length typically refers to the number of nucleotides in the fragment. Nucleic acid fragment length also is sometimes referred to as nucleic acid fragment size. In some implementations, a length-based separation method is performed without measuring lengths of individual fragments. In some implementations, a length based separation method is performed in conjunction with a method for determining length of individual fragments. In some implementations, length-based separation refers to a size fractionation procedure where all or part of the fractionated pool can be isolated (e.g., retained) and/or analyzed. Size fractionation procedures are known in the art (e.g., separation on an array, separation by a molecular sieve, separation by gel electrophoresis, separation by column chromatography (e.g., size-exclusion columns), and microfluidics-based approaches). In certain instances, length-based separation approaches can include selective sequence tagging approaches, fragment circularization, chemical treatment (e.g., formaldehyde, polyethylene glycol (PEG) precipitation), mass spectrometry and/or size-specific nucleic acid amplification, for example.

In some implementations, nucleic acid is enriched for fragments associated with one or more nucleic acid binding proteins. Example enrichment methods include but are not limited to chromatin immunoprecipitation (ChIP), cross-linked ChIP (XCHIP), native ChIP (NChIP), bead-free ChIP, carrier ChIP (CChIP), fast ChIP (qChIP), quick and quantitative ChIP (Q²ChIP), microchip (µChIP), matrix ChIP, pathology-ChIP (PAT-ChIP), ChIP-exo, ChIP-on-chip, RIP-ChIP, HiChIP, ChlA-PET, and HiChIRP.

In some implementations, a method herein includes enriching an RNA species in a mixture of RNA species. For example, a method herein may comprise enriching messenger RNA (mRNA) present in a mixture of mRNA and ribosomal RNA (rRNA). Any suitable mRNA enrichment method may be used, which includes rRNA depletion and/or mRNA enrichment methods such as rRNA depletion with magnetic beads (e.g., Ribo-zero^{™}, Ribominus^{™}, and MICROBExpress^{™}, which use rRNA depletion probes in combination with magnetic beads to deplete rRNAs from a sample, thus enriching mRNAs), oligo(dT)-based poly(A) enrichment (e.g., BioMag^{®} Oligo (dT)20), nuclease-based rRNA depletion (e.g., digestion of rRNA with Terminator^{™} 5'-Phosphate Dependent Exonuclease), and combinations thereof.

Enrichment strategies can increase the relative abundance (e.g., as assessed by percent of sequencing reads) of the targeted nucleic acids by at least 5%, 10%, 20%, 30%, 40%, 50%, 60%, 70%, 80%, 90%, 100%, 200%, 300%, 400%, 500%, 600%, 700%, 800%, 900%, 1000%, 1100%, 1200%, 1300%, 1400%, 1500%, 1600%, 1700%, 1800%, 1900%, 2000%, 3000%, 4000%, 5000%, 6000%, 7000%, 8000%, 9000%, 10000%, or more.

### Length-based separation

In some implementations, a method herein comprises separating target nucleic acids (e.g., ssNAs) according to fragment length. For example, target nucleic acids (e.g., ssNAs) may be enriched for a particular nucleic acid fragment length, range of lengths, or lengths under or over a particular threshold or cutoff using one or more length-based separation methods. Nucleic acid fragment length typically refers to the number of nucleotides in the fragment. Nucleic acid fragment length also may be referred to as nucleic acid fragment size. In some implementations, a length-based separation method is performed without measuring lengths of individual fragments. In some implementations, a length based separation method is performed in conjunction with a method for determining length of individual fragments. In some implementations, length-based separation refers to a size fractionation procedure where all or part of the fractionated pool can be isolated (e.g., retained) and/or analyzed. Size fractionation procedures are known in the art (e.g., separation on an array, separation by a molecular sieve, separation by gel electrophoresis, separation by column chromatography (e.g., size-exclusion columns), and microfluidics-based approaches). In some implementations, length-based separation approaches can include fragment circularization, chemical treatment (e.g., formaldehyde, polyethylene glycol (PEG)), mass spectrometry and/or size-specific nucleic acid amplification, for example. In some implementations, length based-separation is performed using Solid Phase Reversible Immobilization (SPRI) beads.

In some implementations, nucleic acid fragments of a certain length, range of lengths, or lengths under or over a particular threshold or cutoff are separated from the sample. In some implementations, fragments having a length under a particular threshold or cutoff (e.g., 500 bp, 400 bp, 300 bp, 200 bp, 150 bp, 100 bp) are referred to as "short" fragments and fragments having a length over a particular threshold or cutoff (e.g., 500 bp, 600 bp, 700 bp, 800 bp, 900 bp, 1000 bp) are referred to as "long" fragments, large fragments, and/or high molecular weight (HMW) fragments. In some implementations, fragments of a certain length, range of lengths, or lengths under or over a particular threshold or cutoff are retained for analysis while fragments of a different length or range of lengths, or lengths over or under the threshold or cutoff are not retained for analysis. In some implementations, fragments that are less than about 500 bp are retained. In some implementations, fragments that are less than about 400 bp are retained. In some implementations, fragments that are less than about 300 bp are retained. In some implementations, fragments that are less than about 200 bp are retained. In some implementations, fragments that are less than about 150 bp are retained. For example, fragments that are less than about 190 bp, 180 bp, 170 bp, 160 bp, 150 bp, 140 bp, 130 bp, 120 bp, 110 bp or 100 bp are retained. In some implementations, fragments that are about 100 bp to about 200 bp are retained. For example, fragments that are about 190 bp, 180 bp, 170 bp, 160 bp, 150 bp, 140 bp, 130 bp, 120 bp or 110 bp are retained. In some implementations, fragments that are in the range of about 100 bp to about 200 bp are retained. For example, fragments that are in the range of about 110 bp to about 190 bp, 130 bp to about 180 bp, 140 bp to about 170 bp, 140 bp to about 150 bp, 150 bp to about 160 bp, or 145 bp to about 155 bp are retained.

In some implementations, target nucleic acids (e.g., ssNAs) having fragment lengths of less than about 1000 bp are combined with a plurality or pool of scaffold adapter species, or components of scaffold adapter species, described herein. In some implementations, target nucleic acids (e.g., ssNAs) having fragment lengths of less than about 500 bp are combined with a plurality or pool of scaffold adapter species, or components of scaffold adapter species, described herein. In some implementations, target nucleic acids (e.g., ssNAs) having fragment lengths of less than about 400 bp are combined with a plurality or pool of scaffold adapter species, or components of scaffold adapter species, described herein. In some implementations, target nucleic acids (e.g., ssNAs) having fragment lengths of less than about 300 bp are combined with a plurality or pool of scaffold adapter species, or components of scaffold adapter species, described herein. In some implementations, target nucleic acids (e.g., ssNAs) having fragment lengths of less than about 200 bp are combined with a plurality or pool of scaffold adapter species, or components of scaffold adapter species, described herein. In some implementations, target nucleic acids (e.g., ssNAs) having fragment lengths of less than about 100 bp are combined with a plurality or pool of scaffold adapter species, or components of scaffold adapter species, described herein.

In some implementations, target nucleic acids (e.g., ssNAs) having fragment lengths of about 100 bp or more are combined with a plurality or pool of scaffold adapter species, or components of scaffold adapter species, described herein. In some implementations, target nucleic acids (e.g., ssNAs) having fragment lengths of about 200 bp or more are combined with a plurality or pool of scaffold adapter species, or components of scaffold adapter species, described herein. In some implementations, target nucleic acids (e.g., ssNAs) having fragment lengths of about 300 bp or more are combined with a plurality or pool of scaffold adapter species, or components of scaffold adapter species, described herein. In some implementations, target nucleic acids (e.g., ssNAs) having fragment lengths of about 400 bp or more are combined with a plurality or pool of scaffold adapter species, or components of scaffold adapter species, described herein. In some implementations, target nucleic acids (e.g., ssNAs) having fragment lengths of about 500 bp or more are combined with a plurality or pool of scaffold adapter species, or components of scaffold adapter species, described herein. In some implementations, target nucleic acids (e.g., ssNAs) having fragment lengths of about 1000 bp or more are combined with a plurality or pool of scaffold adapter species, or components of scaffold adapter species, described herein.

In some implementations, target nucleic acids (e.g., ssNAs) having any fragment length or any combination of fragment lengths are combined with a plurality or pool of scaffold adapter species, or components of scaffold adapter species, described herein. For example, target nucleic acids (e.g., ssNAs) having fragment lengths of less than 500 bp and fragments lengths of 500 bp or more may be combined with a plurality or pool of scaffold adapter species, or components of scaffold adapter species, described herein.

Certain length-based separation methods that can be used with methods described herein employ a selective sequence tagging approach, for example. In such methods, a fragment size species (e.g., short fragments) nucleic acids are selectively tagged in a sample that includes long and short nucleic acids. Such methods typically involve performing a nucleic acid amplification reaction using a set of nested primers which include inner primers and outer primers. In some implementations, one or both of the inner can be tagged to thereby introduce a tag onto the target amplification product. The outer primers generally do not anneal to the short fragments that carry the (inner) target sequence. The inner primers can anneal to the short fragments and generate an amplification product that carries a tag and the target sequence. Typically, tagging of the long fragments is inhibited through a combination of mechanisms which include, for example, blocked extension of the inner primers by the prior annealing and extension of the outer primers. Enrichment for tagged fragments can be accomplished by any of a variety of methods, including for example, exonuclease digestion of single stranded nucleic acid and amplification of the tagged fragments using amplification primers specific for at least one tag.

Another length-based separation method that can be used with methods described herein involves subjecting a nucleic acid sample to polyethylene glycol (PEG) precipitation. Examples of methods include those described in International Patent Application Publication Nos. WO2007/140417 and WO2010/115016. This method in general entails contacting a nucleic acid sample with PEG in the presence of one or more monovalent salts under conditions sufficient to substantially precipitate large nucleic acids without substantially precipitating small (e.g., less than 300 nucleotides) nucleic acids.

Another length-based enrichment method that can be used with methods described herein involves circularization by ligation, for example, using circligase. Short nucleic acid fragments typically can be circularized with higher efficiency than long fragments. Non-circularized sequences can be separated from circularized sequences, and the enriched short fragments can be used for further analysis.

### Nucleic acid library

Methods herein may include preparing a nucleic acid library and/or modifying nucleic acids for a nucleic acid library. In some implementations, ends of nucleic acid fragments are modified such that the fragments, or amplified products thereof, may be incorporated into a nucleic acid library. Generally, a nucleic acid library refers to a plurality of polynucleotide molecules (e.g., a sample of nucleic acids) that are prepared, assembled and/or modified for a specific process, non-limiting examples of which include immobilization on a solid phase (e.g., a solid support, a flow cell, a bead), enrichment, amplification, cloning, detection and/or for nucleic acid sequencing. In certain implementations, a nucleic acid library is prepared prior to or during a sequencing process. A nucleic acid library (e.g., sequencing library) can be prepared by a suitable method as known in the art. A nucleic acid library can be prepared by a targeted or a non-targeted preparation process.

In some implementations, a library of nucleic acids is modified to comprise a chemical moiety (e.g., a functional group) configured for immobilization of nucleic acids to a solid support. In some implementations a library of nucleic acids is modified to comprise a biomolecule (e.g., a functional group) and/or member of a binding pair configured for immobilization of the library to a solid support, non-limiting examples of which include thyroxin-binding globulin, steroid-binding proteins, antibodies, antigens, haptens, enzymes, lectins, nucleic acids, repressors, protein A, protein G, avidin, streptavidin, biotin, complement component C1q, nucleic acid-binding proteins, receptors, carbohydrates, oligonucleotides, polynucleotides, complementary nucleic acid sequences, the like and combinations thereof. Some examples of specific binding pairs include, without limitation: an avidin moiety and a biotin moiety; an antigenic epitope and an antibody or immunologically reactive fragment thereof; an antibody and a hapten; a digoxigenin moiety and an anti-digoxigenin antibody; a fluorescein moiety and an anti-fluorescein antibody; an operator and a repressor; a nuclease and a nucleotide; a lectin and a polysaccharide; a steroid and a steroid-binding protein; an active compound and an active compound receptor; a hormone and a hormone receptor; an enzyme and a substrate; an immunoglobulin and protein A; an oligonucleotide or polynucleotide and its corresponding complement; the like or combinations thereof.

In some implementations, a library of nucleic acids is modified to comprise one or more polynucleotides of known composition, non-limiting examples of which include an identifier (e.g., a tag, an indexing tag), a capture sequence, a label, an adapter, a restriction enzyme site, a promoter, an enhancer, an origin of replication, a stem loop, a complimentary sequence (e.g., a primer binding site, an annealing site), a suitable integration site (e.g., a transposon, a viral integration site), a modified nucleotide, a unique molecular identifier (UMI) described herein, a palindromic sequence described herein, the like or combinations thereof. Polynucleotides of known sequence can be added at a suitable position, for example on the 5' end, 3' end or within a nucleic acid sequence. Polynucleotides of known sequence can be the same or different sequences. In some implementations, a polynucleotide of known sequence is configured to hybridize to one or more oligonucleotides immobilized on a surface (e.g., a surface in flow cell). For example, a nucleic acid molecule comprising a 5' known sequence may hybridize to a first plurality of oligonucleotides while the 3' known sequence may hybridize to a second plurality of oligonucleotides. In some implementations, a library of nucleic acid can comprise chromosome-specific tags, capture sequences, labels and/or adapters (e.g., oligonucleotide adapters described herein). In some implementations, a library of nucleic acids comprises one or more detectable labels. In some implementations one or more detectable labels may be incorporated into a nucleic acid library at a 5' end, at a 3' end, and/or at any nucleotide position within a nucleic acid in the library. In some implementations, a library of nucleic acids comprises hybridized oligonucleotides. In certain implementations hybridized oligonucleotides are labeled probes. In some implementations, a library of nucleic acids comprises hybridized oligonucleotide probes prior to immobilization on a solid phase.

In some implementations, a polynucleotide of known sequence comprises a universal sequence. A universal sequence is a specific nucleotide sequence that is integrated into two or more nucleic acid molecules or two or more subsets of nucleic acid molecules where the universal sequence is the same for all molecules or subsets of molecules that it is integrated into. A universal sequence is often designed to hybridize to and/or amplify a plurality of different sequences using a single universal primer that is complementary to a universal sequence. In some implementations two (e.g., a pair) or more universal sequences and/or universal primers are used. A universal primer often comprises a universal sequence. In some implementations adapters (e.g., universal adapters) comprise universal sequences. In some implementations one or more universal sequences are used to capture, identify and/or detect multiple species or subsets of nucleic acids.

In certain implementations of preparing a nucleic acid library, (e.g., in certain sequencing by synthesis procedures), nucleic acids are size selected and/or fragmented into lengths of several hundred base pairs, or less (e.g., in preparation for library generation). In some implementations, library preparation is performed without fragmentation (e.g., when using cell-free DNA).

In certain implementations, a ligation-based library preparation method is used (e.g., ILLUMINA TRUSEQ, Illumina, San Diego CA). Ligation-based library preparation methods often make use of an adapter (e.g., a methylated adapter) design which can incorporate an index sequence (e.g., a sample index sequence to identify sample origin for a nucleic acid sequence) at the initial ligation step and often can be used to prepare samples for single-read sequencing, paired-end sequencing and multiplexed sequencing. For example, nucleic acids (e.g., fragmented nucleic acids or cell-free DNA) may be end repaired by a fill-in reaction, an exonuclease reaction or a combination thereof. In some implementations, the resulting blunt-end repaired nucleic acid can then be extended by a single nucleotide, which is complementary to a single nucleotide overhang on the 3' end of an adapter/primer. Any nucleotide can be used for the extension/overhang nucleotides. In some implementations, end repair is omitted and scaffold adapters (e.g., scaffold adapters described herein) are ligated directly to the native ends of nucleic acids (e.g., single-stranded nucleic acids, fragmented nucleic acids, and/or cell-free DNA).

In some implementations, nucleic acid library preparation comprises ligating a scaffold adapter, or component thereof, (e.g., to a sample nucleic acid, to a sample nucleic acid fragment, to a template nucleic acid, to a target nucleic acid, to an ssNA), such as a scaffold adapter described herein. Scaffold adapters, or components thereof, may comprise sequences complementary to flow-cell anchors, and sometimes are utilized to immobilize a nucleic acid library to a solid support, such as the inside surface of a flow cell, for example. In some implementations, a scaffold adapter, or component thereof, comprises an identifier, one or more sequencing primer hybridization sites (e.g., sequences complementary to universal sequencing primers, single end sequencing primers, paired end sequencing primers, multiplexed sequencing primers, and the like), or combinations thereof (e.g., adapter/sequencing, adapter/identifier, adapter/identifier/sequencing). In some implementations, a scaffold adapter, or component thereof, comprises one or more of primer annealing polynucleotide, also referred to herein as priming sequence or primer binding domain, (e.g., for annealing to flow cell attached oligonucleotides and/or to free amplification primers), an index polynucleotide (e.g., sample index sequence for tracking nucleic acid from different samples; also referred to as a sample ID), a barcode polynucleotide (e.g., single molecule barcode (SMB) for tracking individual molecules of sample nucleic acid that are amplified prior to sequencing; also referred to as a molecular barcode or a unique molecular identifier (UMI)). In some implementations, a primer annealing component (or priming sequence or primer binding domain) of a scaffold adapter, or component thereof, comprises one or more universal sequences (e.g., sequences complementary to one or more universal amplification primers). In some implementations, an index polynucleotide (e.g., sample index; sample ID) is a component of a scaffold adapter, or component thereof. In some implementations, an index polynucleotide (e.g., sample index; sample ID) is a component of a universal amplification primer sequence.

In some implementations, scaffold adapters, or components thereof, when used in combination with amplification primers (e.g., universal amplification primers) are designed generate library constructs comprising one or more of: universal sequences, molecular barcodes, sample ID sequences, spacer sequences, and a sample nucleic acid sequence (e.g., ssNA sequence). In some implementations, scaffold adapters, or components thereof, when used in combination with universal amplification primers are designed to generate library constructs comprising an ordered combination of one or more of: universal sequences, molecular barcodes, sample ID sequences, spacer sequences, and a sample nucleic acid sequence (e.g., ssNA sequence). For example, a library construct may comprise a first universal sequence, followed by a second universal sequence, followed by first molecular barcode, followed by a spacer sequence, followed by a template sequence (e.g., sample nucleic acid sequence; ssNA sequence), followed by a spacer sequence, followed by a second molecular barcode, followed by a third universal sequence, followed by a sample ID, followed by a fourth universal sequence. In some implementations, scaffold adapters, or components thereof, when used in combination with amplification primers (e.g., universal amplification primers) are designed generate library constructs for each strand of a template molecule (e.g., sample nucleic acid molecule; ssNA molecule). In some implementations, scaffold adapters are duplex adapters.

An identifier can be a suitable detectable label incorporated into or attached to a nucleic acid (e.g., a polynucleotide) that allows detection and/or identification of nucleic acids that comprise the identifier. In some implementations, an identifier is incorporated into or attached to a nucleic acid during a sequencing method (e.g., by a polymerase). In some implementations, an identifier is incorporated into or attached to a nucleic acid prior to a sequencing method (e.g., by an extension reaction, by an amplification reaction, by a ligation reaction). Non-limiting examples of identifiers include nucleic acid tags, nucleic acid indexes or barcodes, a radiolabel (e.g., an isotope), metallic label, a fluorescent label, a chemiluminescent label, a phosphorescent label, a fluorophore quencher, a dye, a protein (e.g., an enzyme, an antibody or part thereof, a linker, a member of a binding pair), the like or combinations thereof. In some implementations, an identifier (e.g., a nucleic acid index or barcode) is a unique, known and/or identifiable sequence of nucleotides or nucleotide analogues. In some implementations, identifiers are six or more contiguous nucleotides. A multitude of fluorophores are available with a variety of different excitation and emission spectra. Any suitable type and/or number of fluorophores can be used as an identifier. In some implementations 1 or more, 2 or more, 3 or more, 4 or more, 5 or more, 6 or more, 7 or more, 8 or more, 9 or more, 10 or more, 20 or more, 30 or more or 50 or more different identifiers are utilized in a method described herein (e.g., a nucleic acid detection and/or sequencing method). In some implementations, one or two types of identifiers (e.g., fluorescent labels) are linked to each nucleic acid in a library. Detection and/or quantification of an identifier can be performed by a suitable method, apparatus or machine, non-limiting examples of which include flow cytometry, quantitative polymerase chain reaction (qPCR), gel electrophoresis, a luminometer, a fluorometer, a spectrophotometer, a suitable genechip or microarray analysis, Western blot, mass spectrometry, chromatography, cytofluorimetric analysis, fluorescence microscopy, a suitable fluorescence or digital imaging method, confocal laser scanning microscopy, laser scanning cytometry, affinity chromatography, manual batch mode separation, electric field suspension, a suitable nucleic acid sequencing method and/or nucleic acid sequencing apparatus, the like and combinations thereof.

In some implementations, an identifier, a sequencing-specific index/barcode, and a sequencer-specific flow-cell binding primer sites are incorporated into a nucleic acid library by single-primer extension (e.g., by a strand displacing polymerase).

In some implementations, a nucleic acid library or parts thereof are amplified (e.g., amplified by a PCR-based method) under amplification conditions. In some implementations, a sequencing method comprises amplification of a nucleic acid library. A nucleic acid library can be amplified prior to or after immobilization on a solid support (e.g., a solid support in a flow cell). Nucleic acid amplification includes the process of amplifying or increasing the numbers of a nucleic acid template and/or of a complement thereof that are present (e.g., in a nucleic acid library), by producing one or more copies of the template and/or its complement. Amplification can be carried out by a suitable method. A nucleic acid library can be amplified by a thermocycling method or by an isothermal amplification method. In some implementations, a rolling circle amplification method is used. In some implementations, amplification takes place on a solid support (e.g., within a flow cell) where a nucleic acid library or portion thereof is immobilized. In certain sequencing methods, a nucleic acid library is added to a flow cell and immobilized by hybridization to anchors under suitable conditions. This type of nucleic acid amplification is often referred to as solid phase amplification. In some implementations of solid phase amplification, all or a portion of the amplified products are synthesized by an extension initiating from an immobilized primer. Solid phase amplification reactions are analogous to standard solution phase amplifications except that at least one of the amplification oligonucleotides (e.g., primers) is immobilized on a solid support. In some implementations, modified nucleic acid (e.g., nucleic acid modified by addition of adapters) is amplified.

In some implementations, solid phase amplification comprises a nucleic acid amplification reaction comprising only one species of oligonucleotide primer immobilized to a surface. In certain implementations, solid phase amplification comprises a plurality of different immobilized oligonucleotide primer species. In some implementations, solid phase amplification may comprise a nucleic acid amplification reaction comprising one species of oligonucleotide primer immobilized on a solid surface and a second different oligonucleotide primer species in solution. Multiple different species of immobilized or solution-based primers can be used. Non-limiting examples of solid phase nucleic acid amplification reactions include interfacial amplification, bridge amplification, emulsion PCR, WildFire amplification (e.g., U.S. Patent Application Publication No. 2013/0012399), the like or combinations thereof.

### Nucleic acid sequencing

In some implementations, nucleic acid (e.g., nucleic acid fragments, sample nucleic acid, cell-free nucleic acid, single-stranded nucleic acid, single-stranded DNA, single-stranded RNA) is sequenced. In some implementations, ssNA hybridized to scaffold adapters provided herein ("hybridization products") are sequenced by a sequencing process. In some implementations, ssNA ligated to oligonucleotide components provided herein ("single-stranded ligation products") are sequenced by a sequencing process. In some implementations, hybridization products and/or single-stranded ligation products are amplified by an amplification process, and the amplification products are sequenced by a sequencing process. In some implementations, hybridization products and/or single-stranded ligation products are not amplified by an amplification process, and the hybridization products and/or single-stranded ligation products are sequenced without prior amplification by a sequencing process. In some implementations, the sequencing process generates sequence reads (or sequencing reads). In some implementations, a method herein comprises determining the sequence of a single-stranded nucleic acid molecule based on the sequence reads.

For certain sequencing platforms (e.g., paired-end sequencing), generating sequence reads may include generating forward sequence reads and generating reverse sequence reads. For example, sequencing using certain paired-end sequencing platforms sequence each nucleic acid fragment from both directions, generally resulting in two reads per nucleic acid fragment, with the first read in a forward orientation (forward read) and the second read in reverse-complement orientation (reverse read). For certain platforms, a forward read is generated off a particular primer within a sequencing adapter (e.g., ILLUMINA adapter, P5 primer), and a reverse read is generated off a different primer within a sequencing adapter (e.g., ILLUMINA adapter, P7 primer).

Nucleic acid may be sequenced using any suitable sequencing platform including a Sanger sequencing platform, a high throughput or massively parallel sequencing (next generation sequencing (NGS)) platform, or the like, such as, for example, a sequencing platform provided by Illumina^{®} (e.g., HiSeq^{™}, MiSeq^{™} and/or Genome Analyzer^{™} sequencing systems); Oxford Nanopore^{™} Technologies (e.g., MinION sequencing system), Ion Torrent^{™} (e.g., Ion PGM^{™} and/or Ion Proton^{™} sequencing systems); Pacific Biosciences (e.g., PACBIO RS II sequencing system); Life Technologies^{™} (e.g., SOLiD sequencing system); Roche (e.g., 454 GS FLX+ and/or GS Junior sequencing systems); or any other suitable sequencing platform. In some implementations, the sequencing process is a highly multiplexed sequencing process. In certain instances, a full or substantially full sequence is obtained and sometimes a partial sequence is obtained. Nucleic acid sequencing generally produces a collection of sequence reads. As used herein, "reads" (e.g., "a read," "a sequence read") are short sequences of nucleotides produced by any sequencing process described herein or known in the art. Reads can be generated from one end of nucleic acid fragments (single-end reads), and sometimes are generated from both ends of nucleic acid fragments (e.g., paired-end reads, double-end reads). In some implementations, a sequencing process generates short sequencing reads or "short reads." In some implementations, the nominal, average, mean or absolute length of short reads sometimes is about 10 continuous nucleotides to about 250 or more contiguous nucleotides. In some implementations, the nominal, average, mean or absolute length of short reads sometimes is about 50 continuous nucleotides to about 150 or more contiguous nucleotides.

The length of a sequence read is often associated with the particular sequencing technology utilized. High-throughput methods, for example, provide sequence reads that can vary in size from tens to hundreds of base pairs (bp). Nanopore sequencing, for example, can provide sequence reads that can vary in size from tens to hundreds to thousands of base pairs. In some implementations, sequence reads are of a mean, median, average or absolute length of about 15 bp to about 900 bp long. In certain implementations sequence reads are of a mean, median, average or absolute length of about 1000 bp or more. In some implementations sequence reads are of a mean, median, average or absolute length of about 1500, 2000, 2500, 3000, 3500, 4000, 4500, or 5000 bp or more. In some implementations, sequence reads are of a mean, median, average or absolute length of about 100 bp to about 200 bp.

In some implementations, the nominal, average, mean or absolute length of single-end reads sometimes is about 10 continuous nucleotides to about 250 or more contiguous nucleotides, about 15 contiguous nucleotides to about 200 or more contiguous nucleotides, about 15 contiguous nucleotides to about 150 or more contiguous nucleotides, about 15 contiguous nucleotides to about 125 or more contiguous nucleotides, about 15 contiguous nucleotides to about 100 or more contiguous nucleotides, about 15 contiguous nucleotides to about 75 or more contiguous nucleotides, about 15 contiguous nucleotides to about 60 or more contiguous nucleotides, 15 contiguous nucleotides to about 50 or more contiguous nucleotides, about 15 contiguous nucleotides to about 40 or more contiguous nucleotides, and sometimes about 15 contiguous nucleotides or about 36 or more contiguous nucleotides. In certain implementations the nominal, average, mean or absolute length of single-end reads is about 20 to about 30 bases, or about 24 to about 28 bases in length. In certain implementations the nominal, average, mean or absolute length of single-end reads is about 1, 2, 3, 4, 5, 6, 7, 8, 9, 10, 11, 12, 13, 14, 15, 16, 17, 18, 19, 21, 22, 23, 24, 25, 26, 27, 28 or about 29 bases or more in length. In certain implementations the nominal, average, mean or absolute length of single-end reads is about 20 to about 200 bases, about 100 to about 200 bases, or about 140 to about 160 bases in length. In certain implementations the nominal, average, mean or absolute length of single-end reads is about 30, 40, 50, 60, 70, 80, 90, 100, 110, 120, 130, 140, 150, 160, 170, 180, 190, or about 200 bases or more in length. In certain implementations, the nominal, average, mean or absolute length of paired-end reads sometimes is about 10 contiguous nucleotides to about 25 contiguous nucleotides or more (e.g., about 10, 11, 12, 13, 14, 15, 16, 17, 18, 19, 20, 21, 22, 23, 24 or 25 nucleotides in length or more), about 15 contiguous nucleotides to about 20 contiguous nucleotides or more, and sometimes is about 17 contiguous nucleotides or about 18 contiguous nucleotides. In certain implementations, the nominal, average, mean or absolute length of paired-end reads sometimes is about 25 contiguous nucleotides to about 400 contiguous nucleotides or more (e.g., about 25, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, or 400 nucleotides in length or more), about 50 contiguous nucleotides to about 350 contiguous nucleotides or more, about 100 contiguous nucleotides to about 325 contiguous nucleotides, about 150 contiguous nucleotides to about 325 contiguous nucleotides, about 200 contiguous nucleotides to about 325 contiguous nucleotides, about 275 contiguous nucleotides to about 310 contiguous nucleotides, about 100 contiguous nucleotides to about 200 contiguous nucleotides, about 100 contiguous nucleotides to about 175 contiguous nucleotides, about 125 contiguous nucleotides to about 175 contiguous nucleotides, and sometimes is about 140 contiguous nucleotides to about 160 contiguous nucleotides. In certain implementations, the nominal, average, mean, or absolute length of paired-end reads is about 150 contiguous nucleotides, and sometimes is 150 contiguous nucleotides.

Reads generally are representations of nucleotide sequences in a physical nucleic acid. For example, in a read containing an ATGC depiction of a sequence, "A" represents an adenine nucleotide, "T" represents a thymine nucleotide, "G" represents a guanine nucleotide and "C" represents a cytosine nucleotide, in a physical nucleic acid. Sequence reads obtained from a sample from a subject can be reads from a mixture of a minority nucleic acid and a majority nucleic acid. For example, sequence reads obtained from the blood of a cancer patient can be reads from a mixture of cancer nucleic acid and non-cancer nucleic acid. In another example, sequence reads obtained from the blood of a pregnant female can be reads from a mixture of fetal nucleic acid and maternal nucleic acid. In another example, sequence reads obtained from the blood of a patient having an infection or infectious disease can be reads from a mixture of host nucleic acid and pathogen nucleic acid. In another example, sequence reads obtained from the blood of a transplant recipient can be reads from a mixture of host nucleic acid and transplant nucleic acid. In another example, sequence reads obtained from a sample can be reads from a mixture of nucleic acid from microorganisms collectively comprising a microbiome (e.g., microbiome of gut, microbiome of blood, microbiome of mouth, microbiome of spinal fluid, microbiome of feces) in a subject. In another example, sequence reads obtained from a sample can be reads from a mixture of nucleic acid from microorganisms collectively comprising a microbiome (e.g., microbiome of gut, microbiome of blood, microbiome of mouth, microbiome of spinal fluid, microbiome of feces), and nucleic acid from the host subject. A mixture of relatively short reads can be transformed by processes described herein into a representation of genomic nucleic acid present in the subject, and/or a representation of genomic nucleic acid present in a tumor, a fetus, a pathogen, a transplant, or a microbiome.

In certain implementations, "obtaining" nucleic acid sequence reads of a sample from a subject and/or "obtaining" nucleic acid sequence reads of a biological specimen from one or more reference persons can involve directly sequencing nucleic acid to obtain the sequence information. In some implementations, "obtaining" can involve receiving sequence information obtained directly from a nucleic acid by another.

In some implementations, some or all nucleic acids in a sample are enriched and/or amplified (e.g., non-specifically, e.g., by a PCR based method) prior to or during sequencing. In certain implementations, specific nucleic acid species or subsets in a sample are enriched and/or amplified prior to or during sequencing. In some implementations, a species or subset of a pre-selected pool of nucleic acids is sequenced randomly. In some implementations, nucleic acids in a sample are not enriched and/or amplified prior to or during sequencing.

In some implementations, a representative fraction of a genome is sequenced and is sometimes referred to as "coverage" or "fold coverage." For example, a 1-fold coverage indicates that roughly 100% of the nucleotide sequences of the genome are represented by reads. In some instances, fold coverage is referred to as (and is directly proportional to) "sequencing depth." In some implementations, "fold coverage" is a relative term referring to a prior sequencing run as a reference. For example, a second sequencing run may have 2-fold less coverage than a first sequencing run. In some implementations, a genome is sequenced with redundancy, where a given region of the genome can be covered by two or more reads or overlapping reads (e.g., a "fold coverage" greater than 1, e.g., a 2-fold coverage). In some implementations, a genome (e.g., a whole genome) is sequenced with about 0.01-fold to about 100-fold coverage, about 0.1-fold to 20-fold coverage, or about 0.1-fold to about 1-fold coverage (e.g., about 0.015-, 0.02-, 0.03-, 0.04-, 0.05-, 0.06-, 0.07-, 0.08-, 0.09-, 0.1-, 0.2-, 0.3-, 0.4-, 0.5-, 0.6-, 0.7-, 0.8-, 0.9-, 1-, 2-, 3-, 4-, 5-, 6-, 7-, 8-, 9-, 10-, 15-, 20-, 30-, 40-, 50-, 60-, 70-, 80-, 90-fold or greater coverage). In some implementations, specific parts of a genome (e.g., genomic parts from targeted methods) are sequenced and fold coverage values generally refer to the fraction of the specific genomic parts sequenced (i.e., fold coverage values do not refer to the whole genome). In some instances, specific genomic parts are sequenced at 1000-fold coverage or more. For example, specific genomic parts may be sequenced at 2000-fold, 5,000-fold, 10,000-fold, 20,000-fold, 30,000-fold, 40,000-fold or 50,000-fold coverage. In some implementations, sequencing is at about 1 ,000-fold to about 100,000-fold coverage. In some implementations, sequencing is at about 10,000-fold to about 70,000-fold coverage. In some implementations, sequencing is at about 20,000-fold to about 60,000-fold coverage. In some implementations, sequencing is at about 30,000-fold to about 50,000-fold coverage.

In some implementations, one nucleic acid sample from one individual is sequenced. In certain implementations, nucleic acids from each of two or more samples are sequenced, where samples are from one individual or from different individuals. In certain implementations, nucleic acid samples from two or more biological samples are pooled, where each biological sample is from one individual or two or more individuals, and the pool is sequenced. In the latter implementations, a nucleic acid sample from each biological sample often is identified by one or more unique identifiers.

In some implementations, a sequencing method utilizes identifiers that allow multiplexing of sequence reactions in a sequencing process. The greater the number of unique identifiers, the greater the number of samples and/or chromosomes for detection, for example, that can be multiplexed in a sequencing process. A sequencing process can be performed using any suitable number of unique identifiers (e.g., 4, 8, 12, 24, 48, 96, or more).

A sequencing process sometimes makes use of a solid phase, and sometimes the solid phase comprises a flow cell on which nucleic acid from a library can be attached and reagents can be flowed and contacted with the attached nucleic acid. A flow cell sometimes includes flow cell lanes, and use of identifiers can facilitate analyzing a number of samples in each lane. A flow cell often is a solid support that can be configured to retain and/or allow the orderly passage of reagent solutions over bound analytes. Flow cells frequently are planar in shape, optically transparent, generally in the millimeter or sub-millimeter scale, and often have channels or lanes in which the analyte/reagent interaction occurs. In some implementations, the number of samples analyzed in a given flow cell lane is dependent on the number of unique identifiers utilized during library preparation and/or probe design. Multiplexing using 12 identifiers, for example, allows simultaneous analysis of 96 samples (e.g., equal to the number of wells in a 96 well microwell plate) in an 8-lane flow cell. Similarly, multiplexing using 48 identifiers, for example, allows simultaneous analysis of 384 samples (e.g., equal to the number of wells in a 384 well microwell plate) in an 8-lane flow cell. Non-limiting examples of commercially available multiplex sequencing kits include Illumina's multiplexing sample preparation oligonucleotide kit and multiplexing sequencing primers and PhiX control kit (e.g., Illumina's catalog numbers PE-400-1001 and PE-400-1002, respectively).

Any suitable method of sequencing nucleic acids can be used, non-limiting examples of which include Maxim & Gilbert, chain-termination methods, sequencing by synthesis, sequencing by ligation, sequencing by mass spectrometry, microscopy-based techniques, the like or combinations thereof. In some implementations, a first-generation technology, such as, for example, Sanger sequencing methods including automated Sanger sequencing methods, including microfluidic Sanger sequencing, can be used in a method provided herein. In some implementations, sequencing technologies that include the use of nucleic acid imaging technologies (e.g., transmission electron microscopy (TEM) and atomic force microscopy (AFM)), can be used. In some implementations, a high-throughput sequencing method is used. High-throughput sequencing methods generally involve clonally amplified DNA templates or single DNA molecules that are sequenced in a massively parallel fashion, sometimes within a flow cell. Next generation (e.g., 2nd and 3rd generation) sequencing techniques capable of sequencing DNA in a massively parallel fashion can be used for methods described herein and are collectively referred to herein as "massively parallel sequencing" (MPS). In some implementations, MPS sequencing methods utilize a targeted approach, where specific chromosomes, genes or regions of interest are sequenced. In certain implementations, a non-targeted approach is used where most or all nucleic acids in a sample are sequenced, amplified and/or captured randomly.

In some implementations a targeted enrichment, amplification and/or sequencing approach is used. A targeted approach often isolates, selects and/or enriches a subset of nucleic acids in a sample for further processing by use of sequence-specific oligonucleotides. In some implementations, a library of sequence-specific oligonucleotides are utilized to target (e.g., hybridize to) one or more sets of nucleic acids in a sample. Sequence-specific oligonucleotides and/or primers are often selective for particular sequences (e.g., unique nucleic acid sequences) present in one or more chromosomes, genes, exons, introns, and/or regulatory regions of interest. Any suitable method or combination of methods can be used for enrichment, amplification and/or sequencing of one or more subsets of targeted nucleic acids. In some implementations targeted sequences are isolated and/or enriched by capture to a solid phase (e.g., a flow cell, a bead) using one or more sequence-specific anchors. In some implementations targeted sequences are enriched and/or amplified by a polymerase-based method (e.g., a PCR-based method, by any suitable polymerase-based extension) using sequence-specific primers and/or primer sets. Sequence specific anchors often can be used as sequence-specific primers.

MPS sequencing sometimes makes use of sequencing by synthesis and certain imaging processes. A nucleic acid sequencing technology that may be used in a method described herein is sequencing-by-synthesis and reversible terminator-based sequencing (e.g., Illumina's Genome Analyzer; Genome Analyzer II; HISEQ 2000; HISEQ 2500 (Illumina, San Diego CA)). With this technology, millions of nucleic acid (e.g., DNA) fragments can be sequenced in parallel. In one example of this type of sequencing technology, a flow cell is used which contains an optically transparent slide with 8 individual lanes on the surfaces of which are bound oligonucleotide anchors (e.g., adapter primers).

Sequencing by synthesis generally is performed by iteratively adding (e.g., by covalent addition) a nucleotide to a primer or preexisting nucleic acid strand in a template directed manner. Each iterative addition of a nucleotide is detected and the process is repeated multiple times until a sequence of a nucleic acid strand is obtained. The length of a sequence obtained depends, in part, on the number of addition and detection steps that are performed. In some implementations of sequencing by synthesis, one, two, three or more nucleotides of the same type (e.g., A, G, C or T) are added and detected in a round of nucleotide addition. Nucleotides can be added by any suitable method (e.g., enzymatically or chemically). For example, in some implementations a polymerase or a ligase adds a nucleotide to a primer or to a preexisting nucleic acid strand in a template directed manner. In some implementations of sequencing by synthesis, different types of nucleotides, nucleotide analogues and/or identifiers are used. In some implementations, reversible terminators and/or removable (e.g., cleavable) identifiers are used. In some implementations, fluorescent labeled nucleotides and/or nucleotide analogues are used. In certain implementations sequencing by synthesis comprises a cleavage (e.g., cleavage and removal of an identifier) and/or a washing step. In some implementations the addition of one or more nucleotides is detected by a suitable method described herein or known in the art, non-limiting examples of which include any suitable imaging apparatus, a suitable camera, a digital camera, a CCD (Charge Couple Device) based imaging apparatus (e.g., a CCD camera), a CMOS (Complementary Metal Oxide Silicon) based imaging apparatus (e.g., a CMOS camera), a photo diode (e.g., a photomultiplier tube), electron microscopy, a field-effect transistor (e.g., a DNA field-effect transistor), an ISFET ion sensor (e.g., a CHEMFET sensor), the like or combinations thereof.

Any suitable MPS method, system or technology platform for conducting methods described herein can be used to obtain nucleic acid sequence reads. Non-limiting examples of MPS platforms include ILLUMINA/SOLEX/HISEQ (e.g., Illumina's Genome Analyzer; Genome Analyzer II; HISEQ 2000; HISEQ), SOLiD, Roche/454, PACBIO and/or SMRT, Helicos True Single Molecule Sequencing, Ion Torrent and Ion semiconductor-based sequencing (e.g., as developed by Life Technologies), WildFire, 5500, 5500xl W and/or 5500xl W Genetic Analyzer based technologies (e.g., as developed and sold by Life Technologies, U.S. Patent Application Publication No. 2013/0012399); Polony sequencing, Pyrosequencing, Massively Parallel Signature Sequencing (MPSS), RNA polymerase (RNAP) sequencing, LaserGen systems and methods, Nanopore-based platforms, chemical-sensitive field effect transistor (CHEMFET) array, electron microscopy-based sequencing (e.g., as developed by ZS Genetics, Halcyon Molecular), nanoball sequencing, the like or combinations thereof. Other sequencing methods that may be used to conduct methods herein include digital PCR, sequencing by hybridization, nanopore sequencing, chromosome-specific sequencing (e.g., using DANSR (digital analysis of selected regions) technology.

In some implementations, nucleic acid is sequenced and the sequencing product (e.g., a collection of sequence reads) is processed prior to, or in conjunction with, an analysis of the sequenced nucleic acid. For example, sequence reads may be processed according to one or more of the following: aligning, mapping, filtering, counting, normalizing, weighting, generating a profile, and the like, and combinations thereof. Certain processing steps may be performed in any order and certain processing steps may be repeated.

Methods of the present disclosure can be used to reduce sequencing error rates. In some implementations, prior to an initial denaturing, double-stranded molecules can be labeled with a barcode such that, after subsequent denaturing, single-stranded library preparation, and sequencing, sequences from nucleic acid molecules that were originally paired together can be associated. In some implementations, after initial ligation of scaffold adapters, a pool of index primers is used to conduct index PCR such that copies are generated of both original sample nucleic acid molecules and nucleic acids from initial PCR first strand synthesis that both comprise the same barcode or UMI (or the complement thereof). By these or other means of associating strands that were originally hybridized (and therefore have complementary sequences), sequencing read information for both strands can be compared and used to reduce the sequencing error rate.

### Mapping reads

Sequence reads can be mapped and the number of reads mapping to a specified nucleic acid region (e.g., a chromosome or portion thereof) are referred to as counts. Any suitable mapping method (e.g., process, algorithm, program, software, module, the like or combination thereof) can be used. Certain aspects of mapping processes are described hereafter.

Mapping nucleotide sequence reads (i.e., sequence information from a fragment whose physical genomic position is unknown) can be performed in a number of ways, and often comprises alignment of the obtained sequence reads with a matching sequence in a reference genome. In such alignments, sequence reads generally are aligned to a reference sequence and those that align are designated as being "mapped," as "a mapped sequence read" or as "a mapped read." In certain implementations, a mapped sequence read is referred to as a "hit" or "count." In some implementations, mapped sequence reads are grouped together according to various parameters and assigned to particular genomic portions, which are discussed in further detail below.

The terms "aligned," "alignment," or "aligning" generally refer to two or more nucleic acid sequences that can be identified as a match (e.g., 100% identity) or partial match. Alignments can be done manually or by a computer (e.g., a software, program, module, or algorithm), non-limiting examples of which include the Efficient Local Alignment of Nucleotide Data (ELAND) computer program distributed as part of the ILLUMINA Genomics Analysis pipeline. Alignment of a sequence read can be a 100% sequence match. In some instances, an alignment is less than a 100% sequence match (i.e., non-perfect match, partial match, partial alignment). In some implementations an alignment is about a 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76% or 75% match. In some implementations, an alignment comprises a mismatch. In some implementations, an alignment comprises 1, 2, 3, 4 or 5 mismatches. Two or more sequences can be aligned using either strand (e.g., sense or antisense strand). In certain implementations a nucleic acid sequence is aligned with the reverse complement of another nucleic acid sequence.

Various computational methods can be used to map each sequence read to a portion. Non-limiting examples of computer algorithms that can be used to align sequences include, without limitation, BLAST, BLITZ, FASTA, BOWTIE 1, BOWTIE 2, ELAND, MAQ, PROBEMATCH, SOAP, BWA or SEQMAP, or variations thereof or combinations thereof. In some implementations, sequence reads can be aligned with sequences in a reference genome. In some implementations, sequence reads can be found and/or aligned with sequences in nucleic acid databases known in the art including, for example, GenBank, dbEST, dbSTS, EMBL (European Molecular Biology Laboratory) and DDBJ (DNA Databank of Japan). BLAST or similar tools can be used to search identified sequences against a sequence database. Search hits can then be used to sort the identified sequences into appropriate portions (described hereafter), for example.

In some implementations, a read may uniquely or non-uniquely map to portions in a reference genome. A read is considered as "uniquely mapped" if it aligns with a single sequence in the reference genome. A read is considered as "non-uniquely mapped" if it aligns with two or more sequences in the reference genome. In some implementations, non-uniquely mapped reads are eliminated from further analysis (e.g. quantification). A certain, small degree of mismatch (0-1) may be allowed to account for single nucleotide polymorphisms that may exist between the reference genome and the reads from individual samples being mapped, in certain implementations. In some implementations, no degree of mismatch is allowed for a read mapped to a reference sequence.

As used herein, the term "reference genome" can refer to any particular known, sequenced or characterized genome, whether partial or complete, of any organism or virus which may be used to reference identified sequences from a subject. For example, a reference genome used for human subjects as well as many other organisms can be found at the National Center for Biotechnology Information at World Wide Web URL ncbi.nlm.nih.gov. A "genome" refers to the complete genetic information of an organism or virus, expressed in nucleic acid sequences. As used herein, a reference sequence or reference genome often is an assembled or partially assembled genomic sequence from an individual or multiple individuals. In some implementations, a reference genome is an assembled or partially assembled genomic sequence from one or more human individuals. In some implementations, a reference genome comprises sequences assigned to chromosomes.

In certain implementations, mappability is assessed for a genomic region (e.g., portion, genomic portion). Mappability is the ability to unambiguously align a nucleotide sequence read to a portion of a reference genome, typically up to a specified number of mismatches, including, for example, 0, 1, 2 or more mismatches. For a given genomic region, the expected mappability can be estimated using a sliding-window approach of a preset read length and averaging the resulting read-level mappability values. Genomic regions comprising stretches of unique nucleotide sequence sometimes have a high mappability value.

For paired-end sequencing, reads may be mapped to a reference genome by use of a suitable mapping and/or alignment program or algorithm, non-limiting examples of which include BWA (Li H. and Durbin R. (2009)Bioinformatics 25, 1754-60), Novoalign [Novocraft (2010)], Bowtie (Langmead B, et al., (2009) Genome Biol. 10:R25), SOAP2 (Li R, et al., (2009) Bioinformatics 25, 1966-67), BFAST (Homer N, et al., (2009) PLoS ONE 4, e7767), GASSST (Rizk, G. and Lavenier, D. (2010) Bioinformatics 26, 2534-2540), and MPscan (Rivals E., et al. (2009) Lecture Notes in Computer Science 5724, 246-260), and the like. Reads can be trimmed and/or merged by use of a suitable trimming and/or merging program or algorithm, non-limiting examples of which include Cutadapt, trimmomatic, SeqPrep, and usearch. Some paired-end reads, such as those from nucleic acid templates that are shorter than the sequencing read length, can have portions sequenced by both the forward read and the reverse read; in such instances, the forward and reverse reads can be merged into a single read using the overlap between the forward and reverse reads. Reads that do not overlap or that do not overlap sufficiently can remain unmerged and be mapped as paired reads. Paired-end reads may be mapped and/or aligned using a suitable short read alignment program or algorithm. Non-limiting examples of short read alignment programs include BarraCUDA, BFAST, BLASTN, BLAT, Bowtie, BWA, CASHX, CUDA-EC, CUSHAW, CUSHAW2, drFAST, ELAND, ERNE, GNUMAP, GEM, GensearchNGS, GMAP, Geneious Assembler, iSAAC, LAST, MAQ, mrFAST, mrsFAST, MOSAIK, MPscan, Novoalign, NovoalignCS, Novocraft, NextGENe, Omixon, PALMapper, Partek , PASS, PerM, QPalma, RazerS, REAL, cREAL, RMAP, rNA, RTG, Segemehl, SeqMap, Shrec, SHRiMP, SLIDER, SOAP, SOAP2, SOAP3, SOCS, SSAHA, SSAHA2, Stampy, SToRM, Subread, Subjunc, Taipan, UGENE, VelociMapper, TimeLogic, XpressAlign, ZOOM, the like or combinations thereof. Paired-end reads are often mapped to opposing ends of the same polynucleotide fragment, according to a reference genome. In some implementations, read mates are mapped independently. In some implementations, information from both sequence reads (i.e., from each end) is factored in the mapping process. A reference genome is often used to determine and/or infer the sequence of nucleic acids located between paired-end read mates. The term "discordant read pairs" as used herein refers to a paired-end read comprising a pair of read mates, where one or both read mates fail to unambiguously map to the same region of a reference genome defined, in part, by a segment of contiguous nucleotides. In some implementations discordant read pairs are paired-end read mates that map to unexpected locations of a reference genome. Non-limiting examples of unexpected locations of a reference genome include (i) two different chromosomes, (ii) locations separated by more than a predetermined fragment size (e.g., more than 300 bp, more than 500 bp, more than 1000 bp, more than 5000 bp, or more than 10,000 bp), (iii) an orientation inconsistent with a reference sequence (e.g., opposite orientations), the like or a combination thereof. In some implementations discordant read mates are identified according to a length (e.g., an average length, a predetermined fragment size) or expected length of template polynucleotide fragments in a sample. For example, read mates that map to a location that is separated by more than the average length or expected length of polynucleotide fragments in a sample are sometimes identified as discordant read pairs. Read pairs that map in opposite orientation are sometimes determined by taking the reverse complement of one of the reads and comparing the alignment of both reads using the same strand of a reference sequence. Discordant read pairs can be identified by any suitable method and/or algorithm known in the art or described herein (e.g., SVDetect, Lumpy, BreakDancer, BreakDancerMax, CREST, DELLY, the like or combinations thereof).

### Sequence read quantification

Sequence reads that are mapped or partitioned based on a selected feature or variable can be quantified to determine the amount or number of reads that are mapped to one or more portions (e.g., portion of a reference genome). In certain implementations, the quantity of sequence reads that are mapped to a portion or segment is referred to as a count or read density.

A count often is associated with a genomic portion. In some implementations a count is determined from some or all of the sequence reads mapped to (i.e., associated with) a portion. In certain implementations, a count is determined from some or all of the sequence reads mapped to a group of portions (e.g., portions in a segment or region).

A count can be determined by a suitable method, operation or mathematical process. A count sometimes is the direct sum of all sequence reads mapped to a genomic portion or a group of genomic portions corresponding to a segment, a group of portions corresponding to a sub-region of a genome (e.g., copy number variation region, copy number alteration region, copy number duplication region, copy number deletion region, microduplication region, microdeletion region, chromosome region, autosome region, sex chromosome region) and/or sometimes is a group of portions corresponding to a genome. A read quantification sometimes is a ratio, and sometimes is a ratio of a quantification for portion(s) in region a to a quantification for portion(s) in region b. Region a sometimes is one portion, segment region, copy number variation region, copy number alteration region, copy number duplication region, copy number deletion region, microduplication region, microdeletion region, chromosome region, autosome region and/or sex chromosome region. Region b independently sometimes is one portion, segment region, copy number variation region, copy number alteration region, copy number duplication region, copy number deletion region, microduplication region, microdeletion region, chromosome region, autosome region, sex chromosome region, a region including all autosomes, a region including sex chromosomes and/or a region including all chromosomes.

In some implementations, a count is derived from raw sequence reads and/or filtered sequence reads. In certain implementations a count is an average, mean or sum of sequence reads mapped to a genomic portion or group of genomic portions (e.g., genomic portions in a region). In some implementations, a count is associated with an uncertainty value. A count sometimes is adjusted. A count may be adjusted according to sequence reads associated with a genomic portion or group of portions that have been weighted, removed, filtered, normalized, adjusted, averaged, derived as a mean, derived as a median, added, or combination thereof.

A sequence read quantification sometimes is a read density. A read density may be determined and/or generated for one or more segments of a genome. In certain instances, a read density may be determined and/or generated for one or more chromosomes. In some implementations a read density comprises a quantitative measure of counts of sequence reads mapped to a segment or portion of a reference genome. A read density can be determined by a suitable process. In some implementations a read density is determined by a suitable distribution and/or a suitable distribution function. Non-limiting examples of a distribution function include a probability function, probability distribution function, probability density function (PDF), a kernel density function (kernel density estimation), a cumulative distribution function, probability mass function, discrete probability distribution, an absolutely continuous univariate distribution, the like, any suitable distribution, or combinations thereof. A read density may be a density estimation derived from a suitable probability density function. A density estimation is the construction of an estimate, based on observed data, of an underlying probability density function. In some implementations a read density comprises a density estimation (e.g., a probability density estimation, a kernel density estimation). A read density may be generated according to a process comprising generating a density estimation for each of the one or more portions of a genome where each portion comprises counts of sequence reads. A read density may be generated for normalized and/or weighted counts mapped to a portion or segment. In some instances, each read mapped to a portion or segment may contribute to a read density, a value (e.g., a count) equal to its weight obtained from a normalization process described herein. In some implementations read densities for one or more portions or segments are adjusted. Read densities can be adjusted by a suitable method. For example, read densities for one or more portions can be weighted and/or normalized.

Reads quantified for a given portion or segment can be from one source or different sources. In one example, reads may be obtained from nucleic acid from a subject having cancer or suspected of having cancer. In such circumstances, reads mapped to one or more portions often are reads representative of both healthy cells (i.e., non-cancer cells) and cancer cells (e.g., tumor cells). In certain implementations, some of the reads mapped to a portion are from cancer cell nucleic acid and some of the reads mapped to the same portion are from non-cancer cell nucleic acid. In another example, reads may be obtained from a nucleic acid sample from a pregnant female bearing a fetus. In such circumstances, reads mapped to one or more portions often are reads representative of both the fetus and the mother of the fetus (e.g., a pregnant female subject). In certain implementations some of the reads mapped to a portion are from a fetal genome and some of the reads mapped to the same portion are from a maternal genome.

### Classifications and uses thereof

Methods described herein can provide an outcome indicative of one or more characteristics of a sample or source described above. Methods described herein sometimes provide an outcome indicative of a phenotype and/or presence or absence of a medical condition for a test sample (e.g., providing an outcome determinative of the presence or absence of a medical condition and/or phenotype). An outcome often is part of a classification process, and a classification (e.g., classification of one or more characteristics of a sample or source; and/or presence or absence of a genotype, phenotype, genetic variation and/or medical condition for a test sample) sometimes is based on and/or includes an outcome. An outcome and/or classification sometimes is based on and/or includes a result of data processing for a test sample that facilitates determining one or more characteristics of a sample or source and/or presence or absence of a genotype, phenotype, genetic variation, genetic alteration, and/or medical condition in a classification process (e.g., a statistic value). An outcome and/or classification sometimes includes or is based on a score determinative of, or a call of, one or more characteristics of a sample or source and/or presence or absence of a genotype, phenotype, genetic variation, genetic alteration, and/or medical condition. In certain implementations, an outcome and/or classification includes a conclusion that predicts and/or determines one or more characteristics of a sample or source and/or presence or absence of a genotype, phenotype, genetic variation, genetic alteration, and/or medical condition in a classification process.

Any suitable expression of an outcome and/or classification can be provided. An outcome and/or classification sometimes is based on and/or includes one or more numerical values generated using a processing method described herein in the context of one or more considerations of probability. Non-limiting examples of values that can be utilized include a sensitivity, specificity, standard deviation, median absolute deviation (MAD), measure of certainty, measure of confidence, measure of certainty or confidence that a value obtained for a test sample is inside or outside a particular range of values, measure of uncertainty, measure of uncertainty that a value obtained for a test sample is inside or outside a particular range of values, coefficient of variation (CV), confidence level, confidence interval (e.g., about 95% confidence interval), standard score (e.g., z-score), chi value, phi value, result of a t-test, p-value, ploidy value, fitted minority species fraction, area ratio, median level, the like or combination thereof. In some implementations, an outcome and/or classification comprises a read density, a read density profile and/or a plot (e.g., a profile plot). In certain implementations, multiple values are analyzed together, sometimes in a profile for such values (e.g., z-score profile, p-value profile, chi value profile, phi value profile, result of a t-test, value profile, the like, or combination thereof). A consideration of probability can facilitate determining one or more characteristics of a sample or source and/or whether a subject is at risk of having, or has, a genotype, phenotype, genetic variation and/or medical condition, and an outcome and/or classification determinative of the foregoing sometimes includes such a consideration.

In certain implementations, an outcome and/or classification is based on and/or includes a conclusion that predicts and/or determines a risk or probability of the presence or absence of a genotype, phenotype, genetic variation and/or medical condition for a test sample. A conclusion sometimes is based on a value determined from a data analysis method described herein (e.g., a statistics value indicative of probability, certainty and/or uncertainty (e.g., standard deviation, median absolute deviation (MAD), measure of certainty, measure of confidence, measure of certainty or confidence that a value obtained for a test sample is inside or outside a particular range of values, measure of uncertainty, measure of uncertainty that a value obtained for a test sample is inside or outside a particular range of values, coefficient of variation (CV), confidence level, confidence interval (e.g., about 95% confidence interval), standard score (e.g., z-score), chi value, phi value, result of a t-test, p-value, sensitivity, specificity, the like or combination thereof). An outcome and/or classification sometimes is expressed in a laboratory test report for particular test sample as a probability (e.g., odds ratio, p-value), likelihood, or risk factor, associated with the presence or absence of a genotype, phenotype, genetic variation and/or medical condition. An outcome and/or classification for a test sample sometimes is provided as "positive" or "negative" with respect a particular genotype, phenotype, genetic variation and/or medical condition. For example, an outcome and/or classification sometimes is designated as "positive" in a laboratory test report for a particular test sample where presence of a genotype, phenotype, genetic variation and/or medical condition is determined, and sometimes an outcome and/or classification is designated as "negative" in a laboratory test report for a particular test sample where absence of a genotype, phenotype, genetic variation and/or medical condition is determined. An outcome and/or classification sometimes is determined and sometimes includes an assumption used in data processing.

There typically are four types of classifications generated in a classification process: true positive, false positive, true negative and false negative. The term "true positive" as used herein refers to presence of a genotype, phenotype, genetic variation, or medical condition correctly determined for a test sample. The term "false positive" as used herein refers to presence of a genotype, phenotype, genetic variation, or medical condition incorrectly determined for a test sample. The term "true negative" as used herein refers to absence of a genotype, phenotype, genetic variation, or medical condition correctly determined for a test sample. The term "false negative" as used herein refers to absence of a genotype, phenotype, genetic variation, or medical condition incorrectly determined for a test sample. Two measures of performance for a classification process can be calculated based on the ratios of these occurrences: (i) a sensitivity value, which generally is the fraction of predicted positives that are correctly identified as being positives; and (ii) a specificity value, which generally is the fraction of predicted negatives correctly identified as being negative.

In certain implementations, a laboratory test report generated for a classification process includes a measure of test performance (e.g., sensitivity and/or specificity) and/or a measure of confidence (e.g., a confidence level, confidence interval). A measure of test performance and/or confidence sometimes is obtained from a clinical validation study performed prior to performing a laboratory test for a test sample. In certain implementations, one or more of sensitivity, specificity and/or confidence are expressed as a percentage. In some implementations, a percentage expressed independently for each of sensitivity, specificity or confidence level, is greater than about 90% (e.g., about 90, 91, 92, 93, 94, 95, 96, 97, 98 or 99%, or greater than 99% (e.g., about 99.5%, or greater, about 99.9% or greater, about 99.95% or greater, about 99.99% or greater)). A confidence interval expressed for a particular confidence level (e.g., a confidence level of about 90% to about 99.9% (e.g., about 95%)) can be expressed as a range of values, and sometimes is expressed as a range or sensitivities and/or specificities for a particular confidence level. Coefficient of variation (CV) in some implementations is expressed as a percentage, and sometimes the percentage is about 10% or less (e.g., about 10, 9, 8, 7, 6, 5, 4, 3, 2 or 1%, or less than 1% (e.g., about 0.5% or less, about 0.1% or less, about 0.05% or less, about 0.01 % or less)). A probability (e.g., that a particular outcome and/or classification is not due to chance) in certain implementations is expressed as a standard score (e.g., z-score), a p-value, or result of a t-test. In some implementations, a measured variance, confidence level, confidence interval, sensitivity, specificity and the like (e.g., referred to collectively as confidence parameters) for an outcome and/or classification can be generated using one or more data processing manipulations described herein.

An outcome and/or classification for a test sample often is ordered by, and often is provided to, a health care professional or other qualified individual (e.g., physician or assistant) who transmits an outcome and/or classification to a subject from whom the test sample is obtained. In certain implementations, an outcome and/or classification is provided using a suitable visual medium (e.g., a peripheral or component of a machine, e.g., a printer or display). A classification and/or outcome often is provided to a healthcare professional or qualified individual in the form of a report. A report typically comprises a display of an outcome and/or classification (e.g., a value, one or more characteristics of a sample or source, or an assessment or probability of presence or absence of a genotype, phenotype, genetic variation and/or medical condition), sometimes includes an associated confidence parameter, and sometimes includes a measure of performance for a test used to generate the outcome and/or classification. A report sometimes includes a recommendation for a follow-up procedure (e.g., a procedure that confirms the outcome or classification). A report sometimes includes a visual representation of a chromosome or portion thereof (e.g., a chromosome ideogram or karyogram), and sometimes shows a visualization of a duplication and/or deletion region for a chromosome (e.g., a visualization of a whole chromosome for a chromosome deletion or duplication; a visualization of a whole chromosome with a deleted region or duplicated region shown; a visualization of a portion of chromosome duplicated or deleted; a visualization of a portion of a chromosome remaining in the event of a deletion of a portion of a chromosome) identified for a test sample.

A report can be displayed in a suitable format that facilitates determination of presence or absence of a genotype, phenotype, genetic variation and/or medical condition by a health professional or other qualified individual. Non-limiting examples of formats suitable for use for generating a report include digital data, a graph, a 2D graph, a 3D graph, and 4D graph, a picture (e.g., a jpg, bitmap (e.g., bmp), pdf, tiff, gif, raw, png, the like or suitable format), a pictograph, a chart, a table, a bar graph, a pie graph, a diagram, a flow chart, a scatter plot, a map, a histogram, a density chart, a function graph, a circuit diagram, a block diagram, a bubble map, a constellation diagram, a contour diagram, a cartogram, spider chart, Venn diagram, nomogram, and the like, or combination of the foregoing.

A report may be generated by a computer and/or by human data entry, and can be transmitted and communicated using a suitable electronic medium (e.g., via the internet, via computer, via facsimile, from one network location to another location at the same or different physical sites), or by another method of sending or receiving data (e.g., mail service, courier service and the like). Non-limiting examples of communication media for transmitting a report include auditory file, computer readable file (e.g., pdf file), paper file, laboratory file, medical record file, or any other medium described in the previous paragraph. A laboratory file or medical record file may be in tangible form or electronic form (e.g., computer readable form), in certain implementations. After a report is generated and transmitted, a report can be received by obtaining, via a suitable communication medium, a written and/or graphical representation comprising an outcome and/or classification, which upon review allows a healthcare professional or other qualified individual to make a determination as to one or more characteristics of a sample or source, or presence or absence of a genotype, phenotype, genetic variation and/or or medical condition for a test sample.

An outcome and/or classification may be provided by and obtained from a laboratory (e.g., obtained from a laboratory file). A laboratory file can be generated by a laboratory that carries out one or more tests for determining one or more characteristics of a sample or source and/or presence or absence of a genotype, phenotype, genetic variation and/or medical condition for a test sample. Laboratory personnel (e.g., a laboratory manager) can analyze information associated with test samples (e.g., test profiles, reference profiles, test values, reference values, level of deviation, patient information) underlying an outcome and/or classification. For calls pertaining to presence or absence of a genotype, phenotype, genetic variation and/or medical condition that are close or questionable, laboratory personnel can re-run the same procedure using the same (e.g., aliquot of the same sample) or different test sample from a test subject. A laboratory may be in the same location or different location (e.g., in another country) as personnel assessing the presence or absence of a genotype, phenotype, genetic variation and/or a medical condition from the laboratory file. For example, a laboratory file can be generated in one location and transmitted to another location in which the information for a test sample therein is assessed by a healthcare professional or other qualified individual, and optionally, transmitted to the subject from which the test sample was obtained. A laboratory sometimes generates and/or transmits a laboratory report containing a classification of presence or absence of genomic instability, a genotype, phenotype, a genetic variation and/or a medical condition for a test sample. A laboratory generating a laboratory test report sometimes is a certified laboratory, and sometimes is a laboratory certified under the Clinical Laboratory Improvement Amendments (CLIA).

An outcome and/or classification sometimes is a component of a diagnosis for a subject, and sometimes an outcome and/or classification is utilized and/or assessed as part of providing a diagnosis for a test sample. For example, a healthcare professional or other qualified individual may analyze an outcome and/or classification and provide a diagnosis based on, or based in part on, the outcome and/or classification. In some implementations, determination, detection or diagnosis of a medical condition, disease, syndrome or abnormality comprises use of an outcome and/or classification determinative of presence or absence of a genotype, phenotype, genetic variation and/or medical condition. Thus, provided herein are methods for diagnosing presence or absence of a genotype, phenotype, a genetic variation and/or a medical condition for a test sample according to an outcome or classification generated by methods described herein, and optionally according to generating and transmitting a laboratory report that includes a classification for presence or absence of the genotype, phenotype, a genetic variation and/or a medical condition for the test sample.

### Machines, software and interfaces

Certain processes and methods described herein (e.g., selecting a subset of sequence reads, generating a sequence reads profile, processing sequence read data, processing sequence read quantifications, determining one or more characteristics of a sample based on sequence read data or a sequence read profile) often are too complex for performing in the mind and cannot be performed without a computer, microprocessor, software, module or other machine. Methods described herein may be computer-implemented methods, and one or more portions of a method sometimes are performed by one or more processors (e.g., microprocessors), computers, systems, apparatuses, or machines (e.g., microprocessor-controlled machine).

Computers, systems, apparatuses, machines and computer program products suitable for use often include, or are utilized in conjunction with, computer readable storage media. Non-limiting examples of computer readable storage media include memory, hard disk, CD-ROM, flash memory device and the like. Computer readable storage media generally are computer hardware, and often are non-transitory computer-readable storage media. Computer readable storage media are not computer readable transmission media, the latter of which are transmission signals per se.

Provided herein are computer readable storage media with an executable program stored thereon, where the program instructs a microprocessor to perform a method described herein. Provided also are computer readable storage media with an executable program module stored thereon, where the program module instructs a microprocessor to perform part of a method described herein. Also provided herein are systems, machines, apparatuses and computer program products that include computer readable storage media with an executable program stored thereon, where the program instructs a microprocessor to perform a method described herein. Provided also are systems, machines and apparatuses that include computer readable storage media with an executable program module stored thereon, where the program module instructs a microprocessor to perform part of a method described herein.

Also provided are computer program products. A computer program product often includes a computer usable medium that includes a computer readable program code embodied therein, the computer readable program code adapted for being executed to implement a method or part of a method described herein. Computer usable media and readable program code are not transmission media (i.e., transmission signals per se). Computer readable program code often is adapted for being executed by a processor, computer, system, apparatus, or machine.

In some implementations, methods described herein (e.g., selecting a subset of sequence reads, generating a sequence reads profile, processing sequence read data, processing sequence read quantifications, determining one or more characteristics of a sample based on sequence read data or a sequence read profile) are performed by automated methods. In some implementations, one or more steps of a method described herein are carried out by a microprocessor and/or computer, and/or carried out in conjunction with memory. In some implementations, an automated method is embodied in software, modules, microprocessors, peripherals and/or a machine comprising the like, that perform methods described herein. As used herein, software refers to computer readable program instructions that, when executed by a microprocessor, perform computer operations, as described herein.

Machines, software and interfaces may be used to conduct methods described herein. Using machines, software and interfaces, a user may enter, request, query or determine options for using particular information, programs or processes (e.g., processing sequence read data, processing sequence read quantifications, and/or providing an outcome), which can involve implementing statistical analysis algorithms, statistical significance algorithms, statistical algorithms, iterative steps, validation algorithms, and graphical representations, for example. In some implementations, a data set may be entered by a user as input information, a user may download one or more data sets by suitable hardware media (e.g., flash drive), and/or a user may send a data set from one system to another for subsequent processing and/or providing an outcome (e.g., send sequence read data from a sequencer to a computer system for sequence read processing; send processed sequence read data to a computer system for further processing and/or yielding an outcome and/or report).

A system typically comprises one or more machines. Each machine comprises one or more of memory, one or more microprocessors, and instructions. Where a system includes two or more machines, some or all of the machines may be located at the same location, some or all of the machines may be located at different locations, all of the machines may be located at one location and/or all of the machines may be located at different locations. Where a system includes two or more machines, some or all of the machines may be located at the same location as a user, some or all of the machines may be located at a location different than a user, all of the machines may be located at the same location as the user, and/or all of the machine may be located at one or more locations different than the user.

A system sometimes comprises a computing machine and a sequencing apparatus or machine, where the sequencing apparatus or machine is configured to receive physical nucleic acid and generate sequence reads, and the computing apparatus is configured to process the reads from the sequencing apparatus or machine. The computing machine sometimes is configured to determine an outcome from the sequence reads (e.g., a characteristic of a sample).

A user may, for example, place a query to software which then may acquire a data set via internet access, and in certain implementations, a programmable microprocessor may be prompted to acquire a suitable data set based on given parameters. A programmable microprocessor also may prompt a user to select one or more data set options selected by the microprocessor based on given parameters. A programmable microprocessor may prompt a user to select one or more data set options selected by the microprocessor based on information found via the internet, other internal or external information, or the like. Options may be chosen for selecting one or more data feature selections, one or more statistical algorithms, one or more statistical analysis algorithms, one or more statistical significance algorithms, iterative steps, one or more validation algorithms, and one or more graphical representations of methods, machines, apparatuses, computer programs or a non-transitory computer-readable storage medium with an executable program stored thereon.

Systems addressed herein may comprise general components of computer systems, such as, for example, network servers, laptop systems, desktop systems, handheld systems, personal digital assistants, computing kiosks, and the like. A computer system may comprise one or more input means such as a keyboard, touch screen, mouse, voice recognition or other means to allow the user to enter data into the system. A system may further comprise one or more outputs, including, but not limited to, a display screen (e.g., CRT or LCD), speaker, FAX machine, printer (e.g., laser, ink jet, impact, black and white or color printer), or other output useful for providing visual, auditory and/or hardcopy output of information (e.g., outcome and/or report).

In a system, input and output components may be connected to a central processing unit which may comprise among other components, a microprocessor for executing program instructions and memory for storing program code and data. In some implementations, processes may be implemented as a single user system located in a single geographical site. In certain implementations, processes may be implemented as a multi-user system. In the case of a multi-user implementation, multiple central processing units may be connected by means of a network. The network may be local, encompassing a single department in one portion of a building, an entire building, span multiple buildings, span a region, span an entire country or be worldwide. The network may be private, being owned and controlled by a provider, or it may be implemented as an internet based service where the user accesses a web page to enter and retrieve information. Accordingly, in certain implementations, a system includes one or more machines, which may be local or remote with respect to a user. More than one machine in one location or multiple locations may be accessed by a user, and data may be mapped and/or processed in series and/or in parallel. Thus, a suitable configuration and control may be utilized for mapping and/or processing data using multiple machines, such as in local network, remote network and/or "cloud" computing platforms.

A system can include a communications interface in some implementations. A communications interface allows for transfer of software and data between a computer system and one or more external devices. Non-limiting examples of communications interfaces include a modem, a network interface (such as an Ethernet card), a communications port, a PCMCIA slot and card, and the like. Software and data transferred via a communications interface generally are in the form of signals, which can be electronic, electromagnetic, optical and/or other signals capable of being received by a communications interface. Signals often are provided to a communications interface via a channel. A channel often carries signals and can be implemented using wire or cable, fiber optics, a phone line, a cellular phone link, an RF link and/or other communications channels. Thus, in an example, a communications interface may be used to receive signal information that can be detected by a signal detection module.

Data may be input by a suitable device and/or method, including, but not limited to, manual input devices or direct data entry devices (DDEs). Non-limiting examples of manual devices include keyboards, concept keyboards, touch sensitive screens, light pens, mouse, tracker balls, joysticks, graphic tablets, scanners, digital cameras, video digitizers and voice recognition devices. Non-limiting examples of DDEs include bar code readers, magnetic strip codes, smart cards, magnetic ink character recognition, optical character recognition, optical mark recognition, and turnaround documents.

In some implementations, output from a sequencing apparatus or machine may serve as data that can be input via an input device. In certain implementations, sequence read information may serve as data that can be input via an input device. In certain implementations, mapped sequence reads may serve as data that can be input via an input device. In certain implementations, nucleic acid fragment size (e.g., length) may serve as data that can be input via an input device. In certain implementations, output from a nucleic acid capture process (e.g., genomic region origin data) may serve as data that can be input via an input device. In certain implementations, a combination of nucleic acid fragment size (e.g., length) and output from a nucleic acid capture process (e.g., genomic region origin data) may serve as data that can be input via an input device. In certain implementations, simulated data is generated by an in silico process and the simulated data serves as data that can be input via an input device. The term "in silico" refers to research and experiments performed using a computer. In silico processes include, but are not limited to, mapping sequence reads and processing mapped sequence reads according to processes described herein.

A system may include software useful for performing a process or part of a process described herein, and software can include one or more modules for performing such processes (e.g., sequencing module, logic processing module, data display organization module). The term "software" refers to computer readable program instructions that, when executed by a computer, perform computer operations. Instructions executable by the one or more microprocessors sometimes are provided as executable code, that when executed, can cause one or more microprocessors to implement a method described herein. A module described herein can exist as software, and instructions (e.g., processes, routines, subroutines) embodied in the software can be implemented or performed by a microprocessor. For example, a module (e.g., a software module) can be a part of a program that performs a particular process or task. The term "module" refers to a self-contained functional unit that can be used in a larger machine or software system. A module can comprise a set of instructions for carrying out a function of the module. A module can transform data and/or information. Data and/or information can be in a suitable form. For example, data and/or information can be digital or analogue. In certain implementations, data and/or information sometimes can be packets, bytes, characters, or bits. In some implementations, data and/or information can be any gathered, assembled or usable data or information. Non-limiting examples of data and/or information include a suitable media, pictures, video, sound (e.g. frequencies, audible or non-audible), numbers, constants, a value, objects, time, functions, instructions, maps, references, sequences, reads, mapped reads, levels, ranges, thresholds, signals, displays, representations, or transformations thereof. A module can accept or receive data and/or information, transform the data and/or information into a second form, and provide or transfer the second form to a machine, peripheral, component or another module. A microprocessor can, in certain implementations, carry out the instructions in a module. In some implementations, one or more microprocessors are required to carry out instructions in a module or group of modules. A module can provide data and/or information to another module, machine or source and can receive data and/or information from another module, machine or source.

A computer program product sometimes is embodied on a tangible computer-readable medium, and sometimes is tangibly embodied on a non-transitory computer-readable medium. A module sometimes is stored on a computer readable medium (e.g., disk, drive) or in memory (e.g., random access memory). A module and microprocessor capable of implementing instructions from a module can be located in a machine or in a different machine. A module and/or microprocessor capable of implementing an instruction for a module can be located in the same location as a user (e.g., local network) or in a different location from a user (e.g., remote network, cloud system). In implementations in which a method is carried out in conjunction with two or more modules, the modules can be located in the same machine, one or more modules can be located in different machine in the same physical location, and one or more modules may be located in different machines in different physical locations.

A machine, in some implementations, comprises at least one microprocessor for carrying out the instructions in a module. Sequence read quantifications (e.g., counts) sometimes are accessed by a microprocessor that executes instructions configured to carry out a method described herein. Sequence read quantifications that are accessed by a microprocessor can be within memory of a system, and the sequence read counts can be accessed and placed into the memory of the system after they are obtained. In some implementations, a machine includes a microprocessor (e.g., one or more microprocessors) which microprocessor can perform and/or implement one or more instructions (e.g., processes, routines and/or subroutines) from a module. In some implementations, a machine includes multiple microprocessors, such as microprocessors coordinated and working in parallel. In some implementations, a machine operates with one or more external microprocessors (e.g., an internal or external network, server, storage device and/or storage network (e.g., a cloud)). In some implementations, a machine comprises a module (e.g., one or more modules). A machine comprising a module often is capable of receiving and transferring one or more of data and/or information to and from other modules.

In certain implementations, a machine comprises peripherals and/or components. In certain implementations, a machine can comprise one or more peripherals or components that can transfer data and/or information to and from other modules, peripherals and/or components. In certain implementations, a machine interacts with a peripheral and/or component that provides data and/or information. In certain implementations, peripherals and components assist a machine in carrying out a function or interact directly with a module. Non-limiting examples of peripherals and/or components include a suitable computer peripheral, I/O or storage method or device including but not limited to scanners, printers, displays (e.g., monitors, LED, LCT or CRTs), cameras, microphones, pads (e.g., ipads, tablets), touch screens, smart phones, mobile phones, USB I/O devices, USB mass storage devices, keyboards, a computer mouse, digital pens, modems, hard drives, jump drives, flash drives, a microprocessor, a server, CDs, DVDs, graphic cards, specialized I/O devices (e.g., sequencers, photo cells, photo multiplier tubes, optical readers, sensors, etc.), one or more flow cells, fluid handling components, network interface controllers, ROM, RAM, wireless transfer methods and devices (Bluetooth, WiFi, and the like,), the world wide web (www), the internet, a computer and/or another module.

Software often is provided on a program product containing program instructions recorded on a computer readable medium, including, but not limited to, magnetic media including floppy disks, hard disks, and magnetic tape; and optical media including CD-ROM discs, DVD discs, magneto-optical discs, flash memory devices (e.g., flash drives), RAM, floppy discs, the like, and other such media on which the program instructions can be recorded. In online implementation, a server and web site maintained by an organization can be configured to provide software downloads to remote users, or remote users may access a remote system maintained by an organization to remotely access software. Software may obtain or receive input information. Software may include a module that specifically obtains or receives data (e.g., a data receiving module that receives sequence read data and/or mapped read data) and may include a module that specifically processes the data (e.g., a processing module that processes received data (e.g., filters, normalizes, provides an outcome and/or report). The terms "obtaining" and "receiving" input information refers to receiving data (e.g., sequence reads, mapped reads) by computer communication means from a local, or remote site, human data entry, or any other method of receiving data. The input information may be generated in the same location at which it is received, or it may be generated in a different location and transmitted to the receiving location. In some implementations, input information is modified before it is processed (e.g., placed into a format amenable to processing (e.g., tabulated)).

Software can include one or more algorithms in certain implementations. An algorithm may be used for processing data and/or providing an outcome or report according to a finite sequence of instructions. An algorithm often is a list of defined instructions for completing a task. Starting from an initial state, the instructions may describe a computation that proceeds through a defined series of successive states, eventually terminating in a final ending state. The transition from one state to the next is not necessarily deterministic (e.g., some algorithms incorporate randomness). By way of example, and without limitation, an algorithm can be a search algorithm, sorting algorithm, merge algorithm, numerical algorithm, graph algorithm, string algorithm, modeling algorithm, computational genometric algorithm, combinatorial algorithm, machine learning algorithm, cryptography algorithm, data compression algorithm, parsing algorithm and the like. An algorithm can include one algorithm or two or more algorithms working in combination. An algorithm can be of any suitable complexity class and/or parameterized complexity. An algorithm can be used for calculation and/or data processing, and in some implementations, can be used in a deterministic or probabilistic/predictive approach. An algorithm can be implemented in a computing environment by use of a suitable programming language, non-limiting examples of which are C, C++, Java, Perl, Python, Fortran, and the like. In some implementations, an algorithm can be configured or modified to include margin of errors, statistical analysis, statistical significance, and/or comparison to other information or data sets (e.g., applicable when using a neural net or clustering algorithm).

In certain implementations, several algorithms may be implemented for use in software. These algorithms can be trained with raw data in some implementations. For each new raw data sample, the trained algorithms may produce a representative processed data set or outcome. A processed data set sometimes is of reduced complexity compared to the parent data set that was processed. Based on a processed set, the performance of a trained algorithm may be assessed based on sensitivity and specificity, in some implementations. An algorithm with the highest sensitivity and/or specificity may be identified and utilized, in certain implementations.

In certain implementations, simulated (or simulation) data can aid data processing, for example, by training an algorithm or testing an algorithm. In some implementations, simulated data includes hypothetical various samplings of different groupings of sequence reads. Simulated data may be based on what might be expected from a real population or may be skewed to test an algorithm and/or to assign a correct classification. Simulated data also is referred to herein as "virtual" data. Simulations can be performed by a computer program in certain implementations. One possible step in using a simulated data set is to evaluate the confidence of identified results, e.g., how well a random sampling matches or best represents the original data. One approach is to calculate a probability value (p-value), which estimates the probability of a random sample having better score than the selected samples. In some implementations, an empirical model may be assessed, in which it is assumed that at least one sample matches a reference sample (with or without resolved variations). In some implementations, another distribution, such as a Poisson distribution for example, can be used to define the probability distribution.

A system may include one or more microprocessors in certain implementations. A microprocessor can be connected to a communication bus. A computer system may include a main memory, often random access memory (RAM), and can also include a secondary memory. Memory in some implementations comprises a non-transitory computer-readable storage medium. Secondary memory can include, for example, a hard disk drive and/or a removable storage drive, representing a floppy disk drive, a magnetic tape drive, an optical disk drive, memory card and the like. A removable storage drive often reads from and/or writes to a removable storage unit. Non-limiting examples of removable storage units include a floppy disk, magnetic tape, optical disk, and the like, which can be read by and written to by, for example, a removable storage drive. A removable storage unit can include a computer-usable storage medium having stored therein computer software and/or data.

A microprocessor may implement software in a system. In some implementations, a microprocessor may be programmed to automatically perform a task described herein that a user could perform. Accordingly, a microprocessor, or algorithm conducted by such a microprocessor, can require little to no supervision or input from a user (e.g., software may be programmed to implement a function automatically). In some implementations, the complexity of a process is so large that a single person or group of persons could not perform the process in a timeframe short enough for determining one or more characteristics of a sample.

In some implementations, secondary memory may include other similar means for allowing computer programs or other instructions to be loaded into a computer system. For example, a system can include a removable storage unit and an interface device. Non-limiting examples of such systems include a program cartridge and cartridge interface (such as that found in video game devices), a removable memory chip (such as an EPROM, or PROM) and associated socket, and other removable storage units and interfaces that allow software and data to be transferred from the removable storage unit to a computer system.

### Methods for analyzing nucleic acids

Provided herein are methods for analyzing nucleic acids.

Provided herein are methods for assessing the purity and/or quality of nucleic acid. Purity and/or quality of nucleic acid may be assessed using a single-stranded library preparation method described herein.

In some implementations, a single-stranded library preparation method described herein may be used to assess the purity and/or quality of single-stranded nucleic acid (ssNA). ssNAs may include a single ssNA species (e.g., ssNAs having the same sequence and length) or may include a pool of ssNA species (e.g., ssNAs having different sequences and/or lengths). In some implementations, ssNA comprises single-stranded oligonucleotides. In some implementations, single-stranded oligonucleotides are commercially produced. In some implementations, single-stranded oligonucleotides are produced by the user. In some implementations, ssNA comprises single-stranded probes. In some implementations, single-stranded probes are commercially produced. In some implementations, single-stranded probes are produced by the user.

In some implementations, a single-stranded library preparation method described herein may be used to assess the purity and/or quality of single-stranded ribonucleic acid (ssRNA). ssRNAs may include a single ssRNA species (e.g., ssRNAs having the same sequence and length) or may include a pool of ssRNA species (e.g., ssRNAs having different sequences and/or lengths). In some implementations, ssRNA comprises single-stranded RNA oligonucleotides. In some implementations, single-stranded RNA oligonucleotides are commercially produced. In some implementations, single-stranded RNA oligonucleotides are produced by the user. In some implementations, ssRNA comprises single-stranded RNA probes. In some implementations, single-stranded RNA probes are commercially produced. In some implementations, single-stranded RNA probes are produced by the user.

In some implementations, a single-stranded library preparation method described herein may be used to assess the purity and/or quality of single-stranded complementary deoxyribonucleic acid (sscDNA). sscDNAs may include a single sscDNA species (e.g., sscDNAs having the same sequence and length) or may include a pool of sscDNA species (e.g., sscDNAs having different sequences and/or lengths). In some implementations, sscDNA comprises single-stranded cDNA oligonucleotides. In some implementations, single-stranded cDNA oligonucleotides are commercially produced. In some implementations, single-stranded cDNA oligonucleotides are produced by the user. In some implementations, sscDNA comprises single-stranded cDNA probes. In some implementations, single-stranded cDNA probes are commercially produced. In some implementations, single-stranded cDNA probes are produced by the user.

The purity and/or quality of ssNA, ssRNA, and/or sscDNA may be assessed according to an assessment of fragment length. Fragment length may be determined using any suitable method for determining fragment length. In some implementations, fragment length is determined according to the length of a single-end sequencing read (e.g., where the read length covers the length of the entire fragment). In some implementations, fragment length is determined according to mapped positions of paired-end sequencing reads. In some implementations, the purity and/or quality of ssNA, ssRNA, and/or sscDNA is assessed according to a fragment length profile. A fragment length profile may include quantifications of fragments having particular lengths. Example fragment length profiles are provided in Fig. 31. In some implementations, the purity and/or quality of ssNA, ssRNA, and/or sscDNA is assessed according to an amount of a major ssNA, ssRNA, and/or sscDNA species and an amount of a minor ssNA, ssRNA, and/or sscDNA species in the fragment length profile. A major species generally refers to the fragment length most abundant in the sample. A major species may refer to the intended or expected fragment length of the ssNA, ssRNA, and/or sscDNA being assessed. For example, for an oligonucleotide designed to include exactly 50 nucleotides, an assessment of the purity and/or quality of that oligonucleotide may yield a major species length of 50 nucleotides. A minor species generally refers to the remaining fragment lengths that are not the major species. A minor species may refer to the unintended or unexpected fragment lengths of the ssNA, ssRNA, and/or sscDNA being assessed. For example, for an oligonucleotide designed to include exactly 50 nucleotides, an assessment of the purity and/or quality of that oligonucleotide may yield a minor species having lengths greater than 50 and/or less than 50, but not exactly 50 nucleotides. The purity and/or quality of ssNA, ssRNA, and/or sscDNA may be expressed as a ratio or percentage. For example, an oligonucleotide may be considered 90% pure for the major species if 90% of the oligonucleotides in the sample are of the major species fragment length and 10% of the oligonucleotides in the sample (collectively) are of minor species fragment length.

The amount of nicked DNA in a sample can be estimated or measured. For example, sequencing libraries can be prepared from a sample before and after nick repair. Sequencing results for the two libraries can be compared and the amount of nicked DNA can be estimated or measured. Nicked DNA can be cfDNA, for example generated due to endo and exonuclease activity on genomic DNA within cells undergoing apoptosis and subsequently in the blood stream. The initial nuclease activity can involve endonuclease activity between nucleosomes or nicking activity of DNasel on the nucleosomes. Understanding the nucleic acid regions that are susceptible for nicking can be informative of nucleosome occupancy. Other sources of nicked DNA include but are not limited to FFPE samples, hair, degraded samples, and in vitro tests of nickase enzymes. Single-stranded library preparation methods such as those of the present disclosure can capture nicked fragments. Additionally, methods of the present disclosure retain the end generated from nicking. Performing methods of the present disclosure directly on a nicked molecule would generate 3 strands of different length - 1 long and 2 shorter molecules (e.g., FIG. 53B). Treatment with a nick-sealing enzyme (e.g. HiFi Taq ligase) would ligate the two nicked strands; subsequent performance of methods of the present disclosure with this sealed dsDNA would yield 2 strands of similar lengths without visibility into the ends generated at the nicks (e.g., FIG. 53A). Comparison of sequences (and fragment ends) obtained from the two libraries would show that in the library where the nicks were sealed, there are fewer short fragments and fewer reads that have sequences that flank the nicked region.

In an example, known nicks were generated in gDNA using N.BstNBI that generates nicks at 5'GAGTCNNNN^N3'. One portion of the nicked gDNA sample was nick-sealed with HiFi Taq ligase and one portion were not. Single-stranded library preparation was conducted on both as discussed herein, and libraries were sequenced and compared. Control gDNA that was never nicked showed 0.07% of sequence reads ending in GAGTCNNNN; nicked DNA that was not sealed showed 15.74% of sequence reads ending in GAGTCNNNN; nicked and nick-sealed DNA showed 10.67% of sequence reads ending in GAGTCNNNN.

Pools of nucleic acids (e.g., aptamers, siRNAs, oligonucleotide probes) can be sequenced without need for the nucleic acids to comprise flanking regions such as primer binding sites, which may affect their properties. Pools of nucleic acids for a given purpose can be generated, subjected to one or more rounds of selection for desired properties, and sequenced via single-stranded library preparation methods of the present disclosure. For example, a random pool of aptamers or siRNAs can be generated, subjected to one or more rounds of positive and/or negative selection (e.g., positive selection for binding to desired targets, negative selection for off-target binding), and successful candidates can be sequenced via methods of the present disclosure without need for the random aptamers or siRNAs to include flank regions for sequencing; the presence of such flanking regions may impact aptamer or siRNA performance.

In an example, a random pool of nucleic acids (e.g., is synthesized (e.g., aptamers, siRNAs, oligonucleotide probes) via chemical synthesis or transcription from synthesized DNA. The random pool is then subjected to one or more rounds of positive selection and/or one or more rounds of negative selection. Positive selection can include incubation with a desired binding target under increasingly stringent binding conditions. Negative selection can include incubation with off-target binding substrates under increasingly favorable binding conditions. Binding conditions can include but are not limited to temperature, salt concentration, pH, magnetic field, crowding agents, competitive binding agents, inhibitors, and other conditions. Sequencing via methods of the present disclosure can be performed before selection, in between rounds of selection, and/or after selection is complete to allow for bioinformatic analysis of the pool and changes thereto. UMls or other barcodes can be used to get a numeric or absolute count of the relative quantities of nucleic acid species in the pool. For example, positive selection can be conducted for n rounds in the presence of a desired binding target, with sequencing conducted on each bound pool separately to monitor how the bound sequence pool changes with different selection stringencies. Different clusters of nucleic acid sequences can be found during different rounds of selection. In some instances, bound nucleic acids from each positive selection round can go through the rest of selection and library preparation process separately to monitor how the bound nucleic acid pool changes with different selection stringencies, as different clusters of nucleic acid sequences can be found during different rounds of selection.

### Kits

Provided in certain implementations are kits. The kits may include any components and compositions described herein (e.g., scaffold adapters and components/subcomponents thereof, oligonucleotides, oligonucleotide components/regions, scaffold polynucleotides, scaffold polynucleotide components/regions, nucleic acids, single-stranded nucleic acids, primers, single-stranded binding proteins, enzymes) useful for performing any of the methods described herein, in any suitable combination. Kits may further include any reagents, buffers, or other components useful for carrying out any of the methods described herein. For example, a kit may include one or more of a plurality of scaffold adapter species or a plurality of scaffold polynucleotide species and corresponding oligonucleotide components, a kinase adapted to 5' phosphorylate nucleic acids (e.g., a polynucleotide kinase (PNK)), a DNA ligase, and any combination thereof.

Kits may include components for capturing single-stranded DNA and/or single-stranded RNA. Kits for capturing single-stranded DNA may be configured such that a user provides double-stranded or single-stranded DNA. Kits for capturing single-stranded RNA may be configured such that a user provides cDNA (either single or double stranded), or provides RNA (e.g., total RNA or rRNA-depleted RNA). A kit for capturing single-stranded RNA may include rRNA depletion reagents, mRNA enrichment reagents, fragmentation reagents, cDNA synthesis reagents, and/or RNA digestion reagents.

Components of a kit may be present in separate containers, or multiple components may be present in a single container. Suitable containers include a single tube (e.g., vial), one or more wells of a plate (e.g., a 96-well plate, a 384-well plate, and the like), and the like.

Kits may also comprise instructions for performing one or more methods described herein and/or a description of one or more components described herein. For example, a kit may include instructions for using scaffold adapters described herein, or components thereof, to capture single-stranded nucleic acid fragments and/or to produce a nucleic acid library. Instructions and/or descriptions may be in printed form and may be included in a kit insert. In some implementations, instructions and/or descriptions are provided as an electronic storage data file present on a suitable computer readable storage medium, e.g., portable flash drive, DVD, CD-ROM, diskette, and the like. A kit also may include a written description of an internet location that provides such instructions or descriptions.

### Examples

The examples set forth below illustrate certain implementations and do not limit the technology.

### Example 1: Single-stranded specific library prep for cell-free DNA (cfDNA)

In this Example, a single-stranded DNA (ssDNA) library preparation method and modifications thereto are described. The ssDNA library preparation method described below captures both double-stranded (dsDNA) and ssDNA molecules by making all DNA single-stranded prior to adapter ligation.

### Base protocol

1) Create and maintain ssDNA by heating the DNA to 95°C for 3 minutes in the presence of a thermal-stable single-stranded DNA binding protein (SSB) and then snap cool the tube on ice.
2) Prepare appropriate dilution of scaffold adapters and add a) 6x excess of both P5 and P7 scaffold adapter combination and b) a phosphorylation/ligation master mix resulting in 18.5% PEG 8000 final concentration, 1 mM final ATP, 11 mM final DTT, 10 mM final MgCl₂, 50 mM Tris-HCl pH 7.5, 2000 units of T4 DNA ligase, and 10 units of PNK, in an 80 µl reaction volume.
3) Incubate 1 hour at 37°C.
4) Perform a column purification clean using the Qiagen MINELUTE PCR Purification Kit.
5) Perform index PCR.

### Modifications and improvements to base protocol

Described below are various modifications to the base protocol described above. Certain modifications resulted in improvements to the protocol such as, for example, better library quality, increased yield, faster library generation, fewer or lower dose reagents, fewer steps, and the like.

### Ligase

T4 DNA ligase input amounts from 800 units to 2000 units were tested. The results showed 800 units was more than sufficient to produce results similar to 2000 units.

### Incubation time

Various incubation times were tested. Reaction times tested were 5 minutes - 60 minutes.

Decent libraries were produced with incubation times as short as 5 minutes. Increasing incubation time to 60 minutes increased DNA yield but the increase from 30 minutes to 60 minutes was minimal.

### Purification after ssDNA adapter ligation/phosphorylation reaction

Different purification methods were tested. The results showed purification method did not matter. Solid Phase Reversible Immobilization (SPRI) magnetic bead purification worked as well as column purification. Multiple SPRI bead manufacturers were tested, and all worked well. Also tested was proceeding from the ligation/phosphorylation reaction straight into index PCR without a purification step. While the method without purification worked, it produced less yield and more problematic DNA size profiles post index PCR.

### Maintaining ssDNA

The method was performed with and without single stranded binding proteins (SSBs). The results showed SSBs were not necessary for the protocol, even in low complexity mixtures such as oligo pools. The results showed the DNA remained sufficiently single-stranded after heat denaturation and snap cool, even in the absence of SSB. SSB titrations and SSBs from various suppliers were tested and no difference was observed between any of the conditions tested and the no SSB controls. Parameters analyzed included adapter dimer %, DNA library yield, % fragments between 30-130 base pairs (bp), % mapping rate, % duplication rate, % reads pass filter, pass filter reads mapping rate, and library product size distribution. Results of the SSB vs. no SSB testing are shown in Figs. 2A, 2B, 3A, and 3B.

### Scaffold adapters: substrate DNA ratio

Various scaffold adapter to substrate DNA ratios were tested. The results showed the ideal ratio of scaffold adapters to substrate DNA was around 30x. The results also showed a 6x ratio in the base protocol was too low.

### Scaffold adapter modifications

Pre-treating the scaffold adapters with a phosphatase prior to performing the ssDNA ligation/phosphorylation reaction improved the results, increasing yield and lowering adapter dimers. The improvements are shown in Fig. 4.

Also tested were various ligation/extension blocking modifications on the terminal ends of the scaffold adapters. All modifications tested worked equally well.

### Ligation/phosphorylation master mix

The ingredients for the adapter ligation/phosphorylation reaction were premade and stored together for eases of use. The master mix included: tris buffer pH8, DTT, MgCl₂, ATP, PEG 8000, T4 DNA ligase, and T4 PNK.

### Phosphorylation

The phosphorylation (e.g., PNK) part of the ligation/phosphorylation reaction was not necessary to produce quality libraries. The PNK was omitted from the protocol completely or placed upstream of the ligation step, and quality libraries were produced either way. Also, as long as the PNK is included upstream or co-occurring with the ligation step, the substrate DNA can be dephosphorylated.

### Index PCR

Quality libraries were produced with various high-fidelity thermostable polymerases. Quality libraries were produced with primer concentrations anywhere between 0.2 µM - 4 µM final. 1 µM was selected as a final primer concentration.

### PCR Free ssDNA libraries

If scaffold adapters are synthesized to contain all requisite DNA sequences necessary for sequencer compatibility (referred to as "full-length adapters"), quality libraries could be constructed without index PCR. See Figs. 5A and 5B. In Fig. 5A, the scaffold polynucleotide (bottom strand) and the oligonucleotide (top strand) of the scaffold adapters each include a flow cell binding region. In Fig. 5B, the oligonucleotide (top strand) of the scaffold adapters includes a flow cell binding region and the scaffold polynucleotide (bottom strand) of the scaffold adapters excludes a flow cell binding region.

### PEG concentration

Final PEG 8000 concentrations were tested from 0 -30% in the ligation/phosphorylation reaction and the results showed 18.5% was the ideal amount to include.

### Adapter dimer reduction

One issue with the single-stranded DNA protocol described above is that it can produce a high percent of adapter dimers, most likely due to the structure of the scaffold adapters. To combat adapter dimer formation, several different techniques were developed and tested, which had variable levels of success. One technique was to pretreat the scaffold adapters with a phosphatase prior to performing the ssDNA ligation/phosphorylation reaction (described above). Another technique was to perform volumetric titrations of the ligation/phosphorylation reaction (described below). Other techniques tested are described below.

Post-index PCR SPRI (e.g., 18% PEG 8000) titrations, and serial and sequential SPRI cleans, were performed. For example, two or more serial 1.2x SPRls performed back-to back lowered the % adapter dimers and increased the relative amount of library to adapter dimer. Fig. 18 shows a gel image and table demonstrating reduction of dimers after each of four serial 1.2x SPRI cleans. The effect of serial SPR! on the sequence data reduced the number of reads that were discarded due to adapter artifacts, thereby increasing the amount of usable data and mappability to the human genome. There was a slight loss in the smallest fragment length categories (i.e., <100 bp). Sequential SPRI - which involves incubating the sample with 0.6x SPRI for some minutes followed by the second addition of 0.6x beads to a final amount of 1.2x SPRI, also reduced dimers and increased the library size of interest. Fig. 19 shows a gel and an Agilent Tapestation trace of a sequential SPRI on cfDNA, which shows disproportionate recovery of library sized molecules relative to adapter dimer sized molecules.

Another approach was to employ the use of a selective restriction enzyme that discriminately digests the dimers pre-index PCR in conjunction with a complementary oligo to the dimers, and post index PCR with or without the complementary oligo. In this example, a short oligo that is complementary only to a presumed adapter dimer was added and incubated after hybridization and ligation, forming a double-stranded DNA fragment, followed by the addition of nuclease Xbal, which cuts at T*CTAGA recognition site present within the double-stranded adapter dimer DNA. Fig. 20 shows examples of presumed adapter dimer formations, the single-stranded form of an adapter dimer (or oligo dimer), and the addition of an oligo that anneals only to a single-stranded dimer. In this example, an Xbal recognition site formed when the double-stranded hybridization product was created. During PCR, an adapter dimer will become double-stranded. For this reason, Xbal treatment can be performed after PCR without the above oligo. While reduction in adapter dimer was seen following use of Xbal with and without the oligo, one risk or tradeoff with this approach is the depletion of Xbal sites in the genomic DNA of interest. The retention of Xbal sites may be increased by denaturing the sample and reannealing before Xbal treatment. The high complexity of genomic DNA may prevent the reannealing of the gDNA of interest while the low complexity of adapter dimers may cause higher rates of reannealing, rendering the adapter dimers ripe for targeting by the nuclease.

In certain configurations, the structure of the scaffold adapter was modified to form a hairpin (see Fig. 8) or modified by adding phosphorothioate bonds to increase the rigidity of the scaffold adapter and help prevent nuclease degradation.

### Ligation/phosphorylation reaction volume

Volumetric titrations were performed for the ligation/phosphorylation reaction. The results showed lowering the entire reaction volume increased yield and suppressed adapter dimer formation. Without being limited by theory, this may be the result of the ratio of DNA molecule ends to adapter/scaffold molecules per unit of volume in the presence of 18.5% PEG. The ratio that produced the highest quality libraries from cfDNA with the best insert distribution and minimal adapter dimer formation was when the substrate DNA ends were at 0.4 femtomoles per microliter (fmol/µl) in the presence of 18.5% PEG 8000 and each scaffold adapter was at 10 fmol/µl. This ratio can be achieved at any volume and restrictions can be overcome by increasing the volume or input DNA mass as needed. This ratio may be expressed in many different ways and units. For example, one protocol includes a 25 µl final reaction volume, 1 ng of cell-free DNA input, and the addition of 1.6 picomoles of each scaffold adapter.

### Input (substrate) DNA titration

Input DNA amounts from 5 ng to 100 pg were tested. Libraries were made from all input amounts, but quality dropped off below 250 pg.

### Ligation/phosphorylation reaction temperature

Reaction temperatures from 16°C - 37°C were tested. The best temperature was 37°C with decreasing DNA yield and higher adapter dimer % as temperature decreased.

### Unique molecular identifier (UMI)

Scaffold adapters were modified to include a UMI polynucleotide. A few configurations were designed to integrate UMIs into the scaffold adapters. For example, UMIs were added either in short adapters directly flanking the insert using Ns or inosines as the random bases, or, placed next to an indexing barcode in the full-length adapters (see Fig. 6A or 6B). In the configuration shown in Fig. 6B, a full-length P5 and P7 adapter is ligated during the reaction. UMls can comprise universal bases, random bases, or known bases. UMIs can be 1, 2, 3, 4, 5, 6, 7, 8, 9, 10, or more bases in length. In certain configurations, short P5/P7 were ligated and the adapters were made full-length during PCR amplification. The method of placing a UMI of random N bases next to the index in the P7 adapter worked well.

A UMI polynucleotide can be incorporated into the single-stranded ligation product or library prior to index PCR using a single primer extension method and a strand displacing polymerase, e.g. Bst polymerase. Primers can each contain a sequence that anneals to and primes from a portion of the scaffold adapter located 3' of the ssNA, and a UMI polynucleotide. In some instances, the primers also include a sequencing index or barcode sequence and flow cell binding sites (see Fig. 7). The primers may or may not include a blocking modification at the 5' end. In one example workflow, the ssNA-scaffold adapter ligation product can be denatured, releasing the scaffold polynucleotide, resulting in a single-stranded ligation product (ssNA ligated to the oligonucleotide component of the scaffold adapter). The free scaffold polynucleotide and the single-stranded ligation product each contain blocking modifications and cannot be extended. The UMI or index-containing primers can be extended (the ssNA ligation product acting as a template) with a strand displacing polymerase to create a library strand complete only on the P7 side of the molecule. After extension, SPRI purification can be performed. To complete the full indexed library molecule, PCR can be performed using IS4 or another version of the P5 index primer and a primer complementary to the flow cell binding sites (see Fig. 7). In one variation of the workflow, excess scaffold polynucleotides containing deoxyuridine cleavage sites, released from the ssNA ligation product during denaturation, are degraded with DNA-uracil glycosylase to prevent them from annealing.

### Hairpin adapters

To reduce the formation of adapter dimers, a hairpin structure for the scaffold adapters was designed (see Fig. 8). The method using hairpin adapters worked but did not perform better than the original adapter design.

### Alternative adapter designs

Figs. 9, 10 and 11 describe modifications to the scaffold adapters that involve alternative order of operation, delayed addition of enzymes or other reagents, terminal DNA modifications, and/or other ligase types.

One adapter configuration and workflow includes staged ligation and enzyme delay (Fig. 9). Non-phosphorylated or dephosphorylated P5 and P7 scaffold adapters are combined with single-stranded DNA template. A ligation master mix that excludes PNK is added to the adapter/template combination and is incubated at room temperature for 5 minutes. The P5 adapter ligates to DNA template having a 5' phosphate. Following P5 ligation, PNK is added to the reaction and the temperature is brought to 37°C. The P7 adapter is phosphorylated by the PNK and the 5' phosphorylated end of the P7 adapter ligates to the 3' end of the DNA template. This workflow was performed and reduced dimers by half and roughly doubled the yield compared to the SOP. One variation of this workflow includes delaying addition of the P7 adapter until after the P5 adapter has ligated to the DNA template, or delaying addition of the P5 adapter until after the P7 adapter has ligated to the DNA template. One variation of this workflow includes first adding a phosphorylated P7, but delaying the addition of P5 and PNK by 20 minutes. This workflow also was performed and reduced dimers by half and roughly doubled the yield compared to the SOP.

Another adapter configuration and workflow includes staged ligation, a 5'App P7 adapter, and ATP delay (Fig. 10). The 5'App P7 scaffold adapters are combined with single-stranded DNA template. A ligation master mix that excludes ATP is added to the adapter/template combination and is incubated at room temperature for 5 minutes. The 5'App end of the P7 adapter ligates to the 3' end of the DNA template without ATP. Following P7 ligation, non-phosphorylated or dephosphorylated P5 scaffold adapters and ATP are added to the reaction. The P5 adapter ligates to DNA template having a 5' phosphate.

Another adapter configuration and workflow includes staged ligation and a single-stranded P5 adapter with a 3' phosphate (Fig. 11). Template DNA is dephosphorylated and combined with the single-stranded P5 adapters with a 3' phosphate. A ligase that can ligate a DNA 3' phosphate end to a 5' OH end and prefers to ligate single strands (e.g., RtcB) is added to the adapter/template combination. The 3' phosphate end of the P5 adapter ligates to the 5' OH end of the DNA template. 5' phosphorylated P7 scaffold adapters and T4 DNA ligase are then added to the reaction, and the 5' phosphorylated end of the P7 adapter ligates to the 3' end of the DNA template. One variation of this workflow includes combining the 5' phosphorylated P7 scaffold adapters with the DNA template when the single-stranded P5 adapters are added.

### Example 2: Single-stranded specific library prep for RNA

The single-stranded DNA library preparation method described in Example 1 was modified for the conversion of RNA molecules into sequencing libraries. In one configuration, first strand DNA synthesis products generated from rRNA depleted or mRNA enriched total RNA are incorporated into the single-stranded DNA library preparation methods described herein. In another configuration, rRNA depleted or mRNA enriched total RNA is directly incorporated into the single-stranded DNA library preparation methods described herein, followed by first strand DNA synthesis. In addition to fewer enzymatic steps, time savings, and reagent savings, applying the single-stranded DNA library preparation method to RNA has several biological benefits as well over existing technology. For example, due to the single-stranded nature of the technology described herein, second strand synthesis can be omitted altogether. The resulting RNA sequencing libraries can produce stranded RNA sequencing libraries, resulting in more accurate transcript mapping.

A general workflow for one configuration is shown in Fig. 15 and includes the following. Total RNA is extracted and proceeds through an rRNA depletion kit or an mRNA enrichment kit. RNA is then fragmented and first strand synthesis cDNA is created using random primers and a reverse transcriptase enzyme. RNaseH digestion is performed to ensure removal of RNA from the RNA-DNA hybrids. The first strand cDNA is fed into a single-stranded DNA library preparation kit with few modifications. Often, cDNA is denatured to remove secondary structures. Single-stranded binding protein may be included, but generally is not necessary. Scaffold adapters described herein are ligated to the first strand cDNA in the presence of PNK to phosphorylate the DNA ends. Other than phosphorylation, there is no alteration to the native termini. The ligation products are subjected to clean-up and purification, followed by amplification by PCR (e.g., index PCR). The amplification products are then subjected to clean-up and purification.

In a specific example of the workflow described above, total RNA is extracted and proceeds through an rRNA depletion kit or an mRNA enrichment kit. RNA is then fragmented, heat denatured (94°C for 15 minutes), and first strand synthesis cDNA is created using random hexamers/ random octamers/polyT primers or commercial random primers, and an M-MLV reverse transcriptase enzyme. RNaseH digestion is performed (at 37°C for 30 minutes) to ensure removal of RNA from the RNA-DNA hybrids. cDNA is heat denatured to remove secondary structures, then snap cooled. Scaffold adapters described herein are ligated to the first strand cDNA in the presence of PNKto phosphorylate the DNA ends. The ligation products are subjected to clean-up and purification, followed by amplification by index PCR. This method was performed and libraries were successfully constructed. Results of an analysis of the libraries is provided in Figs. 12, 13, and 14.

The above protocol may include one or more variations. For example, scaffold adapters can be ligated directly to the RNA post rRNA depletion (or mRNA enrichment) and RNA fragmentation. Then first strand synthesis can use a single-stranded adapter specific primer. This alternative protocol also negates second strand synthesis but involves additional RNA processing (fragmentation) upstream of cDNA creation (see the workflow of Fig. 16). One variation of this method was performed and libraries were successfully constructed (see workflow shown in Fig. 50). In this workflow, total RNA input undergoes mRNA enrichment and/or rRNA depletion, RNA is sheared (e.g., BIORUPTOR or other fragmentation method), RNA fragments are ligated to DNA scaffold adapters described herein and SPRI purified, and a combined reverse transcriptase and PCR amplification step is performed. In the combined step, the DNA/RNA hybrid ligation products are converted to cDNA by a reverse transcriptase (e.g., M-MLV reverse transcriptase), and the cDNA molecules are amplified by PCR (e.g., using NEB's ONETAQ One-Step RT-PCR and PCR primers that prime off of ssPrep Illumina-compatible adapters).

Other variations of the protocol described in this Example may include 1) performing the method with or without rRNA depletion (mRNA enrichment) where mRNA enrichment, fragmentation, and cDNA synthesis occur in one step by eliminating ribosomal RNA binding oligos in the random primer cocktail; 2) performing the method with or without RNA fragmentation (e.g., if using sequencers other than Illumina); 3) performing the method with or without RNase H digestion; 4) performing the method with or without single-stranded DNA binding protein; 5) performing the method using rSAP-treated adapters or non-rSAP treated adapters; 6) performing the method where PNK treatment is decoupled and put upstream or not; 7) performing the method using any type of clean-up or without clean-up before PCR; and 8) performing the method with various RNA input amounts. Other variations include combining certain steps within a workflow. For example, Fig. 17 shows modifications A, B, and C which combine certain steps in a workflow. Fig. 50 shows an example workflow where reverse transcription of the RNA/DNA hybrid ligation product and amplification of the cDNA product are combined in a single step.

### Example 3: Directional RNA-Seq Library Preparation NGS assay (ssPrep for RNA)

RNA-Seq is a next generation sequencing (NGS) workflow used for gene expression profiling and whole transcriptome analyses. Single-stranded library prep (ssPrep) for RNA described herein and shown in Fig. 21 is a directional RNA-Seq library preparation method that uses unique NGS adapters to generate libraries directly from first strand cDNA, eliminating second strand synthesis and DNA end-repair. The result is improved library quality with significant reductions in cost and time.

Features of ssPrep for RNA include: workflow directly from first strand cDNA to Illumina^{®} sequence-ready library in ~2 hours; user-preferred first strand cDNA synthesis protocol may be used; kit contains reagents for adapter ligation, indexing PCR and magnetic beads for bead purification steps; optimized for 10 ng of mRNA, minimal hands-on time, and sequence-ready libraries produced with very few PCR cycles and reduced bias. ssPrep for RNA works in a one-step reaction that simultaneously prepares template first strand cDNA molecules for ligation without end-polishing and ligates scaffold adapters (i.e., scaffold adapters described herein) for sequencing on Illumina^{®} platforms. Downstream applications may include gene expression profiling, isoform analysis, and splice variant discovery, for example.

### mRNA input range

In this Example, improved quality metrics for RNA-Seq libraries made with an ssPrep for RNA protocol are demonstrated. Metrics for an ssPrep for RNA method were compared to those of a commercially available double-stranded (dsPrep) method (i.e., NEBNEXT ULTRA II Directional library preparation method). Using a spike-in mRNA control, ssPrep for RNA generated libraries that had high concordance between replicates, retained library strandedness, and captured true mRNA GC composition.

Total RNA was extracted from lung cancer cell line H2126 using Qiagen All Prep DNA, RNA, Protein kit. mRNA was isolated with NEBNEXT PolyA magnetic mRNA isolation module. 1-20 ng of mRNA was used as input for first strand cDNA synthesis using the NEBNEXT ULTRA II first strand synthesis module. Following bead purification, libraries were amplified in an index PCR reaction following the ssPrep for RNA protocol and sequenced on an ILLUMINA MISEQ. Starting from mRNA, sequence-ready libraries were generated from 1 - 20 ng inputs in ~4 hours (yield shown in Fig. 22); after first strand cDNA synthesis and clean-up, the protocol took about ~2 hours. ssPrep for RNA required fewer number of PCR cycles in comparison to other commercial RNA-Seq kits to generate sequence ready libraries (Fig. 23).

### Comparison with commercial double-stranded prep (dsPrep) kit

The performance of ssPrep for RNA was compared to the performance of a commercial double-stranded prep (dsPrep) kit (i.e., NEBNEXT ULTRA II Directional kit), which dsPrep requires 2nd strand synthesis. The 2nd strand synthesis step incorporates dUTP into the complementary strand, followed by dA-tailing, end-filling and adapter ligation. Subsequent enzymatic digestion of the dUTP-containing strand maintains the original strand information. Unlike conventional RNAseq methods, ssPrep for RNA occurs directly on the first strand cDNA, naturally maintaining the transcript directionality.

Following first strand synthesis (NEBNEXT First Strand Synthesis Module), each library was completed according to the manufacturer's protocol. To evaluate the relative performance of ssPrep for RNA, a spike-in control available from the External RNA Control Consortium (ERCC) was used (1000 amoles of control per 10 ng replicate mRNA sample). This control is a mixture of 92 transcripts of known concentration, length, and sequence that mimic eukaryotic mRNA but do not map to the human genome. ERCC controls were designed to evaluate deviation from expected transcriptome complexity.

Each library was sequenced to a depth >10 million reads (ILLUMINA MISEQ 2x76 bp). Reads were mapped to the human or ERCC control reference genome using STAR v 2.6.1d. Both library methods showed comparable mapping metrics: >90% uniquely mapped reads, minimal ribosomal reads, and maintenance of strandedness (Fig. 25, top panel). Normalized read counts were determined for both human and ERCC reads. A high concordance was observed between replicates and the two methods for both human (Fig. 25, bottom panel) and ERCC reads (not shown).

Further analyses showed that ssPrep for RNA captured sequences uniformly across the gene body whereas the commercial kit libraries had a slight 5' bias (Fig. 26, top panel). This bias was also reflected in the genomic composition of the transcriptome, observed as a higher % recovery of untranslated regions (Fig. 26, middle panel). The libraries generated by the two methods had slightly different human DNA GC composition, however by comparing the observed versus expected (44.5%) GC composition of the spike-in control it was shown that ssPrep for RNA more closely matched its expected value than the commercial kit (Fig. 26, bottom panel, inset). Spike-in control analyses also showed almost 100% of the mapped ERCC reads arose from the correct strand for both libraries (Fig. 27).

### Example 4: Single-stranded approach to NGS library preparation for the analysis of cell-free DNA and single-stranded oligos

In this Example, a simple and efficient ligation-based ssDNA library preparation engineered to produce complex libraries from low inputs of cfDNA without alteration to the native ends of template molecules is presented. This method, sometimes referred to as ssPrep, works in a one-step combined phosphorylation/ligation reaction. The ssPrep method prepares template DNA molecules for ligation without end-polishing, ligating uniquely designed Next Generation Sequencing (NGS) scaffold-adapters. The ssPrep method is a fast and efficient single-stranded library method with protocol time and sequencing results comparable to the most efficient double-stranded library preparation DNA methods. The utility of ssPrep's native termini retention is demonstrated using two independent groups of synthetic oligos, and the ability of ssPrep to assay single-stranded oligos for purity is showcased. Finally, it is demonstrated that cfDNA Next-Generation Sequencing data generated from ssPrep can be used to analyze nucleosome positioning and transcription factor binding sites from healthy individuals. Accordingly, ssPrep is a fast and versatile tool for converting fragmented DNA molecules, like cfDNA fragments, into sequencing libraries that retain the native lengths and ends.

A fast, simple, and efficient ligation-based single-stranded DNA library preparation method (ssPrep) engineered to produce complex NGS libraries from one nanogram (ng) of DNA without altering the native ends of template molecules is described in this Example. The ssPrep method requires no exotic reagents, can be completed in 2.5 hours, and works in a one-step combined phosphorylation/ligation reaction that simultaneously prepares template DNA molecules for ligation without end-polishing while ligating ILLUMINA adapters.

Standard sequencing metrics produced by ssPrep libraries made from healthy human cfDNA donors are presented and compared to results from a traditional end-polished dsDNA library method. ssDNA libraries generated using ssPrep are compared to dsDNA preps using synthetic duplexed oligonucleotides. Next, the ability of ssPrep to capture short length ssDNA fragments is demonstrated, and the ability to assay oligonucleotide purity using single-stranded synthesized oligos of varying length and known sequence is demonstrated. Finally, it is demonstrated how ssPrep libraries empower improved analyses of cfDNA data by capturing a wide range of DNA fragment lengths without altering their native 5-prime and 3-prime termini. Given its efficiency and ease of use, ssPrep may replace both ssDNA and dsDNA library preparation methods for many applications.

### Methods

The following methods were used in this Example.

### Human cell-free DNA preparation

Whole blood from deidentified donors was obtained for in vitro investigational use from the Stanford Blood Center in Palo Alto, CA. Blood plasma was extracted from whole blood by spinning the blood collection tubes at 1800 g for 10 minutes at 4°C. Without disturbing the cell layer, the supernatant was transferred to microfuge tubes under sterile conditions in 2 ml aliquots and spun again at 16000 g for 10 minutes at 4°C to remove cell debris. cfDNA was prepared from 4 ml plasma using the Circulating Cell-free DNA kit (Qiagen Technologies) following manufacturer's protocol. Concentration of the purified cell-free DNA (cfDNA) was measured using the QUANT-IT high sensitivity dsDNA Assay Kit and a Qubit Fluorometer (ThermoFisher Scientific). cfDNA size distribution was analyzed using TapeStation and associated D5000 or D1000 high sensitivity products (Agilent; Figs. 33A and 33B).

### Synthetic oligo preparation

Double-stranded synthetic oligos (shown in Table 1 below) were designed using a random sequence generator at 50% GC content; sequences matching any known organism in public databases were removed. Each dsDNA oligo (n=12) was a unique 50 nt sequence of double-stranded DNA with one blunt-end, and one 3-prime or 5-prime single-stranded overhang of random sequence, 1 to 6 nucleotides in length. Oligos were synthesized using standard desalting purification and duplexed by Integrated DNA Technologies (IDT); all random nucleotides were 'hand-mixed' to reduce synthesis bias. Control oligos were pooled together in an equimolar ratio for single-stranded library preparation (ssPrep).

| **Table 1: Synthetic duplexed oligo sequences** | | |
|---|---|---|
| **Overhang Type** | **Sequence 1** | **Sequence 2** |
| 3' 1bp | CCATACTGTGGTCGTCACCTATTACCCC GCGTAAAGGTAGGCTATGTCATN1 | ATGACATAGCCTACCTTTACGCGGGGTAATA GGTGACGACCACAGTATGG |
| 3' 2bp | GTGAATTGTTGATGTCCTGGGTGCCTCG TCCCAAAAGCTGTCCTCACGACN2 | GTCGTGAGGACAGCTTTTGGGACGAGGCAC CCAGGACATCAACAATTCAC |
| 3' 3bp | GCTTCTCGAACCCGCGATCCGGCCGAT CCGGCATAATGGGTTGATTTAGAN3 | TCTAAATCAACCCATTATGCCGGATCGGCCG GATCGCGGGTTCGAGAAGC |
| 3' 4bp | CGACACGGATATTCCATCAAGAGACGG GCCTATGGTCCCTGTGATGATGTN4 | ACATCATCACAGGGACCATAGGCCCGTCTC TTGATGGAATATCCGTGTCG |
| 3' 5bp | ACCTTGTGTGTTGCTGAAGCAAAGCCG CGTGACCGTTTTAACCAGCGAACN5 | GTTCGCTGGTTAAAACGGTCACGCGGCTTT GCTTCAGCAACACACAAGGT |
| 3' 6bp | ATTTTACCACGAGTTCCTTACGACGGCT GTGATGCCACGGTAGGCAGGTAN6 | TACCTGCCTACCGTGGCATCACAGCCGTCG TAAGGAACTCGTGGTAAAAT |
| 5' 1bp | N₁CGCTTTACGGTCCTGGGCCGGGGT GCGATACCTTGCAGAAATCGAGGCC | GGCCTCGATTTCTGCAAGGTATCGCACCCC GGCCCAGGACCCGTAAAGCG |
| 5' 2bp | N2AGGACTCTGCCGTCGACGAGTTCGTT AATTCACGGCATCACGTGCGTAGT | ACTACGCACGTGATGCCGTGAATTAACGAA CTCGTCGACGGCAGAGTCCT |
| 5' 3bp | N3ACCTCCGTCGCGCTATGTTCTGTTGC ATTCGACCTTCTCCGTTCTGTGGG | CCCACAGAACGGAGAAGGTCGAATGCAACA GAACATAGCGCGACGGAGGT |
| 5' 4bp | N₄ACAAGAGGAGCATCCGTATTACCGCC TATATCGCCTACGTTTAGAGCATT | AATGCTCTAAACGTAGGCGATATAGGCGGT AATACGGATGCTCCTCTTGT |
| 5' 5bp | N5GTAAATCCCACACAGCTGTCGGCTTA TATGGTCATTGGACGGCGTAATAG | CTATTACGCCGTCCAATGACCATATAAGCCG ACAGCTGTGTGGGATTTAC |
| 5' 6bp | N6CCAGACAGCCATAGAGGTTACAAGCA TAGCAATTTGCATCAGTTCGCAGA | TCTGCGAACTGATGCAAATTGCTATGCTTGT AACCTCTATGGCTGTCTGG |

Single-stranded synthetic oligos (shown in Table 2 below) were generated in the same way as the double-stranded control oligos. Unless otherwise noted, oligos were synthesized using standard desalting purification for ssDNA oligos 20 - 80 nt in length and Ultramer purification for ssDNA oligos 90 -120 nt in length.

| **Table 2: Synthetic single-stranded oligo sequences** | |
|---|---|
| **Oligo** | **Sequence (5' -> 3')** |
| 20mer | GTA AAG GTA GGC TAT GTC AT |
| 30mer | GTG CCT CGT CCC AAA AGC TGT CCT CAC GAC |
| 40mer | GCT TCT CGA ACC CGC GAT CCG GCC GAT CCG GCA TAA TGG G |
| 50mer | CGA CAC GGA TAT TCC ATC AAG AGA CGG GCC TAT GGT CCC TGT GAT GAT GT |
| 60mer | ATT TTA CCA CAC CTT GTG TGT TGC TGA AGC AAA GCC GCG TGA CCG TTT TAA CCA GCG AAC |
| 70mer | CCA TTC GGG CAT AAT ATG AAC TAT ACG CAG CTT ATC CCG GGC CCG TAA CAA ACA ATT TGC GTG AGG TAT G |
| 80mer | GTC CCA CTC AGA GAA TTA GCA GCC CTG GTC TAG CGA GGG ATG CCG CTT AGC GTC GGT TGA ATT TCG CTG CAC TAC AGA CG |
| 90mer | CGC TTT ACG GGT CCT GGG CCG GGG TGC GAT ACC TTG CAG AAT CTG CGC CTC TTG GTG GCG CCC CAT CAG TAG TGT CTA CAC GGG CGC TGT |
| 100mer | GTA AAT CCC ACA CAG CTG TCG GCT TAT ATG GTC ATT GGA CGG CGT AAT AGA CAA GAG GAG CAT CCG TAT TAC CGC CTA TAT CGC CTA CGT TTA GAG CAT T |
| 110mer | GGT TCC TAA CAG GTG ATT ACC AGT GCA GTT AGC CAT TTA TCC TCG TCA AAA AGC CAC GTT CCA GAC AGC CAT AGA GGT TAC AAG CAT AGC AAT TTG CAT CAG TTC GCA GA |
| 120mer | GAC GGC CCT AGT CTG CTT CTC GAG ACA ATC TGC TAG AAC TCG GAC GCC TCG CAC TGT ACT GAT GCA TGG TCC GTA ATC GAG GTG AAA ACT ACA CGG TAT GAC ATC AGC GAT AAC TGG TTT |

### ssPrep adapter preparation

The forward (P5) ssPrep adapter as well as the reverse (P7) ssPrep adapter were both double-stranded scaffold adapters. The forward ssPrep adapter contained a 5-prime overhang in the scaffold portion of the adapter and a free 3-prime OH end on the ligating end; all other ends contained ligation and extension blocking modifications. The reverse ssPrep adapter contained a 3-prime overhang in the scaffold portion of the adapter and a phosphorylated 5-prime end on the ligating end; all other ends contained ligation and extension blocking modifications (Table 3 below). The ssPrep adapters were synthesized using standard desalting purification and duplexed by Integrated DNA Technologies (IDT). Working stocks of the adapters were made by diluting the adapters in TE + 50mM NaCl.

| **Table 3: ssPrep adapter design** | | |
|---|---|---|
| **Adapter** | **Sequence 1 (adapter)** | **Sequence 2 (scaffold)** |
| Forward (P5) | /5AmMC12/ACACTCTTTCCCTACACGACG CTCTTCCGATCT | /5AmMC6/NNNNNNNAGATCGGAAGAGCGT CGTGTAGGGAAAGAGTGT/3AmMO/ |
| Reverse (P7) | /5Phos/AGATCGGAAGAGCACACGTCTGA ACTCCAGTCA/3ddC/ | /5AmMC12/GTGACTGGAGTTCAGACGTGTG CTCTTCCGATCTNNNNNNN/3AmMO/ |

### ssPrep library preparation

1 ng of purified cfDNA or 5 ng of synthesized oligos, as measured by the QUANT-IT, was combined with 10 mM Tris pH8.0 and 8 ng of ET SSB (New England Biolabs) in a 22 µl denaturation reaction, on ice. The reaction was placed in a thermocycler preheated to 95°C and incubated for 3 minutes before immediately being placed back on ice for at least 2 minutes. 1 pmol of the forward and 1 pmol of the reverse ssPrep adapters were added to the denaturation reaction, on ice, as well as PEG-8000, T4 DNA ligase Buffer, T4 PNK, and T4 DNA ligase (all New England Biolabs) to a final volume of 50 µl. PEG-8000 was added to a final concentration of 18.5% v/v. T4 DNA ligase buffer was added to a final concentration of 1X. T4 PNK and T4 DNA ligase were added to a final concentration of 10 units and 800 units, respectively. This ligation reaction was incubated at 37°C for one hour and purified using the MINELUTE PCR Purification Kit (Qiagen) and manufacturer's instructions with the following changes: The initial binding spin was performed at 6000 rpm on a desktop centrifuge. The wash spin was repeated for a total of two wash spins and both washes were performed at 6000 rpm. The DNA was eluted in 15 µl 10 mM Tris pH8.0.

ssPrep libraries were indexed by combining the purified ligated DNA with 1x KAPA HIFI HOTSTART READYMIX (Roche) and 2 mM final concentration of universal primer and 2 mM final concentration of an index primer in a 50 µl reaction and amplified using the following thermal cycling conditions: 3 minutes at 98°C for initial denaturation followed by 10 cycles at 98°C for 20 seconds, 68°C for 30 seconds, 72°C for 30 seconds, and finally an elongation step of 1 minute at 72°C. After index PCR, ssPrep libraries were purified with a 1.2x AMPURE clean (Beckman Coulter) and eluted in 20 µl 10 mM Tris pH8.0. Final molarity estimates were calculated using fragment length distribution and dsDNA concentration (Agilent Tapestation 4200 and Qubit Fluorometric Quantitation unit).

### Double-stranded DNA library preparation

1 ng of purified cfDNA or 5 ng of synthesized oligos, as measured by the QUANT-IT, was taken through library preparation (end-polishing, adapter ligation, index PCR) as outlined in the NEBNEXT ULTRA II manual using the supplied reagents, recommended AMPURE cleanup ratios, and recommended index PCR cycles.

### Sequencing

All cfDNA libraries were sequenced on an ILLUMINA HISEQX at a 2 x 151 read length by Fulgent Genetics. All synthetic oligo libraries were sequenced on an in-house ILLUMINA MISEQ benchtop sequencer at a read length of 2 x 151 bp following manufacturer's instructions.

### Read processing

Sequencing data was first aligned to the PhiX genome using bwa mem with default parameters. Extracted reads that did not map to PhiX (samtools fastq -f 12) were used for downstream analyses. Next adapter sequences were removed and reads were merged simultaneously. This process included collapsing forward and reverse reads into single sequences, based on sequence similarity, while trimming ends of reads that match known ILLUMINA adapter sequences using SeqPrep (github.com/jstjohn/SeqPrep). Merged reads that remained after filtering were aligned to either the hg19 human reference genome (Tables 4 and 5 below) downloaded from the UCSC genome browser, or to a custom fasta file corresponding to the synthesized oligo sequence (Table 1). Bwa aln and bwa sampe were used with default parameters for alignment and mapping. Mapping rates, for human libraries, were determined from samtools flagstat. Duplicate reads were then removed using samtools rmdup.

| **Table 4: ssPrep human cfDNA extract NGS statistics** | | | | | | |
|---|---|---|---|---|---|---|
| **Library ID** | **cfDNA extract** | **Raw read pairs** | **Pass filter read pairs** | **Merged read pairs** | **Mapped read pairs** | **Duplicate read pairs** |
| A1 | sample A | 94,786,943 | 86,884,321 (91.7%) | 74,059,050 (85.2%) | 69,735,053 (77.7%) | 6,646,784 (9.5%) |
| A2 | sample A | 94,297,123 | 89,775,122 (95.2%) | 75,364,887 (83.9%) | 74,408,496 (85.6%) | 7,342,661 (9.9%) |
| A3 | sample A | 81,474,103 | 77,874,288 (95.6%) | 65,201,784 (83.7%) | 64,727,039 (83.1%) | 5,958,851 (9.2%) |
| A4 | sample A | 98,450,841 | 90,642,659 (92.1%) | 76,197,502 (84.1 %) | 74,101,090 (81.8%) | 6,981,901 (9.4%) |
| A5 | sample A | 115,200,247 | 105,758,818 (91.8%) | 86,567,929 (81.9%) | 85,410,946 (80.8%) | 9,571,162 (11.2%) |
| All | sample A | 484,209,257 | 450,935,208 (93.1%) | 377,391,152 (83.7%) | 368,382,624 (81.7%) | 36,501,359 (9.9%) |
| B6 | sample B | 84,140,424 | 80,948,813 (96.2%) | 71,429,415 (88.2%) | 68,958,103 (85.2%) | 9,122,834 (13.2%) |
| B7 | sample B | 74,670,157 | 71,559,425 (95.8%) | 63,111,643 (88.2%) | 61,087,692 (85.4%) | 7,380,490 (12.1%) |
| B8 | sample B | 77,438,201 | 74,583,049 (96.3%) | 65,654,147 (88.0) | 63,686,313 (85.4%) | 8,372,356 (13.1%) |
| B9 | sample B | 84,600,059 | 81,361,847 (96.2%) | 72,265,939 (88.8%) | 70,187,322 (86.3%) | 8,495,259 (12.1%) |
| B10 | sample B | 77,177,608 | 74,493,904 (96.5%) | 66,365,109 (89.1%) | 64,450,156 (86.5%) | 8,256,944 (12.8%) |
| All | sample B | 398,026,449 | 382,947,038 (96.2%) | 338,826,253 (88.5%) | 328,369,586 (85.7%) | 41,627,883 (12.7%) |

| **Table 5: dsPrep human cfDNA extract NGS statistics** | | | | | | | |
|---|---|---|---|---|---|---|---|
| **Librar y ID** | **Preparatio n kit** | **cfDN A extra ct** | **Raw read pairs** | **Pass filter read pairs** | **Merged read pairs** | **Mapped read pairs** | **Duplicat e read pairs** |
| ds1A | NEB ULTRA II | sampl e A | 56,804,74 2 | 56,581,21 6 (99.6%) | 47,014,20 4 (83.1%) | 49,075,27 4 (86.7%) | 4,286,898 (8.7%) |
| ds2A | NEB ULTRA II | sampl e A | 62,159,09 2 | 61,939,78 0 (99.6%) | 51,079,84 1 (82.5%) | 53,969,24 0 (87.1%) | 5,281,009 (9.8%) |
| ds3A | NEB ULTRA II | sampl e A | 54,665,14 8 | 54,415,55 2 (99.5%) | 44,943,92 3 (82.6%) | 47,100,88 1 (86.6%) | 3,917,226 (8.3%) |
| ds4A | NEB ULTRA II | sampl e B | 45,040,36 9 | 44,945,09 2 (99.8%) | 39,385,03 6 (87.6%) | 40,229,49 9 (89.5%) | 4,519,419 (11.2%) |
| ds5A | NEB ULTRA II | sampl e B | 40,276,52 8 | 40,208,80 0 (99.8%) | 34,083,41 7 (84.8%) | 35,894,02 3 (89.3%) | 3,951,972 (11.0%) |
| ds6A | NEB ULTRA II | sampl e B | 38,184,95 1 | 38,107,03 2 (99.8%) | 33,641,06 6 (88.3%) | 34,066,68 9 (89.4%) | 3,653,615 (10.7%) |
| ds1B | NEB ULTRA II | sampl e A | 145,586,4 38 | 145,391,6 8 (99.9%) | 124,430,8 80 (85.6%) | 130,678,6 20 (89.9%) | 21,702,62 9 (16.6%) |
| ds2B | NEB ULTRA II | sampl e A | 167,080,6 44 | 166,862,8 22 (99.9%) | 141,337,1 08 (84.7%) | 150,007,1 51 (89.9%) | 27,522,31 4 (18.3%) |
| ds3B | NEB ULTRA II | sampl e A | 149,861,1 98 | 149,652,9 29 (99.9%) | 128,254,2 14 (85.7%) | 134,494,1 74 (89.9%) | 21,894,09 1 (16.3%) |
| ds4B | NEB ULTRA II | sampl e B | 135,165,1 44 | 134,915,9 60 (99.8%) | 117,427,6 39 (87.0%) | 121,157,3 80 (89.8%) | 20,317,67 6 (16.8%) |
| ds5B | NEB ULTRA II | sampl e B | 134,972,5 63 | 134,765,6 78 (99.8%) | 112,962,0 99 (83.8%) | 120,756,5 17 (89.6%) | 20,380,32 1 (16.9%) |
| ds6B | NEB ULTRA II | sampl e B | 118,888,5 14 | 118,686,2 58 (99.8%) | 10,4471,0 69 (88.0%) | 106,579,3 41 (89.8%) | 17,229,56 9 (16.2%) |

### QC metrics

For most analyses bam files from individual libraries of same preparation method and same cfDNA extract were merged into sample- and library-specific bam files using samtools merge prior to analysis. For insert length distribution of merged reads, for the same preparation method and cfDNA extract insert length information was parsed from the bam files of individual libraries that were generated using samtools view -q20 -f66 and combined using a concatenate command. Frequency of reads per length was calculated and plotted as the percent reads of total library. Normalized genome coverage was extracted from down-sampled merged duplicate removed bam files using samtools view -s such that all libraries had the same coverage. Data was obtained by pipping downsampled bam files from samtools view -q20 -b into bedtools genomecov. Preseq complexity estimates were obtained by combining only 3 libraries for each cfDNA input sample per library preparation method prior to downsampling in order to not artificially inflate the complexity of ssPrep, which had more libraries per cfDNA extract than NEBNEXT ULTRA II. Libraries combined for ssPrep sample A were: A1, A2, A3. Libraries combined for ssPrep sample B were: B6, B7, B8. Libraries combined for NEBNEXT ULTRA II for sample A and sample B were ds1A-ds3A and ds4A-ds6A, respectively. After combining and downsampling, complexity estimates and extrapolation were performed using preseq Icextract. GC coverage was obtained from down-sampled merged duplicate removed bam files utilizing Picard Tools (Broad Institute) CollectGCBiasMetrics. For each library type, fragment terminal nucleotide analysis was done by calculating the proportion of each base i.e., the base composition, at every position for a region spanning from -2 to +34 bases on both reads of a fragment. The base composition per position was normalized with the mode for that base along the length of the region and log-2 transformed. The normalized, log-transformed proportions were calculated for both library types, for both reads and plotted. All plots were generated in R utilizing ggplot2

### Synthetic oligo analysis

Double-stranded synthetic oligo sequencing coverage at each position in the oligo was determined utilizing a custom script akin to samtools depth and plotted in R utilizing ggplot2 as a function of percent across the length of the oligos in 0 base coordinates

Fragment length analysis of single-stranded synthetic oligos was conducted analogous to that for cfDNA.

### Biological analysis of cfDNA

For dinucleotide frequency calculations merged bam files from combined sample A and sample B libraries for each library preparation method were parsed using samtools view -bh -F 0X10 -m -M -q 20 to extract forward reads of specific insert lengths: 167 bp (chromatosome-wrapped DNA length), 144 bp (core particle-wrapped DNA length, and 83 bp (a shorter DNA length that occurs as a peak in Fig. 29, Panel A). For each insert length, the dinucleotide counts around both fragmentation points were estimated using a custom python script for all 16 2-mer combination for either a 100 bp or 11 bp window, where 100 bp or 11 bp of genomic context at both 5-prime and 3-prime fragmentation points were added respectively. For the data generated with a 100 bp flanking window on both ends, the overlapping regions (which justifiably had the same counts) were removed. The data was normalized using a median filter and dinucleotide frequency was plotted for weak (AA/AT/TA/TT) vs strong (CC/CG/GC/GG) dinucleotide interaction such that the center of the insert was at 0 and the regions upstream of the fragmentation point had negative values and downstream had positive values. For the data generated with a 11 bp flanking window, the data was normalized with a median filter and dinucleotide frequencies of weak vs strong dinucleotide were plotted for 5-prime and 3-prime ends using R.

The WPS score for each position in the genome was determined by collecting the reads which align in a window around that position (as described in Snyder et al., Cell 164, 57-68 (2016)), 120 bp in the case of large fragment analysis and 35 bp in the case of short fragment analysis. The score was calculated as follows: Every time an insert starts or end in that window, one is subtracted from the score. If an insert does not start or end in that window, but aligns to it nevertheless, one is added to the score. The normalized WPS score was calculated by taking the WPS scores over non-overlapping 1000 bp segments and adjusting to a median score of zero by subtracting the median WPS score. The scores were then smoothed by the Savitzky-Golay filter: second-order polynomials were fitted to median-adjusted scores over a 21 bp window at each position. The smoothed score is the value of that polynomial at that position. The Average WPS score was calculated over a set of regions of equal length by calculating the mean of the WPS scores over each position in each of the regions in the set, where position 1 is the first nucleotide of each region in the set, position 2 is the second nucleotide in each region, etc. CTCF sites were chosen as described in Snyder et al., Cell 164, 57-68 (2016). A bed file containing a list of putative TF binding sites was downloaded from the JASPAR2018 table (hub_186875_JasparTFBS) from the UCSC Genome Browser Table Browser into a bed file and filtered to include only CTCF sites. These sites were compared with CTCF Chipseq data from 19 cell lines. Putative binding sites with overlapping chipseq peaks in all 19 cell lines were used for further analysis.

### Abbreviations

cfDNA: cell-free DNA; NGS: Next-Generation Sequencing; ssDNA: single-stranded DNA; dsDNA: double-stranded DNA; bp: base-pair; nt: nucleotide; SSB: single-stranded binding protein; FFPE: formalin-fixed paraffin-embedded; ctDNA: circulating-tumor DNA; WPS: window protection score.

### Library construction

The ssPrep method described in this Example creates ILLUMINA sequencing libraries from fragmented or degraded template DNA (Fig. 28). Template DNA, which can be a complex mixture of dsDNA, ssDNA, and nicked dsDNA, is first heat denatured and then immediately cold shocked in order to render all template DNA molecules uniformly single-stranded. The DNA is maintained as single-stranded throughout the ligation reaction by the inclusion of a thermostable single-stranded binding protein (SSB). Next, the template DNA, which is now uniformly single-stranded and coated with SSB, is placed in a phosphorylation/ligation dual reaction with directional dsDNA NGS adapters that contain single-stranded overhangs.

Both the forward and reverse sequencing adapters share similar structures but differ in which termini is unblocked in order to facilitate proper ligations. Both sequencing adapters are dsDNA, except for a random 7 base-pair (bp) single-stranded scaffold overhang that occurs on the 3-prime termini of the bottom strand of forward adapter and the 5-prime termini of the bottom strand of the reverse adapter. In this way, the forward (P5) ILLUMINA adapter is always delivered to the 5-prime end of template molecules and the reverse (P7) ILLUMINA adapter is always delivered to the 3-prime end of template molecules.

During the dual phosphorylation/ligation reaction, T4 polynucleotide Kinase (PNK) prepares template DNA termini for ligation by phosphorylating 5-prime termini and dephosphorylating 3-prime termini. T4 PNK works on both ssDNA and dsDNA molecules and has no activity on the phosphorylation state of proteins. Simultaneously, the random bases of the scaffold adapter anneal to the single-stranded template molecule. This creates a short, localized dsDNA molecule, enabling ligation of template to adapter with T4 DNA ligase, which has high ligation efficiency on double-stranded DNA templates but low efficiency on ssDNA. After the single phosphorylation/ligation reaction is complete, the library DNA is purified and placed directly into standard NGS indexing PCR, compatible with both single and dual index primers.

### Performance of the ssPrep protocol

To evaluate the quality and quantity of data produced by ssPrep several sequencing libraries were generated from two plasma cfDNA extracts obtained from two healthy human individuals (sample A and sample B) using both ssPrep and a standard end-polishing dsDNA library kit (New England Biolabs NEBNEXT ULTRA II; also referred to as a commercial kit or dsPrep). After library preparation and quantification (Figs. 33A and 33B), libraries were paired-end sequenced on ILLUMINA HISEQ X (2 x 150 bp) to roughly 400 million read pairs per cfDNA extract. Sequencing data from libraries generated from the same cfDNA extract and library preparation method were combined for analysis. Forward and reverse sequence reads were merged when these reads overlapped to generate single reads representing the original DNA fragment. Since the majority of sequence reads from cfDNA are about 167 bp long, only merged reads (where read 1 and read 2 overlapped by at least 30 bp of complementarity) were used for downstream analyses (Table 4 and Table 5 above). The data generated resulted in about 15-fold coverage of the human genome for both ssPrep and dsPrep samples per cfDNA extract.

Libraries generated by ssPrep and dsPrep (commercial kit) cfDNA had length distribution features typical of cfDNA fragments. They both showed fragment length distributions centered around the chromatosome length at 167 bp. They both showed a sawtooth pattern in shorter fragments that are the result of DNase I cleaving the exposed minor grove of nucleosome bound DNA at a periodicity of 10.4 bp (Fig. 29, Panel A; Fig. 34). However, as shown in Fig. 29, Panel A, and its inset, the two preparation methods differed in the proportion of reads captured at different fragment lengths, as well as the length distribution of the sub-peaks present in the sawtooth pattern. ssPrep libraries had a higher abundance of shorter, i.e. sub-nucleosome length, reads with shorter sub-peaks in the sawtooth pattern versus dsPrep. The increased proportion of sub-nucleosome-sized reads reflected the increased ability of the ssDNA method to convert short and/or nicked DNA fragments into sequence library molecules.

Without being limited by theory, the difference in sub-nucleosome peak sizes is likely due to the ability of ssPrep to retain native termini compared to dsDNA methods. In dsDNA library methods, 5-prime overhangs are filled in and 3-prime overhangs are removed. Thus, the observed length of a given DNA molecule will be dependent on what type of overhangs are present. This information is lost during the end-polishing step required in dsDNA library preps.

Read coverage, GC content, and complexity (number of unique molecules in the library) of ssPrep versus dsPrep (commercial kit) libraries were compared for both cfDNA extracts. Fig. 29, Panel B, shows that ssPrep produces fold-coverage similar to that of the dsPrep kit and that both methods produce relatively uniform genomic coverage. Fig. 29, Panel C, shows that the GC content of ssPrep libraries is similar to that of the dsPrep kit which mirrors that of the human genome reference (histogram, plotted in gray). The differences shown in regions of low GC content between ssPrep and dsPrep could be either the result of the differences in the polymerase used during index PCR (NEB Q5 versus KAPA HIFI HS RM) or GC-rich biases in the synthesis of the random portion of the ssPrep scaffold adapters. Fig. 29, Panel D, shows that at a sequencing depth of 300 million reads, or roughly one HISEQ sequencing lane, ssPrep libraries are estimated to have higher molecular complexity than dsPrep libraries. Without being limited by theory, this difference might be a reflection of ssPrep's ability to recover nicked and ssDNA strands lost to traditional dsDNA library preparation.

Most dsDNA library preps, including the dsPrep kit used in this Example, perform end-polishing on the input DNA molecules. Because the ssPrep method delivers sequencing adapters to the native termini of DNA fragments, the base composition at and around the exact 5-prime and 3-prime end of each DNA fragment can be examined with single nucleotide resolution. Note that the end-polishing procedure retains the native 5-prime end of molecules. However, the 5-prime overhang "fill-in" and the 3-prime overhang exonuclease activity of T4 DNA polymerase generates a 3-prime end that is not representative of the original molecule when overhangs of either type are present. In this way, the end-polishing procedure is expected to make all 3-prime ends mirror what is present at the 5-prime end of the complementary strand.

To test differences in DNA termini information, base composition per position across the start coordinates was compared for both the forward (read 1) and reverse (read 2) reads, inferred from the merged read dataset, for both the ssPrep and the dsPrep cfDNA libraries (Fig. 29, Panel E). There were four notable findings. First, for both ssPrep and dsPrep there was significant deviation from the average base composition at the start of each read, as well as upstream of the biological fragmentation point. Second, unlike the dsDNA library data, the average base composition for the start of the forward reads and the start of reverse reads differed in ssPrep libraries. This indicates that cfDNA fragments often contain overhangs that are altered during the end-polishing steps of dsDNA library prep. Third, the average base composition for the start of the forward read in dsPrep libraries were exactly the reverse-complement of the average base composition for the start of the reverse read (dsPrep generates molecules that are uniformly blunt ended, the byproduct of end-polishing). Finally, the average base composition for the start of the forward read in ssPrep libraries was nearly identical to that of dsPrep libraries (end-polishing retains the native 5-prime ends, as does the ssPrep direct ligation procedure).

### Assessing the features of ssPrep

### 5-prime and 3-prime overhangs

Given the base composition differences in cfDNA at the 5-prime and 3-prime ends, an experiment was designed to test whether ssPrep and dsDNA library preparation methods, like NEBNEXT ULTRA II, are altering (or not altering) input DNA fragments. Pools of 12 synthetic duplexed oligos were constructed, at equimolar concentrations, each having a specific length and type (5-prime or 3-prime) overhang. Each duplex contained a 50 nucleotide (nt) core sequence, unique to each overhang type and had a common structure: blunt terminus on one side, and a 5-prime or 3-prime overhang of a specific length of random sequence (one to six nt) on the other side (Fig. 30, Panel A; Table 1).

ssPrep and dsPrep libraries were generated by spiking this pool of oligos into cfDNA extracts. From the sequencing data, reads that originate from the oligo pool were identified by mapping the libraries to a reference file containing the known unique 50 nt core sequences of each oligo. Depth of coverage was calculated at every position for each oligo in the pool, including the overhangs. The results (Fig. 30, Panel B) show that ssPrep produced reduced coverage across the overhanging regions compared to the double-stranded regions of the synthetic oligos illustrating the method's ability to yield stranded data that accurately characterizes the input DNA. By contrast, the libraries produced by dsPrep demonstrated a result of end-polishing. Five-prime overhangs were filled-in, resulting in almost full coverage on the complementary strand of molecules with known 5-prime overhangs. Three-prime exonuclease activity, on the other hand, caused nearly complete loss of the 3-prime overhang sequence when it was present.

### Single-stranded oligo libraries

To test the efficiency of ssPrep on a defined range of input DNA template lengths, a set of 11 single-stranded oligos (standard desalt purification) of lengths ranging from 20 to 120 nucleotides at 10 nt length intervals (Table 2) were designed. A pool was made using equimolar concentrations of each and ssPrep libraries were generated from this pool. Analysis of the proportion of template lengths from sequencing these libraries showed that the ssPrep protocol generated ssDNA libraries across this length range (Fig. 31, Panel A). As a control, an attempt was made to generate dsPrep libraries from this pool of single-stranded oligos; this protocol failed to generate any library at all using a template of exclusively single-stranded input DNA (libraries contained adapter dimers but no detectable yield at expected size distributions).

There were several noteworthy observations from the ssPrep data analysis. First, the shortest test oligos (20 nt and 30 nt length) were under-presented in the libraries. This was likely due to the bead clean-up step after the ligation, which has a length bias against DNA oligos in this size range. Second, there was some variation in library conversion efficiency amongst the longer (>= 40 nt) test oligos. This variation is likely due to subtle biases in the test oligos, which are a single, fixed sequence for each length. Finally, a continuous background fraction of oligo lengths that do not correspond to the input oligo lengths was observed, and at least some reads of every length between 20 and 120 were observed.

To test whether these reads of unexpected length were due to truncated and incomplete oligo synthesis or due to labile breakage of the longer single-stranded oligos, all reads in the ssPrep libraries were mapped to their respective oligo reference (Fig. 31, Panel B; Table 6 below). Truncation products were present for each oligo. These truncated DNA fragments had lengths that were nearly uniformly distributed across the length of the oligo. The fraction of correct, full-length read mapping to each oligo decreased as a function of oligo length. Such observations indicate limits of the phosphoroamadite method of oligo synthesis. These observations are consistent with a model where nucleotide incorporation is less than 100% efficient in each chemical cycle of base addition.

| **Table 6: Synthetic single-stranded oligo raw read counts** | | | | | |
|---|---|---|---|---|---|
| **Library ID** | **Oligo length** | **Raw mapped reads** | **Library ID** | **Oligo Length** | **Raw mapped reads** |
| Replicate 1 | 20 bp | 1241 | Replicate 2 | 20 bp | 876 |
| Replicate 1 | 30 bp | 2864 | Replicate 2 | 30 bp | 2918 |
| Replicate 1 | 40 bp | 9802 | Replicate 2 | 40 bp | 10144 |
| Replicate 1 | 50 bp | 9340 | Replicate 2 | 50 bp | 6481 |
| Replicate 1 | 60 bp | 10437 | Replicate 2 | 60 bp | 8761 |
| Replicate 1 | 70 bp | 15275 | Replicate 2 | 70 bp | 13055 |
| Replicate 1 | 80 bp | 14465 | Replicate 2 | 80 bp | 9531 |
| Replicate 1 | 90 bp | 8229 | Replicate 2 | 90 bp | 5678 |
| Replicate 1 | 100 bp | 18801 | Replicate 2 | 100 bp | 14240 |
| Replicate 1 | 110 bp | 20577 | Replicate 2 | 110 bp | 13938 |
| Replicate 1 | 120 bp | 17567 | Replicate 2 | 120 bp | 12004 |
| Replicate 1 | All | 128598 (96.47%) | Replicate 2 | All | 97626 (96.24%) |
| Replicate 1 | 60 bp HPLC | 4686 | Replicate 2 | 60 bp HPLC | 2966 |
| Replicate 1 | 60 bp PAGE | 4038 | Replicate 2 | 60 bp PAGE | 3520 |

To test whether ssPrep can assess the purity of oligos subjected to various purification methods, a 60 nt oligo was purified using three common schemes: standard desalt, HPLC, and PAGE purification. ssPrep libraries were constructed, in duplicate, using the 60 nt oligo from all three purification methods. Mapping the sequence data to the 60 nt reference sequence (Fig. 31, Panel C) showed that the proportion of reads attributed to the expected full length sequence increased in both the HPLC and PAGE purified oligo libraries while truncation products, defined as reads at lengths shorter than 60 nt, decreased compared to the libraries generated from standard desalt oligos. These results are consistent with the projected quality of each purification method based on phosphoramidite synthesis (Integrated DNA Technologies Product Literature) and indicate that ssPrep can be used as a simple and sensitive assay to determine the purity of chemically synthesized DNA oligos.

### Analysis of ssPrep cfDNA libraries

The majority of cfDNA fragments derive from DNA wrapped around a nucleosome, a configuration that protects the DNA from nuclease degradation during cell death. Thus, the genomic map positions of cfDNA fragments can be used to infer the positions of histones and other DNA binding proteins in the tissues that have given rise to a population of cfDNA molecules. Single-stranded DNA library methods, like ssPrep described in this Example, retain the native ends of cfDNA fragments and are thus maximally useful for inferring the positions of histones and other DNA-binding proteins insofar as these proteins protect the DNA from endonuclease activity. ssPrep data from two healthy individuals (sample A and sample B) was combined to obtain 30-fold average genome coverage. From these data, the ability of ssPrep libraries to reveal aspects of the positioning of nucleosomes and other DNA-binding proteins was examined.

Nucleosome positioning is at least partially encoded by the genome. For DNA bound to histones, A/T dinucleotdes generally are favored when the minor grove faces towards the histone and G/C dinucleotides generally are favored when the minor grove faces outwards. Therefore, when analyzed in aggregate, DNA fragments originating from nucleosome protected DNA should contain an oscillating pattern of an A/T rich and G/C depleted region directly followed by a G/C rich and A/T depleted region within captured fragments, compared to the surrounding genomic regions. To test whether this oscillation pattern is present in the ssPrep data, the A/T and G/C genomic dinucleotide in molecules of three fragment lengths, 167, 144, and 83 bp, including bases 100 nts upstream and downstream of each of the three read lengths (Fig. 32, Panel A) was examined. Each was centered on the midpoint of the sequence. As noted, 167 bp corresponds to the length of DNA wrapped around a nucleosome core particle plus the associated linker region, 144 bp represents the length of DNA wrapped around the nucleosome core particle only, and 83 bp may represent a degradation product originating from nucleosome-associated DNA.

An oscillation enrichment for A/T and G/C dinucleotides within the sequenced molecule length compared to the surrounding genomic regions was observed. A strong oscillation signal for ~55 bp upstream of the 83 bp fragment length also was observed, indicating that these molecules are likely derived from degraded nucleosomal associated DNA. This dinucleotide oscillation within the defined fragment lengths for the dsPrep method was observed as well (Fig. 32, Panel C). However, the upstream oscillation signal in the 83 bp fragment length for the dsPrep data was not observed. This may be due to low recovery of short fragments in the dsDNA preparation methods or other differences in the ability of dsDNA preps to convert fragmented or nicked DNA into sequencing libraries.

An additional feature of dinucleotide-mediated histone wrapping is that DNase I mediated nicking occurs when the minor grove is accessible. This phenomenon leads to a specific enrichment for G/C dinculeotides at the terminal ends of nucleosome-associated fragments (Fig. 32, Panels A-D). Due to the dsDNA end-polishing step, the terminal profile of the 5-prime and 3-prime ends in dsPrep data were mirror images of each other (Fig. 32, Panel D). The dinucleotide frequency at 3-prime termini differing considerably between ssPrep and dsPrep indicates a substantial population of diverse overhangs occurs in a population of nucleosome-associated cfDNA fragments (Fig. 32, Panels B and D).

Next, nucleosome positioning was examined using the window protection score (WPS). The WPS is a measure of whether a position in the genome tends to be protected from endonuclease activity or enriched for endonuclease activity. It is a function of how many reads span the given position (and thus were not cut) versus how many reads begin or end at that position (and thus were cut). The normalized WPS was calculated using ssPrep data at a region comprised of well-positioned nucleosomes on chromosome 12. Comparing the WPS results with previous results using an alternative ssDNA library protocol, good concordance with respect to the location of the peaks and troughs was observed (Fig. 32, Panel E); Overall Pearons Correlation: r = 0.80, p < 0.0001).

A second WPS validation of the ssPrep data was performed by calculating normalized WPSs for fragments whose lengths fell into a long-sized bin (120 -180 bp, the range of fragments lengths presumed to derive from histone protection) and a short-sized bin (35 -80 bp, presumed to be enriched for fragments protected by other DNA-binding proteins) within 1 kb upstream or 1 kb downstream of experimentally determined binding sites for the transcription factor CTCF (Fig. 32, Panel F). CTCF is a DNA-binding protein that occludes histones where it is bound and organizes histone positioning upstream and downstream. The long fragment WPS showed a depression centered at the putative CTCF binding site (position 0) and oscillation patterns extending outward in both directions at a periodicity of ~180 bp indicating well-positioned nucleosomes. The short fragment results showed a strong peak centered at the putative CTCF binding site, presumably due to CTCF-protection from endonuclease activity. Upstream and downstream, the smaller amplitude oscillations were consistent with the absence of DNA-binding proteins other than nucleosomes.

### Example 5: ssPrep kit for cell-free DNA

In this Example, a kit for preparing an NGS library from single-stranded cell-free DNA is described. An example workflow for an ssPrep kit is illustrated in Fig. 36.

Features of the ssPrep kit for cell-free DNA include: duplex DNA recovery as well as single-stranded and nicked duplex DNA lost to standard preps; low input of 1 ng produces complex libraries and saves precious samples; single reaction reduces failures due to error, reduces bench time; no end-polishing preserves the natural ends of all DNA fragments; superior recovery of short fragments lengths; no downstream data trimming ensures pipeline compatibility; from DNA to sequence-ready Illumina^{®} library in under 3 hours; each kit provides reagents for library preparation, indexing PCR, and bead purification; options available for single and dual-indexing, as well as unique molecular identifier (UMI) incorporation; optimized for 1 ng of cell-free DNA with input concentrations as low as 50 pg/µl; and optimized master mixes and minimal pipetting steps ensures nominal hands on time. Downstream applications of the ssPrep kit include: exome sequencing, panel enrichment, nucleosome positioning, SNP calling, and novel discovery. Fields of use for the ssPrep kit include: liquid biopsy, oncology, prenatal testing, and transplant medicine.

Cell-free DNA found circulating in blood plasma and other bodily fluids contains a wealth of clinically relevant biological information and can be recovered from minimally-invasive procedures. NGS data obtained from cfDNA can reveal aspects of cellular biology such as prenatal health, organ transplant reception or rejection, cancer detection and progression, and a multitude of other diseases.

The majority of DNA fragments extracted from blood plasma cfDNA are centered around 167 base-pairs (bp) in length and often are the result of histone monomer bound DNA that is protected from nucleases degradation. In addition, cfDNA derived from blood plasma contains a valuable minority of short length sized DNA fragments (30-100 bp) that harbor footprints of transcription factors, other DNA binding proteins, mitochondrial DNA, and microbial derived DNA, all of which adds detail to cfDNA sequence data (Fig. 37).

The benefits of single-stranded library preparation methods includes the ability to capture (1) shorter and more damaged fragments and (2) more diverse DNA molecules than dsDNA preps, without losing duplexed DNA strands. Despite these advantages, however, widespread adoption by the NGS community has been hindered by existing single-stranded DNA library preparation methods and kits that are more time consuming than traditional dsDNA methods, require exotic or single-source enzymes, and in some instances produce sequencing artifacts.

The ssPrep kit described in this Example is a simple and efficient ligation-based single-stranded DNA library preparation method that is engineered to produce complex libraries from 1 ng of input cfDNA without alteration to the native ends of template molecules. ssPrep rivals yields, complexity, and preparation time of traditional dsDNA kits and provides additional information dsDNA kits cannot, such as enhanced coverage of short fragments, and retention of native termini, all in a single reaction (see Fig. 38).

### ssPrep libraries produce high DNA yields

Post-index library yield measures the success of the library and is an indirect measurement of overall conversion performance. ssPrep returns high DNA yields to allow users more flexibility with sequencing and downstream enrichment prior to sequencing (see Fig. 39).

### ssPrep libraries mapping rates and short fragment retention

Mapping performance of ssPrep cfDNA libraries is on par with the best commercial kits on the market. ssPrep goes above and beyond other commercial kits by capturing a higher percentage of reads with short inserts (30-100 bp) to help researchers leverage the valuable biology encoded in short cfDNA fragments (see Figs. 40 and 41).

### ssPrep libraries mapped insert length details biological signals

ssPrep libraries produce canonical cfDNA molecular length profiles showcasing a prominent fragment length distribution centered around the histone monomer at ~167 bp and a sawtooth pattern revealing a periodicity of 10.4 bp that is likely the result of DNase I cleaving the exposed minor grove of nucleosome-bound DNA. ssPrep distinguishes itself from dsDNA preps (Fig. 42A) by (1) its ability to capture an increased proportion of sub-nucleosome-sized fragments and retain short DNA fragments, and (2) omitting DNA end-polishing steps to recover native DNA ends, revealing the true fragment length profile as demonstrated by sub-peaks that are slightly shorter than dsDNA counterparts. Unlike ssPrep or the dsDNA methods, Swift Accel NGS 1S Plus data requires read trimming prior to mapping due to the additional of non-template nucleotides during its prep process. This abolishes both the biological signal (no sawtooth pattern) and artificially shifts the insert distribution smaller (Fig. 42B).

### ssPrep generates complex libraries

ssPrep retains native termini without sacrificing library complexity. ssPrep generates complex libraries from 1 ng input on par with the best commercial kits on the market, with no end-polishing required (see Fig. 43).

### ssPrep libraries produce uniform GC coverage

Another measure of library quality is coverage across the GC spectrum. ssPrep shows genome coverage in low GC rich regions at rates similar to that of the NEBNext Ultra II kit, mostly uniform coverage across the bulk of human genomes GC content bins, and enhanced coverage of areas with high GC content relative to the other kits (see Fig. 44).

### ssPrep facilitates cfDNA biological discovery

Cell-free DNA contains nucleosome positioning information that can be leveraged to explore a multitude of biological signals, such as tissue of origin investigation, expression correlation, and cancer evolution. Shown in Fig. 45 is the normalized window protection score (WPS) from ssPrep cfDNA libraries for a strongly conserved region of well positioned nucleosomes from chromosome 12. The sinusoidal curve indicates ordered nucleosome positioning and the peaks are where nucleosome protection of cfDNA fragments is strongest.

Localized dinucleotide composition is important for the rotational positioning of nucleosomes. Periodic oscillation between G/C and A/T dinucleotides facilitates the histone wrapping by DNA. Furthermore, DNA nicking by DNase I occurs in G/C rich dinucleotide regions since that is when the minor grove is facing outwards and accessible to Dnase I. ssPrep libraries preserve these features of nucleosome positioning as can be seen by the oscillation frequency within the read length and the enrichment for G/C dyads at the fragment termination points in Fig. 46.

### Example 6: Nucleic acid fragment size enrichment

In some variations of the single-stranded library prep methods described in certain Examples above, ligation products (e.g., single-stranded sample fragments ligated to scaffold adapters (or components thereof) described herein) are purified prior to amplification using SPRI purification. In some variations described in this Example, the volume of the ligation products is increased using a certain amount of elution buffer (EB; i.e., 10 mM Tris buffer) prior to SPRI purification. In some variations, the volume of buffer was replaced with isopropanol to increase retention of smaller fragments. The addition of isopropanol at this part of the purification step allows for fine-grained cutoffs at the lower end of degraded DNA (e.g., degraded human DNA) size distributions, without compromising on the exclusion of adapter artifacts (e.g., adapter dimers). If a less stringent SPRI purification is performed in attempt to recover the smallest human fragments, more adapter artifacts are recovered. Using various increments of isopropanol (e.g., 2 µl, 5 µl), a desired size distribution may be tailored.

Libraries were generated from 150 pg DNA obtained from a degraded, 25-year-old hair sample. Isopropanol was added to certain buffer-sample mixtures during the post-ligation SPRI bead purification step according to the following protocol:
1) Add 50 µl of EB to each reaction or replace a portion of the EB volume with isopropanol (to retain a higher proportion of small fragments)
2) Add 72.6 µl of 18% PEG SPRI bead solution (i.e., 1.2x ratio of 18% SPRI beads to sample, for 50 µl of sample) and perform SPRI purification
3) Elute in 20 µl EB

Purified ligation products were amplified by PCR using 50 µl AMPLITAQ GOLD, and the amplification products were purified using SPRI bead purification at 1.2x. The post-PCR purifications were at 1.2x using 18% SPRI beads (18% PEG in the SPRI solution). The post-ligation purifications were at 1.01x v/v ratio (72.6 ul of 18% SPRI given a final reaction volume of 75ul). Generally, the final concentration of PEG in the post-ligation purifications was ~12% in this example, as the ligation products already contained PEG.

Fig. 48A to Fig. 48D show traces of DNA libraries after PCR amplification and a 1.2x 18% SPRI purification. Each trace differs based on the post-ligation SPRI conditions used prior to amplification. The fragment size distributions shown in Fig. 48A to Fig. 48D and Fig 49A to Fig. 49E show the shift in retained fragments at different volumes of isopropanol spiked into the EB (i.e., Tris buffer). In particular, the peak between 100 and 148 bp remains proportionally high with increasing isopropanol, while the peak from 148 to 350 proportionally decreases, bringing down the average fragment size. One observation is an increase in adapter dimer retention under certain conditions. For example, Fig. 48A shows amplified DNA libraries with 14.8% adapter dimers, an average fragment length of 206 bp, and an amplified ligation product (excluding adapter dimer) concentration of 60.7 nmol/µl following a post-ligation SPRI purification (72.6 µl of 18% SPRI) with 50 µl Tris buffer added to 25 µl of ligation products. Fig. 48B shows 26.4% adapter dimers, an average fragment length of 197 bp, and an amplified ligation product concentration of 28.5 nmol/µl following a post-ligation SPRI purification (72. 6µl of 18% SPRI) with 25 µl isopropanol and 25 µl Tris buffer added to 25 µl of ligation products. Fig. 48C shows 26.9% adapter dimers, an average fragment length of 193 bp, and an amplified ligation product concentration of of 27.8 nmol/µl following a post-ligation SPRI purification (72.6 µl of 18% SPRI) with 50 µl isopropanol added to 25ul of ligation products. Fig. 48D shows 27.8% adapter dimers, an average fragment length of 192 bp, and 32.8 nmol/µl for ligation products purified using SPRI purification (72.6 µl of 38% PEG) with 50 µl Tris buffer added to 25µl of ligation products.

In another experiment, libraries were generated from 150 pg DNA obtained from a fresh hair sample. Certain parameters for this experiment included: Adapter Hyb = 1:1.4 (Ad:Sp); P5 = 1.6 p rSAP; P7 = 0.4 p phosph, no rSAP; SSB = 64 ng. During preparation of the double-stranded adapters, the top strand of the P5 adapter was annealed to the scaffold strand of the P5 adapter at a ratio of 1:1.4 (i.e., more scaffold strand is added during annealing); and the top strand of the P7 adapter was annealed to the scaffold strand of the P7 adapter at a ratio of 1:1.4. 1.6 pmol of unphosphorylated P5 adapter and 0.4 pmol of phosphorylated P7 adapter were added. The DNA template was combined with 64 ng of SSB before the adapter ligation step. Isopropanol was added to certain buffer-sample mixtures during the post-ligation SPRI bead purification step according to the following protocol:
1) Add 50 µl of EB to each reaction or replace a portion of the EB volume with isopropanol (to retain a higher proportion of small fragments)
2) Add 72.6 µl of 18% PEG SPRI bead solution and perform SPRI purification
3) Elute in 20 µl EB

Purified ligation products were amplified by PCR using 100 µl AMPLITAQ GOLD (16 cycles), and the amplification products were purified using 18% PEG SPRI bead purification at 1.2x. The post-PCR purifications were at 1.2x using 18% SPRI beads (18% PEG in the SPRI solution). The post-ligation purifications were at 1.01x v/v ratio (72.6 ul of 18% SPRI given a final reaction volume of 75ul). Generally, the final concentration of PEG in the post-ligation purifications was ~12% in this example, as the ligation products already contained PEG.

Fig 49A to Fig. 49E show traces of DNA libraries after PCR amplification and a 1.2x 18% SPRI purification. Each trace differs based on the post-ligation SPRI conditions used prior to amplification. The fragment size distributions shown in Fig 49A to Fig. 49E show the shift in retained fragments at different volumes of isopropanol spiked into the EB (i.e., Tris buffer). Fig. 49A shows 1.16% adapter dimers and an average fragment length of 263 bp for ligation products purified using SPRI purification (72.6 µl of 18% SPRI) with 50 µl Tris buffer added to 25ul of ligation products. Fig. 49B shows 6.13% adapter dimers and an average fragment length of 232 bp for ligation products purified using column purification. Fig. 49C shows 1.26% adapter dimers and an average fragment length of 256 bp for ligation products purified using SPRI purification (72.6 µl of 18% SPRI) with 5 µl isopropanol and 45 µl Tris buffer added to 25ul of ligation products. Fig. 49D shows 1.66% adapter dimers and an average fragment length of 236 bp for ligation products purified using SPRI purification (72.6 µl of 18% SPRI) with 10 µl isopropanol and 40 µl Tris buffer added to 25ul of ligation products. Fig. 49E shows 7.53% adapter dimers and an average fragment length of 227 bp for ligation products purified using SPRI purification(72.6 µl of 18% SPRI) with 20 µl isopropanol and 30 µl Tris buffer added to 25ul of ligation products.

Average fragments lengths, percent adapter dimers, and post amplification yields (conc.) for this experiment are provided in Table 7 below.

| **Table 7** | | | | | |
|---|---|---|---|---|---|
| **Clean Method** | **isopropanol (µl)** | **EB (Tris) (µl)** | **conc. (ng/µl)** | **% adapter dimers** | **average length (bp)** |
| 18% SPRI | 0 | 50 | 39.2 | 1.16 | 263 |
| column (MINELUTE) | N/A | N/A | 29.3 | 6.13 | 232 |
| 18% SPRI 5 µl isopropanol | 5 | 45 | 37.1 | 1.27 | 254 |
| 18% SPRI 10 µl isopropanol | 10 | 40 | 39.8 | 1.78 | 237 |
| 18% SPRI 20 µl isopropanol | 20 | 30 | 27.3 | 7.54 | 221 |

In a further experiment, libraries were generated from 150 pg DNA obtained from a fresh hair sample. Certain parameters for this experiment included: Adapter Hyb = 1:1.4 (Ad:Sp); P5 = 1.6 p rSAP; P7 = 0.4 p phosph, no rSAP; SSB = 64 ng. During preparation of the double-stranded adapters, the top strand of the P5 adapter was annealed to the scaffold strand of the P5 adapter at a ratio of 1:1.4 (i.e., more scaffold strand is added during annealing); and the top strand of the P7 adapter was annealed to the scaffold strand of the P7 adapter at a ratio of 1:1.4. 1.6 pmol of unphosphorylated P5 adapter and 0.4 pmol of phosphorylated P7 adapter were added. The DNA template was combined with 64 ng of SSB before the adapter ligation step. Isopropanol was added to the buffer-sample mixtures during the post-ligation SPRI bead purification step according to the following protocol:
1) Add varying amounts of isopropanol and EB to the 25 µl of sample for a total volume of 75 µl for each reaction
2) Add 72.6 µl of 18% PEG SPRI bead solution and perform SPRI purification
3) Elute in 20 µl EB

Purified ligation products were amplified by PCR using 100 µl AMPLITAQ GOLD (15 cycles), and the amplification products were purified using 18% PEG SPRI bead purification at 1.2x. The post-PCR purifications were at 1.2x using 18% SPRI beads (18% PEG in the SPRI solution). The post-ligation purifications were at 1.01x v/v ratio (72.6 ul of 18% SPRI given a final reaction volume of 75ul). Generally, the final concentration of PEG in the post-ligation purifications was ~12% in this example, as the ligation products already contained PEG.

Average fragments lengths, percent adapter dimers, and post amplification yields (conc.) for this experiment are provided in Table 8 below.

| **Table 8** | | | | | |
|---|---|---|---|---|---|
| **Clean Method** | **isopropanol (µl)** | **EB (Tris) (µl)** | **conc. (ng/µl)** | **% adapter dimers** | **average length (bp)** |
| 18% SPRI 10 µl isopropanol | 10 | 40 | 27.1 | 2.81 | 240 |
| 18% SPRI 12 µl isopropanol | 12 | 38 | 22 | 4.57 | 237 |
| 18% SPRI 14 µl isopropanol | 14 | 36 | 20.2 | 6.29 | 232 |
| 18% SPRI 16 µl isopropanol | 16 | 34 | 21.7 | 6.57 | 228 |
| 18% SPRI 18 µl isopropanol | 18 | 32 | 19 | 7.83 | 228 |
| 18% SPRI 20 µl isopropanol | 20 | 30 | 17.8 | 9.77 | 225 |

The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams.

Certain embodiments of the technology are set forth in the claim(s) that follow(s).

## Claims

1. A method of producing a nucleic acid library, comprising:
combining (i) a nucleic acid composition comprising single-stranded nucleic acid (ssNA), (ii) a first oligonucleotide, and (iii) a plurality of first scaffold polynucleotide species, wherein:
(a) each polynucleotide in the plurality of first scaffold polynucleotide species comprises an ssNA hybridization region and a first oligonucleotide hybridization region;
(b) the nucleic acid composition, the first oligonucleotide, and the plurality of first scaffold polynucleotide species are combined under conditions in which a molecule of the first scaffold polynucleotide species is hybridized to (i) a first ssNA terminal region and (ii) a molecule of the first oligonucleotide, thereby forming hybridization products in which an end of the molecule of the first oligonucleotide is adjacent to an end of the first ssNA terminal region;
(c) the ssNA is not combined with a single-stranded nucleic acid binding protein (SSB) prior to the combining or during the combining; and
(d) the ssNA is not modified prior to the combining.

2. The method of claim 1, further comprising combining the nucleic acid composition with (iv) a second oligonucleotide, and (v) a plurality of second scaffold polynucleotide species, wherein:
(e) each polynucleotide in the plurality of second scaffold polynucleotide species comprises an ssNA hybridization region and a second oligonucleotide hybridization region; and
(f) the nucleic acid composition, the second oligonucleotide, and the plurality of second scaffold polynucleotide species are combined under conditions in which a molecule of the second scaffold polynucleotide species is hybridized to (i) a second ssNA terminal region and (ii) a molecule of the second oligonucleotide, thereby forming hybridization products in which an end of the molecule of the second oligonucleotide is adjacent to an end of the second ssNA terminal region.

3. The method of claim 2, wherein prior to the combining, each of the first scaffold polynucleotide species is hybridized to a first oligonucleotide to form a plurality of first scaffold duplex species, and each of the second scaffold polynucleotide species is hybridized to a second oligonucleotide to form a plurality of second scaffold duplex species.

4. The method of claim 2 or 3, further comprising covalently linking the adjacent ends of the first oligonucleotide and the first ssNA terminal region, and covalently linking the adjacent ends of the second oligonucleotide and the second ssNA terminal region, thereby generating covalently linked hybridization products.

5. The method of claim 4, wherein the covalently linking comprises contacting the hybridization products with an agent comprising a ligase activity under conditions in which an end of the first ssNA terminal region is covalently linked to an end of the first oligonucleotide and an end of the second ssNA terminal region is covalently linked to an end of the second oligonucleotide.

6. The method of claim 3, wherein some or all of the duplexes in the plurality of first scaffold duplex species comprise an adenylation modification at the 5' end of the first oligonucleotide, and the plurality of first scaffold duplex species are combined with and covalently linked to the ssNA in the absence of ATP, thereby forming intermediate covalently linked hybridization products, wherein the intermediate covalently linked hybridization products are combined with and covalently linked to the plurality of second scaffold duplex species and ATP, thereby forming covalently linked hybridization products.

7. The method of any one of claims 1 to 6, wherein the ssNA hybridization region of each of the first polynucleotide species is different than the ssNA hybridization region in other first polynucleotide species in the plurality of first polynucleotide species, and the ssNA hybridization region of each of the second polynucleotide species is different than the ssNA hybridization region in other second polynucleotide species in the plurality of second polynucleotide species.

8. The method of any one of claims 1 to 7, wherein:
(1) the ssNA hybridization region comprises a random sequence, and/or
(2) the ssNA hybridization region comprises one or more universal bases.

9. The method of any one of claims 2 to 6 wherein:
(1) the first oligonucleotide comprises one or more modified nucleotides, some or all of the first scaffold polynucleotide species comprise one or more modified nucleotides, or the first oligonucleotide comprises one or more modified nucleotides and some or all of the first scaffold polynucleotide species comprise one or more modified nucleotides; and/or
(2) the second oligonucleotide comprises one or more modified nucleotides, some or all of the second scaffold polynucleotide species comprise one or more modified nucleotides, or the second oligonucleotide comprises one or more modified nucleotides and some or all of the second scaffold polynucleotide species comprise one or more modified nucleotides,
wherein the one or more modified nucleotides are capable of blocking covalent linkage of the oligonucleotide to another oligonucleotide, polynucleotide, or nucleic acid molecule, and wherein the oligonucleotide comprises the one or more modified nucleotides at an end not adjacent to the ssNA, and/or
wherein the one or more modified nucleotides are capable of blocking covalent linkage of the scaffold polynucleotide to another oligonucleotide, polynucleotide, or nucleic acid molecule, and wherein the scaffold polynucleotide comprises the one or more modified nucleotides at one or both ends of the polynucleotide.

10. The method of claim 9, wherein the one or more modified nucleotides comprise a ligation-blocking modification.

11. The method of any one of claims 4 to 10, further comprising denaturing the covalently linked hybridization products, thereby generating single-stranded ligation products.

12. The method of claim 11, further comprising:
(1) combining the single-stranded ligation products with a third oligonucleotide under conditions in which the third oligonucleotide is hybridized to a dimer of the first oligonucleotide and the second oligonucleotide, thereby forming an oligonucleotide dimer hybridization product, wherein the oligonucleotide dimer hybridization product comprises a cleavage site
(2) contacting the oligonucleotide dimer hybridization product with a cleavage agent.

13. The method of any one of claims 1 to 12, wherein the nucleic acid composition comprises single-stranded DNA (ssDNA), single-stranded RNA (ssRNA), or ssDNA and ssRNA.

14. The method of any one of claims 1 to 13, wherein one or both native ends of the ssNA are present when the ssNA is combined with the first oligonucleotide and the plurality of first scaffold polynucleotide species.

15. The method of any one of claims 1 to 14, wherein the ssNA is from cell-free nucleic acid.

## Patentansprüche

1. Verfahren zur Herstellung einer Nukleinsäurebibliothek, umfassend:
Kombinieren (i) einer Nukleinsäurezusammensetzung, die einzelsträngige Nukleinsäuren (ssNA) umfasst, (ii) eines ersten Oligonukleotids und (iii) einer Vielzahl von ersten Gerüst-Polynukleotidspezies, wobei:
(a) jedes Polynukleotid in der Vielzahl der ersten Gerüst-Polynukleotidspezies eine ssNA-Hybridisierungsregion und eine Hybridisierungsregion für das erste Oligonukleotid umfasst;
(b) die Nukleinsäurezusammensetzung, das erste Oligonukleotid und die Vielzahl der ersten Gerüst-Polynukleotidspezies unter Bedingungen kombiniert werden, bei denen ein Molekül der ersten Gerüst-Polynukleotidspezies mit (i) einer ersten ssNA-terminalen Region und (ii) einem Molekül des ersten Oligonukleotids hybridisiert wird, wodurch Hybridisierungsprodukte gebildet werden, bei denen ein Ende des Moleküls des ersten Oligonukleotids benachbart zu einem Ende der ersten ssNA-terminalen Region ist;
(c) die ssNA vor dem Kombinieren oder während des Kombinierens nicht mit einem Nukleinsäure-Einzelstrang-bindenden Protein (SSB) kombiniert wird; und
(d) die ssNA vor dem Kombinieren nicht modifiziert wird.

2. Verfahren nach Anspruch 1, ferner umfassend das Kombinieren der Nukleinsäurezusammensetzung mit (iv) einem zweiten Oligonukleotid und (v) einer Vielzahl von zweiten Gerüst-Polynukleotidspezies, wobei:
(e) jedes Polynukleotid in der Vielzahl der zweiten Gerüst-Polynukleotidspezies eine ssNA-Hybridisierungsregion und eine Hybridisierungsregion für das zweite Oligonukleotid umfasst; und
(f) die Nukleinsäurezusammensetzung, das zweite Oligonukleotid und die Vielzahl der zweiten Gerüstpolynukleotid-Spezies unter Bedingungen kombiniert werden, unter denen ein Molekül der zweiten Gerüstpolynukleotid-Spezies mit (i) einer zweiten ssNA-terminalen Region und (ii) einem Molekül des zweiten Oligonukleotids hybridisiert wird, wodurch Hybridisierungsprodukte gebildet werden, bei denen ein Ende des Moleküls des zweiten Oligonukleotids benachbart zu einem Ende der zweiten ssNA-terminalen Region ist.

3. Verfahren nach Anspruch 2, wobei vor dem Kombinieren jede der ersten Gerüst-Polynukleotidspezies an ein erstes Oligonukleotid hybridisiert wird, um eine Vielzahl von ersten Gerüstduplex-Spezies zu bilden, und jede der zweiten Gerüst-Polynukleotidspezies an ein zweites Oligonukleotid hybridisiert wird, um eine Vielzahl von zweiten Gerüstduplex-Spezies zu bilden.

4. Verfahren nach Anspruch 2 oder 3, ferner umfassend die kovalente Verknüpfung der benachbarten Enden des ersten Oligonukleotids und der ersten ssNA-terminalen Region und die kovalente Verknüpfung der benachbarten Enden des zweiten Oligonukleotids und der zweiten ssNA-terminalen Region, wodurch kovalent verknüpfte Hybridisierungsprodukte erzeugt werden.

5. Verfahren nach Anspruch 4, wobei die kovalente Verknüpfung das Inkontaktbringen der Hybridisierungsprodukte mit einem Wirkstoff umfasst, der eine Ligaseaktivität umfasst, unter Bedingungen, bei denen ein Ende der ersten ssNA-terminalen Region kovalent mit einem Ende des ersten Oligonukleotids verknüpft wird und ein Ende der zweiten ssNA-terminalen Region kovalent mit einem Ende des zweiten Oligonukleotids verknüpft wird.

6. Verfahren nach Anspruch 3, wobei einige oder alle der Duplexe in der Vielzahl der ersten Gerüst-Duplex-Spezies eine Adenylierungsmodifikation am 5'-Ende des ersten Oligonukleotids umfassen und die Vielzahl der ersten Gerüst-Duplex-Spezies mit der ssNA in Abwesenheit von ATP kombiniert und kovalent daran gebunden werden, wodurch intermediäre kovalent verknüpfte Hybridisierungsprodukte gebildet werden, wobei die intermediären kovalent verknüpften Hybridisierungsprodukte mit der Vielzahl der zweiten Gerüst-Duplex-Spezies und ATP kombiniert und kovalent an diese gebunden werden, wodurch kovalent verknüpfte Hybridisierungsprodukte gebildet werden.

7. Verfahren nach einem der Ansprüche 1 bis 6, wobei die ssNA-Hybridisierungsregion jeder der ersten Polynukleotidspezies sich von der ssNA-Hybridisierungsregion in anderen ersten Polynukleotidspezies in der Vielzahl der ersten Polynukleotidspezies unterscheidet, und die ssNA-Hybridisierungsregion jeder der zweiten Polynukleotidspezies sich von der ssNA-Hybridisierungsregion in anderen zweiten Polynukleotidspezies in der Vielzahl der zweiten Polynukleotidspezies unterscheidet.

8. Verfahren nach einem der Ansprüche 1 bis 7, wobei:
(1) Die ssNA-Hybridisierungsregion eine zufällige Sequenz umfasst, und/oder
(2) Die ssNA-Hybridisierungsregion eine oder mehrere universelle Basen umfasst.

9. Verfahren nach einem der Ansprüche 2 bis 6, wobei:
(1) das erste Oligonukleotid ein oder mehrere modifizierte Nukleotide umfasst, einige oder alle der ersten Gerüst-Polynukleotidspezies ein oder mehrere modifizierte Nukleotide umfassen, oder das erste Oligonukleotid ein oder mehrere modifizierte Nukleotide umfasst und einige oder alle der ersten Gerüst-Polynukleotidspezies ein oder mehrere modifizierte Nukleotide umfassen; und/oder
(2) das zweite Oligonukleotid ein oder mehrere modifizierte Nukleotide umfasst, einige oder alle der zweiten Gerüst-Polynukleotidspezies ein oder mehrere modifizierte Nukleotide umfassen, oder das zweite Oligonukleotid ein oder mehrere modifizierte Nukleotide umfasst und einige oder alle der zweiten Gerüst-Polynukleotidspezies ein oder mehrere modifizierte Nukleotide umfassen,
wobei das eine oder die mehreren modifizierten Nukleotide in der Lage sind, die kovalente Bindung des Oligonukleotids an ein anderes Oligonukleotid, Polynukleotid oder Nukleinsäuremolekül zu blockieren, und wobei das Oligonukleotid das eine oder die mehreren modifizierten Nukleotide an einem Ende umfasst, das nicht an die ssNA angrenzt, und/oder
wobei das eine oder die mehreren modifizierten Nukleotide in der Lage sind, die kovalente Bindung des Gerüst-Polynukleotids an ein anderes Oligonukleotid, Polynukleotid oder Nukleinsäuremolekül zu blockieren, und wobei das GerüstPolynukleotid das eine oder die mehreren modifizierten Nukleotide an einem oder beiden Enden des Polynukleotids umfasst.

10. Verfahren nach Anspruch 9, wobei das eine oder die mehreren modifizierten Nukleotide eine ligationsblockierende Modifikation umfassen.

11. Verfahren nach einem der Ansprüche 4 bis 10, ferner umfassend die Denaturierung der kovalent verbundenen Hybridisierungsprodukte, wodurch einzelsträngige Ligationsprodukte erzeugt werden.

12. Verfahren nach Anspruch 11, ferner umfassend:
(1) Kombinieren der einzelsträngigen Ligationsprodukte mit einem dritten Oligonukleotid unter Bedingungen, unter denen das dritte Oligonukleotid an ein Dimer des ersten Oligonukleotids und des zweiten Oligonukleotids hybridisiert wird, wodurch ein Oligonukleotid-Dimer-Hybridisierungsprodukt gebildet wird, wobei das Oligonukleotid-Dimer-Hybridisierungsprodukt eine Spaltungsstelle umfasst
(2) Inkontaktbringen des Oligonukleotid-Dimer-Hybridisierungsprodukts mit einem Spaltungsmittel.

13. Verfahren nach einem der Ansprüche 1 bis 12, wobei die Nukleinsäurezusammensetzung einzelsträngige DNA (ssDNA), einzelsträngige RNA (ssRNA) oder ssDNA und ssRNA umfasst.

14. Verfahren nach einem der Ansprüche 1 bis 13, wobei ein oder beide native Enden der ssNA vorhanden sind, wenn die ssNA mit dem ersten Oligonukleotid und der Vielzahl an ersten Gerüst-Polynukleotidspezies kombiniert wird.

15. Verfahren nach einem der Ansprüche 1 bis 14, wobei die ssNA aus zellfreier Nukleinsäure stammt.

## Revendications

1. Méthode de production d'une bibliothèque d'acides nucléiques, comprenant:
la combinaison (i) d'une composition d'acides nucléiques comprenant un acide nucléique simple brin (ANsb), (ii) un premier oligonucléotide, et (iii) une pluralité de premières espèces de polynucléotide d'échafaudage, dans laquelle:
(a) chaque polynucléotide dans la pluralité de premières espèces de polynucléotide d'échafaudage comprend une région d'hybridation ANsb et une première région d'hybridation d'oligonucléotide;
(b) la composition d'acides nucléiques, le premier oligonucléotide, et la pluralité de premières espèces de polynucléotide d'échafaudage sont combinés dans des conditions dans lesquelles une molécule de première espèce de polynucléotide d'échafaudage est hybridée à (i) une première région terminale d'ANsb et (ii) une molécule du premier oligonucléotide, ce qui permet de former des produits d'hybridation dans lesquels une extrémité de la molécule du premier oligonucléotide est adjacente à une extrémité de la première région terminale d'ANsb;
(c) l'ANsb n'est pas combiné avec une protéine de liaison d'acide nucléique simple brin (LSB) avant la combinaison ou pendant la combinaison; et
(d) l'ANsb n'est pas modifié avant la combinaison.

2. Méthode selon la revendication 1, comprenant en outre la combinaison de la composition d'acide nucléique avec (iv) un deuxième oligonucléotide, et (v) une pluralité de deuxièmes espèces de polynucléotide d'échafaudage, dans laquelle:
(e) chaque polynucléotide dans la pluralité de deuxièmes espèces de polynucléotide d'échafaudage comprend une région d'hybridation d'ANsb et une deuxième région d'hybridation d'oligonucléotide; et
(f) la composition d'acide nucléique, le deuxième oligonucléotide, et la pluralité de deuxièmes espèces de polynucléotide d'échafaudage sont combinés dans des conditions dans lesquelles une molécule de la deuxième espèce de polynucléotide d'échafaudage est hybridée à (i) une deuxième région terminale d'ANsb et (ii) une molécule du deuxième oligonucléotide, ce qui permet de former des produits d'hybridation dans lesquels une extrémité de la molécule du deuxième oligonucléotide est adjacente à une extrémité de la deuxième région terminale d'ANsb.

3. Méthode selon la revendication 2, dans laquelle, avant la combinaison, chacune des premières espèces de polynucléotide d'échafaudage est hybridée à un premier oligonucléotide pour former une pluralité de premières espèces duplexes d'échafaudage, et chacune des deuxièmes espèces de polynucléotide d'échafaudage est hybridée à un deuxième oligonucléotide pour former une pluralité de deuxièmes espèces duplexes d'échafaudage.

4. Méthode selon la revendication 2 ou 3, comprenant en outre la liaison covalente des extrémités adjacentes du premier oligonucléotide et de la première région terminale d'ANsb, et la liaison covalente des extrémités adjacentes du deuxième oligonucléotide et de la deuxième région terminale d'ANsb, ce qui permet de générer des produits d'hybridation liés de manière covalente.

5. Méthode selon la revendication 4, dans laquelle la liaison covalente comprend la mise en contact des produits d'hybridation avec un agent comprenant une activité de ligase dans des conditions dans lesquelles une extrémité de la première région terminale d'ANsb est liée de manière covalente à une extrémité du premier oligonucléotide et une extrémité de la deuxième région terminale d'ANsb est liée de manière covalente à une extrémité du deuxième oligonucléotide.

6. Méthode selon la revendication 3, dans laquelle certains ou tous les duplexes dans la pluralité de premières espèces duplexes d'échafaudage comprennent une modification d'adénylation à l'extrémité 5' du premier oligonucléotide, et la pluralité des premières espèces duplexes d'échafaudage sont combinées avec et liées de manière covalente à l'ANsb en l'absence d'ATP, ce qui permet de former des produits d'hybridation liés de manière covalente intermédiaires, les produits d'hybridation liés de manière covalente intermédiaires étant combinés avec et liés à la pluralité de deuxièmes espèces duplexes d'échafaudage et l'ATP, ce qui permet de former des produits d'hybridation liés de manière covalente.

7. Méthode selon l'une quelconque des revendications 1 à 6, dans laquelle la région d'hybridation d'ANsb de chacune des premières espèces de polynucléotide est différente de la région d'hybridation d'ANsb dans l'autre première espèce de polynucléotide dans la pluralité des premières espèces de polynucléotide, et la région d'hybridation d'ANsb de chacune des deuxièmes espèces de polynucléotide est différente de la région d'hybridation d'ANsb dans l'autre deuxième espèce de polynucléotide dans la pluralité des deuxièmes espèces de polynucléotide.

8. Méthode selon l'une quelconque des revendications 1 à 7, dans laquelle:
(1) la région d'hybridation d'ANsb comprend une séquence aléatoire, et/ou
(2) la région d'hybridation d'ANsb comprend une ou plusieurs bases universelles.

9. Méthode selon l'une quelconque des revendications 2 à 6, dans laquelle:
(1) le premier oligonucléotide comprend un ou plusieurs nucléotides modifiés, certaines ou toutes les premières espèces de polynucléotide d'échafaudage comprennent un ou plusieurs nucléotides modifiés, ou le premier oligonucléotide comprend un ou plusieurs nucléotides modifiés et certaines ou toutes les premières espèces de polynucléotide d'échafaudage comprennent un ou plusieurs nucléotides modifiés; et/ou
(2) le deuxième oligonucléotide comprend un ou plusieurs nucléotides modifiés, certaines ou toutes les deuxièmes espèces de polynucléotide d'échafaudage comprennent un ou plusieurs nucléotides modifiés, ou le deuxième oligonucléotide comprend un ou plusieurs nucléotides modifiés et certaines ou toutes les deuxièmes espèces de polynucléotide d'échafaudage comprennent un ou plusieurs nucléotides modifiés,
dans laquelle les un ou plusieurs nucléotides modifiés sont capables de bloquer la liaison covalente de l'oligonucléotide à l'autre oligonucléotide, polynucléotide, ou molécule d'acide nucléique, et dans laquelle l'oligonucléotide comprend les un ou plusieurs nucléotides modifiés à une extrémité non adjacentes à l'ANsb, et/ou
dans laquelle les un ou plusieurs nucléotides modifiés sont capables de bloquer la liaison covalente du polynucléotide d'échafaudage à l'autre oligonucléotide, polynucléotide, ou molécule d'acide nucléique, et dans laquelle le polynucléotide d'échafaudage comprend les un ou plusieurs nucléotides modifiés à une ou aux deux extrémités du polynucléotide.

10. Méthode selon la revendication 9, dans laquelle les un ou plusieurs nucléotides modifiés comprennent une modification de ligature-blocage.

11. Méthode selon l'une quelconque des revendications 4 à 10, comprenant en outre la dénaturation des produits d'hybridation liés de manière covalente, ce qui permet de générer des produits de ligature simple brin.

12. Méthode selon la revendication 11, comprenant en outre:
(1) la combinaison des produits de ligature simple brin avec un troisième oligonucléotide dans des conditions dans lesquelles le troisième oligonucléotide est hybridé à un dimère du premier oligonucléotide et du deuxième oligonucléotide, ce qui permet de former un produit d'hybridation dimère d'oligonucléotide, où le produit d'hybridation dimère d'oligonucléotide comprend un site de clivage
(2) la mise en contact du produit d'hybridation dimère d'oligonucléotide avec un agent de clivage.

13. Méthode selon l'une quelconque des revendications 1 à 12, dans laquelle la composition d'acide nucléique comprend de l'ADN simple brin (ADNsb), de l'ARN simple brin (ARNsb), ou de l'ADNsb et de l'ARNsb.

14. Méthode selon l'une quelconque des revendications 1 à 13, dans laquelle une ou les deux extrémités natives de l'ANsb sont présentes lorsque l'ANsb est combiné avec le premier oligonucléotide et la pluralité des premières espèces de polynucléotide d'échafaudage.

15. Méthode selon l'une quelconque des revendications 1 à 14, dans laquelle l'ANsb provient d'acide nucléique acellulaire.
